# EUROPEAN PATENT APPLICATION

(11) **EP 4 365 300 A2**
(43) Date of publication of application: **08.05.2024**
(21) Application number: 23215487.2
(22) Date of filing: 14.07.2020
(51) Int. Cl.: C12N 15/861

(54) **TREATMENT/PREVENTION OF DISEASE BY LINC COMPLEX INHIBITION**

(30) Priority: 17.07.2019 SG 10201906637U
(62) Divisional of application: 20839620.0
(71) Applicant: Agency for Science, Technology and Research, Singapore 138632 (SG); National University of Singapore, Singapore 119077 (SG)
(72) Inventor: STEWART, Colin Lawson, 138648 Singapore (SG); SINGARAJA, Roshni, 138648 Singapore (SG); BURKE, Brian, 138648 Singapore (SG); LEE, Yin Loon, 138648 Singapore (SG); TAN, Yann Chong, 138672 Singapore (SG)
(74) Representative: Mewburn Ellis LLP

(57) **Abstract**

Methods for the treatment and prevention of laminopathies and diseases characterised by hyperlipidemia through LINC complex inhibition are disclosed, as well as LINC complex inhibitors for use in such methods.

## Description

This application claims priority from SG 10201906637U filed 17 July 2019, the contents and elements of which are herein incorporated by reference for all purposes.

### Field of the Invention

The present invention relates to the treatment and prevention of disease, via LINC complex inhibition.

### Background to the Invention

Dilated Cardiomyopathy (DCM) is the most common disease affecting heart muscle, accounting for approximately 60% of all cardiomyopathies. It is characterised by reduced systolic (contractile) function due to enlargement and thinning of the left ventricular wall or in some cases both ventricles. DCM is associated with sudden heart failure and cardiac death, resulting in high rates of hospital admission, the need for heart transplantation and consequently a high cost burden (J. L. Jefferies and J. A. Towbin, Lancet 375: 752-762 (2010); R. E. Hershberger, et al., Nat Rev Cardiol 10: 531-547 (2013)). The causes of DCM are varied, but include a variety of extrinsic factors, (viral, autoimmune infiltration, alcohol, and drugs). However 30-40% of all cases have a monogenic basis, with mutations in some 40 genes being linked to DCM. The most frequently mutated gene in DCM is *TTN,* that encodes the giant sarcomeric protein titin, with truncating variants in *TTN* accounting for almost 15-25% of all congenital forms of DCM (D. S. Herman et al., N Engl J Med 366: 619-628 (2012); U. Tayal, S. et al., Genome Med 9: 20 (2017)). The second most frequently mutated gene is Lamin A (*LMNA*) accounts for as many as 6-8% of congenital DCM patients (U. Tayal, S. et al., Genome Med 9: 20 (2017)).

*LMNA* mutation-associated DCM is characterised by cardiac conduction disease manifested by electrophysiological (ECG) abnormalities, including atrioventricular block, ventricular arrhythmias and fibrillation. The risk of sudden cardiac death is greater in patients with *LMNA* mutation-associated cardiomyopathy compared to patients with other forms of DCM (J. H. Van Berlo et al., Hum Mol Genet 14: 2839-2849 (2005)). Some 450 different mutations have been identified in the *LMNA* gene, most being missense, resulting in the majority of DCM cases having autosomal dominant inheritance, and the diversity of *LMNA* mutations giving rise to DCM has complicated genetic approaches to treating *LMNA* mutation-associated DCM. To a limited extent *LMNA* mutation-associated DCM can be treated by fitting a pacemaker. Ultimately, however, effective treatment at present is accomplished by heart transplantation (R. E. Hershberger and A. Morales, in GeneReviews, M. P. Adam et al., Eds. (Seattle (WA), 1993); G. Captur et al., Heart 104: 468-479 (2018)).

Mouse lines carrying *Lmna* mutations usually die within a few weeks after birth (T. Sullivan et al., J Cell Biol 147: 913-920 (1999); A. T. Bertrand et al., Hum Mol Genet 21: 1037-1048 (2012); V. Nikolova et al., J Clin Invest 113: 357-369 (2004); A. S. Wang, et al., Differentiation; research in biological diversity, (2015)). The cause of early death in mice lacking *Lmna* is uncertain due to multiple tissues being affected. Cardiac myopathy is thought to be a major contributing cause, as *Lmna* mutant mice develop DCM with conduction abnormalities and focal myocyte degeneration (V. Nikolova et al., J Clin Invest 113: 357-369 (2004); L. C. Mounkes, et al., Hum Mol Genet 14: 2167-2180 (2005)), although effects on other, as yet undefined, skeletal muscles may contribute to early postnatal death.

Lamins are nuclear intermediate filament proteins, and the principal constituents of the nuclear lamina, the proteinaceous matrix underlying the inner nuclear membrane (INM). The lamina consists of the A-type lamins, consisting of 2 predominant forms, lamin A and lamin C, derived by alternate splicing of *LMNA* RNA, whereas the two B type lamins (LMNB 1 and 2) are encoded by two genes: *LMNB1* and *LMNB2* (B. Burke and C. L. Stewart, Nat Rev Mol Cell Biol 14: 13-24 (2013)). The lamina provides structural and mechanical integrity to the nucleus, maintains nuclear shape and position within the cell, as well as being a determinant of chromatin organization (T. Sullivan et al., J Cell Biol 147: 913-920 (1999); I. Solovei et al., Cell 152: 584-598 (2013)). Lamins interact with numerous INM proteins, including emerin, the lamina-associated polypeptides (LAPs) and SUN domain-containing proteins (B. van Steensel and A. S. Belmont, Cell 169: 780-791 (2017)), many of which when either mutated or present as a variant are linked to heart disease (H. J. Worman, et al., Cold Spring Harbor perspectives in biology 2: a000760 (2010); C. L. Stewart, et al., Exp Cell Res 313: 2144-2156 (2007)). Together these proteins form an integrated protein network, centered on the lamina, where loss or mutation of the lamins can result in either the mis-localisation or changes in the level of expression of many lamina-associated proteins (e.g. emerin, SUN1, LBR and Lap2α) (T. Sullivan et al., J Cell Biol 147: 913-920 (1999); I. Solovei et al., Cell 152: 584-598 (2013); C. Y. Chen et al., Cell 149: 565-577 (2012); T. V. Cohen et al., Hum Mol Genet 22: 2852-2869 (2013); F. Haque et al., J Biol Chem 285: 3487-3498 (2010)). Among these proteins, where expression is affected by the loss of or mutation to *Lmna,* are SUN1 and Lap2, both of whose levels are increased. In the case of SUN1 the increased level is due to reduced turnover, rather than increased expression, resulting in high levels accumulating in the Golgi, which was thought to be cytotoxic at least in the *Lmna-*/*-* and *Lmna*Δ9 mouse disease models (C. Y. Chen et al., Cell 149: 565-577 (2012); C. Stewart and B. Burke, WO/2013/158046). However, genetic ablation of SUN1 in mice with *Lmna* mutations increases longevity 3-fold and ameliorates much of the pathology (C. Y. Chen et al., Cell 149: 565-577 (2012); C. Stewart and B. Burke, WO/2013/158046). The median survival of wildtype or *Sun1-*/*-* is > 210 days in a 7 month follow up; *Lmna-*/*-* mice had median survival of 41 days; *Lmna-*/*- Sun1+*/*-* mice had a median survival of 54 days; *Lmna-*/*- Sun1-*/*-* mice had a median survival of 104 days (p < 0.01 comparing *Lmna-*/*-* and *Lmna-*/*- Sun1-*/*-).* Likewise, whereas all *Lmna*Δ9 mice expired by 30 days after birth, their *Lmna*Δ*9Sun1-l-* littermates thrived past this date, and most achieved life spans more than twice this duration (C. Y. Chen et al., Cell 149: 565-577 (2012)). At the cellular level, human fibroblasts harbouring a *LMNA* mutation resulting in Hutchison-Gilford Progeria Syndrome also exhibited increased SUN1 levels. Depleting SUN1 in these cells alleviated nuclear morphology defects, again suggesting that excess Sun1 resulting from *LMNA* mutation is cytotoxic (C. Y. Chen et al., Cell 149: 565-577 (2012); C. Stewart and B. Burke, WO/2013/158046).

The SUN (Sad1p, UNC-84) domain proteins share a conserved C-terminal SUN domain and localize to the INM (C. J. Malone, et al., Development 126: 3171-3181 (1999)). In mammals, SUN1 and SUN2 are the 2 principal SUN proteins that are widely expressed in virtually all tissues. In the perinuclear space, between the INM and outer nuclear membrane (ONM), the C-termini of SUN1 and/or 2 bind to the C-termini (KASH domains) of the different Nesprins/SYNE/KASH proteins that traverse the ONM. Together these 2 families of proteins comprise the LINC complexes that physically couple the interphase nuclei to the cytoskeleton (M. Crisp et al., J Cell Biol 172: 41-53 (2006); E. C. Tapley and D. A. Starr, Curr Opin Cell Biol 25: 57-62 (2013)). The N-termini of the SUN domain proteins protrude into the nucleoplasm and with SUN1, this region interacts with pre-laminA and nuclear pore complexes. Whether the N-terminus of SUN2 interacts with any nucleoplasmic/NE protein is unclear. By contrast, the bulk of the Nesprins/KASH domain proteins extend into the cytoplasm adjacent to the ONM. There, depending on the particular Nesprin/KASH protein, they interact directly or indirectly with all 3 cytoskeletal protein networks (microtubules, actin microfilaments and intermediate filaments) (H. F. Horn, Current topics in developmental biology 109: 287-321 (2014)). Together, the SUN and KASH/Nesprin proteins of the LINC complex establish a direct physical connection between the cytoplasmic cytoskeletal networks (and their connections e.g. cell adhesion complexes at the cell membrane) and the interphase nuclear interior or nucleoplasm. The LINC complex is thought to mediate force transmission between the nucleus and cytoskeleton and consequently regulate changes in gene expression/chromatin organization in response to mechanical/physical stimuli (S. G. Alam et al., Scientific reports 6: 38063 (2016)). Although loss of either SUN1 or SUN2 alone has no overt effect on postnatal growth and viability, SUN1 null mice are infertile and deaf (H. F. Horn et al., J Cell Biol. 2013 Sep 30;202(7):1023-39; H. F. Horn et al., J Clin Invest. 2013 Feb;123(2):740-50). Simultaneous loss of Sun1 and Sun2 results in perinatal lethality, indicating a degree of redundancy during embryogenesis (K. Lei et al., Proc Natl Acad Sci USA 106: 10207-10212 (2009)).

Importantly, whilst Chen et al. Cell (2012) 149, 565-577 discloses that Sun1 knockout in mice reduces pathology and extends lifespan of *Lmna*^{*-*/*-*} of *Lmna*Δ9 mice, this effect is attributed to the prevention of over-accumulation of Sun1 in the Golgi apparatus. Cellular toxicity as a result of accumulation of SUN proteins in the Golgi was one of several mechanisms proposed to be responsible for laminopathies. Other proposed mechanisms included hyperactive DNA damage following over-accumulation SUN1 in the nuclear envelope, and abnormal transcriptional activity driven by the SUN1 nucleoplasmic domain (see Starr, Curr Biol. (2012) 22(17): R678-R680).

Recently, Kim et al. Sci Transl Med (2018) 10 reported that expression of a polypeptide comprising EGFP and the KASH domain of Nesprin-2 (KASH2) under the control of a smooth muscle cell-specific promoter reduced aortic pathology in a *Lmna*^{G609G/G609G} mouse model of HGPS.

However, a very large number of studies have suggested that inhibition of the level/function of LINC complex proteins would cause or exacerbate laminopathy-related pathology.

Mutations in *SYNE1,* the gene encoding Nesprin-1, have been implicated in dilated cardiomyopathy (Zhou et al., Hum Mol Genet. (2017) 26(12): 2258-2276; Haskell et al., Circ Cardiovasc Genet. (2017) 10(3) pii: e001443; Puckelwartz et al., J. Mol. Cell. (2010) Cardiol. 48, 600-60). Furthermore, the majority of cardiomyopathy-associated mutations to *SYNE1* and *SYNE2* are located in the C-terminus Nesprins-1 and -2 (Stroud, Biophys Rev (2018) 10, 1033-1051), implicating disruption of KASH function in disease pathology. Moreover, cardiac-specific ablation of Nesprin-1 and Nesprin-2 KASH domains has been reported to result in early onset cardiac dysfunction, fibrosis and fetal gene re-expression (Banjeree PLOS Genetics (2014) 10, e1004114), whilst global disruption of Nesprin-1 and Nesprin-2 has been shown to result in perinatal lethality in mice (Zhang et al., Development (2007) 134, 901-908).

Similarly, SUN1 and SUN2 have been suggested to be modifier genes that exacerbate the effects of muscular dystrophies associated with lamin A/C mutation in humans (Meinke et al., PLoS Genet. (2014) 10, e1004605), and some EDMD patient harbour mutations to *SUN1*which reduce interaction between SUN1 and lamins A/C (Lie et al., Human Mutation (2014) 35, 452-461). Global disruption of SUN1 and SUN2 has been shown to result in perinatal lethality in mice, and results in genomic instability and increased DNA damage (Lei et al. Proc. Natl. Acad. Sci. U.S.A. (2009) 106, 10207-10212).

In view of the role of Nesprin and SUN proteins in laminopathy EDMD in addition to lamin A/C, disruption of the LINC complex has been suggested to contribute to laminopathies (Chang et al., Nucleus (2015) 6, 77-88), and LINC complex disruption has been proposed to result in defects in cellular structure and function that may contribute to the development of muscular dystrophies and cardiomyopathies (Lombardi et al. J Biol Chem (2011) 286, 26743-26753).

### Summary of the Invention

The present invention is based on the inventors' unexpected finding that LlNC complex inhibition ameliorates the symptoms of a broad spectrum of diseases caused by *LMNA* mutations having different functional consequences. The inventors demonstrate in the experimental examples herein that LlNC complex disruption (using dominant-negative versions of LINC complex proteins, or through targeted disruption of domains of LINC complex proteins through which the LINC complex proteins interact to form LINC complexes) ameliorates the laminopathy associated with reduced levels of lamin A/C, as well as progerin-associated laminopathy.

Thus LINC complex disruption is shown to be a viable strategy for the treatment and prevention of laminopathies resulting from a broad spectrum of *LMNA* mutations.

The inventors have also unexpectedly discovered that LINC complex inhibition can ameliorate the symptoms of diseases characterised by hyperlipidemia. The inventors show that LlNC complex disruption reduces the symptoms of atherosclerosis.

In a first aspect, the present invention provides a LINC complex inhibitor for use in a method of treating or preventing a laminopathy.

The present invention also provides the use of a LINC complex inhibitor in the manufacture of a medicament for use in a method of treating or preventing a laminopathy.

The present invention also provides a method of treating or preventing a laminopathy, comprising administering a therapeutically or prophylactically effective amount of a LINC complex inhibitor to a subject.

In some embodiments the laminopathy is characterised by one or more of myopathy, cardiomyopathy, dilated cardiomyopathy, muscular dystrophy, cardiac muscular dystrophy, skeletal muscular dystrophy, progeria, neuropathy, lipoatrophy, skeletal dysplasia, lipodystrophy, leukodystrophy or dermopathy.

In some embodiments the laminopathy is associated with mutation to LMNA.

In some embodiments the laminopathy is selected from Hutchinson-Gilford Progeria Syndrome; Dilated Cardiomyopathy; Muscular Dystrophy, Congenital, Lmna-Related; Emery-Dreifuss Muscular Dystrophy 2, Autosomal Dominant; Muscular Dystrophy; Mandibuloacral Dysplasia with Type a Lipodystrophy; Cardiomyopathy, Dilated, 1a; Charcot-Marie-Tooth Disease; Limb-Girdle Muscular Dystrophy; Cardiomyopathy, Dilated, with Hypergonadotropic Hypogonadism; Emery-Dreifuss Muscular Dystrophy 3, Autosomal Recessive; Lipodystrophy, Familial Partial, Type 2; Emery-Dreifuss Muscular Dystrophy; Charcot-Marie-Tooth Disease, Axonal, Type 2b1; Heart-Hand Syndrome, Slovenian Type; Aging; Familial Partial Lipodystrophy; Restrictive Dermopathy, Lethal; Arrhythmogenic Right Ventricular Cardiomyopathy; Tooth Disease; Heart Disease; Werner Syndrome; Hypertrophic Cardiomyopathy; Left Ventricular Noncompaction; Atrioventricular Block; Calcinosis; Acroosteolysis; Autosomal Dominant Limb-Girdle Muscular Dystrophy; Diabetes Mellitus, Noninsulin-Dependent; Osteoporosis; Atrial Fibrillation; Atrial Standstill 1; Acanthosis Nigricans; Cardiac Conduction Defect; Catecholaminergic Polymorphic Ventricular Tachycardia; Mandibular Hypoplasia, Deafness, Progeroid Features, and Lipodystrophy Syndrome; Sick Sinus Syndrome; Pelger-Huet Anomaly; Charcot-Marie-Tooth Disease, Axonal, Type 2e; Congenital Generalized Lipodystrophy; Restrictive Cardiomyopathy; Congenital Fiber-Type Disproportion; Lipodystrophy, Congenital Generalized, Type 1; Myofibrillar Myopathy; Lipodystrophy, Familial Partial, Type 1; Axonal Neuropathy; Atypical Werner Syndrome; Ovarian Cystadenoma; Fanconi Anemia, Complementation Group a; Body Mass Index Quantitative Trait Locus 11; Skin Disease; Rigid Spine Muscular Dystrophy 1; Neuromuscular Disease; Hallermann-Streiff Syndrome; Bethlem Myopathy 1; Acquired Generalized Lipodystrophy; Cardiomyopathy, Dilated, 1e; Lipodystrophy, Congenital Generalized, Type 4; Undifferentiated Pleomorphic Sarcoma; Lipodystrophy, Familial Partial, Type 3; Muscular Dystrophy, Congenital Merosin-Deficient, 1a; Proximal Spinal Muscular Atrophy; Muscular Dystrophy-Dystroglycanopathy , Type B, 5; Muscular Dystrophy, Congenital, 1b; Reynolds Syndrome; Wiedemann-Rautenstrauch Syndrome; Emery-Dreifuss Muscular Dystrophy 1, X-Linked; Lipodystrophy, Congenital Generalized, Type 2; Monogenic Diabetes; Cardiomyopathy, Dilated, 1d; Myopathy, Proximal, and Ophthalmoplegia; Muscle Tissue Disease; Lipodystrophy, Familial Partial, Type 4; Cardiomyopathy, Dilated, 1h; Second-Degree Atrioventricular Block; Median Neuropathy; Intrinsic Cardiomyopathy; Prolapse of Female Genital Organ; Complete Generalized Lipodystrophy; Rigid Spine Muscular Dystrophy; Emerinopathy; Ulnar Nerve Lesion; Limb-Girdle Muscular Dystrophy Type 1b; Lmna-Related Dilated Cardiomyopathy; Pelvic Muscle Wasting; Generalized Lipodystrophy-Associated Progeroid Syndrome; Muscular Disease; Cardiomyopathy, Dilated, 1b; Autosomal Genetic Disease; Familial Isolated Arrhythmogenic Ventricular Dysplasia, Right Dominant Form; Familial Isolated Arrhythmogenic Ventricular Dysplasia, Biventricular Form; Familial Isolated Arrhythmogenic Ventricular Dysplasia, Left Dominant Form; Lmna-Related Cardiocutaneous Progeria Syndrome; and Autosomal Semi-Dominant Severe Lipodystrophic Laminopathy.

The present invention also provides a LINC complex inhibitor for use in a method of treating or preventing a disease characterised by hyperlipidemia.

The present invention also provides the use of a LINC complex inhibitor in the manufacture of a medicament for use in a method of treating or preventing a disease characterised by hyperlipidemia.

The present invention also provides a method of treating or preventing a disease characterised by hyperlipidemia, comprising administering a therapeutically or prophylactically effective amount of a LINC complex inhibitor to a subject.

In some embodiments the disease characterised by hyperlipidemia is selected from atherosclerosis, cardiovascular disease, stroke and a familial hyperlipidemia.

In some embodiments in accordance with various aspects of the present invention, the LINC complex inhibitor is capable of binding to a LINC complex, a LINC complex protein or an interaction partner for a LINC complex protein, or wherein the LINC complex inhibitor is capable of reducing expression of a LINC complex protein.

In some embodiments the LINC complex inhibitor is capable of inhibiting interaction between a LINC complex protein and an interaction partner for a LINC complex protein.

In some embodiments the LINC complex inhibitor is a peptide/polypeptide, nucleic acid or small molecule.

In some embodiments the LINC complex inhibitor is capable of modifying a gene encoding a LINC complex protein to reduce its expression.

In some embodiments the LINC complex inhibitor comprises a site-specific nuclease (SSN) targeting a gene encoding a LINC complex protein.

In some embodiments the LINC complex inhibitor is an inhibitory nucleic acid capable of reducing expression of a LINC complex protein by RNA interference (RNAi).

In some embodiments the method comprises administering nucleic acid encoding the LINC complex inhibitor, or nucleic acid encoding factors required for production of the LINC complex inhibitor, to the subject.

### Description

### LINC complex structure and function

Linker of nucleoskeleton and cytoskeleton (LINC) complexes are polypeptide complexes comprising SUN domain-containing proteins and KASH domain-containing proteins. LINC complex structure is reviewed in e.g. in Sosa et al., Curr Opin Struct Biol. (2013) 23(2):285-91 and Hieda, Cells (2017) 6(1):3, both of which are hereby incorporated by reference in their entirety.

LINC complexes connect the inner nuclear membrane (INM) and the outer nuclear membrane (ONM) or the nuclear envelope. SUN domain-containing proteins span the INM, and are associated with nuclear lamins and chromatin-binding proteins on the nucleoplasmic side of the INM, and with KASH domain-containing proteins on the perinuclear side of the INM. KASH domain-containing proteins span the ONM, and are associated with cytoskeletal structural components such as actin filaments, microtubule motors and intermediate filaments on the cytoplasmic side of the ONM, and with SUN domain-containing proteins on the perinuclear side of the ONM. SUN domain proteins function as translumenal tethers for KASH domain proteins in the ONM.

Herein, a "SUN domain-containing protein" refers to any polypeptide comprising a SUN domain. SUN (Sad1 and UNC-84) domain proteins are important INM components comprising conserved, carboxy terminal SUN domains which localise to the perinuclear space. SUN domains comprise ~175 residues and are provided at the end of helical stalk regions. The nucleoplasmic domains of SUN proteins interact with structural components of the nucleoskeleton.

A SUN domain may comprise or consist of the amino acid sequence shown in SEQ ID NO:82, 83, 84, 85, 86 or 87, or an amino acid sequence having at least 70%, preferably one of 80%, 85%, 90%, 91%, 92%, 93%, 94%, 95%, 96%, 97%, 98%, 99% or 100% amino acid sequence identity to the amino acid sequence shown in SEQ ID NO:82, 83, 84, 85, 86 or 87.

In some embodiments a SUN domain-containing protein is selected from SUN1, SUN2, SUN3, SUN5, SPAG4 and SUCO. In some embodiments the SUN domain-containing protein is SUN1 or SUN2.

In some embodiments a SUN domain-containing protein is capable of forming a LINC complex. In some embodiments a SUN domain-containing protein is capable of interacting with a KASH domain and/or a KASH domain-containing protein.

Human SUN1 is the polypeptide identified by UniProtKB 094901, the amino acid sequence of which is shown in SEQ ID NO:88. Human SUN2 is the polypeptide identified by UniProtKB Q9UH99, the amino acid sequence of which is shown in SEQ ID NO:89. Human SUN3 is the polypeptide identified by UniProtKB Q8TAQ9, the amino acid sequence of which is shown in SEQ ID NO:90. Human SUN5 is the polypeptide identified by UniProtKB A9Z1W8, the amino acid sequence of which is shown in SEQ ID NO:91. Human SPAG4 is the polypeptide identified by UniProtKB Q9NPE6, the amino acid sequence of which is shown in SEQ ID NO:92. Human SUCO is the polypeptide identified by UniProtKB Q9UBS9, the amino acid sequence of which is shown in SEQ ID NO:93.

In this specification "SUN1", "SUN2, "SUN3", "SUN5" "SPAG4" and "SUCO" respectively refer to SUN1, SUN2, SUN3, SUN5, SPAG4 and SUCO from any species and include isoforms, fragments, variants or homologues thereof.

As used herein, a "fragment", "variant" or "homologue" of a protein may optionally be characterised as having at least 60%, preferably one of 70%, 75%, 80%, 85%, 90%, 91%, 92%, 93%, 94%, 95%, 96%, 97%, 98%, 99% or 100% amino acid sequence identity to the amino acid sequence of the reference protein (e.g. a reference isoform of the reference protein). In some embodiments fragments/variants/ isoforms/homologues may be characterised by ability to perform a function performed by the reference protein.

A "fragment" generally refers to a fraction of the reference protein. A "variant" generally refers to a protein having an amino acid sequence comprising one or more amino acid substitutions, insertions, deletions or other modifications relative to the amino acid sequence of the reference protein, but retaining a considerable degree of sequence identity (e.g. at least 60%) to the amino acid sequence of the reference protein. An "isoform" generally refers to a variant of the reference protein expressed by the same species as the species of the reference protein. A "homologue" generally refers to a variant of the reference protein produced by a different species as compared to the species of the reference protein. Homologues include orthologues.

A "fragment" may be of any length (by number of amino acids), although may optionally be at least 20% of the length of the reference protein (that is, the protein from which the fragment is derived) and may have a maximum length of one of 50%, 75%, 80%, 85%, 90%, 91%, 92%, 93%, 94%, 95%, 96%, 97%, 98%, or 99% of the length of the reference protein.

Isoforms, fragments, variants or homologues may optionally be functional isoforms, fragments, variants or homologues, e.g. having a functional property/activity of the reference protein, as determined by analysis by a suitable assay for the functional property/activity.

In this specification, reference to "SUN1" refers to the protein having the amino acid sequence shown in SEQ ID NO:88, and fragments, variants or homologues thereof. In some embodiments SUN1 comprises, or consists of, an amino acid sequence having at least 70%, preferably one of 80%, 85%, 90%, 91%, 92%, 93%, 94%, 95%, 96%, 97%, 98%, 99% or 100% amino acid sequence identity to SEQ ID NO:88.

In this specification, reference to "SUN2" refers to the protein having the amino acid sequence shown in SEQ ID NO:89, and fragments, variants or homologues thereof. In some embodiments SUN2 comprises, or consists of, an amino acid sequence having at least 70%, preferably one of 80%, 85%, 90%, 91%, 92%, 93%, 94%, 95%, 96%, 97%, 98%, 99% or 100% amino acid sequence identity to SEQ ID NO:89.

In this specification, reference to "SUN3" refers to the protein having the amino acid sequence shown in SEQ ID NO:90, and fragments, variants or homologues thereof. In some embodiments SUN3 comprises, or consists of, an amino acid sequence having at least 70%, preferably one of 80%, 85%, 90%, 91%, 92%, 93%, 94%, 95%, 96%, 97%, 98%, 99% or 100% amino acid sequence identity to SEQ ID NO:90.

In this specification, reference to "SUN5" refers to the protein having the amino acid sequence shown in SEQ ID NO:91, and fragments, variants or homologues thereof. In some embodiments SUN5 comprises, or consists of, an amino acid sequence having at least 70%, preferably one of 80%, 85%, 90%, 91%, 92%, 93%, 94%, 95%, 96%, 97%, 98%, 99% or 100% amino acid sequence identity to SEQ ID NO:91.

In this specification, reference to "SPAG4" refers to the protein having the amino acid sequence shown in SEQ ID NO:92, and fragments, variants or homologues thereof. In some embodiments SPAG4 comprises, or consists of, an amino acid sequence having at least 70%, preferably one of 80%, 85%, 90%, 91%, 92%, 93%, 94%, 95%, 96%, 97%, 98%, 99% or 100% amino acid sequence identity to SEQ ID NO:92.

In this specification, reference to "SUCO" refers to the protein having the amino acid sequence shown in SEQ ID NO:93, and fragments, variants or homologues thereof. In some embodiments SUCO comprises, or consists of, an amino acid sequence having at least 70%, preferably one of 80%, 85%, 90%, 91%, 92%, 93%, 94%, 95%, 96%, 97%, 98%, 99% or 100% amino acid sequence identity to SEQ ID NO:93.

Herein, a "KASH domain-containing protein" refers to any polypeptide comprising a KASH domain. KASH (Klarsicht, ANC-1, Syne homology) domain proteins are carboxy terminal-anchored membrane proteins which are targeted to the nuclear envelope. The 50-60 amino acid KASH domain is found at the C-terminus. KASH domains are hydrophobic, and comprise a single-membrane spanning helix which spans the ONM, and a ~30 amino acid region which extends into the perinuclear space.

A KASH domain may comprise or consist of the amino acid sequence shown in SEQ ID NO:97, 98, 99 or 100, or an amino acid sequence having at least 70%, preferably one of 80%, 85%, 90%, 91%, 92%, 93%, 94%, 95%, 96%, 97%, 98%, 99% or 100% amino acid sequence identity to the amino acid sequence shown in SEQ ID NO:97, 98, 99, 100 or 101.

In some embodiments a KASH domain-containing protein is selected from Nesprin-1 , Nesprin-2, Nesprin-3, Nesprin-4 (also known as SYNE1 , SYNE2, SYNE3 and SYNE4, respectively), KASH5 and LRMP. In some embodiments a KASH domain-containing protein is Nesprin-1, Nesprin-2 or Nesprin-3.

In some embodiments a KASH domain-containing protein is capable of forming a LINC complex. In some embodiments a KASH domain-containing protein is capable of interacting with a SUN domain and/or a SUN domain-containing protein.

Human Nesprin-1 is the polypeptide identified by UniProtKB Q8NF91, the amino acid sequence of which is shown in SEQ ID NO:102. Human Nesprin-2 is the polypeptide identified by UniProtKB Q8WXH0, the amino acid sequence of which is shown in SEQ ID NO:103. Human Nesprin-3 is the polypeptide identified by UniProtKB Q6ZMZ3, the amino acid sequence of which is shown in SEQ ID NO:104. Human Nesprin-4 is the polypeptide identified by UniProtKB Q8N205, the amino acid sequence of which is shown in SEQ ID NO:105. Human KASH5 is the polypeptide identified by UniProtKB Q8N6L0, the amino acid sequence of which is shown in SEQ ID NO:106. Human LRMP is the polypeptide identified by UniProtKB Q12912, the amino acid sequence of which is shown in SEQ ID NO:113.

In this specification "Nesprin-1", "Nesprin-2", "Nesprin-3", "Nesprin-4", "KASH5" and "LRMP" respectively refer to Nesprin-1, Nesprin-2, Nesprin-3, Nesprin-4, KASH5 and LRMP from any species and include isoforms, fragments, variants or homologues thereof.

In this specification, reference to "Nesprin-1" refers to the protein having the amino acid sequence shown in SEQ ID NO:102, and fragments, variants or homologues thereof. In some embodiments Nesprin-1 comprises, or consists of, an amino acid sequence having at least 70%, preferably one of 80%, 85%, 90%, 91%, 92%, 93%, 94%, 95%, 96%, 97%, 98%, 99% or 100% amino acid sequence identity to SEQ ID NO:102.

In this specification, reference to "Nesprin-2" refers to the protein having the amino acid sequence shown in SEQ ID NO:103, and fragments, variants or homologues thereof. In some embodiments Nesprin-2 comprises, or consists of, an amino acid sequence having at least 70%, preferably one of 80%, 85%, 90%, 91%, 92%, 93%, 94%, 95%, 96%, 97%, 98%, 99% or 100% amino acid sequence identity to SEQ ID NO:103.

In this specification, reference to "Nesprin-3" refers to the protein having the amino acid sequence shown in SEQ ID NO:104, and fragments, variants or homologues thereof. In some embodiments Nesprin-3 comprises, or consists of, an amino acid sequence having at least 70%, preferably one of 80%, 85%, 90%, 91%, 92%, 93%, 94%, 95%, 96%, 97%, 98%, 99% or 100% amino acid sequence identity to SEQ ID NO:104.

In this specification, reference to "Nesprin-4" refers to the protein having the amino acid sequence shown in SEQ ID NO:105, and fragments, variants or homologues thereof. In some embodiments Nesprin-4 comprises, or consists of, an amino acid sequence having at least 70%, preferably one of 80%, 85%, 90%, 91%, 92%, 93%, 94%, 95%, 96%, 97%, 98%, 99% or 100% amino acid sequence identity to SEQ ID NO:105.

In this specification, reference to "KASH5" refers to the protein having the amino acid sequence shown in SEQ ID NO:106, and fragments, variants or homologues thereof. In some embodiments KASH5 comprises, or consists of, an amino acid sequence having at least 70%, preferably one of 80%, 85%, 90%, 91%, 92%, 93%, 94%, 95%, 96%, 97%, 98%, 99% or 100% amino acid sequence identity to SEQ ID NO:106.

In this specification, reference to "LRMP" refers to the protein having the amino acid sequence shown in SEQ ID NO:113, and fragments, variants or homologues thereof. In some embodiments LMRP comprises, or consists of, an amino acid sequence having at least 70%, preferably one of 80%, 85%, 90%, 91%, 92%, 93%, 94%, 95%, 96%, 97%, 98%, 99% or 100% amino acid sequence identity to SEQ ID NO:113.

As used herein, a "LINC complex" refers to a polypeptide complex comprising a SUN domain-containing protein and a KASH domain-containing protein.

LINC complexes are formed by protein-protein interactions between SUN domain-containing proteins and KASH domain-containing proteins. The LINC complex may comprise non-covalent and/or covalent interactions between SUN domains and KASH domains. Non-covalent interactions include e.g. hydrogen bonds, ionic interaction, Van der Waals forces and hydrophobic bonds. Covalent interactions include e.g. disulphide bonds.

SUN domain proteins are thought to oligomerise to form trimers via interaction between their stalk regions to form coiled-coil triple helix (Zhou et al., J. Biol. Chem. (2012) 287: 5317-5326). Deletion of SUN domain protein stalk regions has been shown to disrupt LINC complex formation. SUN domains assume a β sandwich structure and SUN domains of the trimer interact extensively with one another in part through protruding β sheets known as KASH lids; the KASH lid of one SUN domain partially overlaps the β sandwich of the adjacent SUN domain (Sosa et al., Cell (2012) 149:1035-1047).

KASH domain proteins can also oligomerise, which may involve protein-protein interactions between the transmembrane helices. A single KASH domain interacts with two adjacent SUN domains along the groove formed between the KASH lid of one SUN domain and the upper region of the β sandwich of the adjacent SUN domain. In this way, SUN and KASH domains are thought to interact to form a 3:3 hexameric heterocomplex. The 2-3 proline residues immediately prior to the C-terminus of the KASH domain are thought to be accommodated in a deep pocket within the surface of a SUN domain. This region of KASH is important for SUN-KASH interactions; extension of the C-terminus by only a single amino acid disrupts LINC complex formation. Conserved cysteine residues of SUN and KASH domains form disulphide bonds, further stabilising the SUN-KASH complex. The disulphide bonds may be important for force transmission through the LINC complex (Jahed et al., Biophys. J. (2015) 109:501-509).

As explained hereinabove, LINC complexes are thought to form through the interaction of the SUN domains of three SUN domain-containing proteins and the KASH domains of three KASH domain-containing proteins. Interaction between the SUN and KASH domain proteins is thought to be promiscuous; SUN1 and SUN2 have been shown to interact with Nesprin-1 , Nesprin-2 and Nesprin-3.

A LINC complex according to the present invention may comprise any SUN domain-containing protein and any KASH domain-containing protein. The SUN domain-containing proteins of the LINC complex may be identical or non-identical. The KASH domain-containing proteins of the LINC complex may be identical or non-identical.

LINC complex function is reviewed in e.g. in Hieda, Cells (2017) 6(1):3 (incorporated by reference hereinabove), and Stroud, Biophys Rev. (2018) 10(4):1033-1051, hereby incorporated by reference its entirety.

The LINC complex performs diverse functions, including providing structural support to the nucleus, shaping and positioning the nucleus, maintaining connection between the centrosome and the nucleus and spacing of the nuclear membrane, DNA repair, cell migration and moving chromosome within the nucleus during meiosis.

The LINC complex has a mechanosensory role to translate mechanical stimuli and changes in the extracellular matrix into signals allowing the cell to adapt to its environment by modulation of cytoskeleton organization, gene expression, nuclear organisation, and structure.

Integrins mediate the transduction of forces from the external microenvironment to the intracellular cytoskeleton, and nucleo-cytoskeletal molecular connections transmit the forces to chromosomal organisations in the nucleus. The nuclear lamina triggers the deformation of nuclear structures, and initiates changes in gene regulation.

The nuclear envelope is a key structure in such processes. On the nucleoplasmic side of the INM, the nuclear lamina (composed of A-type and B-type lamins) forms a lattice structure which contributes to the mechanical stress resistance of the nucleus, and which is essential to the structural integrity of the nuclear envelope. Nuclear lamins are involved in processes critical to cell function and viability, including maintenance of nuclear integrity, regulation of cell cycle, mechanotransduction, cell signalling and DNA repair.

Deviations from normal expression and/or function of nuclear envelope proteins, and deviations from normal expression and/or function of factors directly or indirectly associated with the nuclear envelope, are implicated in a variety of diseases including muscular dystrophies, cardiomyopathies, lipodystrophy, progeria, cancer, and neurological diseases.

### LINC complex inhibition

The present invention is concerned with LINC complex inhibition. As used herein "LINC complex inhibition" encompass inhibition of formation of a LINC complex (i.e. inhibition of LINC complex assembly), disruption/degradation of a LINC complex, and inhibition of LINC complex activity/function.

In some embodiments, formation of a LINC complex may be inhibited by inhibiting the gene and/or protein expression of a constituent protein of a LINC complex. Constituent proteins of LINC complexes include SUN domain-containing proteins and KASH domain-containing proteins.

For conciseness, in the present specification "a constituent protein of a LINC complex" may be referred to simply as "a LINC complex protein".

In some embodiments, inhibiting formation of a LINC complex comprises one or more of: inhibiting the gene or protein expression of encoding a LINC complex protein; modifying a gene encoding a LINC complex protein to reduce/prevent its expression; reducing the level of RNA encoding a LINC complex protein; inhibiting transcription of nucleic acid encoding a LINC complex protein; increasing degradation of RNA encoding a LINC complex protein; reducing the level of a LINC complex protein; disrupt normal post-transcriptional processing (e.g. splicing, translation, post-translational processing) of RNA encoding a LINC complex protein; and increasing degradation of a LINC complex protein.

Gene expression can be determined by means well known to the skilled person. The level of RNA encoding constituent proteins of LINC complexes can be determined e.g. by techniques such as RT-qPCR, northern blot, etc. A reduction in the level of RNA encoding a constituent proteins of a LINC complex may e.g. be the result of reduced transcription of nucleic acid encoding the LINC complex protein, or increased degradation of RNA encoding the LINC complex protein.

Reduced transcription of nucleic acid encoding a LINC complex protein may be a consequence of inhibition of assembly and/or activity of factors required for transcription of the DNA encoding the LINC complex protein. Increased degradation of RNA encoding a LINC complex protein may be a consequence of increased enzymatic degradation of RNA encoding the LINC complex protein, e.g. as a consequence of RNA interference (RNAi), and/or reduced stability of RNA encoding the LINC complex protein.

Protein expression can be determined by means well known to the skilled person. The levels of constituent proteins of LINC complexes can be determined e.g. by antibody-based methods including western blot, immunohisto/cytochemistry, flow cytometry, ELISA, or by reporter-based methods.

Protein degradation can be evaluated e.g. by detection of, or analysis of the level/proportion of, ubiquitinated protein, association with ubiquitin ligase and/or proteosomal localisation.

A reduction in the level of a LINC complex protein may e.g. be the result of a reduced level of RNA encoding the LINC complex protein, reduced post-transcriptional processing of RNA encoding the LINC complex protein, or increased degradation of the LINC complex protein.

Disruption to normal post-transcriptional processing of a LINC complex protein may e.g. be reduced/altered splicing of pre-mRNA to mature mRNA encoding the LINC complex protein, reduced translation of mRNA encoding the LINC complex protein, or reduced/altered post-translational processing of the LINC complex protein.

Reduced/altered splicing of pre-mRNA to mature mRNA encoding the LINC complex protein may be a consequence of inhibition of assembly and/or activity of factors required for normal splicing. Reduced translation of mRNA encoding the LINC complex protein may be a consequence of inhibition of assembly and/or activity of factors required for translation. Reduced/altered post-translational processing (e.g. enzymatic processing, folding) may be a consequence of inhibition of assembly and/or activity of factors required for normal post-translational processing of the LINC complex protein. Increased degradation of the LINC complex protein may be a consequence of increased enzymatic (e.g. protease-mediated) degradation of the protein, which may e.g. be associated with misfolding.

In some embodiments, formation of a LINC complex may be inhibited by inhibiting trafficking of, and/or disrupting normal subcellular localisation of, constituent proteins of a LINC complex (e.g. SUN domain-containing proteins and/or KASH domain-containing proteins). In some embodiments, inhibiting formation of a LINC complex comprises one or more of: reducing the level/proportion of a LINC complex protein localised to the nuclear envelope; reducing the level/proportion of SUN domain-containing protein associated with inner nuclear membrane; reducing the level/proportion of KASH domain-containing protein associated with outer nuclear membrane; increasing retention of a LINC complex protein in the endoplasmic reticulum; and increasing the level/proportion of a LINC complex protein localised to the endoplasmic reticulum.

The subcellular localisation of constituent proteins of LINC complexes within cells can be analysed using techniques known to the person skilled in the art. Such techniques include e.g. analysis by immunocytochemistry and reporter-based methods. For example, Boni et al., J. Cell Biology (2015) 209(5):705-720 and Smoyer et al., J. Cell Biology (2016) 215(4):575-590 describe a reporter system permitting imaging of proteins in the ER, INM and ONM. Such methods can be employed to analyse the levels/proportions of constituent proteins of LINC complexes in the nuclear envelope, inner nuclear membrane and an outer nuclear membrane.

In some embodiments, formation of a LINC complex may be inhibited by inhibiting interaction between constituent proteins of LINC complexes (e.g. SUN domain-containing proteins and KASH domain-containing proteins). In some embodiments, formation of a LINC complex may be inhibited by inhibiting interaction between constituent proteins of a LINC complex and interaction partners for constituent proteins of a LINC complex.

In some embodiments, inhibiting formation of a LINC complex comprises one or more of: inhibiting interaction between a SUN domain-containing protein and an interaction partner for a SUN domain-containing protein; inhibiting interaction between a KASH domain-containing protein and an interaction partner for a KASH domain-containing protein; inhibiting interaction between a SUN domain-containing protein and a KASH domain-containing protein; inhibiting interaction between a SUN domain-containing protein and a lamin; inhibiting interaction between a SUN domain-containing protein and a chromatin-binding protein; inhibiting interaction between a KASH domain-containing protein and a SUN domain-containing protein; and inhibiting interaction between a KASH domain-containing protein and a cytoskeletal component (e.g. a microfilament or a constituent thereof (e.g. actin), a microtubule or a constituent thereof (e.g. tubulin) or an intermediate filament or a constituent thereof).

In some embodiments, LINC complex inhibition comprises disruption/degradation of a LINC complex.

In some embodiments, LINC complex disruption/degradation comprises one or more of: inhibiting interaction between a SUN domain-containing protein and an interaction partner for a SUN domain-containing protein; inhibiting interaction between a KASH domain-containing protein and an interaction partner for a KASH domain-containing protein; inhibiting interaction between a SUN domain-containing protein and a KASH domain-containing protein; inhibiting interaction between a SUN domain-containing protein and a lamin; inhibiting interaction between a SUN domain-containing protein and a chromatin-binding protein; inhibiting interaction between a KASH domain-containing protein and a SUN domain-containing protein; and inhibiting interaction between a KASH domain-containing protein and a cytoskeletal component (e.g. a microfilament or a constituent thereof (e.g. actin), a microtubule or a constituent thereof (e.g. tubulin) or an intermediate filament or a constituent thereof); increasing LINC complex disassembly; increasing LINC complex degradation; displacing a LINC complex protein from a LINC complex; and reducing the level of a LINC complex.

Herein, an interaction partner for a SUN domain-containing protein may be any molecule (e.g. protein/nucleic acid) with which SUN domain-containing protein interacts. An interaction partner for a SUN domain-containing protein may be a protein capable of forming a complex with a SUN domain-containing protein through protein-protein interaction. In some embodiments an interaction partner for a SUN domain-containing protein may be a KASH domain-containing protein (e.g. Nesprin-1, Nesprin-2, Nesprin-3, Nesprin-4 or KASH5), a SUN domain-containing protein (e.g. SUN1, SUN2, SUN3, SUNS, SPAG4 or SUCO), a nucleoplasmic protein, a lamin (e.g. lamin A, lamin C, lamin B1 or lamin B2) or a chromatin-binding protein.

Herein, an interaction partner for a KASH domain-containing protein may be any molecule (e.g. protein/nucleic acid) with which KASH domain-containing protein interacts. An interaction partner for a KASH domain-containing protein may be a protein capable of forming a complex with a KASH domain-containing protein through protein-protein interaction. In some embodiments an interaction partner for a KASH domain-containing protein may be a SUN domain-containing protein (e.g. SUN1, SUN2, SUN3, SUNS, SPAG4 or SUCO), a KASH domain-containing protein (e.g. Nesprin-1, Nesprin-2, Nesprin-3, Nesprin-4, KASH5 or LRMP), a cytoplasmic protein, a cytoskeletal protein, a microfilament, actin, a microtubule, tubulin, a microtubule motor, or an intermediate filament protein.

Interaction between constituent proteins of LINC complexes and interaction partners for such proteins can be analysed using techniques well known to the skilled person such as co-immunoprecipitation, and resonance energy transfer (RET) assays using appropriately labelled species. Inhibition of interaction can be determined in such assays by detection of a reduction in the level of interaction as compared to a control, uninhibited condition.

LINC complexes and constituent proteins thereof may be detected e.g. using methods well known to the skilled person such as antibody-based methods including western blot, immunohisto/cytochemistry, flow cytometry, ELISA, or by reporter-based methods.

LINC complex inhibition may be characterised by a reduced level of a function of a LINC complex. In some embodiments, LINC complex inhibition may be determined by detection of a reduced level of a correlate of LINC complex function.

In particular embodiments contemplated herein, LINC complex inhibition is achieved by one or more of: modifying a gene encoding a SUN domain-containing protein to reduce/prevent its expression; modifying a gene encoding a KASH domain-containing protein to reduce/prevent its expression; inhibition of expression of a SUN domain-containing protein by RNAi; inhibition of expression of a KASH domain-containing protein by RNAi; or inhibiting interaction between a SUN domain-containing protein and a KASH domain-containing protein.

In some embodiments modifying a gene encoding a KASH domain-containing protein or a SUN domain-containing protein to reduce/prevent its expression is achieved using a site-specific nuclease (SSN) system (e.g. a CRISPR-based system) targeting the relevant gene. In some embodiments inhibition of expression of a KASH domain-containing protein or a SUN domain-containing protein by RNAi is achieved using siRNA, miRNA or shRNA targeting the relevant protein.

In some embodiments inhibiting interaction between a SUN domain-containing protein and a KASH domain-containing protein is achieved using a dominant-negative SUN domain-containing protein, a dominant-negative KASH domain-containing protein, a small molecule inhibitor of interaction between a SUN domain-containing protein and a KASH domain-containing protein, a peptidomimetic of a KASH domain or a peptidomimetic of a SUN domain. It will be appreciated that inhibition of interaction is between an endogenous SUN domain-containing protein and an endogenous KASH domain-containing protein.

### LINC complex inhibitors

Aspects of the present invention comprise LINC complex inhibition using a LINC complex inhibitor.

A "LINC complex inhibitor" refers to any agent capable of achieving LINC complex inhibition. LINC complex inhibitors include agents capable of inhibiting formation of a LINC complex (i.e. inhibiting LINC complex assembly), disrupting/degrading a LINC complex, or inhibiting LINC complex function.

Such agents may be effectors of (i.e. may directly or indirectly cause) LINC complex inhibition as described hereinabove. LINC complex inhibitors may also be referred to herein as LINC complex antagonists.

In some embodiments, a LINC complex inhibitor may: inhibit formation of a LINC complex; disrupt/degrade a LINC complex; inhibit LINC complex activity; inhibit the gene and/or protein expression of a LINC complex protein; modify a gene encoding a LINC complex protein to reduce/prevent its expression; reduce the level of RNA encoding a LINC complex protein; inhibit transcription of nucleic acid encoding a LINC complex protein; increase degradation of RNA encoding a LINC complex protein; reduce the level of a LINC complex protein; disrupt normal post-transcriptional processing (e.g. splicing, translation, post-translational processing) of RNA encoding a LINC complex protein; increase degradation of a LINC complex protein; inhibit trafficking of, and/or disrupt normal subcellular localisation of, a LINC complex protein; reduce the level/proportion of a LINC complex protein localised to the nuclear envelope; reduce the level/proportion of SUN domain-containing protein associated with inner nuclear membrane; reduce the level/proportion of KASH domain-containing protein associated with outer nuclear membrane; increase retention of a LINC complex protein in the endoplasmic reticulum; increase the level/proportion of a LINC complex protein localised to the endoplasmic reticulum; inhibit interaction between constituent proteins of a LINC complex; inhibit interaction between a LINC complex protein and an interaction partner for a LINC complex protein; inhibit interaction between a SUN domain-containing protein and an interaction partner for a SUN domain-containing protein; inhibit interaction between a KASH domain-containing protein and an interaction partner for a KASH domain-containing protein; inhibit interaction between a SUN domain-containing protein and a KASH domain-containing protein; inhibit interaction between a SUN domain-containing protein and a lamin; inhibit interaction between a SUN domain-containing protein and a chromatin-binding protein; inhibit interaction between a KASH domain-containing protein and a SUN domain-containing protein; inhibit interaction between a KASH domain-containing protein and a cytoskeletal component (e.g. a microfilament or a constituent thereof (e.g. actin), a microtubule or a constituent thereof (e.g. tubulin) or an intermediate filament or a constituent thereof); increase disassembly of a LINC complex; increase degradation of a LINC complex; displace a LINC complex protein from a LINC complex; and/or reduce the level of a LINC complex.

It will be appreciated that a given LINC complex inhibitor may display more than one of the properties recited in the preceding paragraph. A given agent may be evaluated for the properties recited in the preceding paragraph using suitable assays. The assays may be e.g. *in vitro* assays, optionally cell-based assays or cell-free assays.

Where assays are cell-based assays, they may comprise treating cells with the test agent in order to determine whether the agent displays one or more of the recited properties. Assays may employ endogenously- or recombinantly-expressed proteins, and may use species labelled with detectable entities in order to facilitate their detection.

Agents capable of reducing gene expression of a LINC complex protein (e.g. reducing the level of RNA encoding a LINC complex protein, reducing transcription of nucleic acid encoding a LINC complex protein and/or increasing degradation of RNA encoding a LINC complex protein) may be identified using assays comprising detecting the level of RNA encoding the relevant protein, e.g. by RT-qPCR. Such assays may comprise treating cells/tissue with the agent, and subsequently comparing the level of RNA encoding the relevant protein in such cells/tissue to the level of RNA encoding the relevant protein in cells/tissue of an appropriate control condition (e.g. untreated/vehicle-treated cells/tissue). Assays for detecting reduced/altered splicing of pre-mRNA of a given protein may comprise detecting and/or quantifying one or more isoforms of the relevant protein, or RNA encoding one or more of said isoforms.

Agents capable of reducing protein expression of a LINC complex protein (e.g. reducing the level of a LINC complex protein, increasing degradation of a LINC complex protein) may be identified using assays comprising detecting the level of the relevant protein, e.g. using antibody/reporter-based methods (western blot, ELISA, immunohisto/cytochemistry, etc.). Such assays may comprise treating cells/tissue with the agent, and subsequently comparing the level of the relevant protein in such cells/tissue to the level of the relevant protein in cells/tissue of an appropriate control condition (e.g. untreated/vehicle-treated cells/tissue). Assays of protein degradation may comprise evaluating e.g. ubiquitination or proteosomal localisation of the relevant protein, and/or the proportion of the relevant protein that is ubiquitinated or localised to the proteasome.

Agents capable of inhibiting trafficking and/or disrupting normal subcellular localisation of a LINC complex protein may be identified using assays comprising detecting the presence of, or determining the proportion of, the relevant protein in a given subcellular location, e.g. using antibody/reporter-based methods (western blot, ELISA, immunohisto/cytochemistry, etc.). Subcellular localisation may be analysed e.g. by immunocytochemistry, or western blot of extracts prepared from different cellular fractions, and may employ organelle markers and/or labelled proteins of known subcellular localisation. Assays may comprise treating cells/tissue with the agent, subsequently comparing the subcellular localisation of the relevant protein in such cells to the subcellular localisation of the relevant protein in cells/tissue of an appropriate control condition (e.g. untreated/vehicle-treated cells/tissue).

Agents capable of inhibiting interaction between a LINC complex protein and an interaction partner for a LINC complex protein may be identified using assays comprising detecting the level of interaction between a LINC complex protein and an interaction partner for a LINC complex protein, e.g. using antibody/reporter-based methods. The level of interaction between a LINC complex protein and an interaction partner for a LINC complex protein can be analysed e.g. using resonance energy transfer techniques (e.g. FRET, BRET), co-immunoprecipitation or methods analysing a correlate of interaction (e.g. a function of a LINC complex). Assays may comprise treating cells/tissue with the agent, and subsequently comparing the level of interaction in such cells/tissue to the level of interaction in cells/tissue of an appropriate control condition (e.g. untreated/vehicle-treated cells/tissue). Interaction between a LINC complex protein and an interaction partner for a LINC complex protein can also be analysed e.g. using techniques such as ELISA, surface plasmon resonance or biolayer interferometry analysis. Assays may comprise comparing the level of interaction in the presence of the agent to the level of interaction in an appropriate control condition (e.g. the absence of the agent).

Agents capable of inhibiting a function of a LINC complex may be identified using assays comprising detecting the level of a correlate of LINC complex function.

A LINC complex inhibitor according to the present disclosure may be any agent/plurality of agents achieving the desired inhibitory activity. In some embodiments, a LINC complex inhibitor may be or comprise a peptide/polypeptide, small molecule, nucleic acid or biomolecule.

In some embodiments, a LINC complex inhibitor is capable of binding to a LINC complex, a LINC complex protein, or an interaction partner for a LINC complex protein.

LINC complex inhibitors may display specific binding to the relevant factor/complex (i.e. a LINC complex protein, or an interaction partner for a LINC complex protein). As used herein, "specific binding" refers to binding which is selective, and which can be discriminated from non-specific binding to non-target molecules. LINC complex inhibitors that specifically bind to a LINC complex protein or an interaction partner for a LINC complex protein preferably binds to the relevant factor with greater affinity, and/or with greater duration than other, non-target molecules; such LINC complex inhibitors may be described as being "specific for" the relevant factor.

In some embodiments, a LINC complex inhibitor is capable of inhibiting interaction between a LINC complex protein and an interaction partner for a LINC complex protein. In some embodiments, a LINC complex inhibitor is capable of inhibiting LINC complex function. In some embodiments a LINC complex inhibitor behaves as a competitive inhibitor of interaction between a LINC complex protein and an interaction partner for a LINC complex protein. The LINC complex inhibitor may occupy, or otherwise reduce access to, a region of a LINC complex protein required for binding to an interaction partner for a LINC complex protein, or may occupy, or otherwise reduce access to, a region of an interaction partner for a LINC complex protein required for binding to a LINC complex protein.

In some embodiments a LINC complex inhibitor mimics an interaction partner for a LINC complex protein.

In some embodiments, a LINC complex inhibitor inhibits interaction between a SUN domain and a KASH domain.

In some embodiments, a LINC complex inhibitor inhibits association between the C-terminal region of a KASH domain and the deep pocket on the surface of a SUN domain. In some embodiments a LINC complex inhibitor binds to a SUN domain and inhibits access of a KASH domain to the deep pocket of the SUN domain. In some embodiments a LINC complex inhibitor binds to a KASH domain and inhibits access of the KASH domain to a deep pocket on the surface of a SUN domain.

In some embodiments, a LINC complex inhibitor inhibits the formation of, or disrupts, disulphide bonds between a SUN domain and a KASH domain.

In some embodiments, a LINC complex inhibitor targets the C-terminal, proline-rich region of a KASH domain-containing protein.

In some embodiments, a LINC complex inhibitor inhibits oligomerisation of SUN domain-containing proteins. In some embodiments, a LINC complex inhibitor targets the stalk region of a SUN domain-containing protein.

In some embodiments, a LINC complex inhibitor inhibits protein-protein interaction between a SUN domain and a KASH domain. In some embodiments, a LINC complex inhibitor inhibits protein-protein interaction between: SUN1 and Nesprin-1, SUN2 and Nesprin-1, SUN1 and Nesprin-2, SUN1 and Nesprin-3, SUN2 and Nesprin-2 or SUN2 and Nesprin-3. In some embodiments a LINC complex inhibitor inhibits protein-protein interaction between SUN1 and Nesprin-1.

The ability of a candidate LINC complex inhibitor to inhibit interaction between a LINC complex protein and an interaction partner for a LINC complex protein can be evaluated e.g. by analysis of interaction in the presence of, or following incubation of one or both of the interaction partners with, the candidate LINC complex inhibitor. An example of a suitable assay to determine whether a given binding agent is capable of inhibiting interaction between a LINC complex protein and an interaction partner for a LINC complex protein is a competition ELISA.

In some embodiments a molecule which binds to a LINC complex protein or an interaction partner for a LINC complex protein inhibits the ability of a LINC complex protein to bind to an interaction partner for a LINC complex protein.

In some embodiments, a LINC complex inhibitor is capable of binding to a LINC complex protein or an interaction partner for a LINC complex protein, and inhibiting interaction between a LINC complex protein and an interaction partner for a LINC complex protein.

LINC complex inhibitors which are capable of binding to a LINC complex protein or an interaction partner for a LINC complex protein, and inhibiting interaction between a LINC complex protein and an interaction partner for a LINC complex protein can be identified using any suitable assay for detecting binding of a molecule to the relevant factor (i.e. the LINC complex protein, or the interaction partner for the LINC complex protein) and inhibition of interaction between a LINC complex protein and an interaction partner for a LINC complex protein. Such assays may comprise e.g. detecting the formation of a complex between the relevant factor and the candidate inhibitor molecule, and/or detecting the formation of a complex between the LINC complex protein and the interaction partner for the LINC complex protein.

In some embodiments, LINC complex inhibitors which are capable of binding to a LINC complex protein or an interaction partner for a LINC complex protein, and inhibiting interaction between a LINC complex protein and an interaction partner for a LINC complex protein may e.g. be peptide/polypeptides.

A LINC complex inhibitor may e.g. be based on an interaction partner for the relevant factor (i.e. the LINC complex protein, or the interaction partner for a LINC complex protein) to which the inhibitor binds.

As used herein, a peptide/polypeptide which is "based on" a reference protein comprises or consists of an amino acid sequence having high sequence identity (e.g. at least 80%, 85% 86%, 87%, 88%, 89%, 90%, 91%, 92%, 93%, 94%, 95%, 96%, 97%, 98%, 99%, or 100% sequence identity) to all or part of the amino acid of the reference protein.

For example, a LINC complex inhibitor which binds to a LINC complex protein may comprise/consist of a peptide/polypeptide fragment of an interaction partner for a LINC complex protein. Similarly, a LINC complex inhibitor which binds to an interaction partner for a LINC complex protein may comprise/consist of a peptide/polypeptide fragment of a LINC complex protein.

Such inhibitors preferably possess the ability to bind to the relevant factor, but lacks or displays a reduced level of one or more other properties of the protein on which they are based. For example, a LINC complex inhibitor may comprise the amino acid sequence(s) required for binding to the relevant factor, and may lack the amino acid sequence(s) required for one or more other properties of the protein on which it is based.

Such peptide/polypeptide LINC complex inhibitors may be referred to as 'decoy', 'dominant-negative' or 'mimetic' versions of the proteins on which they are based, and preferably display competitive inhibition of interaction between a LINC complex protein and an interaction partner for a LINC complex protein. In this way, such peptide/polypeptide LINC complex inhibitors inhibit the formation of LINC complexes and/or disrupt existing LINC complexes via displacement of endogenous interaction partners, forming non-functional complexes/complexes having a reduced level of function.

A dominant-negative version of SUN1 is described e.g. in Crisp et al. J Cell Biol. (2006) 172(1): 41-53. A dominant-negative SUN2 is described e.g. in Stewart-Hutchinson et al. Exp Cell Res. (2008) 314(8):1892-905.

Dominant-negative versions of KASH1 are described e.g. in Stewart-Hutchinson et al. Exp Cell Res. (2008) 314(8):1892-905, Grady et al., Proc. Natl. Acad. Sci. U.S.A. (2005) 102: 4359-4364 and Libotte et al., MBoC (2005) 16: 3411-3424. Dominant-negative versions of KASH2 are described e.g. in Stewart-Hutchinson et al. Exp Cell Res. (2008) 314(8):1892-905, Libotte et al., MBoC (2005) 16: 3411-3424, Zhen et al., J. Cell. Sci. (2002) 115: 3207-3222 and Kim et al. Sci Transl Med (2018) 10. A dominant-negative version of KASH3 is described e.g. in Stewart-Hutchinson et al. Exp Cell Res. (2008) 314(8):1892-905. A dominant-negative version of KASH4 is described e.g. in Roux et al. Proc. Natl. Acad. Sci. U.S.A. (2009) 106: 2194-2199. A dominant-negative version of KASH5 is described e.g. in Horn et al. J Cell Biol (2013) 202: 1023-1039.

In some embodiments, a peptide/polypeptide LINC complex inhibitor is based on a SUN domain-containing protein (e.g. a SUN domain containing-protein as described herein). In some embodiments the peptide/polypeptide LINC complex inhibitor is a decoy/dominant-negative version of a SUN domain-containing protein.

LINC complex inhibitors based on a SUN domain-containing protein may display binding to a KASH domain-containing protein (e.g. a KASH domain-containing protein as described herein), but lacks or displays a reduced level of one or more other properties of the SUN domain-containing protein on which it is based (e.g. binding to a SUN domain-containing protein, nucleoplasmic protein, lamin, and/or chromatin-binding protein).

LINC complex inhibitors based on a SUN domain-containing protein may display binding to a SUN domain-containing protein (e.g. a SUN domain-containing protein as described herein), but lacks or displays a reduced level of one or more other properties of the SUN domain-containing protein on which it is based (e.g. binding to a KASH domain-containing protein, nucleoplasmic protein, lamin, and/or chromatin-binding protein).

LINC complex inhibitors based on a SUN domain-containing protein preferably comprise a SUN domain. In some embodiments a LINC complex consists of, or consists essentially of, a SUN domain. The peptide/polypeptide may lack amino acid sequence(s) of a SUN domain-containing protein constituting protein domains other than a SUN domain. The peptide/polypeptide preferably lacks properties of an endogenous SUN domain-containing protein other than properties mediated by the SUN domain. The peptide/polypeptide may behave as a dominant-negative peptide/polypeptide, capable of inhibiting interaction between an endogenous SUN domain-containing protein and an endogenous interaction partner for a SUN domain-containing protein.

In some embodiments, a LINC complex inhibitor comprises, or consists of, the SUN domain of SUN1, SUN2, SUN3, SUNS, SPAG4 or SUCO, or a SUN domain having at least 70%, preferably one of 80%, 85%, 90%, 91%, 92%, 93%, 94%, 95%, 96%, 97%, 98%, 99% or 100% amino acid sequence identity to the SUN domain of SUN1, SUN2, SUN3, SUNS, SPAG4 or SUCO. In some embodiments, a LINC complex inhibitor comprises, or consists of, the amino acid sequence shown in SEQ ID NO:82, 83, 84, 85, 86 or 87, or an amino acid sequence having at least 70%, preferably one of 80%, 85%, 90%, 91%, 92%, 93%, 94%, 95%, 96%, 97%, 98%, 99% or 100% amino acid sequence identity to the amino acid sequence shown in SEQ ID NO:82, 83, 84, 85, 86 or 87.

In some embodiments a LINC complex inhibitor lacks the full amino acid sequence of SUN1, SUN2, SUN3, SUNS, SPAG4 or SUCO. In some embodiments a LINC complex inhibitor lacks the amino acid sequence according to SEQ ID NO:88, 89, 90, 91, 92 or 93.

In some embodiments a LINC complex inhibitor lacks the amino acid sequence of a SUN domain-containing protein required for binding to a nucleoplasmic protein, a lamin, and/or a chromatin-binding protein. In some embodiments a LINC complex inhibitor lacks the amino acid sequence according to SEQ ID NO:94, 95 or 96.

In some embodiments, a peptide/polypeptide LINC complex inhibitor is based on a KASH domain-containing protein (e.g. a KASH domain containing-protein as described herein). In some embodiments the peptide/polypeptide LINC complex inhibitor is a decoy/dominant-negative version of a KASH domain-containing protein.

LINC complex inhibitors based on a KASH domain-containing protein may display binding to a SUN domain-containing protein (e.g. a SUN domain-containing protein as described herein), but lacks or displays a reduced level of one or more other properties of the KASH domain-containing protein on which it is based (e.g. binding to a KASH domain-containing protein, cytoplasmic protein, cytoskeletal protein, microfilament, actin, microtubule, tubulin, microtubule motor, or intermediate filament protein).

LINC complex inhibitors based on a KASH domain-containing protein may display binding to a KASH domain-containing protein (e.g. a KASH domain-containing protein as described herein), but lacks or displays a reduced level of one or more other properties of the KASH domain-containing protein on which it is based (e.g. binding to a SUN domain-containing protein, cytoplasmic protein, cytoskeletal protein, microfilament, actin, microtubule, tubulin, microtubule motor, or intermediate filament protein).

LINC complex inhibitors based on a KASH domain-containing protein preferably comprise a KASH domain. In some embodiments a LINC complex consists of, or consists essentially of, a KASH domain. The peptide/polypeptide may lack amino acid sequence(s) of a KASH domain-containing protein constituting protein domains other than a KASH domain. The peptide/polypeptide preferably lacks properties of an endogenous KASH domain-containing protein other than properties mediated by the KASH domain. The peptide/polypeptide may behave as a dominant-negative peptide/polypeptide, capable of inhibiting interaction between an endogenous KASH domain-containing protein and an endogenous interaction partner for a KASH domain-containing protein.

In some embodiments, a LINC complex inhibitor comprises, or consists of, the KASH domain of Nesprin-1, Nesprin-2, Nesprin-3, Nesprin-4, KASH5, LRMP or a KASH domain having at least 70%, preferably one of 80%, 85%, 90%, 91%, 92%, 93%, 94%, 95%, 96%, 97%, 98%, 99% or 100% amino acid sequence identity to the KASH domain of Nesprin-1 , Nesprin-2, Nesprin-3, Nesprin-4, KASH5 or LRMP. In some embodiments, a LINC complex inhibitor comprises, or consists of, the amino acid sequence shown in SEQ ID NO:97, 98, 99, 100, 101 or 114, or an amino acid sequence having at least 70%, preferably one of 80%, 85%, 90%, 91%, 92%, 93%, 94%, 95%, 96%, 97%, 98%, 99% or 100% amino acid sequence identity to the amino acid sequence shown in SEQ ID NO:97, 98, 99, 100, 101 or 114.

In some embodiments a LINC complex inhibitor lacks the full amino acid sequence of Nesprin-1, Nesprin-2, Nesprin-3, Nesprin-4, KASH5 or LRMP. In some embodiments a LINC complex inhibitor lacks the amino acid sequence according to SEQ ID NO:102, 103, 104, 105, 106 or 113.

In some embodiments a LINC complex inhibitor lacks the amino acid sequence of a KASH domain-containing protein required for binding to a cytoplasmic protein, a cytoskeletal protein, a microfilament, actin, a microtubule, tubulin, a microtubule motor, or an intermediate filament protein. In some embodiments a LINC complex inhibitor lacks the amino acid sequence according to SEQ ID NO:107 or 108.

In some embodiments, peptide/polypeptide LlNC complex inhibitors capable of binding to a LINC complex/LINC complex protein/interaction partner for a LINC complex protein and inhibiting interaction between a LINC complex protein and an interaction partner for a LINC complex protein and/or LINC complex function include e.g. peptide aptamers, thioredoxins, monobodies, anticalin, Kunitz domains, avimers, knottins, fynomers, atrimers, DARPins, affibodies, nanobodies (i.e. single-domain antibodies (sdAbs)) affilins, armadillo repeat proteins (ArmRPs), OBodies and fibronectin - reviewed e.g. in Reverdatto et al., Curr Top Med Chem. 2015; 15(12): 1082-1101, which is hereby incorporated by reference in its entirety (see also e.g. Boersma et al., J Biol Chem (2011) 286:41273-85 and Emanuel et al., Mabs (2011) 3:38-48). Further peptide/polypeptide LINC complex inhibitors contemplated in connection with the present invention include antibodies (immunoglobulins) such as monoclonal antibodies, polyclonal antibodies, monospecific antibodies, multispecific antibodies (e.g., bispecific antibodies), and fragments and derivatives thereof (e.g. Fv, scFv, Fab, scFab, F(ab')₂, Fab₂, diabodies, triabodies, scFv-Fc, minibodies, single domain antibodies (e.g. VhH), etc.).

Such peptide/polypeptide LINC complex inhibitors can be identified by screening of libraries of the relevant peptides/polypeptides for LINC complex inhibition.

Peptide/polypeptide LlNC complex inhibitors may comprise further amino acids or sequences of amino acids. For example, the peptides/polypeptides may comprise amino acid sequence(s) to facilitate expression, folding, trafficking, processing, purification or detection. For example, the peptide/polypeptide may comprise a sequence encoding a His, (e.g. 6XHis), Myc, GST, MBP, FLAG, HA, E, or Biotin tag, optionally at the N- or C- terminus of the antigen-binding molecule/polypeptide. In some embodiments the peptide/polypeptide comprises a detectable moiety, e.g. a fluorescent, lunminescent, immuno-detectable, radio, chemical, nucleic acid or enzymatic label.

In some embodiments, peptides/polypeptides may comprise an N-terminal signal sequence, a signal peptidase cleavage site, and a C-terminal targeting peptide sequence.

The peptides/polypeptides may additionally comprise a signal peptide (also known as a leader sequence or signal sequence). Signal peptides normally consist of a sequence of 5-30 hydrophobic amino acids, which form a single alpha helix. Secreted proteins and proteins expressed at the cell surface often comprise signal peptides. The signal peptide may be present at the N-terminus of the peptide/polypeptide, and may be present in the newly synthesised peptide/polypeptide. Signal peptides are often removed by cleavage, and thus are not comprised in the mature peptide/polypeptide.

In some embodiments an N-terminal signal sequence is derived from a secretory protein or a Type I transmembrane protein. In some embodiments the secretory protein or Type I transmembrane protein is selected from: human serum albumin, proinsulin, transferrin receptor, EGF receptor, pre-pro-opiomelanocortin, carboxypeptidases, complement proteins, fibrinogen, cytokines, chemokines, , fibrinogen, pancreatic digestive enzymes (for example, proteases, amylases and lipases), endoplasmic reticulum lumenal proteins, for example protein disulphide isomerases and GRP94. In some embodiments the N-terminal signal sequence is derived from human serum albumin. In some embodiments the N-terminal signal sequence is not preceded at its N-terminus by any other tags.

In some embodiments the peptide/polypeptide comprises a signal peptidase cleavage site. In some embodiments the signal peptidase cleavage site is a signal peptidase cleavage site derived from or is one of: human serum albumin, proinsulin, transferrin receptor, EGF receptor, pre-pro-opiomelanocortin, pancreatic digestive enzymes (for example, proteases, amylases and lipases), endoplasmic reticulum lumenal proteins, such as protein disulphide isomerases and GRP94. In some embodiments the signal peptidase cleavage site is derived from human serum albumin.

In some embodiments the peptide/polypeptide comprises a sequence for targeted intracellular trafficking of the peptide/polypeptide to the nuclear envelope. In some embodiments the peptide/polypeptide comprises an endoplasmic-reticulum (ER) retention motif. Suitable endoplasmic-reticulum retention sequences are known in the art. In some embodiments the ER retention motif is a KDEL sequence. A KDEL sequence KDEL or a variant thereof that works to retain a protein in the endoplasmic-reticulum. KDEL variants may comprise or consist of the prosite motif [KRHQSA]-[DENQ]-E-L (described in Hulo et al., 2006, Nucleic acids research 34: D227-D230), or a variant described by in Raykhel et al., 2007 (J. Cell Biol. 179(6):1193-1204) who proposed an expanded prosite motif definition. Raykhel demonstrated endoplasmic retention with variants in which the 4 position (i.e. the K position) included F, WY as an alternative to KRHQSA, a range of 3 position (i.e. the D position) residues that extended far beyond DENQ, For M in the 1 position (the L position), and D in the 2 position (i.e. the E position). For example, the KDEL motif may be CDEL, KCEL or HVEL as proposed by Raykhel et al. Thus, in some aspects disclosed herein the endoplasmic-reticulum (ER) retention motif is KDEL or a variant thereof which exhibitis ER retention activity.

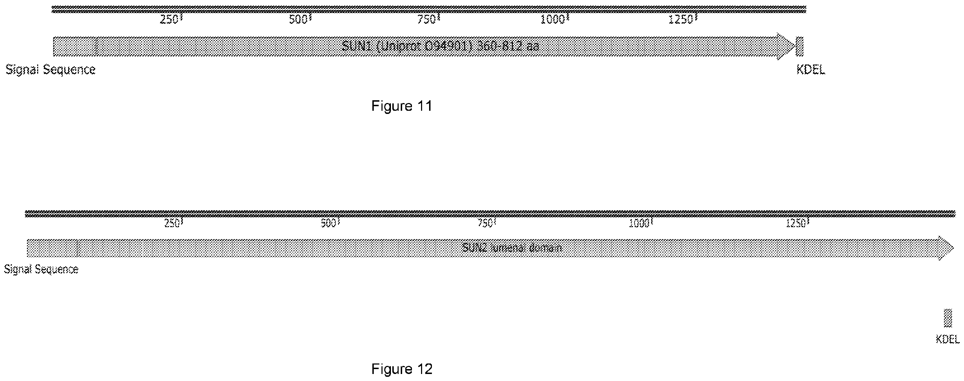

In some embodiments the peptide/polypeptide comprises a C-terminal targeting peptide sequence. In some embodiments the C-terminal targeting peptide sequence prevents secretion of the peptide/polypeptide. In some embodiments, the C-terminal targeting peptide sequence is a KDEL tetrapeptide Golgi retrieval sequence. Examples of such structures are shown in Figures 11 and 12.

In some embodiments the peptide/polypeptide comprises an epitope tag. In some embodiments the epitope tag is N-terminal, or located anywhere in the peptide/polypeptide downstream of (after) the C-terminal targeting peptide sequence [for example an endoplasmi-reticulum retention motif such as a KDEL sequence], or located anywhere in the peptide/polypeptide except upstream of (before) the N-terminal signal sequence. In some embodiments, the epitope tag is selected from: cellulose binding domain (CBD), chloramphenicol acetyl transferase (CAT), dihydrofolate reductase (DHFR), one or more FLAG tags, glutathione S-transferase (GST), green fluorescent protein (GFP), haemagglutinin A (HA), histidine (His), Herpes simplex virus (HSV), luciferase, maltose-binding protein (MBP), c-Myc, Protein A, Protein G, streptavidin, T7, thioredoxin, V5, vesicular stomatitis virus glycoprotein (VSV-G), and combinations thereof. In some embodiments the epitope tag is haemagglutinin A (HA).

In some embodiments, a peptide/polypeptide comprises a signal sequence (i.e. prior to cleavage to remove the signal sequence), a humanized Sun1DN sequence and a KDEL sequence (e.g. as encoded by SEQ ID NO:4). In some embodiments, a peptide/polypeptide comprises a signal sequence, a humanized Sun2DN sequence and a KDEL sequence (e.g. as encoded by SEQ ID NO:5).

For SUN domain constructs it is expected that the SUN domain (crystal structure solved by the Kutay and Schwartz labs [Sosa et al., Cell 149(5):1035-47 (2012)] and an additional upstream 20 amino acid residues corresponding to the alpha-3 region of coiled-coil-2, is sufficient to disrupt the SUN-KASH interaction as it is capable of binding to the KASH domain [Jahed et al., Biophysical Journal 114 (5): 1190-1203 (2018)]. This is instead of the entire lumenal domain (coiled-coil domain and SUN domain). The human SUN1 SUN domain nucleic acid sequence is set forth in SEQ ID NO:80. The presence of the signal sequence and a KDEL sequence may be important for targeting to the perinuclear space.

In some embodiments, a LINC complex inhibitor comprises, or consists of, the amino acid sequence shown in SEQ ID NO:115, or an amino acid sequence having at least 70%, preferably one of 80%, 85%, 90%, 91%, 92%, 93%, 94%, 95%, 96%, 97%, 98%, 99% or 100% amino acid sequence identity to the amino acid sequence shown in SEQ ID NO:115. In some embodiments, a LINC complex inhibitor comprises, or consists of, the amino acid sequence shown in SEQ ID NO:116, or an amino acid sequence having at least 70%, preferably one of 80%, 85%, 90%, 91%, 92%, 93%, 94%, 95%, 96%, 97%, 98%, 99% or 100% amino acid sequence identity to the amino acid sequence shown in SEQ ID NO:116.

In some embodiments peptides/polypeptides comprise either the lumenal domain of a SUN domain-containing protein, or the SUN domain of a SUN domain-containing protein.

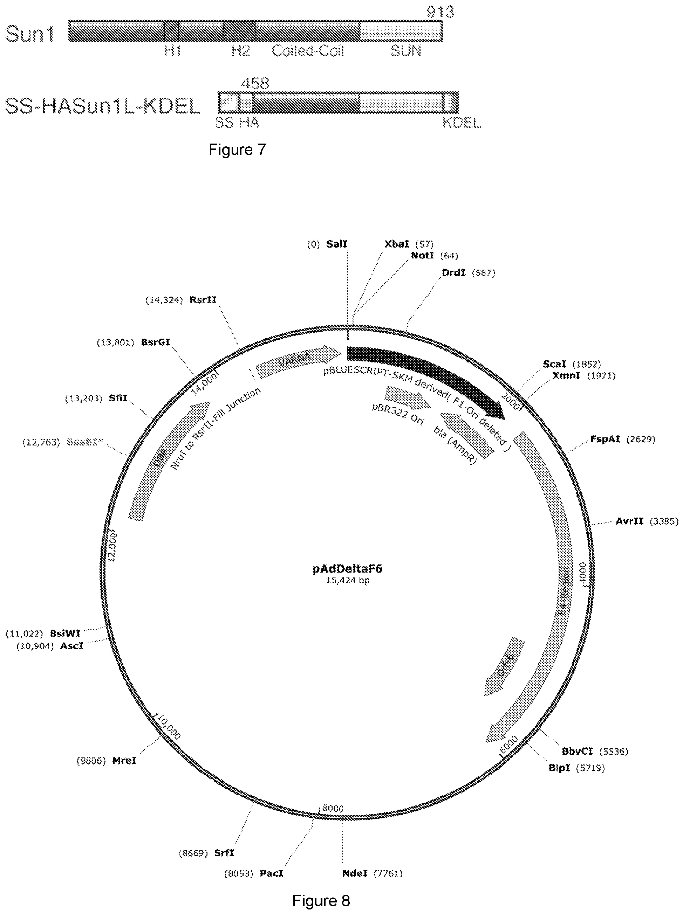

In some embodiments the lumenal domain of Sun1 comprises amino acids 458-913 of full-length mouse Sun1 (UniProt: Q9D666) or its human equivalent comprising the coiled coil domain and the SUN domain and lacking the transmembrane domain. A schematic of the structure of a dominant negative form of Sun1 is shown in Figure 7.

In some embodiments the peptides/polypeptides comprise a humanized Sun1DN sequence or a humanized Sun2DN sequence.

Rather than expressing components of a lumenal domain of a SUN domain-containing protein, a KASH domain may be expressed to disrupt a LINC complex by competing with endogenous Nesprins (which comprise a KASH domain) for binding to SUN1 and SUN2 domains.

Accordingly, in some embodiments the peptides/polypeptides comprise a KASH domain, and an N-terminal stabiliser polypeptide sequence.

In some embodiments the peptide/polypeptide comprises a KASH domain comprising a transmembrane domain and a SUN-interacting peptide. In some embodiments the peptide/polypeptide comprises a KASH domain that traverses the outer nuclear membrane, a SUN-interacting KASH peptide that extends into the perinuclear space at the C-terminus, and an N-terminal stabiliser polypeptide sequence in the cytoplasm.

It will be understood that KASH domain constructs with extensions after the last C-terminal amino acid of the naturally occurring KASH domain are not expected to work i.e. C-terminal tags, or even an additional carboxy-terminal single amino acid, will disrupt KASH interaction with SUN.

In some embodiments, the KASH domain is selected from the group consisting of KASH1 (derived from Nesprin-1 (SYNE1 gene)), KASH2 (derived from Nesprin-2 (SYNE2 gene)), KASH3 (derived from Nesprin-3 (SYNE3 gene)), KASH4 (derived from Nesprin-4 (SYNE4 gene)) and KASH5 (derived from KASH5/CCDC155 (KASH5 gene)).

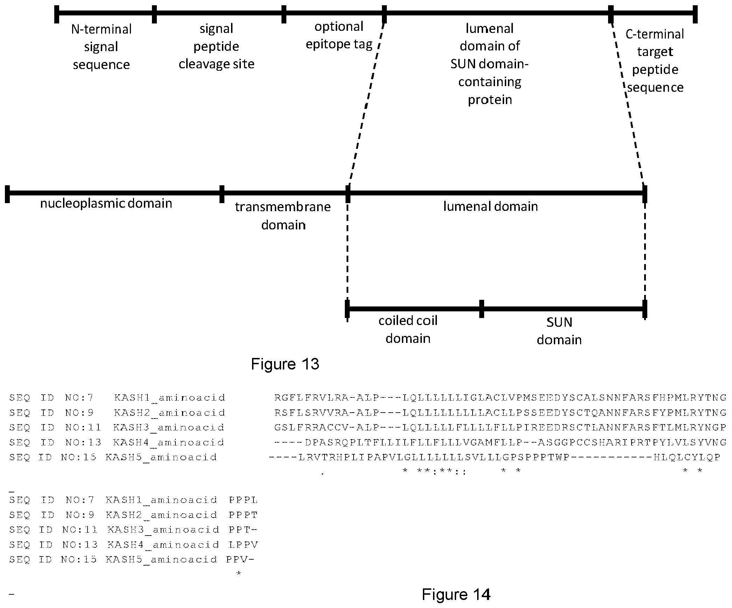

In some embodiments the KASH 1 domain comprises the human amino acid sequence set forth in SEQ ID NO:7; the KASH 2 domain comprises the human amino acid sequence set forth in SEQ ID NO:9; the KASH 3 domain comprises the human amino acid sequence set forth in SEQ ID NO:11; the KASH 4 domain comprises the human amino acid sequence set forth in SEQ ID NO:13; and the KASH 5 domain comprises the human amino acid sequence set forth in SEQ ID NO:15. An alignment of the five KASH amino acid sequences is shown in Figure 14.

In some embodiments the KASH domain nucleic acid sequence has at least 80%, at least 85%, at least 90%, at least 95% sequence identity or 100% sequence identity to the nucleic acid sequence of the KASH1 domain set forth in SEQ ID NO:6; the nucleic acid sequence of the KASH2 domain set forth in SEQ ID NO:8; the nucleic acid sequence of the KASH3 domain set forth in SEQ ID NO:10; the nucleic acid sequence of the KASH4 domain set forth in SEQ ID NO:12; or the nucleic acid sequence of the KASH5 domain set forth in SEQ ID NO:14.

In some embodiments, e.g. for the purposes of clinical use, the KASH domain is the human KASH1 domain of SYNE1 having at least 80%, at least 85%, at least 90%, at least 95% sequence identity or 100% sequence identity to the nucleic acid sequence of the human KASH1 domain set forth in SEQ ID NO:6.

In some embodiments, the KASH domain does not comprise any extensions after the last C-terminal amino acid compared to a naturally occurring KASH domain.

In some embodiments an N-terminal stabiliser polypeptide sequence is selected from the group consisting of green fluorescent protein (GFP), cellulose binding domain (CBD), chloramphenicol acetyl transferase (CAT), dihydrofolate reductase (DHFR), glutathione S-transferase (GST), luciferase, maltose-binding protein (MBP), Protein A, Protein G, streptavidin, thioredoxin, DHFR, including multiples and combinations thereof. In some embodiments, the N-terminal stabiliser polypeptide sequence forms a discretely folded domain.

In some embodiments, the peptide/polypeptide comprises a KASH domain, and an N-terminal stabiliser polypeptide sequence, wherein the KASH domain is selected from the group comprising KASH1, KASH2, KASH3, KASH4 and KASH5. In some embodiments, the N-terminal stabiliser polypeptide sequence is green fluorescent protein (GFP).

In some embodiments a LINC complex inhibitor is a small molecule inhibitor of a LINC complex. As used herein, a "small molecule" refers to a low molecular weight (< 1000 daltons, typically between ~300-700 daltons) organic compound.

A small molecule LINC complex inhibitor may bind to a LINC complex, a LINC complex protein, or an interaction partner for a LINC complex protein. A small molecule inhibitor LINC complex inhibitor may inhibit interaction between a LINC complex protein and an interaction partner for a LINC complex protein. A small molecule inhibitor LINC complex inhibitor may bind to a LINC complex and inhibit LINC complex function.

Suitable small molecule LINC complex inhibitors may be identified e.g. by screening of small molecule libraries, e.g. as described in Example 7 herein.

In some embodiments a LINC complex inhibitor is, or comprises, a nucleic acid.

The nucleic acid may bind to a LINC complex, a LINC complex protein, or an interaction partner for a LINC complex protein. The nucleic acid may inhibit interaction between a LINC complex protein and an interaction partner for a LINC complex protein. The nucleic acid may bind to a LINC complex and inhibit LINC complex function.

Nucleic acid aptamers are reviewed e.g. in Zhou and Rossi Nat Rev Drug Discov. 2017 16(3):181-202. They may be identified and/or produced by the method of Systematic Evolution of Ligands by EXponential enrichment (SELEX), or by developing SOMAmers (slow off-rate modified aptamers) (Gold L et al. (2010) PLoS ONE 5(12):e15004). Aptamers and SELEX are described in Tuerk and Gold, Science (1990) 249(4968):505-10, and in WO 91/19813.

Nucleic acid aptamers may comprise DNA and/or RNA, and may be single-stranded or double-stranded. They may comprise chemically modified nucleic acids, for example in which the sugar and/or phosphate and/or base is chemically modified. Such modifications may improve the stability of the aptamer or make the aptamer more resistant to degradation and may include modification at the 2' position of ribose.

Nucleic acid aptamers may be chemically synthesised, e.g. on a solid support. Solid phase synthesis may use phosphoramidite chemistry. Briefly, a solid supported nucleotide is detritylated, then coupled with a suitably activated nucleoside phosphoramidite to form a phosphite triester linkage. Capping may then occur, followed by oxidation of the phosphite triester with an oxidant, typically iodine. The cycle may then be repeated to assemble the aptamer (e.g., see Sinha, N. D.; Biernat, J.; McManus, J.; Köster, H. Nucleic Acids Res. 1984, 12, 4539; and Beaucage, S. L.; Lyer, R. P. (1992). Tetrahedron 48 (12): 2223).

In some embodiments, a LINC complex inhibitor capable of reducing expression (e.g. gene and/or protein expression) of a LINC complex protein. In some embodiments the LINC complex inhibitor reduces or prevents the expression of an endogenous LINC complex protein.

Inhibition of expression of a LINC complex protein will result in a decrease in the quantity of a LINC complex protein and/or the quantity of LINC complexes comprising the constituent protein in a cell/tissue/organ/organ system/subject. For example, in a given cell the inhibition of expression of a LINC complex protein will result in a decrease in the level of a LINC complex protein and/or of LINC complexes comprising the constituent protein relative to an untreated cell.

Inhibition may be partial. Preferred degrees of inhibition are at least 50%, more preferably one of at least 60%, 70%, 80%, 85% or 90%. A level of inhibition between 90% and 100% is considered a `silencing' of expression or function. Gene and protein expression may be determined as described herein or by methods in the art that are well known to a skilled person.

In some embodiments a LINC complex inhibitor reduces or prevents the expression of SUN1, SUN2, SUN3, SUNS, SPAG4 or SUCO. In some embodiments a LINC complex inhibitor reduces or prevents the expression of SUN1 or SUN2. In some embodiments a LINC complex inhibitor reduces or prevents the expression of a polypeptide according to SEQ ID NO:82, 83, 84, 85, 86 or 87. In some embodiments a LINC complex inhibitor reduces or prevents the expression of a polypeptide according to SEQ ID NO:82 or 83.

In some embodiments a LINC complex inhibitor reduces or prevents the expression of Nesprin-1, Nesprin-2, Nesprin-3, Nesprin-4, KASH5 or LRMP. In some embodiments a LINC complex inhibitor reduces or prevents the expression of a polypeptide according to SEQ ID NO: 102, 103, 104, 105, 106 or 113. In some embodiments a LINC complex inhibitor reduces or prevents the expression of Nesprin-1, Nesprin-2 or Nesprin-3 In some embodiments a LINC complex inhibitor reduces or prevents the expression of a polypeptide according to SEQ ID NO: 102, 103 or 104.

In some embodiments, a LINC complex inhibitor may target a particular domain/region of a LINC complex protein. In some embodiments the LINC complex inhibitor reduces or prevents the expression of an isoform of a LINC complex protein comprising one or more domains/regions of interest.

For example, a domain or region of interest may be or comprise: a SUN domain, a KASH domain and/or a domain required for interaction with an interaction partner for the LINC complex protein. For example, a domain or region of interest may be required for interaction with a KASH domain-containing protein, a SUN domain-containing protein, a nucleoplasmic protein, a lamin, a chromatin-binding protein, a cytoplasmic protein, a cytoskeletal protein, a microfilament, actin, a microtubule, tubulin, a microtubule motor and/or an intermediate filament protein.

In some embodiments, a LINC complex inhibitor may alter splicing of pre-mRNA encoding the LINC complex protein to increase the proportion of mature mRNA encoding isoform(s) lacking the relevant domains/regions, and/or to decrease the proportion of mature mRNA encoding isoform(s) comprising the relevant domains/regions.

In some embodiments, a LINC complex inhibitor may modify nucleic acid encoding the LINC complex protein to increase expression of isoform(s) lacking the relevant domains/regions, and/or may modify the nucleic acid encoding the LINC complex protein to decrease expression of isoform(s) comprising the relevant domains/regions of the protein.

In some embodiments the LINC complex inhibitor is an inhibitory nucleic acid. In some embodiments, the inhibitory nucleic acid is an antisense nucleic acid. In some embodiments the inhibitory nucleic acid is an antisense oligonucleotide (ASO). Antisense oligonucleotides may be single-stranded, and may bind by complementary sequence binding to a target oligonucleotide, e.g. mRNA.

ASOs may be designed to inhibit/prevent expression of a LINC complex protein or particular isoforms thereof.

Oligonucleotides designed to inhibit/prevent expression of a LINC complex protein, or particular isoforms thereof, may have substantial sequence identity to a portion of nucleic acid encoding the LINC complex protein/the relevant isoform, or the complementary sequence thereto.

In some embodiments, the inhibitory nucleic acid reduces expression of a LINC complex protein by RNA interference (RNAi). RNAi involves inhibition of gene expression and translation by targeted neutralisation of mRNA molecules. In some embodiments, the inhibitory nucleic acid is a small interfering RNA (siRNA), a short hairpin RNA (shRNA), or a micro RNA (miRNA).

A role for the RNAi machinery and small RNAs in targeting of heterochromatin complexes and epigenetic gene silencing at specific chromosomal loci has been demonstrated. Double-stranded RNA (dsRNA)-dependent post transcriptional silencing, also known as RNA interference (RNAi), is a phenomenon in which dsRNA complexes can target specific genes of homology for silencing in a short period of time. It acts as a signal to promote degradation of mRNA with sequence identity. A 20-nt siRNA is generally long enough to induce gene-specific silencing, but short enough to evade host response. The decrease in expression of targeted gene products can be extensive with 90% silencing induced by a few molecules of siRNA. RNAi based therapeutics have been progressed into Phase I, II and III clinical trials for a number of indications (Nature 2009 Jan 22; 457(7228):426-433).

In the art, such RNA sequences are termed "short or small interfering RNAs" (siRNAs) or "microRNAs" (miRNAs) depending on their origin. Both types of sequence may be used to down-regulate gene expression by binding to complementary RNAs and either triggering mRNA elimination (RNAi) or arresting mRNA translation into protein. siRNA are derived by processing of long double stranded RNAs and when found in nature are typically of exogenous origin. Micro-interfering RNAs (miRNA) are endogenously encoded small non-coding RNAs, derived by processing of short hairpins.

Both siRNA and miRNA can inhibit the translation of mRNAs bearing partially complimentary target sequences without RNA cleavage and degrade mRNAs bearing fully complementary sequences.

siRNAs are typically double stranded and, in order to optimise the effectiveness of RNA mediated down-regulation of the function of a target gene, it is preferred that the length of the siRNA molecule is chosen to ensure correct recognition of the siRNA by the RISC complex that mediates the recognition by the siRNA of the mRNA target and so that the siRNA is short enough to reduce a host response.

miRNAs are typically single stranded and have regions that are partially complementary enabling the ligands to form a hairpin. miRNAs are RNA genes which are transcribed from DNA, but are not translated into protein. A DNA sequence that codes for a miRNA gene is longer than the miRNA. This DNA sequence includes the miRNA sequence and an approximate reverse complement. When this DNA sequence is transcribed into a single-stranded RNA molecule, the miRNA sequence and its reverse-complement base pair to form a partially double stranded RNA segment. The design of microRNA sequences is discussed e.g. in John et al, PLoS Biology, 11(2), 1862-1879, 2004.

Typically, the oligonucleotides intended to mimic the effects of siRNA or miRNA have between 10 and 40 ribonucleotides (or synthetic analogues thereof), more preferably between 17 and 30 ribonucleotides, more preferably between 19 and 25 ribonucleotides and most preferably between 21 and 23 ribonucleotides. In some embodiments of the invention employing double-stranded siRNA, the molecule may have symmetric 3' overhangs, e.g. of one or two (ribo)nucleotides, typically a UU of dTdT 3' overhang. Based on the disclosure provided herein, the skilled person can readily design suitable siRNA and miRNA sequences, for example using resources such the Ambion siRNA finder. siRNA and miRNA sequences can be synthetically produced and added exogenously to cause gene downregulation or produced using expression systems (e.g. vectors). In some embodiments the siRNA is synthesized synthetically.

siRNA-mediated knockdown of LINC complex proteins and siRNAs for achieving the same are described e.g. in Ostlund et al., Journal of Cell Science (2009) 122:4099-4108, Hatch and Hetzer, J Cell Biol. (2016) 215(1):27-36, Matsumoto et al., Nucleus. (2016) 7(1):68-83, Uzer et al., Stem Cells. (2015) 33(6):2063-76, Thakar et al., Mol Biol Cell. (2017) 28(1): 182-191, Espigat-Georger et al., J Cell Sci. (2016) 129(22):4227-4237, Yang et al., Int J Mol Med. (2013) 32(4):805-12, Rajgor et al., PLoS One. 2012;7(7):e40098, Zhang et al., Exp Cell Res. (2016) 345(2):168-179, Warren et al. J Biol Chem. (2010) 285(2):1311-20 and King et al., Cytoskeleton (Hoboken) (2014) 71(7):423-34, which are hereby incorporated by reference in their entirety.

Longer double stranded RNAs may be processed in the cell to produce siRNAs (see for example Myers (2003) Nature Biotechnology 21:324-328). The longer dsRNA molecule may have symmetric 3' or 5' overhangs, e.g. of one or two (ribo)nucleotides, or may have blunt ends. The longer dsRNA molecules may be 25 nucleotides or longer. Preferably, the longer dsRNA molecules are between 25 and 30 nucleotides long. More preferably, the longer dsRNA molecules are between 25 and 27 nucleotides long. Most preferably, the longer dsRNA molecules are 27 nucleotides in length. dsRNAs 30 nucleotides or more in length may be expressed using the vector pDECAP (Shinagawa et al., Genes and Dev., 17, 1340-5, 2003).

Another alternative is the expression of a short hairpin RNA molecule (shRNA) in the cell. shRNAs are more stable than synthetic siRNAs. A shRNA consists of short inverted repeats separated by a small loop sequence. One inverted repeat is complimentary to the gene target. In the cell the shRNA is processed by DICER into a siRNA which degrades the target gene mRNA and suppresses expression. In some embodiments the shRNA is produced within a cell by transcription from a vector. shRNAs may be produced within a cell by transfecting the cell with a vector encoding the shRNA sequence under control of a RNA polymerase III promoter such as the human H1 or 7SK promoter or a RNA polymerase II promoter. Alternatively, the shRNA may be synthesised exogenously (in vitro) by transcription from a vector. The shRNA may then be introduced directly into the cell. Preferably, the shRNA molecule comprises a partial sequence of a gene encoding a LINC complex protein. Preferably, the shRNA sequence is between 40 and 100 bases in length, more preferably between 40 and 70 bases in length. The stem of the hairpin is preferably between 19 and 30 base pairs in length. The stem may contain G-U pairings to stabilise the hairpin structure.

shRNA-mediated knockdown of LINC complex proteins and shRNAs for achieving the same are described e.g. in Kelkar et al., Nucleus. (2015) 6(6): 479-489, Mroß et al., Nucleus. (2018) 9(1): 503-515, Xing et al., International Journal of Cell Biology (2017) Article ID: 8607532, Arsenovic et al., Biophys J. (2016) 110(1):34-43 and Li et al., Scientific Reports (2017) 7: Article number: 9157, which are hereby incorporated by reference in their entirety.

In some embodiments, the inhibitory nucleic acid is a splice-switching oligonucleotide (SSO). Splice switching oligonucleotides are reviewed e.g. in Haves and Hastings, Nucleic Acids Res. (2016) 44(14): 6549-6563, which is hereby incorporated by reference in its entirety. SSOs disrupt the normal splicing of target RNA transcripts by blocking the RNA-RNA base-pairing and/or protein-RNA binding interactions that occur between components of the splicing machinery and pre-mRNA. SSOs may be employed to alter the number/proportion of mature mRNA transcripts encoding a LINC complex protein or particular isoform(s) thereof. SSOs may be designed to target a specific region of the target transcript, e.g. to effect skipping of exon(s) of interest, e.g. exons encoding domains/regions of interest.

SSOs generally comprise alterations to oligonucleotide sugar-phosphate backbones to prevent RNAse H degradation, and may comprise include e.g. phosphorodiamidate morpholino (PMOs), peptide nucleic acid (PNA), locked nucleic acid (LNA), and/or 2'O-methyl (2'OMe) and 2'-O-methoxyethyl (MOE) ribose modifications.

Inhibitory nucleic acids may be made recombinantly by transcription of a nucleic acid sequence, e.g. contained within vector. Transcription may be performed in cell-free transcription reactions, or in a cell comprising nucleic acid encoding the inhibitory nucleic acid. In some embodiments inhibitory nucleic acids are produced within a cell, e.g. by transcription from a vector. Vectors encoding such molecules may be introduced into cells in any of the ways known in the art. Optionally, expression of the nucleic acid can be regulated using a cell/tissue (e.g. heart, muscle, etc.) specific promoter.

Inhibitory nucleic acids may also be synthesized using standard solid or solution phase synthesis techniques which are known in the art.

In some embodiments, the LINC complex inhibitor is a molecule/plurality of molecules capable of modifying nucleic acid encoding a LINC complex protein to reduce/prevent expression of a LINC complex protein or particular isoform(s) thereof.

Modifying nucleic acid encoding a LINC complex protein may comprise modifying a gene encoding a LINC complex protein. In some embodiments, modifying nucleic acid encoding a LINC complex protein comprises introducing an insertion, substitution or deletion into a nucleic acid sequence encoding the LINC complex protein.

In some embodiments modifying nucleic acid encoding a LINC complex protein comprises modifying the nucleic acid to introduce a premature stop codon in the sequence transcribed from the nucleic acid. In some embodiments modifying nucleic acid encoding a LINC complex protein comprises modifying the nucleic acid to encode a truncated and/or non-functional version of the LINC complex protein. In some embodiments modifying nucleic acid encoding a LINC complex protein comprises modifying the nucleic acid to encode a version of the LINC complex protein which is misfolded and/or degraded.

The modification may be of nucleic acid comprised in a cell, e.g. endogenous nucleic acid encoding a LINC complex protein. The modification causes the cell to have a reduced level of gene and/or protein expression of a LINC complex protein or particular isoform(s) thereof as compared to an equivalent unmodified cell.

In some embodiments, modification may be to a region of the nucleic acid encoding a LINC complex protein involved in (e.g. required for) LINC complex formation. For example, in some embodiments modification may target a region of a gene encoding a SUN domain-containing protein encoding a encoding a SUN domain. In some embodiments modification may target a region of a gene encoding a KASH domain-containing protein encoding a encoding a KASH domain.

In some embodiments the modification is performed *in vitro* or ex *vivo.* In some embodiments the modification is performed *in vivo.*

Methods for modifying nucleic acids encoding proteins of interest and agents for achieving the same are well known in the art, and include e.g. including modification of the target nucleic acid by homologous recombination, and target nucleic acid editing using site-specific nucleases (SSNs). For example, the inventors demonstrate CRISPR/Cas9-mediated disruption of Sun1 and Nesprin-1 in the experimental examples herein.

Suitable methods may employ targeting by homologous recombination, which is reviewed, for example, in Mortensen Curr Protoc Neurosci. (2007) Chapter 4:Unit 4.29 and Vasquez et al., PNAS 2001, 98(15): 8403-8410 both of which are hereby incorporated by reference in their entirety. Targeting by homologous recombination involves the exchange of nucleic acid sequence through crossover events guided by homologous sequences.

In some embodiments the methods employ target nucleic acid editing using SSNs. Gene editing using SSNs is reviewed e.g. in Eid and Mahfouz, Exp Mol Med. 2016 Oct; 48(10): e265, which is hereby incorporated by reference in its entirety. Enzymes capable of creating site-specific double strand breaks (DSBs) can be engineered to introduce DSBs to target nucleic acid sequence(s) of interest. DSBs may be repaired by either error-prone non-homologous end-joining (NHEJ), in which the two ends of the break are rejoined, often with insertion or deletion of nucleotides. Alternatively DSBs may be repaired by highly homology-directed repair (HDR), in which a DNA template with ends homologous to the break site is supplied and introduced at the site of the DSB.

SSNs capable of being engineered to generate target nucleic acid sequence-specific DSBs include zinc-finger nucleases (ZFNs), transcription activator-like effector nucleases (TALENs) and clustered regularly interspaced palindromic repeats/CRISPR-associated-9 (CRISPR/Cas9) systems.

ZFN systems are reviewed e.g. in Umov et al., Nat Rev Genet. (2010) 11(9):636-46, which is hereby incorporated by reference in its entirety. ZFNs comprise a programmable Zinc Finger DNA-binding domain and a DNA-cleaving domain (e.g. a *Fokl* endonuclease domain). The DNA-binding domain may be identified by screening a Zinc Finger array capable of binding to the target nucleic acid sequence.

TALEN systems are reviewed e.g. in Mahfouz et al., Plant Biotechnol J. (2014) 12(8):1006-14, which is hereby incorporated by reference in its entirety. TALENs comprise a programmable DNA-binding TALE domain and a DNA-cleaving domain (e.g. a *Fokl* endonuclease domain). TALEs comprise repeat domains consisting of repeats of 33-39 amino acids, which are identical except for two residues at positions 12 and 13 of each repeat which are repeat variable di-residues (RVDs). Each RVD determines binding of the repeat to a nucleotide in the target DNA sequence according to the following relationship: "HD" binds to C, "NI" binds to A, "NG" binds to T and "NN" or "NK" binds to G (Moscou and Bogdanove, Science (2009) 326(5959):1501.).

CRISPR/Cas9 and related systems e.g. CRISPR/Cpf1, CRISPR/C2c1, CRISPR/C2c2 and CRISPR/C2c3 are reviewed e.g. in Nakade et al., Bioengineered (2017) 8(3):265-273, which is hereby incorporated by reference in its entirety. These systems comprise an endonuclease (e.g. Cas9, Cpf1 etc.) and the single-guide RNA (sgRNA) molecule. The sgRNA can be engineered to target endonuclease activity to nucleic acid sequences of interest.

In some embodiments, LINC complex inhibition employs a site-specific nuclease (SSN) system targeting a LINC complex protein. Accordingly in some embodiments the LINC complex inhibitor comprises or consists of SSN system targeting a LINC complex protein. In some embodiments LINC complex inhibition employs nucleic acid(s) encoding a SSN system targeting a LINC complex protein.

In some embodiments, the SSN system targets a region of the nucleic acid encoding a LINC complex protein involved in (e.g. required for) LINC complex formation. For example, in some embodiments the SSN system may disrupt expression of an exon of a gene encoding a SUN domain-containing protein encoding a SUN domain. In some embodiments the SSN system may disrupt expression of an exon of a gene encoding a KASH domain-containing protein encoding a KASH domain.

In particular embodiments, the SSN system may introduce an insertion/deletion in the nucleic acid sequence of a gene encoding a SUN domain-containing protein upstream of the sequence encoding the conserved tyrosine residue.

In some embodiments the SSN system may introduce an insertion/deletion in the nucleic acid sequence of a gene encoding a SUN domain-containing protein upstream of the sequence encoding the tyrosine at position 154 of SEQ ID NO:82. In some embodiments the SSN system may introduce an insertion/deletion in the nucleic acid sequence of a gene encoding a SUN domain-containing protein upstream of the sequence encoding the tyrosine at position 153 of SEQ ID NO:83. In some embodiments the SSN system may introduce an insertion/deletion in the nucleic acid sequence of a gene encoding a SUN domain-containing protein upstream of the sequence encoding the tyrosine at position 153 of SEQ ID NO:84. In some embodiments the SSN system may introduce an insertion/deletion in the nucleic acid sequence of a gene encoding a SUN domain-containing protein upstream of the sequence encoding the tyrosine at position 151 of SEQ ID NO:85. In some embodiments the SSN system may introduce an insertion/deletion in the nucleic acid sequence of a gene encoding a SUN domain-containing protein upstream of the sequence encoding the tyrosine at position 152 of SEQ ID NO:86.

In particular embodiments, the SSN system may introduce an insertion/deletion in the nucleic acid sequence of a gene encoding a SUN domain-containing protein upstream of the sequence encoding the conserved tyrosine residue.

In some embodiments the SSN system may introduce an insertion/deletion in the nucleic acid sequence of a gene encoding a KASH domain-containing protein upstream of the sequence encoding the proline-rich region at the C-terminus.

In some embodiments the SSN system may introduce an insertion/deletion in the nucleic acid sequence of a gene encoding a KASH domain-containing protein upstream of the sequence encoding the proline at position 57 of SEQ ID NO:97. In some embodiments the SSN system may introduce an insertion/deletion in the nucleic acid sequence of a gene encoding a KASH domain-containing protein upstream of the sequence encoding the proline at position 57 of SEQ ID NO:98. In some embodiments the SSN system may introduce an insertion/deletion in the nucleic acid sequence of a gene encoding a KASH domain-containing protein upstream of the sequence encoding the proline at position 56 of SEQ ID NO:99. In some embodiments the SSN system may introduce an insertion/deletion in the nucleic acid sequence of a gene encoding a KASH domain-containing protein upstream of the sequence encoding the proline at position 56 of SEQ ID NO:100.

In some embodiments the SSN system is a ZFN system, a TALEN system, CRISPR/Cas9 system, a CRISPR/Cpf1 system, a CRISPR/C2c1 system, a CRISPR/C2c2 system or a CRISPR/C2c3 system.

In some embodiments the SSN system is a CRISPR/Cas9 system. In such embodiments, the LINC complex inhibition may employ nucleic acid(s) encoding a CRISPR RNA (crRNA) targeting nucleic acid encoding a LINC complex protein, and a trans-activating crRNA (tracrRNA) for processing the crRNA to its mature form. CRISPR/Cas9 systems for targeted disruption of LINC complex proteins SUN1 and SUN2 are described e.g. in Schaller et al., J Virol. (2017) 91(19): pii: e00463-17, which is hereby incorporated by reference in its entirety.

Rather than expressing components of a lumenal domain of a SUN domain-containing protein or a KASH domain to disrupt a LINC complex by competing for binding with endogenous Nesprins (which comprise a KASH domain) or Sun1 and Sun2 (which comprise a SUN domain), another approach for disrupting the LINC complex is to modify the endogenous SUN domain or KASH domain so that it fails to bind to, or has reduced binding capacity for, its cognate LINC complex binding partner.

As both the SUN domain and the KASH domain are located at the C-termini of their respective proteins, one way of producing a modified SUN or KASH domain is to use a CRISPR/Cas system to modify the genes encoding SUN or KASH domain proteins to generate a premature stop codon at the 3' end of the respective protein sequences following CRISPR-induced non-homologous end joining. This would result in a truncated protein with its C-terminal SUN or KASH domain mutated. The truncated protein would be expressed and membrane-localized, but unable to interact with its cognate LINC complex partners.

Accordingly, in some embodiments a LINC complex inhibitor is a CRISPR-Cas or other synthetic nuclease system capable of modifying nucleic acid that encodes the SUN domain or KASH domain of endogenous Sun or Nesprin protein, respectively.

In some embodiments the CRISPR-Cas system modifies the endogenous SUN domain or KASH domain of Sun1 or Nesprin-1 protein, respectively, to disrupt a LINC complex. The respective nucleic acids are Sun1 and Syne1.

In some embodiments, the CRISPR-Cas system comprises a gRNA nucleic acid sequence comprising 5'-GCACAATAGCCTCGGATGTCG-3' (SEQ ID NO:66), capable of modifying the SUN domain of mouse Sun1.

In some embodiments, the CRISPR-Cas system comprises a gRNA nucleic acid targeting the human SUN1 domain set forth in SEQ ID NO:80. In some embodiments the gRNA nucleic acid sequence targets the end of exon 20 comprising a nucleic acid sequence set forth in SEQ ID NO:81. In some embodiments the gRNA nucleic acid sequence targets a SUN1 nucleic acid sequence selected from the group comprising SEQ ID NO:55; SEQ ID NO:56; SEQ ID NO:57; SEQ ID NO:58; SEQ ID NO:59; SEQ ID NO:60; SEQ ID NO:61; SEQ ID NO:62; SEQ ID NO:63; SEQ ID NO:64 and SEQ ID NO:65 set forth in Table 3.

In some embodiments, the CRISPR-Cas system comprises a gRNA nucleic acid sequence comprising 5'-CCGTTGGTATATCTGAGCAT-3' (SEQ ID NO:34), capable of modifying the KASH domain of mouse Syne-1.

In some embodiments, the CRISPR-Cas system comprises a gRNA nucleic acid sequence targeting the human KASH domain set forth in SEQ ID NO:6. In some embodiments the gRNA nucleic acid sequence comprises a nucleic sequence selected from the group comprising SEQ ID NO:44; SEQ ID NO:45; SEQ ID NO:46; SEQ ID NO:47; SEQ ID NO:48; SEQ ID NO:49; SEQ ID NO:50; SEQ ID NO:51; SEQ ID NO:52; SEQ ID NO:53 and SEQ ID NO:54 set forth in Table 3. In some embodiments, the CRISPR-Cas system is a CRISPR-Cas9 system or variant thereof.

### Administration/delivery of LINC complex inhibitors

Administration of a LINC complex inhibitor to a subject in accordance with the present invention is preferably in a "therapeutically effective" or "prophylactically effective" amount, this being sufficient to show therapeutic/prophylactic benefit to the subject.

The actual amount administered, and rate and time-course of administration, will depend on the nature and severity of the disease to be treated/prevented, and the nature of the LINC complex inhibitor. Prescription of treatment, e.g. decisions on dosage etc., is within the responsibility of general practitioners and other medical doctors, and typically takes account of the disease/condition to be treated, the condition of the individual subject, the site of delivery, the method of administration and other factors known to practitioners. Examples of the techniques and protocols mentioned above can be found in Remington's Pharmaceutical Sciences, 20th Edition, 2000, pub. Lippincott, Williams & Wilkins.

In therapeutic applications, LINC complex inhibitors are preferably formulated as a medicament or pharmaceutical together with one or more other pharmaceutically acceptable ingredients well known to those skilled in the art, including, but not limited to, pharmaceutically acceptable carriers, adjuvants, excipients, diluents, fillers, buffers, preservatives, anti-oxidants, lubricants, stabilisers, solubilisers, surfactants (e.g., wetting agents), masking agents, colouring agents, flavouring agents, and sweetening agents.

The term "pharmaceutically acceptable" as used herein pertains to compounds, ingredients, materials, compositions, dosage forms, etc., which are, within the scope of sound medical judgment, suitable for use in contact with the tissues of the subject in question (e.g., human) without excessive toxicity, irritation, allergic response, or other problem or complication, commensurate with a reasonable benefit/risk ratio. Each carrier, adjuvant, excipient, etc. must also be "acceptable" in the sense of being compatible with the other ingredients of the formulation.

Suitable carriers, adjuvants, excipients, etc. can be found in standard pharmaceutical texts, for example, Remington's Pharmaceutical Sciences, 20th Edition, 2000, pub. Lippincott, Williams & Wilkins; and Handbook of Pharmaceutical Excipients, 2nd edition, 1994.

The formulations may be prepared by any methods well known in the art of pharmacy. Such methods include the step of bringing into association the active compound with a carrier which constitutes one or more accessory ingredients. In general, the formulations are prepared by uniformly and intimately bringing into association the active compound with carriers (e.g., liquid carriers, finely divided solid carrier, etc.), and then shaping the product, if necessary.

The formulations may be prepared for topical, parenteral, systemic, intravenous, intra-arterial, intramuscular, intrathecal, intraocular, intra-conjunctival, subcutaneous, oral or transdermal routes of administration which may include injection. Injectable formulations may comprise the selected agent in a sterile or isotonic medium. The formulation and mode of administration may be selected according to the agent to be administered, and disease to be treated/prevented.

Administration of a LINC complex inhibitor preferably results in modification of a cell or cells to comprise a LINC complex inhibitor as described herein.

LINC complex inhibitors may be formulated to facilitate delivery to and/or uptake by a cell/tissue. LINC complex inhibitors may be linked to a moiety in order to facilitate delivery to and/or uptake by a cell/tissue. Strategies for facilitating intracellular delivery of molecular cargo are reviewed e.g. in Li et al., Int. J. Mol. Sci. (2015) 16: 19518-19536 and Fu et al., Bioconjug Chem. (2014) 25(9): 1602-1608, which are hereby incorporated by reference in their entirety.

In some embodiments a LINC complex inhibitor is formulated with a cationic polymer. In some embodiments a LINC complex inhibitor is encapsulated in a nanoparticle or a liposome.

In some embodiments, a nanoparticle is a nanoparticle described in Chen et al., Mol Ther Methods Clin Dev. (2016) 3:16023, which is hereby incorporated by reference in its entirety. In some embodiments, a nanoparticle is a PLGA, polypeptide, poly(β-amino ester), DOPE, β-cyclodeotrin-containing polycation, linear PEI, PAMAM dendrimer, branched PEI, chitosan or polyphosophoester nanoparticle.

In some embodiments a LINC complex inhibitor is (covalently or non-covalently) associated with a cell-penetrating peptide (e.g. a protein transduction domain, trojan peptide, arginine-rich peptide, vectocell peptide), a cationic polymer, a cationic lipid or a viral carrier. In some embodiments a LINC complex inhibitor is associated with a peptide/polypeptide (e.g. antibody, peptide aptamer, ligand for a cell surface molecule/fragment thereof) or a nucleic acid (e.g. nucleic acid aptamer) capable of binding to a target cell of interest or an antigen thereof.

In some embodiments, a LINC complex inhibitor is administered in the form of nucleic acid encoding the LINC complex inhibitor. For example, the LINC complex inhibitor may be administered in the form of nucleic acid encoding a peptide/polypeptide or nucleic acid LINC complex inhibitor, or an SSN system targeting a LINC complex protein.

In some embodiments, a LINC complex inhibitor is administered in the form of nucleic acid encoding the factors required for production of a LINC complex inhibitor (e.g. nucleic acid encoding a precursor of the LINC complex inhibitor and/or nucleic acid encoding factors required for production/assembly of the LINC complex inhibitor). For example, the LINC complex inhibitor may be administered in the form of nucleic acid encoding factors required for production of a small molecule or biomolecular LINC complex inhibitor.

The nucleic acid may be, or may be comprised in, a vector. A "vector" as used herein is a nucleic acid used as a vehicle to transfer exogenous nucleic acid into a cell. The vector may be a vector for expression of the nucleic acid in the target cell. Such vectors may include a promoter sequence operably linked to the nucleic acid sequence to be expressed. A vector may also include a termination codon and expression enhancers. In this specification the term "operably linked" may include the situation where a selected nucleic acid sequence and regulatory nucleic acid sequence (e.g. promoter and/or enhancer) are covalently linked in such a way as to place the expression of the nucleotide sequence under the influence or control of the regulatory sequence (thereby forming an expression cassette). Thus a regulatory sequence is operably linked to the selected nucleic acid sequence if the regulatory sequence is capable of effecting transcription of the nucleic acid sequence. Where appropriate, the resulting transcript may then be translated into a desired polypeptide.

Any suitable vectors, promoters, enhancers and termination codons known in the art may be used.

Suitable vectors include viral vectors, e.g. retroviral vectors, lentiviral vectors, adenovirus vectors, adeno-associated virus vectors, vaccinia virus vectors and herpesvirus vectors, transposon-based vectors, and artificial chromosomes (e.g. yeast artificial chromosomes), e.g. as described in Maus et al., Annu Rev Immunol (2014) 32:189-225 or Morgan and Boyerinas, Biomedicines 2016 4, 9, which are both hereby incorporated by reference in its entirety. By way of illustration, in Example 5 herein a dominant-negative version of SUN1 is administered using an adeno-associated viral vector.

In some embodiments, a vector is selected based on tropism for a cell type/tissue/organ to which it is desired to deliver the nucleic acid, e.g. a cell type/tissue/organ affected by the disease to be treated/prevented in accordance with the invention (i.e. cells/tissue/an organ in which the symptoms of the disease manifest).

For example, in some embodiments it is desired to deliver nucleic acid encoding a LINC complex inhibitor to muscle cells/tissue (e.g. cardiac and/or skeletal muscle cells/tissue), and vectors having a tropism for such cells/tissue may be employed in such embodiments. In some embodiments, a vector may be cardiotropic. In some embodiments, a vector may be myotropic.

An AAV9 vector has recently been used to deliver Lmna-targeted CRISPR/Cas9-mediated gene therapy for HGPS in a Lmna-G609G mouse model (Santiago-Fernández et al., Nat Med. (2019) 25(3):423-426 and Beyret et al., Nat Med. (2019) 25(3):419-422).

In some embodiments, a vector may be an adeno-associated viral vector. In some embodiments, a vector may be an adeno-associated viral vector of one of the following serotypes: AAV9, AAV1, AAV6, AAV8, AAV2i8, AAV9.45, AAV10 or AAVrh.74.

In some embodiments a vector comprises modification to increase binding to and/or transduction of a cell-type of interest (i.e. as compared to the level of binding/transduction by the unmodified vector). In some embodiments modification is to a capsid protein.

In some embodiments a vector comprises a capsid protein comprising a cell-targeting peptide. In some embodiments the cell-targeting peptide is a cell-targeting peptide described in Büning and Srivastava, Molecular Therapy: Methods & Clinical Development (2019) 12: 248-265, which is hereby incorporated by reference in its entirety, e.g. a cell-targeting peptide shown in Table 1, 2, 3 or 4.

In some embodiments a vector comprises a capsid protein comprising mutation to one or more tyrosine residues, e.g. surface-exposed tyrosine residues. In some embodiments, the tyrosine residues are mutated to phenylalanine. In some embodiments a vector comprises a capsid protein in which tyrosine residues are mutated as described in lida et al., Biomed Res Int. (2013) 2013: 974819, which is hereby incorporated by reference in its entirety.

In some embodiments, a vector may be an adeno-associated viral vector described in Büning and Srivastava, *supra.* In some embodiments, a vector may be an adeno-associated viral vector described in lida et al., *supra.*

In some embodiments the nucleic acid/vector comprises one or more sequences for controlling expression of the nucleic acid. Accordingly, in some embodiments the nucleic acid/vector comprises a control element for inducible expression of the nucleic acid.

A sequence for controlling expression of the nucleic acid may provide for expression of the nucleic acid by cells of a particular type or tissue. For example, expression may be under the control of a cell type- or tissue-specific promoter. By way of illustration, in Example 5 herein the expression of a construct encoding a dominant-negative version of SUN1 is under the control of a cardiomyocyte-specific promoter.

Promoters for cell type- or tissue-specific expression of a nucleic acid in accordance with the present invention can be selected in accordance with the disease to be treated/prevented. For example, the promoter may drive expression in cells/tissue/an organ affected by the disease (i.e. cells/tissue/an organ in which the symptoms of the disease manifest).

In some embodiments a promoter may provide for expression in muscle cells/tissue (e.g. cardiac and/or skeletal muscle cells/tissue). In some embodiments, a promoter may be a cardiac or cardiomyocte-specific promoter (e.g. a cTNT, α-MHC or MLC2v promoter). In some embodiments, a promoter may be a skeletal muscle/striated muscle cell-specific promoter (e.g. a MCK, MHCK7 or desmin promoter).

In some embodiments, a promoter may be a vascular endothelial cell-specific promoter (e.g. a Tie2 promoter). In some embodiments, a promoter may be a vascular smooth muscle cell-specific promoter (e.g. a SM22a promoter). In some embodiments, a promoter may be a monocyte/macrophage-specific promoter (e.g. a LysM promoter).

A sequence for controlling expression of the nucleic acid may provide for expression of the nucleic acid in response to e.g. an agent/signal. For example, expression may be under the control of inducible promoter. The agent may provide for inducible expression of the nucleic acid *in vivo* by administration of the agent to a subject having been administered with a modified cell according to the disclosure, or ex *vivo*/*in vitro* by administration of the agent to cells in culture ex *vivo* or *in vitro.*

In some embodiments the nucleic acid(s)/vector(s) employ a conditional expression system for controlling expression of the nucleic acid encoding a LINC complex inhibitor by cells comprising the nucleic acid(s)/vector(s). "Conditional expression" may also be referred to herein as "inducible expression", and refers to expression contingent on certain conditions, e.g. the presence of a particular agent. Conditional expression systems are well known in the art and are reviewed e.g. in Ryding et al. Journal of Endocrinology (2001) 171, 1-14, which is hereby incorporated by reference in its entirety.

Multiple doses of the LINC complex inhibitor may be provided. One or more, or each, of the doses may be accompanied by simultaneous or sequential administration of another therapeutic agent.

Multiple doses may be separated by a predetermined time interval, which may be selected to be one of 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, or 31 days, or 1, 2, 3, 4, 5, or 6 months. By way of example, doses may be given once every 7, 14, 21 or 28 days (plus or minus 3, 2, or 1 days).

### Treatment/prevention of diseases through LINC complex inhibition

The present invention provides methods and articles (agents and compositions) for the treatment and/or prevention of diseases through LINC complex inhibition. Treatment/prevention of disease is achieved by LINC complex inhibition in e.g. a cell, tissue/organ/organ system/subject.

Aspects of the present invention are concerned with the treatment/prevention of diseases in which LINC complex dysfunction is pathologically implicated. Such disease include e.g. nuclear envelopathies (e.g. laminopathies). The therapeutic utility of the agents and methods of the present invention extends to the treatment and/or prevention of any disease that would derive therapeutic/prophylactic benefit from LINC complex inhibition.

The methods may be aimed at: delaying/preventing the onset of symptoms of the disease; reducing the severity of (i.e. alleviating) the symptoms of the disease; reversing the symptoms of the disease; reducing morbidity of subjects having the disease; reducing mortality of subjects having the disease; and/or delaying/preventing progression the disease (e.g. to a later stage).

Aspects of the invention concern the treatment of diseases associated with mutation to a given gene or plurality of genes. Herein, diseases which are "associated with" mutations to a given gene/genes are diseases which are caused or exacerbated by such mutations, or for which such mutations are a risk factor for the development or progression of the disease. In some embodiments, the mutation gives rise to one or more of the following in cells comprising one or more copies of the mutant allele of the gene as compared to cells comprising two copies of (i.e. homozygous for) the non-mutated (wildtype) reference allele of the gene: a reduced level of a gene product (e.g. RNA and/or protein (or particular isoform thereof)) of the wildtype allele; an increased level of a gene product of a non-wildtype allele; an increased level of a gene product of the wildtype allele.

Aspects of the present invention concern the treatment/prevention of nuclear envelopathies. Nuclear envelopathies are diseases/pathological conditions associated with mutations to genes encoding nuclear envelope proteins (i.e. proteins contained in, or directly/indirectly associated with, the ONM, perinuclear space or INM). Nuclear envelopathies are reviewed e.g. by Chi et al., Journal of Biomedical Science (2009) 16:96, which is hereby incorporated by reference in its entirety. Nuclear envelopathies include diseases/pathological conditions associated with mutations in *LMNA, LMNB1, LMNB2, EMD, LAP2, LBR, ZMPSTE24, SYNE-1* and *NUP62.*

In particular, aspects of the present invention are concerned with the treatment/prevention of laminopathies.

Laminopathies are reviewed e.g. by Burke and Stewart, Nat Rev Mol Cell Biol. (2013) 14(1):13-24, and Hah and Kim, Cells (2019) 8(3): 231, which are both hereby incorporated by reference in their entirety. Laminopathies are commonly associated with tissue-specific defects in load bearing at the nuclear level, which can reduce the tolerance of cells to physical forces. In the experimental examples herein, the inventors demonstrate that LINC complex inhibition ameliorates the symptoms of a range of laminopathies.

As used herein, a "laminopathy" is a disease/pathological condition associated with mutation to a gene encoding a lamin.

Genes encoding lamins include *LMNA* (which encodes lamins A and C), and *LMNB1, LMNB2,* which encode lamins B1 and B2. Accordingly, aspects of the present invention concern the treatment/prevention of diseases associated with mutation to *LMNA, LMNB1* and/or *LMNB2.*

In some embodiments the mutation is known or predicted to reduce the level of a lamin isoform encoded by the wildtype allele of a gene enoding a lamin (e.g. *LMNA, LMNB1* or *LMNB2*)*.* In some embodiments the mutation is a missense mutation. In some embodiments the mutation is known or predicted to result in the production of a truncated version of a lamin encoded by the wildtype allele of a gene enoding a lamin. In some embodiments the mutation is known or predicted to result in the production of a lamin which is misfolded and/or degraded.

In some embodiments the mutation is known or predicted to increase the level of a lamin isoform encoded by the wildtype allele of a gene enoding a lamin (e.g. *LMNA, LMNB1* or *LMNB2*)*.*

In some embodiments the mutation is known or predicted to increase the level of a disease-associated lamin variant (e.g. progerin). In some embodiments the mutation is known or predicted to increase the level of a lamin encoded by a disease-associated allele of a gene enoding a lamin.

In some embodiments the laminopathy is a skeletal muscle laminopathy. In some embodiments the laminopathy is a myopathy. In some embodiments the laminopathy is a *LMNA* mutation-associated myopathy.

In some embodiments, the disease to be treated/prevented in accordance with the present invention is characterised by one or more of myopathy, cardiomyopathy, dilated cardiomyopathy, muscular dystrophy, cardiac muscular dystrophy, skeletal muscular dystrophy, progeria, neuropathy, lipoatrophy, skeletal dysplasia, lipodystrophy, leukodystrophy or dermopathy.

In some embodiments, the disease to be treated/prevented in accordance with the present invention is characterised by one or more of muscular dystrophy, cardiac muscular dystrophy or skeletal muscular dystrophy.

In some embodiments the laminopathy is associated with mutation to *LMNA, LMNB1* or *LMNB2.* In some embodiments the laminopathy is selected from Hutchinson-Gilford Progeria Syndrome; Emery-Dreifuss Muscular Dystrophy; Emery-Dreifuss Muscular Dystrophy 2, Autosomal Dominant; Lipodystrophy, Partial, Acquired; Epilepsy, Progressive Myoclonic, 9; Charcot-Marie-Tooth Disease, Axonal, Type 2e; Muscular Dystrophy; Lipodystrophy, Familial Partial, Type 2; Cardiomyopathy, Dilated, 1h; Pelger-Huet Anomaly; Reynolds Syndrome; Muscular Disease; Leukodystrophy; Dilated Cardiomyopathy; Muscular Dystrophy, Congenital, Lmna-Related; Mandibuloacral Dysplasia with Type a Lipodystrophy; Cardiomyopathy, Dilated, 1a; Restrictive Dermopathy, Lethal; Familial Partial Lipodystrophy; Epilepsy; Lipoatrophy with Diabetes, Leukomelanodermic Papules, Liver Steatosis, and Hypertrophic Cardiomyopathy; Leukodystrophy, Demyelinating, Adult-Onset, Autosomal Dominant; Acquired Generalized Lipodystrophy; Emery-Dreifuss Muscular Dystrophy 3, Autosomal Recessive; Charcot-Marie-Tooth Disease; Charcot-Marie-Tooth Disease, Axonal, Type 2b1; Cardiomyopathy, Dilated, 1b; Atrial Standstill 1; Limb-Girdle Muscular Dystrophy; Cardiomyopathy, Dilated, with Hypergonadotropic Hypogonadism; Heart-Hand Syndrome, Slovenian Type; Monogenic Diabetes; Arrhythmogenic Right Ventricular Cardiomyopathy; Cardiomyopathy, Dilated, 1e; Aging; Mandibular Hypoplasia, Deafness, Progeroid Features, and Lipodystrophy Syndrome; Adrenomyodystrophy; Atypical Werner Syndrome; Endometriosis; Spinocerebellar Ataxia 31; Progressive Muscular Atrophy; Neurogenic Bowel; Autosomal Dominant Leukodystrophy with Autonomic Disease; Werner Syndrome; Myopathy; Lmna-Related Dilated Cardiomyopathy; Muscular Dystrophy, Congenital, 1b; Hypertrophic Cardiomyopathy; Left Ventricular Noncompaction; Diabetes Mellitus, Noninsulin-Dependent; Arrhythmogenic Right Ventricular Dysplasia, Familial, 9; Heart Disease; Atrial Fibrillation; Cardiac Conduction Defect; Myoclonus; Progressive Myoclonus Epilepsy; Myoclonus Epilepsy; Peripheral Nervous System Disease; Tooth Disease; Atrioventricular Block; Myofibrillar Myopathy; Autosomal Dominant Limb-Girdle Muscular Dystrophy; Lmna-Related Cardiocutaneous Progeria Syndrome; Amyotrophic Lateral Sclerosis 1; Neural Tube Defects; Cervical Cancer; Neural Tube Defects, Folate-Sensitive; Cerebral Degeneration; Acanthosis Nigricans; 3-Hydroxyacyl-Coa Dehydrogenase Deficiency; Congenital Fiber-Type Disproportion; Acroosteolysis; Wolff-Parkinson-White Syndrome; Sick Sinus Syndrome; Calcinosis; Undifferentiated Pleomorphic Sarcoma; Ventricular Tachycardia, Catecholaminergic Polymorphic, 1, with or without Atrial Dysfunction and/or Dilated Cardiomyopathy; Lipodystrophy, Familial Partial, Type 1; Axonal Neuropathy; Paroxysmal Ventricular Fibrillation; Brugada Syndrome 5; Rigid Spine Muscular Dystrophy; Limb-Girdle Muscular Dystrophy Type 1b; Insulin-Resistant Acanthosis Nigricans, Type a; Generalized Lipodystrophy-Associated Progeroid Syndrome; Osteoporosis; Rigid Spine Muscular Dystrophy 1; Neuropathy; Catecholaminergic Polymorphic Ventricular Tachycardia; Cataract; Bethlem Myopathy 1; Congenital Generalized Lipodystrophy; Restrictive Cardiomyopathy; Muscular Dystrophy, Congenital Merosin-Deficient, 1a; Proximal Spinal Muscular Atrophy; Muscular Dystrophy-Dystroglycanopathy , Type B, 5; Lipodystrophy, Congenital Generalized, Type 1; Emery-Dreifuss Muscular Dystrophy 1, X-Linked; Cardiomyopathy, Dilated, 1d; Myopathy, Proximal, and Ophthalmoplegia; Muscle Tissue Disease; Ovarian Cystadenoma; Emerinopathy; Fanconi Anemia, Complementation Group a; Body Mass Index Quantitative Trait Locus 11; Myelodysplastic Syndrome; Skin Disease; Anorexia Nervosa; Spinal Muscular Atrophy; Inclusion Body Myositis; Aniridia 1; Myositis; Trichohepatoenteric Syndrome 1; Neuromuscular Disease; Nutritional Deficiency Disease; Thoracic Outlet Syndrome; Muscle Disorders; Muscular Atrophy; Hallermann-Streiff Syndrome; Rere-Related Disorders; Miller-Dieker Lissencephaly Syndrome; Lipodystrophy, Congenital Generalized, Type 4; Lipodystrophy, Familial Partial, Type 3; Wiedemann-Rautenstrauch Syndrome; Lipodystrophy, Congenital Generalized, Type 2; Ataxia Neuropathy Spectrum; Alopecia, Neurologic Defects, and Endocrinopathy Syndrome; Lipodystrophy, Familial Partial, Type 4; Second-Degree Atrioventricular Block; Acute Necrotizing Encephalopathy; Median Neuropathy; Intrinsic Cardiomyopathy; Familial Isolated Arrhythmogenic Ventricular Dysplasia, Right Dominant Form; Prolapse of Female Genital Organ; Familial Isolated Arrhythmogenic Ventricular Dysplasia, Biventricular Form; Familial Isolated Arrhythmogenic Ventricular Dysplasia, Left Dominant Form; Complete Generalized Lipodystrophy; Blood Group--Ahonen; Autosomal Semi-Dominant Severe Lipodystrophic Laminopathy; Ulnar Nerve Lesion; Pelvic Muscle Wasting; Alzheimer Disease; Stroke, Ischemic; Ataxia-Telangiectasia; Spondyloarthropathy 1; Human Immunodeficiency Virus Type 1; Neuroblastoma; Vascular Disease; Nervous System Disease; Respiratory Failure; Turner Syndrome; Carpal Tunnel Syndrome; Barrett Esophagus; Sleep Apnea; Cerebrovascular Disease; Proteasome-Associated Autoinflammatory Syndrome 1; Joubert Syndrome 1; Viral Infectious Disease; Dementia; Personality Disorder; Neuropathy, Hereditary Sensory and Autonomic, Type Iii; Lowe Oculocerebrorenal Syndrome; Diabetes Mellitus; Fatty Liver Disease; Leigh Syndrome; Muscular Dystrophy, Duchenne Type; Hydrocephalus; Dermatomyositis; Hirschsprung Disease 1; Long Qt Syndrome; Angelman Syndrome; Central Nervous System Disease; Congenital Disorder of Glycosylation, Type in; Alacrima, Achalasia, and Mental Retardation Syndrome; Polycystic Ovary Syndrome; Hypoglycemia; Muscle Hypertrophy; Kearns-Sayre Syndrome; Cone-Rod Dystrophy 2; Aicardi-Goutieres Syndrome; Andersen Cardiodysrhythmic Periodic Paralysis; Muscular Dystrophy, Becker Type; Legg-Calve-Perthes Disease; Androgen Insensitivity Syndrome; Ehlers-Danlos Syndrome; Axenfeld-Rieger Syndrome; Muscular Dystrophy-Dystroglycanopathy , Type C, 5; Glomerulonephritis; Seizure Disorder; Chikungunya; West Syndrome; Ullrich Congenital Muscular Dystrophy 1; Focal Segmental Glomerulosclerosis; Walker-Warburg Syndrome; Renal Hypodysplasia/aplasia 1; Popliteal Pterygium Syndrome; Microcephaly; Childhood Type Dermatomyositis; Distal Arthrogryposis; Myocarditis; Arterial Tortuosity Syndrome; Scoliosis; Membranous Nephropathy; Microvascular Complications of Diabetes 3; Epidermolysis Bullosa; Short Syndrome; Hyperekplexia; Nonalcoholic Fatty Liver Disease; Muscular Dystrophy-Dystroglycanopathy , Type a, 4; Congenital Hydrocephalus; Ataxia, Combined Cerebellar and Peripheral, with Hearing Loss and Diabetes Mellitus; Cardiac Arrhythmia; Muscular Dystrophy-Dystroglycanopathy , Type a, 1; Ptosis; Laryngitis; Ablepharon-Macrostomia Syndrome; Supravalvular Aortic Stenosis; Myopathy, Congenital; Metabolic Encephalomyopathic Crises, Recurrent, with Rhabdomyolysis, Cardiac Arrhythmias, and Neurodegeneration; Lissencephaly 1; Polycystic Liver Disease 1 with or without Kidney Cysts; Idiopathic Inflammatory Myopathy; Epidermolysis Bullosa Simplex; Focal Segmental Glomerulosclerosis 1; Gonadal Dysgenesis; Gyrate Atrophy of Choroid and Retina; Syringomyelia; Ichthyosis Vulgaris; Arthrogryposis, Distal, Type 1a; Acute Insulin Response; Brachydactyly; Cerebellar Hypoplasia; Craniometaphyseal Dysplasia, Autosomal Dominant; Alport Syndrome 1, X-Linked; Lissencephaly; Muscular Dystrophy-Dystroglycanopathy , Type B, 6; Diarrhea 5, with Tufting Enteropathy, Congenital; Junctional Epidermolysis Bullosa; Aicardi-Goutieres Syndrome 1; Miyoshi Muscular Dystrophy; Retinitis; Marden-Walker Syndrome; Neuroretinitis; Polyglucosan Body Myopathy 1 with or without Immunodeficiency; Epidermolysis Bullosa, Junctional, Herlitz Type; Macroglossia; Parkinson Disease 15, Autosomal Recessive Early-Onset; Myopathy, Myofibrillar, 3; Microvascular Complications of Diabetes 7; Muscle Eye Brain Disease; Melkersson-Rosenthal Syndrome; Myopathy, X-Linked, with Excessive Autophagy; Choroiditis; Muscular Dystrophy, Limb-Girdle, Autosomal Recessive 8; Crouzon Syndrome with Acanthosis Nigricans; Muscular Dystrophy, Limb-Girdle, Autosomal Recessive 6; Polymicrogyria; Dystrophinopathies; Microvascular Complications of Diabetes 6; Microvascular Complications of Diabetes 4; Hypotonia; Pontocerebellar Hypoplasia; Congenital Fibrosarcoma; Intrauterine Growth Retardation, Metaphyseal Dysplasia, Adrenal Hypoplasia Congenita, and Genital Anomalies; Muscular Dystrophy, Limb-Girdle, Autosomal Recessive 7; Myopathy, Congenital, with Fiber-Type Disproportion; Amelogenesis Imperfecta, Type Ig; Refractory Anemia; Fibrosis of Extraocular Muscles, Congenital, 1; Ataxia and Polyneuropathy, Adult-Onset; Al-Raqad Syndrome; Senile Cataract; Muscular Dystrophy-Dystroglycanopathy , Type C, 1; Neuronal Migration Disorders; Ayme-Gripp Syndrome; Primary Agammaglobulinemia; Autosomal Recessive Limb-Girdle Muscular Dystrophy Type 2a; Cerebritis; Muscular Dystrophy, Congenital, Megaconial Type; Autosomal Recessive Limb-Girdle Muscular Dystrophy; Alkuraya-Kucinskas Syndrome; Muscular Dystrophy-Dystroglycanopathy , Type C, 4; Congenital Muscular Dystrophy Type 1a; Behr Syndrome; Dandy-Walker Complex; Muscular Dystrophy-Dystroglycanopathy , Type C, 2; Emery-Dreifuss Muscular Dystrophy, X-Linked; Muscular Dystrophy, Limb-Girdle, Autosomal Recessive 3; Autosomal Recessive Limb-Girdle Muscular Dystrophy Type 2d; Brain Small Vessel Disease 1 with or without Ocular Anomalies; Familial Isolated Dilated Cardiomyopathy; Epithelial Recurrent Erosion Dystrophy; Muscular Dystrophy-Dystroglycanopathy; Mycobacterium Avium Complex Infections; Autosomal Recessive Limb-Girdle Muscular Dystrophy Type 2I; Visual Epilepsy; Aneurysm of Sinus of Valsalva; Autosomal Recessive Limb-Girdle Muscular Dystrophy Type 2b; Creatine Phosphokinase, Elevated Serum; Spastic Paraplegia, Ataxia, and Mental Retardation; Multinucleated Neurons, Anhydramnios, Renal Dysplasia, Cerebellar Hypoplasia, and Hydranencephaly; Patulous Eustachian Tube; Autosomal Genetic Disease; Ck Syndrome; Neuronitis; Hyperekplexia 1; Reducing Body Myopathy; Polymicrogyria, Bilateral Temporooccipital; Isolated Hyperckemia; Charcot-Marie-Tooth Disease, Axonal, Type 2b2; Cardioneuromyopathy with Hyaline Masses and Nemaline Rods; Congenital Muscular Dystrophy without Intellectual Disability; Blood Group, I System; Salih Myopathy; Adducted Thumbs Syndrome; Dural Sinus Malformation; Blood Group, Dombrock System; Blood Group, Colton System; Arthrochalasia Ehlers-Danlos Syndrome; Lama2-Related Muscular Dystrophy; Muscular Dystrophy, Congenital, with Infantile Cataract and Hypogonadism; Intrauterine Infections; Muscular Dystrophy, Congenital, Merosin-Positive; Congenital Muscular Dystrophy with Cerebellar Involvement; Fukuyama Type Muscular Dystrophy; Chronic Lymphoproliferative Disorder of Natural Killer Cells; Congenital Muscular Dystrophy with Intellectual Disability; Amelogenesis Imperfecta Hypoplastic Type, Ig; Androgen Insensitivity Syndrome, Mild; Muscular Dystrophy, Congenital, Producing Arthrogryposis; Congenital Muscular Alpha-Dystroglycanopathy with Brain and Eye Anomalies; Collagen Vi-Related Myopathy; Emery-Dreifuss Muscular Dystrophy, Dominant Type; Congenital Muscular Dystrophy Due to Dystroglycanopathy; Proximal Myopathy with Focal Depletion of Mitochondria; Infantile Scoliosis.

In some embodiments the laminopathy is a laminopathy associated with mutation to *LMNA.* In some embodiments the laminopathy is selected from Hutchinson-Gilford Progeria Syndrome; Dilated Cardiomyopathy; Muscular Dystrophy, Congenital, Lmna-Related; Emery-Dreifuss Muscular Dystrophy 2, Autosomal Dominant; Muscular Dystrophy; Mandibuloacral Dysplasia with Type a Lipodystrophy; Cardiomyopathy, Dilated, 1a; Charcot-Marie-Tooth Disease; Limb-Girdle Muscular Dystrophy; Cardiomyopathy, Dilated, with Hypergonadotropic Hypogonadism; Emery-Dreifuss Muscular Dystrophy 3, Autosomal Recessive; Lipodystrophy, Familial Partial, Type 2; Emery-Dreifuss Muscular Dystrophy; Charcot-Marie-Tooth Disease, Axonal, Type 2b1; Heart-Hand Syndrome, Slovenian Type; Aging; Familial Partial Lipodystrophy; Restrictive Dermopathy, Lethal; Arrhythmogenic Right Ventricular Cardiomyopathy; Tooth Disease; Heart Disease; Werner Syndrome; Hypertrophic Cardiomyopathy; Left Ventricular Noncompaction; Atrioventricular Block; Calcinosis; Acroosteolysis; Autosomal Dominant Limb-Girdle Muscular Dystrophy; Diabetes Mellitus, Noninsulin-Dependent; Osteoporosis; Atrial Fibrillation; Atrial Standstill 1; Acanthosis Nigricans; Cardiac Conduction Defect; Catecholaminergic Polymorphic Ventricular Tachycardia; Mandibular Hypoplasia, Deafness, Progeroid Features, and Lipodystrophy Syndrome; Sick Sinus Syndrome; Pelger-Huet Anomaly; Charcot-Marie-Tooth Disease, Axonal, Type 2e; Congenital Generalized Lipodystrophy; Restrictive Cardiomyopathy; Congenital Fiber-Type Disproportion; Lipodystrophy, Congenital Generalized, Type 1; Myofibrillar Myopathy; Lipodystrophy, Familial Partial, Type 1; Axonal Neuropathy; Atypical Werner Syndrome; Ovarian Cystadenoma; Fanconi Anemia, Complementation Group a; Body Mass Index Quantitative Trait Locus 11; Skin Disease; Rigid Spine Muscular Dystrophy 1; Neuromuscular Disease; Hallermann-Streiff Syndrome; Bethlem Myopathy 1; Acquired Generalized Lipodystrophy; Cardiomyopathy, Dilated, 1e; Lipodystrophy, Congenital Generalized, Type 4; Undifferentiated Pleomorphic Sarcoma; Lipodystrophy, Familial Partial, Type 3; Muscular Dystrophy, Congenital Merosin-Deficient, 1a; Proximal Spinal Muscular Atrophy; Muscular Dystrophy-Dystroglycanopathy , Type B, 5; Muscular Dystrophy, Congenital, 1b; Reynolds Syndrome; Wiedemann-Rautenstrauch Syndrome; Emery-Dreifuss Muscular Dystrophy 1, X-Linked; Lipodystrophy, Congenital Generalized, Type 2; Monogenic Diabetes; Cardiomyopathy, Dilated, 1d; Myopathy, Proximal, and Ophthalmoplegia; Muscle Tissue Disease; Lipodystrophy, Familial Partial, Type 4; Cardiomyopathy, Dilated, 1h; Second-Degree Atrioventricular Block; Median Neuropathy; Intrinsic Cardiomyopathy; Prolapse of Female Genital Organ; Complete Generalized Lipodystrophy; Rigid Spine Muscular Dystrophy; Emerinopathy; Ulnar Nerve Lesion; Limb-Girdle Muscular Dystrophy Type 1b; Lmna-Related Dilated Cardiomyopathy; Pelvic Muscle Wasting; Generalized Lipodystrophy-Associated Progeroid Syndrome; Muscular Disease; Cardiomyopathy, Dilated, 1b; Autosomal Genetic Disease; Familial Isolated Arrhythmogenic Ventricular Dysplasia, Right Dominant Form; Familial Isolated Arrhythmogenic Ventricular Dysplasia, Biventricular Form; Familial Isolated Arrhythmogenic Ventricular Dysplasia, Left Dominant Form; Lmna-Related Cardiocutaneous Progeria Syndrome; Autosomal Semi-Dominant Severe Lipodystrophic Laminopathy.

In some embodiments the disease to the treated/prevented in accordance with the present invention is selected from a disease associated with a cDNA or protein variant indicated in Table 1.

In some embodiments the disease to the treated/prevented in accordance with the present invention is selected from a disease indicated in Table 1.

In some embodiments the disease to the treated/prevented in accordance with the present invention is selected from a disease indicated in normal font in Table 1.

In some embodiments the disease to the treated/prevented in accordance with the present invention is selected from a disease indicated in bold font in Table 1.

| Database ID | cDNA Variant | cDNA Variant Types | Protein Variant | Protein Variant Types | Domain | Disease Abbreviation | Disease Name | Omim ID | Omim Symbol |
|---|---|---|---|---|---|---|---|---|---|
| 17811 | c.1771T>A | Substitution | p.Cys591Ser | Substitution | Tail | - | Acrogeria, Gottron Type | 201200 | # |
| **18255** | c.418_438dupCTGCTGAAC TCCAAGGAGGCC | **Duplication** | **p.Leu140_Ala146dup** | **Duplication** | **1B** | - | **Arrhythmogenic cardiomyopathy** | - | - |
| **17439** | **c.1039G>A** | **Substitution** | **p.Glu347Lys** | **Substitution** | **2B** | **ARVD7** | **Arrhythmogenic right ventricular cardiomyopathy (ARVD7)** | **609160** | % |
| **18492** | **c.1930C>T** | **Substitution** | **p.Arg644Cys** | **Substitution** | **Tail** | **ARVD7** | **Arrhythmogenic right ventricular cardiomyopathy** | **609160** | % |
| 9157 | c.1718C>T | Substitution | p.Ser573Leu | Substitution | Tail | - | Arthropathy syndrome, autosomal recessive | - | - |
| **18299** | **c.1494G>A** | **Substitution** | **p.Trp498X** | **Substitution** | **Tail** | **AF** | **Atrial fibrillation** | **-** | **-** |
| **18301** | **c.175C>G** | **Substitution** | **p.Leu59Val** | **Substitution** | **1A** | **APS** | **Atypical progeroid syndrome** | **-** | **-** |
| **8885** | **c.169G>C** | **Substitution** | **p.Ala57Pro** | **Substitution** | **1A** | **WRN** | **Atypical Werner syndrome** | **277700** | **#** |
| **8886** | **c.398G>T** | **Substitution** | **p.Arg133Leu** | **Substitution** | **1B** | **WRN** | **Atypical Werner syndrome** | **277700** | **#** |
| **11462** | **c.398G>T** | **Substitution** | **p.Arg133Leu** | **Substitution** | **1B** | **WRN** | **Atypical Werner syndrome** | **277700** | **#** |
| **9232** | **c.398G>T** | **Substitution** | **p.Arg133Leu** | **Substitution** | **1B** | **WRN** | **Atypical Werner syndrome** | **277700** | **#** |
| **11509** | **c.398G>T** | **Substitution** | **p.Arg133Leu** | **Substitution** | **1B** | **WRN** | **Atypical Werner syndrome** | **277700** | **#** |
| **11670** | **c.398G>T** | **Substitution** | **p.Arg133Leu** | **Substitution** | **1B** | **WRN** | **Atypical Werner syndrome** | **277700** | **#** |
| **8888** | **c.419T>G** | **Substitution** | **p.Leu140Arg** | **Substitution** | **1B** | **WRN** | **Atypical Werner syndrome** | **277700** | **#** |
| **13457** | **c.506delT** | **Deletion** | **p.Val169GlyfsX7** | **Frame shift** | **1B** | **WRN** | **Atypical Werner syndrome** | **277700** | **#** |
| **13350** | **c.898G>A** | **Substitution** | **p.Asp300Asn** | **Substitution** | **2B** | **WRN** | **Atypical Werner syndrome** | **277700** | **#** |
| **17875** | **c.898G>A** | **Substitution** | **p.Asp300Asn** | **Substitution** | **2B** | **WRN** | **Atypical Werner syndrome** | **277700** | **#** |
| **11767** | **c.1130G>T** | **Substitution** | **p.Arg377Leu** | **Substitution** | **2B** | **AD-SMA** | **Autosomal dominant spinal muscular dystrophy** | **182980** | **#** |
| **11766** | **c.1477C>T** | **Substitution** | **p.Gln493X** | **Substitution** | **Tail** | **AD-SMA** | **Autosomal dominant spinal muscular dystrophy** | **182980** | **#** |
| **9205** | **c.?** | **Unknown** | **p.Glu33Asp** | **Substitution** | **1A** | **-** | **Axonal neuropathy, muscular dystrophy, cardiac disease** | **-** | **-** |
| **8994** | **c.99G>T** | **Substitution** | **p.Glu33Asp** | **Substitution** | **1A** | **-** | **Axonal neuropathy, muscular dystrophy, cardiac disease, leuconychia** | **-** | **-** |
| **12416** | **c.1621C>T** | **Substitution** | **p.Arg541Cys** | **Substitution** | **Tail** | **-** | **Cardiac arrhythmia** | **-** | **-** |
| **12399** | **c.673C>T** | **Substitution** | **p.Arg225X** | **Substitution** | **L12** | **CCD** | **Cardiac conduction defect** | **115080** | **#** |
| **17808** | **c.695G>T** | **Substitution** | **p.Gly232Val** | **Substitution** | **L12** | **CCD** | **Cardiac conduction defect** | **115080** | **#** |
| **13449** | **c.799T>C** | **Substitution** | **p.Tyr267His** | **Substitution** | **2B** | **CCD** | **Cardiac conduction defect** | **115080** | **#** |
| **14260** | **c.178C>G** | **Substitution** | **p.Arg60Gly** | **Substitution** | **1A** | - | **Cardiomyopathy with advanced AV block and arrhythmia** | - | - |
| **14256** | **c.184C>G** | **Substitution** | **p.Arg62Gly** | **Substitution** | **1A** | - | **Cardiomyopathy with advanced AV block and arrhythmia** | - | - |
| **14018** | **c.497G>C** | **Substitution** | **p.Arg166Pro** | **Substitution** | **1B** | **-** | **Cardiomyopathy with advanced AV block and arrhythmia** | **-** | **-** |
| **14258** | **c.575A>T** | **Substitution** | **p.Asp192Val** | **Substitution** | **1B** | **-** | **Cardiomyopathy with advanced AV block and arrhythmia** | **-** | **-** |
| **14007** | **c.673C>T** | **Substitution** | **p.Arg225X** | **Substitution** | **L12** | **-** | **Cardiomyopathy with advanced AV block and arrhythmia** | **-** | **-** |
| **14015** | **c.775T>C** | **Substitution** | **p.Tyr259His** | **Substitution** | **L2** | **-** | **Cardiomyopathy with advanced AV block and arrhythmia** | **-** | **-** |
| **14016** | **c.775T>C** | **Substitution** | **p.Tyr259His** | **Substitution** | **L2** | **-** | **Cardiomyopathy with advanced AV block and arrhythmia** | **-** | **-** |
| **14011** | c.815_818delACAAinsCCA GAC | **Indel** | **p.Asp272AlafsX208** | **Frame shift** | **2B** | **-** | **Cardiomyopathy with advanced AV block and arrhythmia** | **-** | **-** |
| **14012** | c.815_818delACAAinsCCA GAC | **Indel** | **p.Asp272AlafsX208** | **Frame shift** | **2B** | **-** | **Cardiomyopathy with advanced AV block and arrhythmia** | **-** | **-** |
| 11652 | c.- 3_12delGCCATGGAGACCC CG | Deletion | p.Met1_Pro4del | Deletion | Head | CMT2 | Charcot-Marie-Tooth disease type 2 | 118210 | # |
| 13374 | c.1496_1496delC | Deletion | p.Ala499ValfsX141 | Frame shift | Tail | CMT2 | Charcot-Marie-Tooth disease type 2 | 118210 | # |
| 17199 | c.1910T>C | Substitution | p.Phe637Ser | Substitution | Tail | CMT2 | Charcot-Marie-Tooth disease type 2 | 118210 | # |
| 8840 | c.892C>T | Substitution | p.Arg298Cys | Substitution | 2B | CMT2B1 | Charcot-Marie-Tooth disease type 2B1 | 605588 | # |
| 11415 | c.892C>T | Substitution | p.Arg298Cys | Substitution | 2B | CMT2B1 | Charcot-Marie-Tooth disease type 2B1 | 605588 | # |
| 11416 | c.892C>T | Substitution | p.Arg298Cys | Substitution | 2B | CMT2B1 | Charcot-Marie-Tooth disease type 2B1 | 605588 | # |
| 8997 | c.892C>T | Substitution | p.Arg298Cys | Substitution | 2B | CMT2B1 | Charcot-Marie-Tooth disease type 2B1 | 605588 | # |
| 11840 | c.892C>T | Substitution | p.Arg298Cys | Substitution | 2B | CMT2B1 | Charcot-Marie-Tooth disease type 2B1 | 605588 | # |
| 11839 | c.892C>T | Substitution | p.Arg298Cys | Substitution | 2B | CMT2B1 | Charcot-Marie-Tooth disease type 2B1 | 605588 | # |
| 11838 | c.892C>T | Substitution | p.Arg298Cys | Substitution | 2B | CMT2B1 | Charcot-Marie-Tooth disease type 2B1 | 605588 | # |
| 11837 | c.892C>T | Substitution | p.Arg298Cys | Substitution | 2B | CMT2B1 | Charcot-Marie-Tooth disease type 2B1 | 605588 | # |
| 12087 | c.892C>T | Substitution | p.Arg298Cys | Substitution | 2B | CMT2B1 | Charcot-Marie-Tooth disease type 2B1 | 605588 | # |
| 13414 | c.892C>T | Substitution | p.Arg298Cys | Substitution | 2B | CMT2B1 | Charcot-Marie-Tooth disease type 2B1 | 605588 | # |
| 13415 | c.892C>T | Substitution | p.Arg298Cys | Substitution | 2B | CMT2B1 | Charcot-Marie-Tooth disease type 2B1 | 605588 | # |
| 13416 | c.892C>T | Substitution | p.Arg298Cys | Substitution | 2B | CMT2B1 | Charcot-Marie-Tooth disease type 2B1 | 605588 | # |
| 13417 | c.892C>T | Substitution | p.Arg298Cys | Substitution | 2B | CMT2B1 | Charcot-Marie-Tooth disease type 2B1 | 605588 | # |
| 13418 | c.892C>T | Substitution | p.Arg298Cys | Substitution | 2B | CMT2B1 | Charcot-Marie-Tooth disease type 2B1 | 605588 | # |
| 13419 | c.892C>T | Substitution | p.Arg298Cys | Substitution | 2B | CMT2B1 | Charcot-Marie-Tooth disease type 2B1 | 605588 | # |
| 13420 | c.892C>T | Substitution | p.Arg298Cys | Substitution | 2B | CMT2B1 | Charcot-Marie-Tooth disease type 2B1 | 605588 | # |
| 13421 | c.892C>T | Substitution | p.Arg298Cys | Substitution | 2B | CMT2B1 | Charcot-Marie-Tooth disease type 2B1 | 605588 | # |
| 13422 | c.892C>T | Substitution | p.Arg298Cys | Substitution | 2B | CMT2B1 | Charcot-Marie-Tooth disease type 2B1 | 605588 | # |
| 13423 | c.892C>T | Substitution | p.Arg298Cys | Substitution | 2B | CMT2B1 | Charcot-Marie-Tooth disease type 2B1 | 605588 | # |
| 13424 | c.892C>T | Substitution | p.Arg298Cys | Substitution | 2B | CMT2B1 | Charcot-Marie-Tooth disease type 2B1 | 605588 | # |
| 13425 | c.892C>T | Substitution | p.Arg298Cys | Substitution | 2B | CMT2B1 | Charcot-Marie-Tooth disease type 2B1 | 605588 | # |
| 13426 | c.892C>T | Substitution | p.Arg298Cys | Substitution | 2B | CMT2B1 | Charcot-Marie-Tooth disease type 2B1 | 605588 | # |
| 13427 | c.892C>T | Substitution | p.Arg298Cys | Substitution | 2B | CMT2B1 | Charcot-Marie-Tooth disease type 2B1 | 605588 | # |
| 13428 | c.892C>T | Substitution | p.Arg298Cys | Substitution | 2B | CMT2B1 | Charcot-Marie-Tooth disease type 2B1 | 605588 | # |
| 13429 | c.892C>T | Substitution | p.Arg298Cys | Substitution | 2B | CMT2B1 | Charcot-Marie-Tooth disease type 2B1 | 605588 | # |
| 13430 | c.892C>T | Substitution | p.Arg298Cys | Substitution | 2B | CMT2B1 | Charcot-Marie-Tooth disease type 2B1 | 605588 | # |
| 13431 | c.892C>T | Substitution | p.Arg298Cys | Substitution | 2B | CMT2B1 | Charcot-Marie-Tooth disease type 2B1 | 605588 | # |
| 13432 | c.892C>T | Substitution | p.Arg298Cys | Substitution | 2B | CMT2B1 | Charcot-Marie-Tooth disease type 2B1 | 605588 | # |
| 13433 | c.892C>T | Substitution | p.Arg298Cys | Substitution | 2B | CMT2B1 | Charcot-Marie-Tooth disease type 2B1 | 605588 | # |
| 13434 | c.892C>T | Substitution | p.Arg298Cys | Substitution | 2B | CMT2B1 | Charcot-Marie-Tooth disease type 2B1 | 605588 | # |
| 13435 | c.892C>T | Substitution | p.Arg298Cys | Substitution | 2B | CMT2B1 | Charcot-Marie-Tooth disease type 2B1 | 605588 | # |
| 13436 | c.892C>T | Substitution | p.Arg298Cys | Substitution | 2B | CMT2B1 | Charcot-Marie-Tooth disease type 2B1 | 605588 | # |
| 13437 | c.892C>T | Substitution | p.Arg298Cys | Substitution | 2B | CMT2B1 | Charcot-Marie-Tooth disease type 2B1 | 605588 | # |
| 17886 | c.80C>T | Substitution | p.Thr27Ile | Substitution | Head | CFTDM | Congenital fiber type disproportion | 255310 | # |
| 18472 | c.907T>C | Substitution | p.Ser303Pro | Substitution | 2B | CFTDM | Congenital fiber type disproportion | 255310 | # |
| 18473 | c.907T>C | Substitution | p.Ser303Pro | Substitution | 2B | CFTDM | Congenital fiber type disproportion | 255310 | # |
| 18474 | c.907T>C | Substitution | p.Ser303Pro | Substitution | 2B | CFTDM | Congenital fiber type disproportion | 255310 | # |
| 18475 | c.907T>C | Substitution | p.Ser303Pro | Substitution | 2B | CFTDM | Congenital fiber type disproportion | 255310 | # |
| 18144 | c.91G>A | Substitution | p.Glu31Lys | Substitution | Head | CMD | Congenital muscular dystrophy | - | - |
| 18148 | c.91_93delGAG | Deletion | p.Glu31X | Substitution | Head | CMD | Congenital muscular dystrophy | - | - |
| 17813 | c.93G>C | Substitution | p.Glu31Asp | Substitution | Head | CMD | Congenital muscular dystrophy | - | - |
| 18146 | c.94_96delAAG | Deletion | p.Lys32X | Substitution | 1A | CMD | Congenital muscular dystrophy | - | - |
| 16402 | c.104T>C | Substitution | p.Leu35Pro | Substitution | 1A | CMD | Congenital muscular dystrophy | - | - |
| 16287 | c.115A>T | Substitution | p.Asn39Tyr | Substitution | 1A | CMD | Congenital muscular dystrophy | - | - |
| 18150 | c.117T>G | Substitution | p.Asn39Lys | Substitution | 1A | CMD | Congenital muscular dystrophy | - | - |
| 18153 | c.143G>C | Substitution | p.Arg48Pro | Substitution | 1A | CMD | Congenital muscular dystrophy | - | - |
| 18156 | c.422T>C | Substitution | p.Leu141Pro | Substitution | 1B | CMD | Congenital muscular dystrophy | - | - |
| 13462 | c.745C>T | Substitution | p.Arg249Trp | Substitution | 2A | CMD | Congenital muscular dystrophy | - | - |
| 16404 | c.745C>T | Substitution | p.Arg249Trp | Substitution | 2A | CMD | Congenital muscular dystrophy | - | - |
| 17758 | c.745C>T | Substitution | p.Arg249Trp | Substitution | 2A | CMD | Congenital muscular dystrophy | - | - |
| 18158 | c.745C>T | Substitution | p.Arg249Trp | Substitution | 2A | CMD | Congenital muscular dystrophy | - | - |
| 11816 | c.1072G>A | Substitution | p.Glu358Lys | Substitution | 2B | CMD | Congenital muscular dystrophy | - | - |
| 18161 | c.1117A>G | Substitution | p.Ile373Val | Substitution | 2B | CMD | Congenital muscular dystrophy | - | - |
| 18166 | c.1118T>A | Substitution | p.Ile373Asn | Substitution | 2B | CMD | Congenital muscular dystrophy | - | - |
| 13460 | c.1139T>C | Substitution | p.Leu380Ser | Substitution | 2B | CMD | Congenital muscular dystrophy | - | - |
| 18169 | c.1147G>A | Substitution | p.Glu383Lys | Substitution | 2B | CMD | Congenital muscular dystrophy | - | - |
| 18171 | c.1147G>A | Substitution | p.Glu383Lys | Substitution | 2B | CMD | Congenital muscular dystrophy | - | - |
| 18163 | c.1151A>G | Substitution | p.Glu384Gly | Substitution | 2B | CMD | Congenital muscular dystrophy | - | - |
| 11817 | c.1162C>T | Substitution | p.Arg388Cys | Substitution | Tail | CMD | Congenital muscular dystrophy | - | - |
| 17473 | c.1330_1338dupGAGGTGG AT | Duplication | p.Glu444_Asp446dup | Duplication | Tail | CMD | Congenital muscular dystrophy | - | - |
| 11818 | c.1368_1370delCAA | Deletion | p.Asn456del | Deletion | Tail | CMD | Congenital muscular dystrophy | - | - |
| 18238 | c.1489-14_1489- 7delTTTCTCCT | Deletion | p.? | Unknown | Unknown | CMD | Congenital muscular dystrophy | - | - |
| 16951 | c.1445G>A | Substitution | pArg482Gln | Substitution | Tail | T2D | DIABETES MELLITUS, NONINSULIN-DEPENDENT; NIDDM | 125853 | # |
| 13171 | c.? | Unknown | p.Lys260Asn | Substitution | L2 | CMD1A | Dilated cardiomyopathy 1A | 115200 | # |
| 8832 | c.16C>T | Substitution | p.Gln6X | Substitution | Head | CMD1A | Dilated cardiomyopathy 1A | 115200 | # |
| 9397 | c.28_29insA | Insertion | p.Thr10AsnfsX31 | Frame shift | Head | CMD1A | Dilated cardiomyopathy 1A | 115200 | # |
| 11627 | c.31delC | Deletion | p.Arg11AlafsX85 | Frame shift | Head | CMD1A | Dilated cardiomyopathy 1A | 115200 | # |
| 14254 | c.73C>G | Substitution | p.Arg25Gly | Substitution | Head | CMD1A | Dilated cardiomyopathy 1A | 115200 | # |
| 13155 | c.82C>T | Substitution | p.Arg28Trp | Substitution | Head | CMD1A | Dilated cardiomyopathy 1A | 115200 | # |
| 13563 | c.155T>C | Substitution | p.Leu52Pro | Substitution | 1A | CMD1A | Dilated cardiomyopathy 1A | 115200 | # |
| 13379 | c.176T>G | Substitution | **p.Leu59Arg** | Substitution | 1A | CMD1A | Dilated cardiomyopathy 1A | 115200 | # |
| 13378 | c.176T>G | Substitution | **p.Leu59Arg** | Substitution | 1A | CMD1A | Dilated cardiomyopathy 1A | 115200 | # |
| 8748 | c.178C>G | Substitution | p.Arg60Gly | Substitution | 1A | CMD1A | Dilated cardiomyopathy 1A | 115200 | # |
| 13192 | c.203_208delAGGTGG | Deletion | **p.Glu68_Val69del** | Deletion | 1A | CMD1A | Dilated cardiomyopathy 1A | 115200 | # |
| 17742 | c.232G>A | Substitution | p. Lys78Glu | Substitution | 1B | CMD1A | Dilated cardiomyopathy 1A | 115200 | # |
| 9163 | c.244G>A | Substitution | p.Glu82Lys | Substitution | 1B | CMD1A | Dilated cardiomyopathy 1A | 115200 | # |
| 16139 | c.244G>A | Substitution | p.Glu82Lys | Substitution | 1B | CMD1A | Dilated cardiomyopathy 1A | 115200 | # |
| 8749 | c.254T>G | Substitution | p. Leu85Arg | Substitution | 1B | CMD1A | Dilated cardiomyopathy 1A | 115200 | # |
| 8865 | c.266G>T | Substitution | p.Arg89Leu | Substitution | 1B | CMD1A | Dilated cardiomyopathy 1A | 115200 | # |
| 11618 | c.266G>T | Substitution | p.Arg89Leu | Substitution | 1B | CMD1A | Dilated cardiomyopathy 1A | 115200 | # |
| 13080 | c.266G>T | Substitution | p.Arg89Leu | Substitution | 1B | CMD1A | Dilated cardiomyopathy 1A | 115200 | # |
| 13157 | c.266G>T | Substitution | p.Arg89Leu | Substitution | 1B | CMD1A | Dilated cardiomyopathy 1A | 115200 | # |
| 14030 | c.266G>T | Substitution | p.Arg89Leu | Substitution | 1B | CMD1A | Dilated cardiomyopathy 1A | 115200 | # |
| 16966 | c.266G>T | Substitution | p.Arg89Leu | Substitution | 1B | CMD1A | Dilated cardiomyopathy 1A | 115200 | # |
| 17777 | c.266G>T | Substitution | p.Arg89Leu | Substitution | 1B | CMD1A | Dilated cardiomyopathy 1A | 115200 | # |
| 13613 | c.274C>T | Substitution | p.Leu92Phe | Substitution | 1B | CMD1A | Dilated cardiomyopathy 1A | 115200 | # |
| 8844 | c.289A>G | Substitution | p. Lys97Glu | Substitution | 1B | CMD1A | Dilated cardiomyopathy 1A | 115200 | # |
| 11620 | c.289A>G | Substitution | p. Lys97Glu | Substitution | 1B | CMD1A | Dilated cardiomyopathy 1A | 115200 | # |
| 13159 | c.289A>G | Substitution | p. Lys97Glu | Substitution | 1B | CMD1A | Dilated cardiomyopathy 1A | 115200 | # |
| 13082 | c.302G>C | Substitution | p.Arg101Pro | Substitution | 1B | CMD1A | Dilated cardiomyopathy 1A | 115200 | # |
| 16954 | c.302G>C | Substitution | p.Arg101Pro | Substitution | 1B | CMD1A | Dilated cardiomyopathy 1A | 115200 | # |
| 8843 | **c.331G>T** | Substitution | p.Glu111X | Substitution | 1B | CMD1A | Dilated cardiomyopathy 1A | 115200 | # |
| 11619 | c.331G>T | Substitution | p.Glu111X | Substitution | 1B | CMD1A | Dilated cardiomyopathy 1A | 115200 | # |
| 13199 | c.331G>T | Substitution | p.Glu111X | Substitution | 1B | CMD1A | Dilated cardiomyopathy 1A | 115200 | # |
| 16935 | c.348_349insG | Insertion | p.Lys117GlufsX10 | Frame shift | 1B | CMD1A | Dilated cardiomyopathy 1A | 115200 | # |
| 13089 | **c.357-1G>T** | Substitution | p.? | Unknown | Unknown | CMD1A | Dilated cardiomyopathy 1A | 115200 | # |
| 9148 | c.394G>C | Substitution | p.Ala132Pro | Substitution | 1B | CMD1A | Dilated cardiomyopathy 1A | 115200 | # |
| 17029 | c.394G>C | Substitution | p.Ala132Pro | Substitution | 1B | CMD1A | Dilated cardiomyopathy 1A | 115200 | # |
| 17028 | c.394G>C | Substitution | p.Ala132Pro | Substitution | 1B | CMD1A | Dilated cardiomyopathy 1A | 115200 | # |
| 9297 | c.425_426insGGCACTGGA GGCTCTGCTGAA | Insertion | p.Leu141_Asn142insLys AspLeuAspAlaLeuLeu | Insertion | 1B | CMD1A | Dilated cardiomyopathy 1A | 115200 | # |
| 9010 | c.427T>C | Substitution | p.Ser143Pro | Substitution | 1B | CMD1A | Dilated cardiomyopathy 1A | 115200 | # |
| 11473 | c.427T>C | Substitution | p.Ser143Pro | Substitution | 1B | CMD1A | Dilated cardiomyopathy 1A | 115200 | # |
| 11474 | c.427T>C | Substitution | p.Ser143Pro | Substitution | 1B | CMD1A | Dilated cardiomyopathy 1A | 115200 | # |
| 11475 | c.427T>C | Substitution | p.Ser143Pro | Substitution | 1B | CMD1A | Dilated cardiomyopathy 1A | 115200 | # |
| 11476 | c.427T>C | Substitution | p.Ser143Pro | Substitution | 1B | CMD1A | Dilated cardiomyopathy 1A | 115200 | # |
| 11477 | c.427T>C | Substitution | p.Ser143Pro | Substitution | 1B | CMD1A | Dilated cardiomyopathy 1A | 115200 | # |
| 9150 | c.427T>C | Substitution | p.Ser143Pro | Substitution | 1B | CMD1A | Dilated cardiomyopathy 1A | 115200 | # |
| 16162 | c.427T>C | Substitution | p.Ser143Pro | Substitution | 1B | CMD1A | Dilated cardiomyopathy 1A | 115200 | # |
| 16163 | c.427T>C | Substitution | p.Ser143Pro | Substitution | 1B | CMD1A | Dilated cardiomyopathy 1A | 115200 | # |
| 16164 | c.427T>C | Substitution | p.Ser143Pro | Substitution | 1B | CMD1A | Dilated cardiomyopathy 1A | 115200 | # |
| 16165 | c.427T>C | Substitution | p.Ser143Pro | Substitution | 1B | CMD1A | Dilated cardiomyopathy 1A | 115200 | # |
| 17038 | c.427T>C | Substitution | p.Ser143Pro | Substitution | 1B | CMD1A | Dilated cardiomyopathy 1A | 115200 | # |
| 17037 | c.427T>C | Substitution | p.Ser143Pro | Substitution | 1B | CMD1A | Dilated cardiomyopathy 1A | 115200 | # |
| 17036 | c.427T>C | Substitution | p.Ser143Pro | Substitution | 1B | CMD1A | Dilated cardiomyopathy 1A | 115200 | # |
| 17024 | c.427T>C | Substitution | p.Ser143Pro | Substitution | 1B | CMD1A | Dilated cardiomyopathy 1A | 115200 | # |
| 17022 | c.427T>C | Substitution | p.Ser143Pro | Substitution | 1B | CMD1A | Dilated cardiomyopathy 1A | 115200 | # |
| 17021 | c.427T>C | Substitution | p.Ser143Pro | Substitution | 1B | CMD1A | Dilated cardiomyopathy 1A | 115200 | # |
| 17020 | c.427T>C | Substitution | p.Ser143Pro | Substitution | 1B | CMD1A | Dilated cardiomyopathy 1A | 115200 | # |
| 17019 | c.427T>C | Substitution | p.Ser143Pro | Substitution | 1B | CMD1A | Dilated cardiomyopathy 1A | 115200 | # |
| 17018 | c.427T>C | Substitution | p.Ser143Pro | Substitution | 1B | CMD1A | Dilated cardiomyopathy 1A | 115200 | # |
| 17023 | c.427T>C | Substitution | p.Ser143Pro | Substitution | 1B | CMD1A | Dilated cardiomyopathy 1A | 115200 | # |
| 17661 | c.448A>T | Substitution | p.Thr150Ser | Substitution | 1B | CMD1A | Dilated cardiomyopathy 1A | 115200 | # |
| 8879 | c.481G>A | Substitution | p.Glu161Lys | Substitution | 1B | CMD1A | Dilated cardiomyopathy 1A | 115200 | # |
| 13161 | c.481G>A | Substitution | p.Glu161Lys | Substitution | 1B | CMD1A | Dilated cardiomyopathy 1A | 115200 | # |
| 13162 | c.481G>A | Substitution | p.Glu161Lys | Substitution | 1B | CMD1A | Dilated cardiomyopathy 1A | 115200 | # |
| 13163 | c.481G>A | Substitution | p.Glu161Lys | Substitution | 1B | CMD1A | Dilated cardiomyopathy 1A | 115200 | # |
| 13328 | c.481G>A | Substitution | p.Glu161Lys | Substitution | 1B | CMD1A | Dilated cardiomyopathy 1A | 115200 | # |
| 13617 | c.481G>A | Substitution | p.Glu161Lys | Substitution | 1B | CMD1A | Dilated cardiomyopathy 1A | 115200 | # |
| 13091 | c.497G>C | Substitution | p.Arg166Pro | Substitution | 1B | CMD1A | Dilated cardiomyopathy 1A | 115200 | # |
| 16968 | c.497G>C | Substitution | p.Arg166Pro | Substitution | 1B | CMD1A | Dilated cardiomyopathy 1A | 115200 | # |
| 16969 | c.497G>C | Substitution | p.Arg166Pro | Substitution | 1B | CMD1A | Dilated cardiomyopathy 1A | 115200 | # |
| 13165 | c.548T>C | Substitution | p.Leu183Pro | Substitution | 1B | CMD1A | Dilated cardiomyopathy 1A | 115200 | # |
| 18267 | c.563T>G | Substitution | p.Leu188Arg | Substitution | 1B | CMD1A | Dilated cardiomyopathy 1A | 115200 | # |
| 16160 | c.565C>T | Substitution | p.Arg189Trp | Substitution | 1B | CMD1A | Dilated cardiomyopathy 1A | 115200 | # |
| 8845 | c.568C>T | Substitution | p.Arg190Trp | Substitution | 1B | CMD1A | Dilated cardiomyopathy 1A | 115200 | # |
| 9011 | c.568C>T | Substitution | p.Arg190Trp | Substitution | 1B | CMD1A | Dilated cardiomyopathy 1A | 115200 | # |
| 11507 | c.568C>T | Substitution | p.Arg190Trp | Substitution | 1B | CMD1A | Dilated cardiomyopathy 1A | 115200 | # |
| 9149 | c.568C>T | Substitution | p.Arg190Trp | Substitution | 1B | CMD1A | Dilated cardiomyopathy 1A | 115200 | # |
| 11621 | c.568C>T | Substitution | p.Arg190Trp | Substitution | 1B | CMD1A | Dilated cardiomyopathy 1A | 115200 | # |
| 11782 | c.568C>T | Substitution | p.Arg190Trp | Substitution | 1B | CMD1A | Dilated cardiomyopathy 1A | 115200 | # |
| 11790 | c.568C>T | Substitution | p.Arg190Trp | Substitution | 1B | CMD1A | Dilated cardiomyopathy 1A | 115200 | # |
| 13167 | c.568C>T | Substitution | p.Arg190Trp | Substitution | 1B | CMD1A | Dilated cardiomyopathy 1A | 115200 | # |
| 16167 | c.568C>T | Substitution | p.Arg190Trp | Substitution | 1B | CMD1A | Dilated cardiomyopathy 1A | 115200 | # |
| 17034 | c.568C>T | Substitution | p.Arg190Trp | Substitution | 1B | CMD1A | Dilated cardiomyopathy 1A | 115200 | # |
| 17033 | c.568C>T | Substitution | p.Arg190Trp | Substitution | 1B | CMD1A | Dilated cardiomyopathy 1A | 115200 | # |
| 17032 | c.568C>T | Substitution | p.Arg190Trp | Substitution | 1B | CMD1A | Dilated cardiomyopathy 1A | 115200 | # |
| 17031 | c.568C>T | Substitution | p.Arg190Trp | Substitution | 1B | CMD1A | Dilated cardiomyopathy 1A | 115200 | # |
| 13093 | c.569G>A | Substitution | p.Arg190Gln | Substitution | 1B | CMD1A | Dilated cardiomyopathy 1A | 115200 | # |
| 13330 | c.569G>A | Substitution | p.Arg190Gln | Substitution | 1B | CMD1A | Dilated cardiomyopathy 1A | 115200 | # |
| 16976 | c.569G>A | Substitution | p.Arg190Gln | Substitution | 1B | CMD1A | Dilated cardiomyopathy 1A | 115200 | # |
| 9165 | c.575A>G | Substitution | p.Asp192Gly | Substitution | 1B | CMD1A | Dilated cardiomyopathy 1A | 115200 | # |
| 14200 | c.575A>G | Substitution | p.Asp192Gly | Substitution | 1B | CMD1A | Dilated cardiomyopathy 1A | 115200 | # |
| 8750 | c.585C>G | Substitution | p.Asn195Lys | Substitution | 1B | CMD1A | Dilated cardiomyopathy 1A | 115200 | # |
| 12393 | c.585C>A | Substitution | p.Asn195Lys | Substitution | 1B | CMD1A | Dilated cardiomyopathy 1A | 115200 | # |
| 11668 | c.607G>A | Substitution | p.Glu203Lys | Substitution | 1B | CMD1A | Dilated cardiomyopathy 1A | 115200 | # |
| 16978 | c.607G>A | Substitution | p.Glu203Lys | Substitution | 1B | CMD1A | Dilated cardiomyopathy 1A | 115200 | # |
| 8751 | c.608A>G | Substitution | p.Glu203Gly | Substitution | 1B | CMD1A | Dilated cardiomyopathy 1A | 115200 | # |
| 13332 | c.608A>T | Substitution | p.Glu203Val | Substitution | 1B | CMD1A | Dilated cardiomyopathy 1A | 115200 | # |
| 13095 | c.629T>G | Substitution | p.lle210Ser | Substitution | 1B | CMD1A | Dilated cardiomyopathy 1A | 115200 | # |
| 16971 | c.629T>G | Substitution | p.lle210Ser | Substitution | 1B | CMD1A | Dilated cardiomyopathy 1A | 115200 | # |
| 8901 | c.640-10A>G | Substitution | p.? | Unknown | Unknown | CMD1A | Dilated cardiomyopathy 1A | 115200 | # |
| 8864 | c.644T>C | Substitution | p.Leu215Pro | Substitution | 1B | CMD1A | Dilated cardiomyopathy 1A | 115200 | # |
| 16980 | c.644T>C | Substitution | p.Leu215Pro | Substitution | 1B | CMD1A | Dilated cardiomyopathy 1A | 115200 | # |
| 13169 | c.656A>C | Substitution | p.Lys219Thr | Substitution | 1B | CMD1A | Dilated cardiomyopathy 1A | 115200 | # |
| 13335 | c.656A>C | Substitution | p.Lys219Thr | Substitution | 1B | CMD1A | Dilated cardiomyopathy 1A | 115200 | # |
| 11669 | c.673C>T | Substitution | p.Arg225X | Substitution | L12 | CMD1A | Dilated cardiomyopathy 1A | 115200 | # |
| 17607 | c.673C>T | Substitution | p.Arg225X | Substitution | L12 | CMD1A | Dilated cardiomyopathy 1A | 115200 | # |
| 17609 | c.673C>T | Substitution | p.Arg225X | Substitution | L12 | CMD1A | Dilated cardiomyopathy 1A | 115200 | # |
| 13097 | c.700C>T | Substitution | p.Gln234X | Substitution | L12 | CMD1A | Dilated cardiomyopathy 1A | 115200 | # |
| 13201 | c.736C>T | Substitution | p.Gln246X | Substitution | 2A | CMD1A | Dilated cardiomyopathy 1A | 115200 | # |
| 17786 | c.736C>T | Substitution | p.Gln246X | Substitution | 2A | CMD1A | Dilated cardiomyopathy 1A | 115200 | # |
| 17779 | c.767T>G | Substitution | p.Val256Gly | Substitution | 2A | CMD1A | Dilated cardiomyopathy 1A | 115200 | # |
| 11809 | c.800A>G | Substitution | p.Tyr267Cys | Substitution | 2B | CMD1A | Dilated cardiomyopathy 1A | 115200 | # |
| 13173 | c.800A>G | Substitution | p.Tyr267Cys | Substitution | 2B | CMD1A | Dilated cardiomyopathy 1A | 115200 | # |
| 11810 | c.855delG | Deletion | p.Ala287LeufsX191 | Frame shift | 2B | CMD1A | Dilated cardiomyopathy 1A | 115200 | # |
| 11613 | c.908_909delCT | Deletion | p.Ser303CysfsX26 | Frame shift | 2B | CMD1A | Dilated cardiomyopathy 1A | 115200 | # |
| 12384 | c.908_909delCT | Deletion | p.Ser303CysfsX26 | Frame shift | 2B | CMD1A | Dilated cardiomyopathy 1A | 115200 | # |
| 13206 | c.936+1G>T | Substitution | p.? | Unknown | Unknown | CMD1A | Dilated cardiomyopathy 1A | 115200 | # |
| 16805 | c.937-11C>G | Substitution | p.Leu313GlyfsX31 | Frame shift | 2B | CMD1A | Dilated cardiomyopathy 1A | 115200 | # |
| 8846 | c.949G>A | Substitution | p.Glu317Lys | Substitution | 2B | CMD1A | Dilated cardiomyopathy 1A | 115200 | # |
| 11617 | c.949G>A | Substitution | p.Glu317Lys | Substitution | 2B | CMD1A | Dilated cardiomyopathy 1A | 115200 | # |
| 13175 | c.949G>A | Substitution | p.Glu317Lys | Substitution | 2B | CMD1A | Dilated cardiomyopathy 1A | 115200 | # |
| 13176 | c.949G>A | Substitution | p.Glu317Lys | Substitution | 2B | CMD1A | Dilated cardiomyopathy 1A | 115200 | # |
| 13621 | c.949G>A | Substitution | p.Glu317Lys | Substitution | 2B | CMD1A | Dilated cardiomyopathy 1A | 115200 | # |
| 13100 | c.952G>A | Substitution | p.Ala318Thr | Substitution | 2B | CMD1A | Dilated cardiomyopathy 1A | 115200 | # |
| 16956 | c.952G>A | Substitution | p.Ala318Thr | Substitution | 2B | CMD1A | Dilated cardiomyopathy 1A | 115200 | # |
| 11700 | c.959delT | Deletion | p.Leu320fsX160 | Frame shift | 2B | CMD1A | Dilated cardiomyopathy 1A | 115200 | # |
| 9409 | c.959delT | Deletion | p.Leu320fsX160 | Frame shift | 2B | CMD1A | Dilated cardiomyopathy 1A | 115200 | # |
| 9391 | c.959delT | Deletion | p.Leu320fsX160 | Frame shift | 2B | CMD1A | Dilated cardiomyopathy 1A | 115200 | # |
| 14032 | c.959delT | Deletion | p.Leu320fsX160 | Frame shift | 2B | CMD1A | Dilated cardiomyopathy 1A | 115200 | # |
| **12389** | **c.961C>T** | **Substitution** | **p.Arg321X** | **Substitution** | **2B** | **CMD1A** | **Dilated cardiomyopathy 1A** | **115200** | **#** |
| **14077** | **c.961C>T** | **Substitution** | **p.Arg321X** | **Substitution** | **2B** | **CMD1A** | **Dilated cardiomyopathy 1A** | **115200** | **#** |
| **14093** | **c.961C>T** | **Substitution** | **p.Arg321X** | **Substitution** | **2B** | **CMD1A** | **Dilated cardiomyopathy 1A** | **115200** | **#** |
| **17737** | **c.961C>T** | **Substitution** | **p.Arg321X** | **Substitution** | **2B** | **CMD1A** | **Dilated cardiomyopathy 1A** | **115200** | **#** |
| **17738** | **c.961C>T** | **Substitution** | **p.Arg321X** | **Substitution** | **2B** | **CMD1A** | **Dilated cardiomyopathy 1A** | **115200** | **#** |
| **9155** | **c.976T>A** | **Substitution** | **p.Ser326Thr** | **Substitution** | **2B** | **CMD1A** | **Dilated cardiomyopathy 1A** | **115200** | **#** |
| **11760** | c.[992G>A; =]+[=; 1039G>A] | **Substitution** | p.[Arg331Glu; =]+[=; Glu347Lys] | **Substitution** | **2B** | **CMD1A** | **Dilated cardiomyopathy 1A** | **115200** | **#** |
| **11811** | **c.992G>C** | **Substitution** | **p.Arg331Pro** | **Substitution** | **2B** | **CMD1A** | **Dilated cardiomyopathy 1A** | **115200** | **#** |
| **14075** | **c.992G>A** | **Substitution** | **p.Arg331Gln** | **Substitution** | **2B** | **CMD1A** | **Dilated cardiomyopathy 1A** | **115200** | **#** |
| **17477** | **c.1003C>T** | **Substitution** | **p.Arg335Trp** | **Substitution** | **2B** | **CMD1A** | **Dilated cardiomyopathy 1A** | **115200** | **#** |
| **18486** | **c.1039G>A** | **Substitution** | **p.Glu347Lys** | **Substitution** | **2B** | **CMD1A** | **Dilated cardiomyopathy 1A** | **115200** | **#** |
| **9012** | **c.1046G>T** | **Substitution** | **p.Arg349Leu** | **Substitution** | **2B** | **CMD1A** | **Dilated cardiomyopathy 1A** | **115200** | **#** |
| **13561** | **c.1048G>C** | **Substitution** | **p.Ala350Pro** | **Substitution** | **2B** | **CMD1A** | **Dilated cardiomyopathy 1A** | **115200** | **#** |
| **14204** | **c.1057C>A** | **Substitution** | **p.Gln353Lys** | **Substitution** | **2B** | **CMD1A** | **Dilated cardiomyopathy 1A** | **115200** | **#** |
| **14072** | **c.1063C>T** | **Substitution** | **p.Gln355X** | **Substitution** | **2B** | **CMD1A** | **Dilated cardiomyopathy 1A** | **115200** | **#** |
| **17475** | **c.1070A>C** | **Substitution** | **p.Asp357Ala** | **Substitution** | **2B** | **CMD1A** | **Dilated cardiomyopathy 1A** | **115200** | **#** |
| **13116** | **c.1072G>T** | **Substitution** | **p.Glu358X** | **Substitution** | **2B** | **CMD1A** | **Dilated cardiomyopathy 1A** | **115200** | **#** |
| **9152** | **c.1085_1085delT** | **Deletion** | **p.Leu363TrpfsX117** | **Frame shift** | **2B** | **CMD1A** | **Dilated cardiomyopathy 1A** | **115200** | **#** |
| **17026** | **c.1085_1085delT** | **Deletion** | **p.Leu362TrpfsX117** | **Frame shift** | **2B** | **CMD1A** | **Dilated cardiomyopathy 1A** | **115200** | **#** |
| **16157** | c.1102_1130dupGCCCTGG ACATGGAGATCCACGCCT ACCG | **Duplication** | **p.Lys378ProfsX112** | **Frame shift** | **2B** | **CMD1A** | **Dilated cardiomyopathy 1A** | **115200** | **#** |
| **13102** | **c.1114delG** | **Deletion** | **p.Glu372ArgfsX107** | **Frame shift** | **2B** | **CMD1A** | **Dilated cardiomyopathy 1A** | **115200** | **#** |
| **8866** | **c.1130G>A** | **Substitution** | **p.Arg377His** | **Substitution** | **2B** | **CMD1A** | **Dilated cardiomyopathy 1A** | **115200** | **#** |
| **8869** | **c.1130G>A** | **Substitution** | **p.Arg377His** | **Substitution** | **2B** | **CMD1A** | **Dilated cardiomyopathy 1A** | **115200** | **#** |
| **8880** | **c.1130G>A** | **Substitution** | **p.Arg377His** | **Substitution** | **2B** | **CMD1A** | **Dilated cardiomyopathy 1A** | **115200** | **#** |
| **9160** | **c.1130G>A** | **Substitution** | **p.Arg377His** | **Substitution** | **2B** | **CMD1A** | **Dilated cardiomyopathy 1A** | **115200** | **#** |
| **9162** | **c.1130G>A** | **Substitution** | **p.Arg377His** | **Substitution** | **2B** | **CMD1A** | **Dilated cardiomyopathy 1A** | **115200** | **#** |
| **12330** | **c.1130G>A** | **Substitution** | **p.Arg377His** | **Substitution** | **2B** | **CMD1A** | **Dilated cardiomyopathy 1A** | **115200** | **#** |
| **13178** | **c.1130G>T** | **Substitution** | **p.Arg377Leu** | **Substitution** | **2B** | **CMD1A** | **Dilated cardiomyopathy 1A** | **115200** | **#** |
| 13623 | c.1130G>A | Substitution | p.Arg377His | Substitution | 2B | CMD1A | Dilated cardiomyopathy 1A | 115200 | # |
| 14034 | c.1130G>A | Substitution | p.Arg377His | Substitution | 2B | CMD1A | Dilated cardiomyopathy 1A | 115200 | # |
| 17664 | c.1150G>T | Substitution | p.Glu384X | Substitution | 2B | CMD1A | Dilated cardiomyopathy 1A | 115200 | # |
| 17482 | c.1157+1G>T | Substitution | p.Arg386SerfsX21 | Frame shift | 2B | CMD1A | Dilated cardiomyopathy 1A | 115200 | # |
| 13104 | c.1163G>A | Substitution | p.Arg388His | Substitution | Tail | CMD1A | Dilated cardiomyopathy 1A | 115200 | # |
| 16958 | c.1163G>A | Substitution | p.Arg388His | Substitution | Tail | CMD1A | Dilated cardiomyopathy 1A | 115200 | # |
| 13106 | c.1195C>T | Substitution | p.Arg399Cys | Substitution | Tail | CMD1A | Dilated cardiomyopathy 1A | 115200 | # |
| 16960 | c.1195C>T | Substitution | p.Arg399Cys | Substitution | Tail | CMD1A | Dilated cardiomyopathy 1A | 115200 | # |
| 17782 | c.1197_1240delTGGCCGTG CTTCCTCTCACTCATCCCA GACACAGGGTGGGGGCA | Deletion | p.Gly400ArgfsX11 | Frame shift | Tail | CMD1A | Dilated cardiomyopathy 1A | 115200 | # |
| 17789 | c.1292C>G | Substitution | p.Ser431X | Substitution | Tail | CMD1A | Dilated cardiomyopathy 1A | 115200 | # |
| 14082 | c.1294C>T | Substitution | **p.Gln432X** | Substitution | Tail | CMD1A | Dilated cardiomyopathy 1A | 115200 | # |
| 14084 | c.1294C>T | Substitution | **p.Gln432X** | Substitution | Tail | CMD1A | Dilated cardiomyopathy 1A | 115200 | # |
| 14085 | c.1294C>T | Substitution | **p.Gln432X** | Substitution | Tail | CMD1A | Dilated cardiomyopathy 1A | 115200 | # |
| 14086 | c.1294C>T | Substitution | **p.Gln432X** | Substitution | Tail | CMD1A | Dilated cardiomyopathy 1A | 115200 | # |
| 13108 | c.1307_1308insGCAC | Insertion | p.Ser437HisfsX1 | Frame shift | Tail | CMD1A | Dilated cardiomyopathy 1A | 115200 | # |
| 16962 | c.1307_1308insGCAC | Insertion | p.Ser437HisfsX1 | Frame shift | Tail | CMD1A | Dilated cardiomyopathy 1A | 115200 | # |
| 14088 | c.1318G>A | Substitution | **p.Val440Met** | Substitution | Tail | CMD1A | Dilated cardiomyopathy 1A | 115200 | # |
| 14090 | c.1318G>A | Substitution | **p.Val440Met** | Substitution | Tail | CMD1A | Dilated cardiomyopathy 1A | 115200 | # |
| 14091 | c.1318G>A | Substitution | **p.Val440Met** | Substitution | Tail | CMD1A | Dilated cardiomyopathy 1A | 115200 | # |
| 11812 | **c.1370delA** | Deletion | p.Lys457SerfsX21 | Frame shift | Tail | CMD1A | Dilated cardiomyopathy 1A | 115200 | # |
| 12397 | c.1380+1G>A | Substitution | **p.?** | Unknown | Unknown | CMD1A | Dilated cardiomyopathy 1A | 115200 | # |
| 9413 | **c.1397_1397delA** | Deletion | p.Asn466HefsX14 | Frame shift | Tail | CMD1A | Dilated cardiomyopathy 1A | 115200 | # |
| 9161 | **c.1397_1397delA** | Deletion | p.Asn466HefsX14 | Frame shift | Tail | CMD1A | Dilated cardiomyopathy 1A | 115200 | # |
| 13110 | c.1412G>A | Substitution | p.Arg471His | Substitution | Tail | CMD1A | Dilated cardiomyopathy 1A | 115200 | # |
| 16973 | c.1412G>A | Substitution | p.Arg471His | Substitution | Tail | CMD1A | Dilated cardiomyopathy 1A | 115200 | # |
| 13112 | c.1424_1425insAGA | Insertion | p.Gly474_Asp475insGlu | Insertion | Tail | CMD1A | Dilated cardiomyopathy 1A | 115200 | # |
| 11508 | c.1443C>G | Substitution | p.Tyr481X | Substitution | Tail | CMD1A | Dilated cardiomyopathy 1A | 115200 | # |
| 17791 | c.1443C>G | Substitution | p.Tyr481X | Substitution | Tail | CMD1A | Dilated cardiomyopathy 1A | 115200 | # |
| 17480 | **c.1489-1G>T** | Substitution | **p.Ile497_Glu536del** | Deletion | Tail | CMD1A | Dilated cardiomyopathy 1A | 115200 | # |
| 13180 | c.1492T>A | Substitution | p.Trp498Arg | Substitution | Tail | CMD1A | Dilated cardiomyopathy 1A | 115200 | # |
| 9153 | **c.1493_1493delG** | Deletion | p.Ala499LeufsX47 | Frame shift | Tail | CMD1A | Dilated cardiomyopathy 1A | 115200 | # |
| 12395 | c.1512_1513insAG | Insertion | p.Th r505ArgfsX44 | Frame shift | Tail | CMD1A | Dilated cardiomyopathy 1A | 115200 | # |
| 17784 | c.1526_1527insC | Insertion | p.Thr510TyrfsX42 | Frame shift | Tail | CMD1A | Dilated cardiomyopathy 1A | 115200 | # |
| 17797 | c.1526_1527insA | Insertion | p.Thr510TyrfsX42 | Frame shift | Tail | CMD1A | Dilated cardiomyopathy 1A | 115200 | # |
| 17486 | c.1549C>T | Substitution | **p.Gln517X** | Substitution | Tail | CMD1A | Dilated cardiomyopathy 1A | 115200 | # |
| 17484 | c.1560G>A | Substitution | p.Trp520X | Substitution | Tail | CMD1A | Dilated cardiomyopathy 1A | 115200 | # |
| 13625 | c.1567G>A | Substitution | p.Gly523Arg | Substitution | Tail | CMD1A | Dilated cardiomyopathy 1A | 115200 | # |
| 11624 | c.1579_1580insCTGC | Insertion | p.Arg527ProfsX26 | Frame shift | Tail | CMD1A | Dilated cardiomyopathy 1A | 115200 | # |
| 17558 | c.1583C>T | Substitution | p.Thr528Met | Substitution | Tail | CMD1A | Dilated cardiomyopathy 1A | 115200 | # |
| 9307 | c.1621C>T | Substitution | p.Arg541Cys | Substitution | Tail | CMD1A | Dilated cardiomyopathy 1A | 115200 | # |
| 9283 | c.1621C>A | Substitution | p.Arg541Ser | Substitution | Tail | CMD1A | Dilated cardiomyopathy 1A | 115200 | # |
| 9151 | c.1621C>A | Substitution | p.Arg541Ser | Substitution | Tail | CMD1A | Dilated cardiomyopathy 1A | 115200 | # |
| 13592 | c.1621C>T | Substitution | p.Arg541Cys | Substitution | Tail | CMD1A | Dilated cardiomyopathy 1A | 115200 | # |
| 14202 | c.1621C>A | Substitution | p.Arg541Ser | Substitution | Tail | CMD1A | Dilated cardiomyopathy 1A | 115200 | # |
| 16472 | c.1621C>G | Substitution | p.Arg541Gly | Substitution | Tail | CMD1A | Dilated cardiomyopathy 1A | 115200 | # |
| 17774 | c.1621C>T | Substitution | p.Arg541Cys | Substitution | Tail | CMD1A | Dilated cardiomyopathy 1A | 115200 | # |
| 17775 | c.1621C>T | Substitution | p.Arg541Cys | Substitution | Tail | CMD1A | Dilated cardiomyopathy 1A | 115200 | # |
| 13559 | c.1622G>A | Substitution | p.Arg541His | Substitution | Tail | CMD1A | Dilated cardiomyopathy 1A | 115200 | # |
| 9392 | c.1718C>T | Substitution | p.Ser573Leu | Substitution | Tail | CMD1A | Dilated cardiomyopathy 1A | 115200 | # |
| 13182 | c.1718C>T | Substitution | p.Ser573Leu | Substitution | Tail | CMD1A | Dilated cardiomyopathy 1A | 115200 | # |
| 13183 | c.1718C>T | Substitution | p.Ser573Leu | Substitution | Tail | CMD1A | Dilated cardiomyopathy 1A | 115200 | # |
| 14038 | c.1718C>T | Substitution | p.Ser573Leu | Substitution | Tail | CMD1A | Dilated cardiomyopathy 1A | 115200 | # |
| 17667 | c.1879C>T | Substitution | p.Arg624Cys | Substitution | Tail | CMD1A | Dilated cardiomyopathy 1A | 115200 | # |
| 13565 | c.1904G>A | Substitution | p.Gly635Asp | Substitution | Tail | CMD1A | Dilated cardiomyopathy 1A | 115200 | # |
| 8833 | c.1930C>T | Substitution | p.Arg644Cys | Substitution | Tail | CMD1A | Dilated cardiomyopathy 1A | 115200 | # |
| 9018 | c.1930C>T | Substitution | p.Arg644Cys | Substitution | Tail | CMD1A | Dilated cardiomyopathy 1A | 115200 | # |
| 11500 | c.1930C>T | Substitution | p.Arg644Cys | Substitution | Tail | CMD1A | Dilated cardiomyopathy 1A | 115200 | # |
| 11501 | c.1930C>T | Substitution | p.Arg644Cys | Substitution | Tail | CMD1A | Dilated cardiomyopathy 1A | 115200 | # |
| 11502 | c.1930C>A | Substitution | p.Arg644His | Substitution | Tail | CMD1A | Dilated cardiomyopathy 1A | 115200 | # |
| 13185 | c.1930C>T | Substitution | p.Arg644Cys | Substitution | Tail | CMD1A | Dilated cardiomyopathy 1A | 115200 | # |
| 13337 | c.1930C>T | Substitution | p.Arg644Cys | Substitution | Tail | CMD1A | Dilated cardiomyopathy 1A | 115200 | # |
| 14080 | c.1930C>T | Substitution | p.Arg644Cys | Substitution | Tail | CMD1A | Dilated cardiomyopathy 1A | 115200 | # |
| 13114 | c.1960C>T | Substitution | p.Arg654X | Substitution | Tail | CMD1A | Dilated cardiomyopathy 1A | 115200 | # |
| 16964 | c.1960C>T | Substitution | p.Arg654X | Substitution | Tail | CMD1A | Dilated cardiomyopathy 1A | 115200 | # |
| 11788 | c.? | Unknown | p.Tyr481X | Substitution | Tail | DCM-CD | Dilated cardiomyopathy with conduction **system** defects | - | - |
| 17886 | c.80C>T | Substitution | p. Th r2711e | Substitution | Head | DCM-CD | Dilated cardiomyopathy with conduction system defects | - | - |
| 13486 | c.106C>T | Substitution | p.Gln36X | Substitution | 1A | DCM-CD | Dilated cardiomyopathy with conduction system defects | - | - |
| 11792 | c.158A>T | Substitution | p.Glu53Val | Substitution | 1A | DCM-CD | Dilated cardiomyopathy with conduction system defects | - | - |
| 18301 | c.175C>G | Substitution | p. Leu59Val | Substitution | 1A | DCM-CD | Dilated cardiomyopathy with conduction system defects | - | - |
| 11791 | c.481G>A | Substitution | p.Glu161Lys | Substitution | 1B | DCM-CD | Dilated cardiomyopathy with conduction system defects | - | - |
| 13204 | c.514-1G>A | Substitution | p.? | Unknown | Unknown | DCM-CD | Dilated cardiomyopathy with conduction system defects | - | - |
| 11793 | c.556G>A | Substitution | p.Glu186Lys | Substitution | 1B | DCM-CD | Dilated cardiomyopathy with conduction system defects | - | - |
| 11789 | c.568C>T | Substitution | p.Arg190Trp | Substitution | 1B | DCM-CD | Dilated cardiomyopathy with conduction system defects | - | - |
| 11787 | c.575A>G | Substitution | p.Asp192Gly | Substitution | 1B | DCM-CD | Dilated cardiomyopathy with conduction system defects | - | - |
| 18470 | c.683A>T | Substitution | p.Glu228Val | Substitution | L12 | DCM-CD | Dilated cardiomyopathy with conduction system defects | - | - |
| 17868 | c.871G>A | Substitution | p.Glu291Lys | Substitution | 2B | DCM-CD | Dilated cardiomyopathy with conduction system defects | - | - |
| 17870 | c.949G>A | Substitution | p.Glu317Lys | Substitution | 2B | DCM-CD | Dilated cardiomyopathy with conduction system defects | - | - |
| 13078 | c.1069G>C | Substitution | p.Asp357His | Substitution | 2B | DCM-CD | Dilated cardiomyopathy with conduction system defects | - | - |
| 13208 | c.1157+1G>A | Substitution | p.? | Unknown | Unknown | DCM-CD | Dilated cardiomyopathy with conduction system defects | - | - |
| 18490 | c.1412G>A | Substitution | p.Arg471His | Substitution | Tail | DCM-CD | Dilated cardiomyopathy with conduction system defects | - | - |
| 11814 | c.1526_1527insC | Insertion | p.Thr510TyrfsX42 | Frame shift | Tail | DCM-CD | Dilated cardiomyopathy with conduction system defects | - | - |
| 9008 | c.1621C>T | Substitution | p.Arg541Cys | Substitution | Tail | DCM-CD | Dilated cardiomyopathy with conduction system defects | - | - |
| 17578 | c.1711C>A | Substitution | p.= | Silent | Not affected | DCM-CD | Dilated cardiomyopathy with conduction system defects | - | - |
| 17880 | c.1930C>T | Substitution | p.Arg644Cys | Substitution | Tail | DCM-CD | Dilated cardiomyopathy with conduction system defects | - | - |
| 18305 | c.1774G>A | Substitution | p.Gly592Arg | Substitution | Tail | DAPJ | Distal acroosteolysis, poikiloderma and joint stiffness | - | - |
| 13581 | c.1930C>T | Substitution | p.Arg644Cys | Substitution | Tail | - | Distal motor neuropathy | - | - |
| 17813 | c.93G>C | Substitution | p.Glu31Asp | Substitution | Head | - | Dropped head syndrome | - | - |
| 9020 | c.94_96delAAG | Deletion | p.Lys32del | Deletion | 1A | - | Dropped head syndrome | - | - |
| 13476 | c.116A>G | Substitution | p.Asn39Ser | Substitution | 1A | - | Dropped head syndrome | - | - |
| 13466 | c.149G>C | Substitution | p.Arg50Pro | Substitution | 1A | - | Dropped head syndrome | - | - |
| 13464 | c.745C>T | Substitution | p.Arg249Trp | Substitution | 2A | - | Dropped head syndrome | - | - |
| 13470 | c.905T>C | Substitution | p.Leu302Pro | Substitution | 2B | - | Dropped head syndrome | - | - |
| 13468 | c.1072G>A | Substitution | p.Glu358Lys | Substitution | 2B | - | Dropped head syndrome | - | - |
| 13482 | c.1072G>A | Substitution | p.Glu358Lys | Substitution | 2B | - | Dropped head syndrome | - | - |
| 13484 | c.1072G>A | Substitution | p.Glu358Lys | Substitution | 2B | - | Dropped head syndrome | - | - |
| 13474 | c.1358G>C | Substitution | p.Arg453Pro | Substitution | Tail | - | Dropped head syndrome | - | - |
| 13472 | c.1364G>C | Substitution | p.Arg455Pro | Substitution | Tail | - | Dropped head syndrome | - | - |
| 13478 | c.1366A>G | Substitution | p.Asn456Asp | Substitution | Tail | - | Dropped head syndrome | - | - |
| 13480 | c.1381-2A>G | Substitution | p.? | Unknown | Unknown | - | Dropped head syndrome | - | - |
| 11652 | c.- 3_12delGCCATGGAGACCC CG | Deletion | p.Met1_Pro4del | Deletion | Head | EDMD2 | Emery-Dreifuss muscular dystrophy, autosomal dominant | 181350 | # |
| 8743 | c.16C>T | Substitution | p.Gln6X | Substitution | Head | EDMD2 | Emery-Dreifuss muscular dystrophy, autosomal dominant | 181350 | # |
| 8796 | c.16C>T | Substitution | p.Gln6X | Substitution | Head | EDMD2 | Emery-Dreifuss muscular dystrophy, autosomal dominant | 181350 | # |
| 11746 | c.31delC | Deletion | p.Arg11AlafsX85 | Frame shift | Head | EDMD2 | Emery-Dreifuss muscular dystrophy, autosomal dominant | 181350 | # |
| 9262 | c.73C>G | Substitution | p.Arg25Gly | Substitution | Head | EDMD2 | Emery-Dreifuss muscular dystrophy, autosomal dominant | 181350 | # |
| 9376 | c.74G>C | Substitution | p.Arg25Pro | Substitution | Head | EDMD2 | Emery-Dreifuss muscular dystrophy, autosomal dominant | 181350 | # |
| 9177 | c.94_96delAAG | Deletion | p.Lys32del | Deletion | 1A | EDMD2 | Emery-Dreifuss muscular dystrophy, autosomal dominant | 181350 | # |
| 8998 | c.94_96delAAG | Deletion | p.Lys32del | Deletion | 1A | EDMD2 | Emery-Dreifuss muscular dystrophy, autosomal dominant | 181350 | # |
| 9014 | c.94_96delAAG | Deletion | p.Lys32del | Deletion | 1A | EDMD2 | Emery-Dreifuss muscular dystrophy, autosomal dominant | 181350 | # |
| 13594 | c.98A>G | Substitution | p.Glu33Gly | Substitution | 1A | EDMD2 | Emery-Dreifuss muscular dystrophy, autosomal dominant | 181350 | # |
| 11747 | c.99G>C | Substitution | p.Glu33Asp | Substitution | 1A | EDMD2 | Emery-Dreifuss muscular dystrophy, autosomal dominant | 181350 | # |
| 8999 | c.103C>G | Substitution | p.Leu35Val | Substitution | 1A | EDMD2 | Emery-Dreifuss muscular dystrophy, autosomal dominant | 181350 | # |
| 11748 | c.116A>G | Substitution | p.Asn39Ser | Substitution | 1A | EDMD2 | Emery-Dreifuss muscular dystrophy, autosomal dominant | 181350 | # |
| 16329 | c.116A>G | Substitution | p.Asn39Ser | Substitution | 1A | EDMD2 | Emery-Dreifuss muscular dystrophy, autosomal dominant | 181350 | # |
| 18174 | c.116A>G | Substitution | p.Asn39Ser | Substitution | 1A | EDMD2 | Emery-Dreifuss muscular dystrophy, autosomal dominant | 181350 | # |
| 9377 | c.127G>A | Substitution | p.Ala43Thr | Substitution | 1A | EDMD2 | Emery-Dreifuss muscular dystrophy, autosomal dominant | 181350 | # |
| 8797 | c.134A>G | Substitution | p.Tyr45Cys | Substitution | 1A | EDMD2 | Emery-Dreifuss muscular dystrophy, autosomal dominant | 181350 | # |
| 16331 | c.134A>G | Substitution | p.Tyr45Cys | Substitution | 1A | EDMD2 | Emery-Dreifuss muscular dystrophy, autosomal dominant | 181350 | # |
| 13605 | c.136A>G | Substitution | p.Ile46Val | Substitution | 1A | EDMD2 | Emery-Dreifuss muscular dystrophy, autosomal dominant | 181350 | # |
| 13545 | c.139G>C | Substitution | p.Asp47His | Substitution | 1A | EDMD2 | Emery-Dreifuss muscular dystrophy, autosomal dominant | 181350 | # |
| 9378 | c.148C>A | Substitution | p.Arg50Ser | Substitution | 1A | EDMD2 | Emery-Dreifuss muscular dystrophy, autosomal dominant | 181350 | # |
| 8798 | c.149G>C | Substitution | p.Arg50Pro | Substitution | 1A | EDMD2 | Emery-Dreifuss muscular dystrophy, autosomal dominant | 181350 | # |
| 8799 | c.188T>G | Substitution | p.Ile63Ser | Substitution | 1A | EDMD2 | Emery-Dreifuss muscular dystrophy, autosomal dominant | 181350 | # |
| 9175 | c.188T>A | Substitution | p.Ile63Asn | Substitution | 1A | EDMD2 | Emery-Dreifuss muscular dystrophy, autosomal dominant | 181350 | # |
| 11633 | c.188T>A | Substitution | p.lle63Asn | Substitution | 1A | EDMD2 | Emery-Dreifuss muscular dystrophy, autosomal dominant | 181350 | # |
| 13192 | c.203_208delAGGTGG | Deletion | **p.Glu68_Val69del** | Deletion | 1A | EDMD2 | Emery-Dreifuss muscular dystrophy, autosomal dominant | 181350 | # |
| 12477 | c.265C>T | Substitution | p.Arg89Cys | Substitution | 1B | EDMD2 | Emery-Dreifuss muscular dystrophy, autosomal dominant | 181350 | # |
| 13596 | c.265C>T | Substitution | p.Arg89Cys | Substitution | 1B | EDMD2 | Emery-Dreifuss muscular dystrophy, autosomal dominant | 181350 | # |
| 17193 | c.266G>T | Substitution | p.Arg89Leu | Substitution | 1B | EDMD2 | Emery-Dreifuss muscular dystrophy, autosomal dominant | 181350 | # |
| 9410 | c.334_336delGAG | Deletion | p.Glu112del | Deletion | 1B | EDMD2 | Emery-Dreifuss muscular dystrophy, autosomal dominant | 181350 | # |
| 9000 | c.334_336delGAG | Deletion | p.Glu112del | Deletion | 1B | EDMD2 | Emery-Dreifuss muscular dystrophy, autosomal dominant | 181350 | # |
| 16291 | c.357C>T | Substitution | **p.=** | Silent | Not affected | EDMD2 | Emery-Dreifuss muscular dystrophy, autosomal dominant | 181350 | # |
| 17182 | c.367_369delAAG | Deletion | p.Lys123del | Deletion | 1B | EDMD2 | Emery-Dreifuss muscular dystrophy, autosomal dominant | 181350 | # |
| 9379 | c.398G>C | Substitution | p.Arg133Pro | Substitution | 1B | EDMD2 | Emery-Dreifuss muscular dystrophy, autosomal dominant | 181350 | # |
| 11632 | c.419T>C | Substitution | p.Leu140Pro | Substitution | 1B | EDMD2 | Emery-Dreifuss muscular dystrophy, autosomal dominant | 181350 | # |
| 9015 | c.428C>T | Substitution | p.Ser143Phe | Substitution | 1B | EDMD2 | Emery-Dreifuss muscular dystrophy, autosomal dominant | 181350 | # |
| 12609 | c.428C>T | Substitution | p.Ser143Phe | Substitution | 1B | EDMD2 | Emery-Dreifuss muscular dystrophy, autosomal dominant | 181350 | # |
| 8795 | c.448A>C | Substitution | p.Thr150Pro | Substitution | 1B | EDMD2 | Emery-Dreifuss muscular dystrophy, autosomal dominant | 181350 | # |
| 16334 | c.448A>C | Substitution | p.Thr150Pro | Substitution | 1B | EDMD2 | Emery-Dreifuss muscular dystrophy, autosomal dominant | 181350 | # |
| 13453 | c.485T>C | Substitution | p.Leu162Pro | Substitution | 1B | EDMD2 | Emery-Dreifuss muscular dystrophy, autosomal dominant | 181350 | # |
| 16396 | **c.566_567delGGinsCC** | Indel | p.Arg189Pro | Substitution | 1B | EDMD2 | Emery-Dreifuss muscular dystrophy, autosomal dominant | 181350 | # |
| 16299 | c.568_570dupCGG | Duplication | p.Arg190dup | Duplication | 1B | EDMD2 | Emery-Dreifuss muscular dystrophy, autosomal dominant | 181350 | # |
| 9380 | c.588_596delGCTGCAGAC | Deletion | **p.Arg196_Thr199del**insS er | Indel | 1B | EDMD2 | Emery-Dreifuss muscular dystrophy, autosomal dominant | 181350 | # |
| 16301 | c.618C>G | Substitution | p.Phe206Leu | Substitution | 1B | EDMD2 | Emery-Dreifuss muscular dystrophy, autosomal dominant | 181350 | # |
| 12382 | **c.625delA** | Deletion | p.Asn209ThrfsX271 | Frame shift | 1B | EDMD2 | Emery-Dreifuss muscular dystrophy, autosomal dominant | 181350 | # |
| 8800 | c.665A>C | Substitution | p.His222Pro | Substitution | L12 | EDMD2 | Emery-Dreifuss muscular dystrophy, autosomal dominant | 181350 | # |
| 13547 | c.694G>C | Substitution | p.Gly232Arg | Substitution | L12 | EDMD2 | Emery-Dreifuss muscular dystrophy, autosomal dominant | 181350 | # |
| 8801 | c.695G>A | Substitution | p.Gly232Glu | Substitution | L12 | EDMD2 | Emery-Dreifuss muscular dystrophy, autosomal dominant | 181350 | # |
| 12485 | c.695G>A | Substitution | p.Gly232Glu | Substitution | L12 | EDMD2 | Emery-Dreifuss muscular dystrophy, autosomal dominant | 181350 | # |
| 9001 | c.743T>C | Substitution | p.Leu248Pro | Substitution | 2A | EDMD2 | Emery-Dreifuss muscular dystrophy, autosomal dominant | 181350 | # |
| 16336 | c.745C>T | Substitution | p.Arg249Trp | Substitution | 2A | EDMD2 | Emery-Dreifuss muscular dystrophy, autosomal dominant | 181350 | # |
| 16337 | c.745C>T | Substitution | p.Arg249Trp | Substitution | 2A | EDMD2 | Emery-Dreifuss muscular dystrophy, autosomal dominant | 181350 | # |
| 16338 | c.745C>T | Substitution | p.Arg249Trp | Substitution | 2A | EDMD2 | Emery-Dreifuss muscular dystrophy, autosomal dominant | 181350 | # |
| 16342 | c.[745C>T; 1930C>T] | Substitution | p. [Arg249Trp; **Arg644Cys]** | Substitution | 2A, Tail | EDMD2 | Emery-Dreifuss muscular dystrophy, autosomal dominant | 181350 | # |
| 8783 | c.746G>A | Substitution | p.Arg249Gln | Substitution | 2A | EDMD2 | Emery-Dreifuss muscular dystrophy, autosomal dominant | 181350 | # |
| 11380 | c.746G>A | Substitution | p.Arg249Gln | Substitution | 2A | EDMD2 | Emery-Dreifuss muscular dystrophy, autosomal dominant | 181350 | # |
| 11381 | c.746G>A | Substitution | p.Arg249Gln | Substitution | 2A | EDMD2 | Emery-Dreifuss muscular dystrophy, autosomal dominant | 181350 | # |
| 8802 | c.746G>A | Substitution | p.Arg249Gln | Substitution | 2A | EDMD2 | Emery-Dreifuss muscular dystrophy, autosomal dominant | 181350 | # |
| 9002 | c.746G>A | Substitution | p.Arg249Gln | Substitution | 2A | EDMD2 | Emery-Dreifuss muscular dystrophy, autosomal dominant | 181350 | # |
| 9023 | c.746G>A | Substitution | p.Arg249Gln | Substitution | 2A | EDMD2 | Emery-Dreifuss muscular dystrophy, autosomal dominant | 181350 | # |
| 11631 | c.746G>A | Substitution | p.Arg249Gln | Substitution | 2A | EDMD2 | Emery-Dreifuss muscular dystrophy, autosomal dominant | 181350 | # |
| 11749 | c.746G>A | Substitution | p.Arg249Gln | Substitution | 2A | EDMD2 | Emery-Dreifuss muscular dystrophy, autosomal dominant | 181350 | # |
| 11750 | c.746G>A | Substitution | p.Arg249Gln | Substitution | 2A | EDMD2 | Emery-Dreifuss muscular dystrophy, autosomal dominant | 181350 | # |
| 13552 | c.746G>A | Substitution | p.Arg249Gln | Substitution | 2A | EDMD2 | Emery-Dreifuss muscular dystrophy, autosomal dominant | 181350 | # |
| 16344 | c.746G>A | Substitution | p.Arg249Gln | Substitution | 2A | EDMD2 | Emery-Dreifuss muscular dystrophy, autosomal dominant | 181350 | # |
| 16345 | c.746G>A | Substitution | p.Arg249Gln | Substitution | 2A | EDMD2 | Emery-Dreifuss muscular dystrophy, autosomal dominant | 181350 | # |
| 18176 | c.746G>A | Substitution | p.Arg249Gln | Substitution | 2A | EDMD2 | Emery-Dreifuss muscular dystrophy, autosomal dominant | 181350 | # |
| 11751 | c.775T>G | Substitution | p.Tyr259Asp | Substitution | L2 | EDMD2 | Emery-Dreifuss muscular dystrophy, autosomal dominant | 181350 | # |
| 9640 | c.781_783delAAG | Deletion | p.Lys261del | Deletion | L2 | EDMD2 | Emery-Dreifuss muscular dystrophy, autosomal dominant | 181350 | # |
| 9411 | c.781_783delAAG | Deletion | p.Lys261del | Deletion | L2 | EDMD2 | Emery-Dreifuss muscular dystrophy, autosomal dominant | 181350 | # |
| 9381 | c.781_783delAAG | Deletion | p.Lys261del | Deletion | L2 | EDMD2 | Emery-Dreifuss muscular dystrophy, autosomal dominant | 181350 | # |
| 13584 | c.781_783delAAGinsGTGG AGCAGTATAAGAAA | Indel | p.Lys261delinsValGluGl nTyrLysLys | Indel | L2 | EDMD2 | Emery-Dreifuss muscular dystrophy, autosomal dominant | 181350 | # |
| 16149 | c.788T>C | Substitution | p.Leu263Pro | Substitution | L2 | EDMD2 | Emery-Dreifuss muscular dystrophy, autosomal dominant | 181350 | # |
| 17403 | c.788T>C | Substitution | p.Leu263Pro | Substitution | L2 | EDMD2 | Emery-Dreifuss muscular dystrophy, autosomal dominant | 181350 | # |
| 17751 | c.788T>C | Substitution | p.Leu263Pro | Substitution | L2 | EDMD2 | Emery-Dreifuss muscular dystrophy, autosomal dominant | 181350 | # |
| 17405 | c.799T>C | Substitution | p.Tyr267His | Substitution | 2B | EDMD2 | Emery-Dreifuss muscular dystrophy, autosomal dominant | 181350 | # |
| 9003 | c.800A>G | Substitution | p.Tyr267Cys | Substitution | 2B | EDMD2 | Emery-Dreifuss muscular dystrophy, autosomal dominant | 181350 | # |
| 11809 | c.800A>G | Substitution | p.Tyr267Cys | Substitution | 2B | EDMD2 | Emery-Dreifuss muscular dystrophy, autosomal dominant | 181350 | # |
| 13173 | c.800A>G | Substitution | p.Tyr267Cys | Substitution | 2B | EDMD2 | Emery-Dreifuss muscular dystrophy, autosomal dominant | 181350 | # |
| 16303 | c.802T>C | Substitution | p.Ser268Pro | Substitution | 2B | EDMD2 | Emery-Dreifuss muscular dystrophy, autosomal dominant | 181350 | # |
| 16305 | c.810G>A | Substitution | **p.=** | Silent | Not affected | EDMD2 | Emery-Dreifuss muscular dystrophy, autosomal dominant | 181350 | # |
| 16307 | c.810G>A | Substitution | **p.=** | Silent | Not affected | EDMD2 | Emery-Dreifuss muscular dystrophy, autosomal dominant | 181350 | # |
| 16309 | **c.810+1G>A** | Substitution | **p.?** | Unknown | Unknown | EDMD2 | Emery-Dreifuss muscular dystrophy, autosomal dominant | 181350 | # |
| 16347 | c.812T>C | Substitution | p.Leu271Pro | Substitution | 2B | EDMD2 | Emery-Dreifuss muscular dystrophy, autosomal dominant | 181350 | # |
| 18186 | c.832G>C | Substitution | p.Ala278Pro | Substitution | 2B | EDMD2 | Emery-Dreifuss muscular dystrophy, autosomal dominant | 181350 | # |
| 8803 | c.881A>C | Substitution | **p.Gln294Pro** | Substitution | 2B | EDMD2 | Emery-Dreifuss muscular dystrophy, autosomal dominant | 181350 | # |
| 16349 | c.881A>C | Substitution | **p.Gln294Pro** | Substitution | 2B | EDMD2 | Emery-Dreifuss muscular dystrophy, autosomal dominant | 181350 | # |
| 16311 | c.883T>C | Substitution | p.Ser295Pro | Substitution | 2B | EDMD2 | Emery-Dreifuss muscular dystrophy, autosomal dominant | 181350 | # |
| 8863 | c.907T>C | Substitution | p.Ser303Pro | Substitution | 2B | EDMD2 | Emery-Dreifuss muscular dystrophy, autosomal dominant | 181350 | # |
| 16351 | c.907T>C | Substitution | p.Ser303Pro | Substitution | 2B | EDMD2 | Emery-Dreifuss muscular dystrophy, autosomal dominant | 181350 | # |
| 8786 | c.1007G>A | Substitution | p.Arg336Gln | Substitution | 2B | EDMD2 | Emery-Dreifuss muscular dystrophy, autosomal dominant | 181350 | # |
| 16314 | c.1064_1066delAGC | Deletion | **p.Gln355del** | Deletion | 2B | EDMD2 | Emery-Dreifuss muscular dystrophy, autosomal dominant | 181350 | # |
| 8804 | c.1072G>A | Substitution | p.Glu358Lys | Substitution | 2B | EDMD2 | Emery-Dreifuss muscular dystrophy, autosomal dominant | 181350 | # |
| 11388 | c.1072G>A | Substitution | p.Glu358Lys | Substitution | 2B | EDMD2 | Emery-Dreifuss muscular dystrophy, autosomal dominant | 181350 | # |
| 11816 | c.1072G>A | Substitution | p.Glu358Lys | Substitution | 2B | EDMD2 | Emery-Dreifuss muscular dystrophy, autosomal dominant | 181350 | # |
| 9167 | c.1072G>A | Substitution | p.Glu358Lys | Substitution | 2B | EDMD2 | Emery-Dreifuss muscular dystrophy, autosomal dominant | 181350 | # |
| 11478 | c.1072G>A | Substitution | p.Glu358Lys | Substitution | 2B | EDMD2 | Emery-Dreifuss muscular dystrophy, autosomal dominant | 181350 | # |
| 11479 | c.1072G>A | Substitution | p.Glu358Lys | Substitution | 2B | EDMD2 | Emery-Dreifuss muscular dystrophy, autosomal dominant | 181350 | # |
| 11480 | c.1072G>A | Substitution | p.Glu358Lys | Substitution | 2B | EDMD2 | Emery-Dreifuss muscular dystrophy, autosomal dominant | 181350 | # |
| 11752 | c.1072G>A | Substitution | p.Glu358Lys | Substitution | 2B | EDMD2 | Emery-Dreifuss muscular dystrophy, autosomal dominant | 181350 | # |
| 12479 | c.1072G>A | Substitution | p.Glu358Lys | Substitution | 2B | EDMD2 | Emery-Dreifuss muscular dystrophy, autosomal dominant | 181350 | # |
| 12483 | c.1072G>A | Substitution | p.Glu358Lys | Substitution | 2B | EDMD2 | Emery-Dreifuss muscular dystrophy, autosomal dominant | 181350 | # |
| 13598 | c.1072G>A | Substitution | p.Glu358Lys | Substitution | 2B | EDMD2 | Emery-Dreifuss muscular dystrophy, autosomal dominant | 181350 | # |
| 13603 | c.1072G>A | Substitution | p.Glu358Lys | Substitution | 2B | EDMD2 | Emery-Dreifuss muscular dystrophy, autosomal dominant | 181350 | # |
| 16353 | c.1072G>A | Substitution | p.Glu358Lys | Substitution | 2B | EDMD2 | Emery-Dreifuss muscular dystrophy, autosomal dominant | 181350 | # |
| 16354 | c.1072G>A | Substitution | p.Glu358Lys | Substitution | 2B | EDMD2 | Emery-Dreifuss muscular dystrophy, autosomal dominant | 181350 | # |
| 16355 | c.1072G>A | Substitution | p.Glu358Lys | Substitution | 2B | EDMD2 | Emery-Dreifuss muscular dystrophy, autosomal dominant | 181350 | # |
| 16316 | c.1081G>A | Substitution | **p.Glu361**Lys | Substitution | 2B | EDMD2 | Emery-Dreifuss muscular dystrophy, autosomal dominant | 181350 | # |
| 8806 | c.1112T>A | Substitution | p.Met371Lys | Substitution | 2B | EDMD2 | Emery-Dreifuss muscular dystrophy, autosomal dominant | 181350 | # |
| 18184 | c.1124C>G | Substitution | **p.Ala375Gly** | Substitution | 2B | EDMD2 | Emery-Dreifuss muscular dystrophy, autosomal dominant | 181350 | # |
| 11634 | c.1130G>T | Substitution | p.Arg377Leu | Substitution | 2B | EDMD2 | Emery-Dreifuss muscular dystrophy, autosomal dominant | 181350 | # |
| 11693 | c.1130G>A | Substitution | p.Arg377His | Substitution | 2B | EDMD2 | Emery-Dreifuss muscular dystrophy, autosomal dominant | 181350 | # |
| 12405 | c.1130G>A | Substitution | p.Arg377His | Substitution | 2B | EDMD2 | Emery-Dreifuss muscular dystrophy, autosomal dominant | 181350 | # |
| 18223 | c.1130G>A | Substitution | p.Arg377His | Substitution | 2B | EDMD2 | Emery-Dreifuss muscular dystrophy, autosomal dominant | 181350 | # |
| 12474 | c.1142A>C | Substitution | **p.Glu381Ala** | Substitution | 2B | EDMD2 | Emery-Dreifuss muscular dystrophy, autosomal dominant | 181350 | # |
| 11629 | c.1157G>A | Substitution | p.Arg386Lys | Substitution | 2B | EDMD2 | Emery-Dreifuss muscular dystrophy, autosomal dominant | 181350 | # |
| 13653 | c.1157G>T | Substitution | p.Arg386Met | Substitution | 2B | EDMD2 | Emery-Dreifuss muscular dystrophy, autosomal dominant | 181350 | # |
| 16357 | c.1157G>A | Substitution | p.Arg386Lys | Substitution | 2B | EDMD2 | Emery-Dreifuss muscular dystrophy, autosomal dominant | 181350 | # |
| 16358 | c.1157G>A | Substitution | p.Arg386Lys | Substitution | 2B | EDMD2 | Emery-Dreifuss muscular dystrophy, autosomal dominant | 181350 | # |
| 16319 | c.1158-2A>G | Substitution | **p.?** | Unknown | Unknown | EDMD2 | Emery-Dreifuss muscular dystrophy, autosomal dominant | 181350 | # |
| 11817 | c.1162C>T | Substitution | p.Arg388Cys | Substitution | Tail | EDMD2 | Emery-Dreifuss muscular dystrophy, autosomal dominant | 181350 | # |
| 9281 | c.1187A>G | Substitution | **p.Gln396Arg** | Substitution | Tail | EDMD2 | Emery-Dreifuss muscular dystrophy, autosomal dominant | 181350 | # |
| 8862 | c.1201C>T | Substitution | p.Arg401 Cys | Substitution | Tail | EDMD2 | Emery-Dreifuss muscular dystrophy, autosomal dominant | 181350 | # |
| 9176 | c.1201C>T | Substitution | p.Arg401 Cys | Substitution | Tail | EDMD2 | Emery-Dreifuss muscular dystrophy, autosomal dominant | 181350 | # |
| 9302 | c.1201C>T | Substitution | p.Arg401 Cys | Substitution | Tail | EDMD2 | Emery-Dreifuss muscular dystrophy, autosomal dominant | 181350 | # |
| 9004 | c.1337A>T | Substitution | p.Asp446Val | Substitution | Tail | EDMD2 | Emery-Dreifuss muscular dystrophy, autosomal dominant | 181350 | # |
| 16321 | c.1346G>A | Substitution | p.Gly449Asp | Substitution | Tail | EDMD2 | Emery-Dreifuss muscular dystrophy, autosomal dominant | 181350 | # |
| 18192 | c.1346G>T | Substitution | p.Gly449Val | Substitution | Tail | EDMD2 | Emery-Dreifuss muscular dystrophy, autosomal dominant | 181350 | # |
| 8744 | c.1357C>T | Substitution | p.Arg453Trp | Substitution | Tail | EDMD2 | Emery-Dreifuss muscular dystrophy, autosomal dominant | 181350 | # |
| 8787 | c.1357C>T | Substitution | p.Arg453Trp | Substitution | Tail | EDMD2 | Emery-Dreifuss muscular dystrophy, autosomal dominant | 181350 | # |
| 11382 | c.1357C>T | Substitution | p.Arg453Trp | Substitution | Tail | EDMD2 | Emery-Dreifuss muscular dystrophy, autosomal dominant | 181350 | # |
| 11383 | c.1357C>T | Substitution | p.Arg453Trp | Substitution | Tail | EDMD2 | Emery-Dreifuss muscular dystrophy, autosomal dominant | 181350 | # |
| 11384 | c.1357C>T | Substitution | p.Arg453Trp | Substitution | Tail | EDMD2 | Emery-Dreifuss muscular dystrophy, autosomal dominant | 181350 | # |
| 8807 | c.1357C>T | Substitution | p.Arg453Trp | Substitution | Tail | EDMD2 | Emery-Dreifuss muscular dystrophy, autosomal dominant | 181350 | # |
| 11389 | c.1357C>T | Substitution | p.Arg453Trp | Substitution | Tail | EDMD2 | Emery-Dreifuss muscular dystrophy, autosomal dominant | 181350 | # |
| 11390 | c.1357C>T | Substitution | p.Arg453Trp | Substitution | Tail | EDMD2 | Emery-Dreifuss muscular dystrophy, autosomal dominant | 181350 | # |
| 8836 | c.1357C>T | Substitution | p.Arg453Trp | Substitution | Tail | EDMD2 | Emery-Dreifuss muscular dystrophy, autosomal dominant | 181350 | # |
| 9005 | c.1357C>T | Substitution | p.Arg453Trp | Substitution | Tail | EDMD2 | Emery-Dreifuss muscular dystrophy, autosomal dominant | 181350 | # |
| 9304 | c.1357C>T | Substitution | p.Arg453Trp | Substitution | Tail | EDMD2 | Emery-Dreifuss muscular dystrophy, autosomal dominant | 181350 | # |
| 11614 | c.1357C>T | Substitution | p.Arg453Trp | Substitution | Tail | EDMD2 | Emery-Dreifuss muscular dystrophy, autosomal dominant | 181350 | # |
| 11753 | c.1357C>T | Substitution | p.Arg453Trp | Substitution | Tail | EDMD2 | Emery-Dreifuss muscular dystrophy, autosomal dominant | 181350 | # |
| 12481 | c.1357C>T | Substitution | p.Arg453Trp | Substitution | Tail | EDMD2 | Emery-Dreifuss muscular dystrophy, autosomal dominant | 181350 | # |
| 13590 | c.1357C>T | Substitution | p.Arg453Trp | Substitution | Tail | EDMD2 | Emery-Dreifuss muscular dystrophy, autosomal dominant | 181350 | # |
| 13607 | c.1357C>T | Substitution | p.Arg453Trp | Substitution | Tail | EDMD2 | Emery-Dreifuss muscular dystrophy, autosomal dominant | 181350 | # |
| 13609 | c.1357C>T | Substitution | p.Arg453Trp | Substitution | Tail | EDMD2 | Emery-Dreifuss muscular dystrophy, autosomal dominant | 181350 | # |
| 13611 | c.1357C>T | Substitution | p.Arg453Trp | Substitution | Tail | EDMD2 | Emery-Dreifuss muscular dystrophy, autosomal dominant | 181350 | # |
| 16363 | c.1357C>T | Substitution | p.Arg453Trp | Substitution | Tail | EDMD2 | Emery-Dreifuss muscular dystrophy, autosomal dominant | 181350 | # |
| 16364 | c.1357C>T | Substitution | p.Arg453Trp | Substitution | Tail | EDMD2 | Emery-Dreifuss muscular dystrophy, autosomal dominant | 181350 | # |
| 16365 | c.1357C>T | Substitution | p.Arg453Trp | Substitution | Tail | EDMD2 | Emery-Dreifuss muscular dystrophy, autosomal dominant | 181350 | # |
| 16366 | c.1357C>T | Substitution | p.Arg453Trp | Substitution | Tail | EDMD2 | Emery-Dreifuss muscular dystrophy, autosomal dominant | 181350 | # |
| 16367 | c.1357C>T | Substitution | p.Arg453Trp | Substitution | Tail | EDMD2 | Emery-Dreifuss muscular dystrophy, autosomal dominant | 181350 | # |
| 16368 | c.1357C>T | Substitution | p.Arg453Trp | Substitution | Tail | EDMD2 | Emery-Dreifuss muscular dystrophy, autosomal dominant | 181350 | # |
| 16323 | c.1361T>C | Substitution | p.Leu454Pro | Substitution | Tail | EDMD2 | Emery-Dreifuss muscular dystrophy, autosomal dominant | 181350 | # |
| 9513 | c.1367A>T | Substitution | p.Asn456lle | Substitution | Tail | EDMD2 | Emery-Dreifuss muscular dystrophy, autosomal dominant | 181350 | # |
| 8810 | c.1368C>A | Substitution | p.Asn456Lys | Substitution | Tail | EDMD2 | Emery-Dreifuss muscular dystrophy, autosomal dominant | 181350 | # |
| 11818 | c.1368_1370delCAA | Deletion | p.Asn456del | Deletion | Tail | EDMD2 | Emery-Dreifuss muscular dystrophy, autosomal dominant | 181350 | # |
| 17446 | c.1368_1370delCAA | Deletion | p.Asn456del | Deletion | Tail | EDMD2 | Emery-Dreifuss muscular dystrophy, autosomal dominant | 181350 | # |
| 17447 | c.1368_1370delCAA | Deletion | p.Asn456del | Deletion | Tail | EDMD2 | Emery-Dreifuss muscular dystrophy, autosomal dominant | 181350 | # |
| 16370 | c.1381-2A>G | Substitution | **p.?** | Unknown | Unknown | EDMD2 | Emery-Dreifuss muscular dystrophy, autosomal dominant | 181350 | # |
| 16372 | c.[1381-1G>T; 1381G>T] | Substitution | p.[?; Asp461Tyr] | Unknown, Substitution | Tail | EDMD2 | Emery-Dreifuss muscular dystrophy, autosomal dominant | 181350 | # |
| 16325 | c.1399T>C | Substitution | p.Trp467Arg | Substitution | Tail | EDMD2 | Emery-Dreifuss muscular dystrophy, autosomal dominant | 181350 | # |
| 8791 | c.1406T>C | Substitution | **p.Ile469Thr** | Substitution | Tail | EDMD2 | Emery-Dreifuss muscular dystrophy, autosomal dominant | 181350 | # |
| 18194 | c.1466T>C | Substitution | p.Leu489Pro | Substitution | Tail | EDMD2 | Emery-Dreifuss muscular dystrophy, autosomal dominant | 181350 | # |
| 16327 | c.1488+1G>A | Substitution | **p.?** | Unknown | Unknown | EDMD2 | Emery-Dreifuss muscular dystrophy, autosomal dominant | 181350 | # |
| 13180 | c.1492T>A | Substitution | p.Trp498Arg | Substitution | Tail | EDMD2 | Emery-Dreifuss muscular dystrophy, autosomal dominant | 181350 | # |
| 16375 | c.1526dupC | Duplication | **p.Thr51**0TyrfsX42 | Frame shift | Tail | EDMD2 | Emery-Dreifuss muscular dystrophy, autosomal dominant | 181350 | # |
| 18188 | c.1540T>A | Substitution | p.Trp514Arg | Substitution | Tail | EDMD2 | Emery-Dreifuss muscular dystrophy, autosomal dominant | 181350 | # |
| 18190 | c.1540T>A | Substitution | p.Trp514Arg | Substitution | Tail | EDMD2 | Emery-Dreifuss muscular dystrophy, autosomal dominant | 181350 | # |
| 12434 | c.1558T>G | Substitution | p.Trp520Gly | Substitution | Tail | EDMD2 | Emery-Dreifuss muscular dystrophy, autosomal dominant | 181350 | # |
| 18179 | c.1558T>C | Substitution | p.Trp520Arg | Substitution | Tail | EDMD2 | Emery-Dreifuss muscular dystrophy, autosomal dominant | 181350 | # |
| 8811 | c.1559G>C | Substitution | p.Trp520Ser | Substitution | Tail | EDMD2 | Emery-Dreifuss muscular dystrophy, autosomal dominant | 181350 | # |
| 8745 | c.1580G>C | Substitution | p.Arg527Pro | Substitution | Tail | EDMD2 | Emery-Dreifuss muscular dystrophy, autosomal dominant | 181350 | # |
| 11344 | c.1580G>C | Substitution | p.Arg527Pro | Substitution | Tail | EDMD2 | Emery-Dreifuss muscular dystrophy, autosomal dominant | 181350 | # |
| 8792 | c.1580G>C | Substitution | p.Arg527Pro | Substitution | Tail | EDMD2 | Emery-Dreifuss muscular dystrophy, autosomal dominant | 181350 | # |
| 8812 | c.1580G>C | Substitution | p.Arg527Pro | Substitution | Tail | EDMD2 | Emery-Dreifuss muscular dystrophy, autosomal dominant | 181350 | # |
| 11391 | c.1580G>C | Substitution | p.Arg527Pro | Substitution | Tail | EDMD2 | Emery-Dreifuss muscular dystrophy, autosomal dominant | 181350 | # |
| 8838 | c.1580G>C | Substitution | p.Arg527Pro | Substitution | Tail | EDMD2 | Emery-Dreifuss muscular dystrophy, autosomal dominant | 181350 | # |
| 8859 | c.1580G>C | Substitution | p.Arg527Pro | Substitution | Tail | EDMD2 | Emery-Dreifuss muscular dystrophy, autosomal dominant | 181350 | # |
| 11423 | c.1580G>C | Substitution | p.Arg527Pro | Substitution | Tail | EDMD2 | Emery-Dreifuss muscular dystrophy, autosomal dominant | 181350 | # |
| 11630 | c.1580G>C | Substitution | p.Arg527Pro | Substitution | Tail | EDMD2 | Emery-Dreifuss muscular dystrophy, autosomal dominant | 181350 | # |
| 11754 | c.1580G>C | Substitution | p.Arg527Pro | Substitution | Tail | EDMD2 | Emery-Dreifuss muscular dystrophy, autosomal dominant | 181350 | # |
| 16377 | c.1580G>C | Substitution | p.Arg527Pro | Substitution | Tail | EDMD2 | Emery-Dreifuss muscular dystrophy, autosomal dominant | 181350 | # |
| 18182 | c.1580G>C | Substitution | p.Arg527Pro | Substitution | Tail | EDMD2 | Emery-Dreifuss muscular dystrophy, autosomal dominant | 181350 | # |
| 8793 | c.1583C>A | Substitution | p.Thr528Lys | Substitution | Tail | EDMD2 | Emery-Dreifuss muscular dystrophy, autosomal dominant | 181350 | # |
| 8814 | c.1583C>A | Substitution | p.Thr528Lys | Substitution | Tail | EDMD2 | Emery-Dreifuss muscular dystrophy, autosomal dominant | 181350 | # |
| 11392 | c.1583C>A | Substitution | p.Thr528Lys | Substitution | Tail | EDMD2 | Emery-Dreifuss muscular dystrophy, autosomal dominant | 181350 | # |
| 9006 | c.1583C>G | Substitution | p.Thr528Arg | Substitution | Tail | EDMD2 | Emery-Dreifuss muscular dystrophy, autosomal dominant | 181350 | # |
| 16379 | c.1583C>A | Substitution | p.Thr528Lys | Substitution | Tail | EDMD2 | Emery-Dreifuss muscular dystrophy, autosomal dominant | 181350 | # |
| 16381 | c.1583C>G | Substitution | p.Thr528Arg | Substitution | Tail | EDMD2 | Emery-Dreifuss muscular dystrophy, autosomal dominant | 181350 | # |
| 16382 | c.1583C>G | Substitution | p.Thr528Arg | Substitution | Tail | EDMD2 | Emery-Dreifuss muscular dystrophy, autosomal dominant | 181350 | # |
| 16407 | c.1583C>G | Substitution | p.Thr528Arg | Substitution | Tail | EDMD2 | Emery-Dreifuss muscular dystrophy, autosomal dominant | 181350 | # |
| 18196 | c.1583C>G | Substitution | p.Thr528Arg | Substitution | Tail | EDMD2 | Emery-Dreifuss muscular dystrophy, autosomal dominant | 181350 | # |
| 18297 | c.1588C>T | Substitution | p.Leu530Phe | Substitution | Tail | EDMD2 | Emery-Dreifuss muscular dystrophy, autosomal dominant | 181350 | # |
| 8747 | c.1589T>C | Substitution | p.Leu530Pro | Substitution | Tail | EDMD2 | Emery-Dreifuss muscular dystrophy, autosomal dominant | 181350 | # |
| 16384 | c.1621C>A | Substitution | p.Arg541Ser | Substitution | Tail | EDMD2 | Emery-Dreifuss muscular dystrophy, autosomal dominant | 181350 | # |
| **9007** | c.1622G>A | **Substitution** | **p.Arg541His** | **Substitution** | Tail | EDMD2 | Emery-Dreifuss muscular dystrophy, autosomal dominant | 181350 | # |
| 16387 | c.1622G>C | Substitution | **p.Arg541Pro** | Substitution | Tail | EDMD2 | Emery-Dreifuss muscular dystrophy, autosomal dominant | 181350 | # |
| 13588 | c.1633C>T | Substitution | p.Arg545Cys | Substitution | Tail | EDMD2 | Emery-Dreifuss muscular dystrophy, autosomal dominant | 181350 | # |
| 16389 | c.1804G>A | Substitution | p.Arg541Pro | Substitution | Tail | EDMD2 | Emery-Dreifuss muscular dystrophy, autosomal dominant | 181350 | # |
| 9383 | c.1871G>A | Substitution | p.Arg624His | Substitution | Tail | EDMD2 | Emery-Dreifuss muscular dystrophy, autosomal dominant | 181350 | # |
| 9154 | c.1930C>T | Substitution | p.Arg644Cys | Substitution | Tail | EDMD2 | Emery-Dreifuss muscular dystrophy, autosomal dominant | 181350 | # |
| 13570 | c.1930C>T | Substitution | p.Arg644Cys | Substitution | Tail | EDMD2 | Emery-Dreifuss muscular dystrophy, autosomal dominant | 181350 | # |
| 13571 | c.1930C>T | Substitution | p.Arg644Cys | Substitution | Tail | EDMD2 | Emery-Dreifuss muscular dystrophy, autosomal dominant | 181350 | # |
| 13572 | c.1930C>T | Substitution | p.Arg644Cys | Substitution | Tail | EDMD2 | Emery-Dreifuss muscular dystrophy, autosomal dominant | 181350 | # |
| 13573 | c.1930C>T | Substitution | p.Arg644Cys | Substitution | Tail | EDMD2 | Emery-Dreifuss muscular dystrophy, autosomal dominant | 181350 | # |
| 16392 | c.1930C>T | Substitution | p.Arg644Cys | Substitution | Tail | EDMD2 | Emery-Dreifuss muscular dystrophy, autosomal dominant | 181350 | # |
| 9523 | c.664C>T | Substitution | p.His222Tyr | Substitution | L12 | EDMD3 | Emery-Dreifuss muscular dystrophy, autosomal recessive | 604929 | # |
| 17407 | c.674G>A | Substitution | **p.Arg225Gln** | Substitution | L12 | EDMD3 | Emery-Dreifuss muscular dystrophy, autosomal recessive | 604929 | # |
| 17573 | c.1445G>A | Substitution | p.Arg482Gln | Substitution | Tail | EDMD3 | Emery-Dreifuss muscular dystrophy, autosomal recessive | 604929 | # |
| 13319 | c.1580G>C | Substitution | p.Arg527Pro | Substitution | Tail | EDMD3 | Emery-Dreifuss muscular dystrophy, autosomal recessive | 604929 | # |
| 18345 | c.? | Unknown | p.Arg482Trp | Substitution | Tail | FPLD2 | Familial partial lipodystrophy (Dunnigan type) | 151660 | # |
| 8847 | c.82C>T | Substitution | p.Arg28Trp | Substitution | Head | FPLD2 | Familial partial lipodystrophy (Dunnigan type) | 151660 | # |
| 18291 | c.139G>A | Substitution | p.Asp47Asn | Substitution | 1A | FPLD2 | Familial partial lipodystrophy (Dunnigan type) | 151660 | # |
| 8861 | c.178C>G | Substitution | p.Arg60Gly | Substitution | 1A | FPLD2 | Familial partial lipodystrophy (Dunnigan type) | 151660 | # |
| 14260 | c.178C>G | Substitution | p.Arg60Gly | Substitution | 1A | FPLD2 | Familial partial lipodystrophy (Dunnigan type) | 151660 | # |
| 8848 | c.184C>G | Substitution | p.Arg62Gly | Substitution | 1A | FPLD2 | Familial partial lipodystrophy (Dunnigan type) | 151660 | # |
| 14256 | c.184C>G | Substitution | p.Arg62Gly | Substitution | 1A | FPLD2 | Familial partial lipodystrophy (Dunnigan type) | 151660 | # |
| 12100 | c.398G>T | Substitution | p.Arg133Leu | Substitution | 1B | FPLD2 | Familial partial lipodystrophy (Dunnigan type) | 151660 | # |
| 14258 | c.575A>T | Substitution | p.Asp192Val | Substitution | 1B | FPLD2 | Familial partial lipodystrophy (Dunnigan type) | 151660 | # |
| 17624 | c.667G>A | Substitution | p.Glu223Lys | Substitution | L12 | FPLD2 | Familial partial lipodystrophy (Dunnigan type) | 151660 | # |
| 11775 | c.688G>A | Substitution | p.Asp230Asn | Substitution | L12 | FPLD2 | Familial partial lipodystrophy (Dunnigan type) | 151660 | # |
| 11776 | c.1195C>T | Substitution | p.Arg399Cys | Substitution | Tail | FPLD2 | Familial partial lipodystrophy (Dunnigan type) | 151660 | # |
| 17066 | c.1232G>A | Substitution | p.Gly411Asp | Substitution | Tail | FPLD2 | Familial partial lipodystrophy (Dunnigan type) | 151660 | # |
| 12096 | c.1315C>T | Substitution | p.Arg439Cys | Substitution | Tail | FPLD2 | Familial partial lipodystrophy (Dunnigan type) | 151660 | # |
| 17208 | c.1315C>T | Substitution | p.Arg439Cys | Substitution | Tail | FPLD2 | Familial partial lipodystrophy (Dunnigan type) | 151660 | # |
| 11542 | c.[1318G>A; =]+[=; 1445G>A] | Substitution | p.[Val440Met; =]+[=; Arg482Gln] | Substitution | Tail | FPLD2 | Familial partial lipodystrophy (Dunnigan type) | 151660 | # |
| 8781 | c.1394G>A | Substitution | p.Gly465Asp | Substitution | Tail | FPLD2 | Familial partial lipodystrophy (Dunnigan type) | 151660 | # |
| 12601 | c.1411C>G | Substitution | p.Arg471Gly | Substitution | Tail | FPLD2 | Familial partial lipodystrophy (Dunnigan type) | 151660 | # |
| 8754 | c.1444C>T | Substitution | p.Arg482Trp | Substitution | Tail | FPLD2 | Familial partial lipodystrophy (Dunnigan type) | 151660 | # |
| 11358 | c.1444C>T | Substitution | p.Arg482Trp | Substitution | Tail | FPLD2 | Familial partial lipodystrophy (Dunnigan type) | 151660 | # |
| 11359 | c.1444C>T | Substitution | p.Arg482Trp | Substitution | Tail | FPLD2 | Familial partial lipodystrophy (Dunnigan type) | 151660 | # |
| 11360 | c.1444C>T | Substitution | p.Arg482Trp | Substitution | Tail | FPLD2 | Familial partial lipodystrophy (Dunnigan type) | 151660 | # |
| 11361 | c.1444C>T | Substitution | p.Arg482Trp | Substitution | Tail | FPLD2 | Familial partial lipodystrophy (Dunnigan type) | 151660 | # |
| 11362 | c.1444C>T | Substitution | p.Arg482Trp | Substitution | Tail | FPLD2 | Familial partial lipodystrophy (Dunnigan type) | 151660 | # |
| 11363 | c.1444C>T | Substitution | p.Arg482Trp | Substitution | Tail | FPLD2 | Familial partial lipodystrophy (Dunnigan type) | 151660 | # |
| 11364 | c.1444C>T | Substitution | p.Arg482Trp | Substitution | Tail | FPLD2 | Familial partial lipodystrophy (Dunnigan type) | 151660 | # |
| 11365 | c.1444C>T | Substitution | p.Arg482Trp | Substitution | Tail | FPLD2 | Familial partial lipodystrophy (Dunnigan type) | 151660 | # |
| 8773 | c.1444C>T | Substitution | p.Arg482Trp | Substitution | Tail | FPLD2 | Familial partial lipodystrophy (Dunnigan type) | 151660 | # |
| 11374 | c.1444C>T | Substitution | p.Arg482Trp | Substitution | Tail | FPLD2 | Familial partial lipodystrophy (Dunnigan type) | 151660 | # |
| 11375 | c.1444C>T | Substitution | p.Arg482Trp | Substitution | Tail | FPLD2 | Familial partial lipodystrophy (Dunnigan type) | 151660 | # |
| 11376 | c.1444C>T | Substitution | p.Arg482Trp | Substitution | Tail | FPLD2 | Familial partial lipodystrophy (Dunnigan type) | 151660 | # |
| 11377 | c.1444C>T | Substitution | p.Arg482Trp | Substitution | Tail | FPLD2 | Familial partial lipodystrophy (Dunnigan type) | 151660 | # |
| 11378 | c.1444C>T | Substitution | p.Arg482Trp | Substitution | Tail | FPLD2 | Familial partial lipodystrophy (Dunnigan type) | 151660 | # |
| 11379 | c.1444C>T | Substitution | p.Arg482Trp | Substitution | Tail | FPLD2 | Familial partial lipodystrophy (Dunnigan type) | 151660 | # |
| 8816 | c.1444C>T | Substitution | p.Arg482Trp | Substitution | Tail | FPLD2 | Familial partial lipodystrophy (Dunnigan type) | 151660 | # |
| 11393 | c.1444C>T | Substitution | p.Arg482Trp | Substitution | Tail | FPLD2 | Familial partial lipodystrophy (Dunnigan type) | 151660 | # |
| 11394 | c.1444C>T | Substitution | p.Arg482Trp | Substitution | Tail | FPLD2 | Familial partial lipodystrophy (Dunnigan type) | 151660 | # |
| 11395 | c.1444C>T | Substitution | p.Arg482Trp | Substitution | Tail | FPLD2 | Familial partial lipodystrophy (Dunnigan type) | 151660 | # |
| 11396 | c.1444C>T | Substitution | p.Arg482Trp | Substitution | Tail | FPLD2 | Familial partial lipodystrophy (Dunnigan type) | 151660 | # |
| 11397 | c.1444C>T | Substitution | p.Arg482Trp | Substitution | Tail | FPLD2 | Familial partial lipodystrophy (Dunnigan type) | 151660 | # |
| 8834 | c.1444C>T | Substitution | p.Arg482Trp | Substitution | Tail | FPLD2 | Familial partial lipodystrophy (Dunnigan type) | 151660 | # |
| 9213 | c.1444C>T | Substitution | p.Arg482Trp | Substitution | Tail | FPLD2 | Familial partial lipodystrophy (Dunnigan type) | 151660 | # |
| 9156 | c.1444C>T | Substitution | p.Arg482Trp | Substitution | Tail | FPLD2 | Familial partial lipodystrophy (Dunnigan type) | 151660 | # |
| 11543 | c.1444C>T | Substitution | p.Arg482Trp | Substitution | Tail | FPLD2 | Familial partial lipodystrophy (Dunnigan type) | 151660 | # |
| 11544 | c.1444C>T | Substitution | p.Arg482Trp | Substitution | Tail | FPLD2 | Familial partial lipodystrophy (Dunnigan type) | 151660 | # |
| 11662 | c.1444C>T | Substitution | p.Arg482Trp | Substitution | Tail | FPLD2 | Familial partial lipodystrophy (Dunnigan type) | 151660 | # |
| 11692 | c.1444C>T | Substitution | p.Arg482Trp | Substitution | Tail | FPLD2 | Familial partial lipodystrophy (Dunnigan type) | 151660 | # |
| 11699 | c.1444C>T | Substitution | p.Arg482Trp | Substitution | Tail | FPLD2 | Familial partial lipodystrophy (Dunnigan type) | 151660 | # |
| 12101 | c.1444C>T | Substitution | p.Arg482Trp | Substitution | Tail | FPLD2 | Familial partial lipodystrophy (Dunnigan type) | 151660 | # |
| 12102 | c.1444C>T | Substitution | p.Arg482Trp | Substitution | Tail | FPLD2 | Familial partial lipodystrophy (Dunnigan type) | 151660 | # |
| 12103 | c.1444C>T | Substitution | p.Arg482Trp | Substitution | Tail | FPLD2 | Familial partial lipodystrophy (Dunnigan type) | 151660 | # |
| 12104 | c.1444C>T | Substitution | p.Arg482Trp | Substitution | Tail | FPLD2 | Familial partial lipodystrophy (Dunnigan type) | 151660 | # |
| 12105 | c.1444C>T | Substitution | p.Arg482Trp | Substitution | Tail | FPLD2 | Familial partial lipodystrophy (Dunnigan type) | 151660 | # |
| 14066 | c.1444C>T | Substitution | p.Arg482Trp | Substitution | Tail | FPLD2 | Familial partial lipodystrophy (Dunnigan type) | 151660 | # |
| 16433 | c.1444C>T | Substitution | p.Arg482Trp | Substitution | Tail | FPLD2 | Familial partial lipodystrophy (Dunnigan type) | 151660 | # |
| 16434 | c.1444C>T | Substitution | p.Arg482Trp | Substitution | Tail | FPLD2 | Familial partial lipodystrophy (Dunnigan type) | 151660 | # |
| 16682 | c.1444C>T | Substitution | p.Arg482Trp | Substitution | Tail | FPLD2 | Familial partial lipodystrophy (Dunnigan type) | 151660 | # |
| 17866 | c.1444C>T | Substitution | p.Arg482Trp | Substitution | Tail | FPLD2 | Familial partial lipodystrophy (Dunnigan type) | 151660 | # |
| 17195 | c.1444C>T | Substitution | p.Arg482Trp | Substitution | Tail | FPLD2 | Familial partial lipodystrophy (Dunnigan type) | 151660 | # |
| 17464 | c.1444C>T | Substitution | p.Arg482Trp | Substitution | Tail | FPLD2 | Familial partial lipodystrophy (Dunnigan type) | 151660 | # |
| 17465 | c.1444C>T | Substitution | p.Arg482Trp | Substitution | Tail | FPLD2 | Familial partial lipodystrophy (Dunnigan type) | 151660 | # |
| 17466 | c.1444C>T | Substitution | p.Arg482Trp | Substitution | Tail | FPLD2 | Familial partial lipodystrophy (Dunnigan type) | 151660 | # |
| 17467 | c.1444C>T | Substitution | p.Arg482Trp | Substitution | Tail | FPLD2 | Familial partial lipodystrophy (Dunnigan type) | 151660 | # |
| 17733 | c.1444C>T | Substitution | p.Arg482Trp | Substitution | Tail | FPLD2 | Familial partial lipodystrophy (Dunnigan type) | 151660 | # |
| 17735 | c.1444C>T | Substitution | p.Arg482Trp | Substitution | Tail | FPLD2 | Familial partial lipodystrophy (Dunnigan type) | 151660 | # |
| 17744 | c.1444C>T | Substitution | p.Arg482Trp | Substitution | Tail | FPLD2 | Familial partial lipodystrophy (Dunnigan type) | 151660 | # |
| 17936 | c.1444C>T | Substitution | p.Arg482Trp | Substitution | Tail | FPLD2 | Familial partial lipodystrophy (Dunnigan type) | 151660 | # |
| 18484 | c.1444C>T | Substitution | p.Arg482Trp | Substitution | Tail | FPLD2 | Familial partial lipodystrophy (Dunnigan type) | 151660 | # |
| 8753 | c.1445G>A | Substitution | pArg482Gln | Substitution | Tail | FPLD2 | Familial partial lipodystrophy (Dunnigan type) | 151660 | # |
| 11354 | c.1445G>A | Substitution | pArg482Gln | Substitution | Tail | FPLD2 | Familial partial lipodystrophy (Dunnigan type) | 151660 | # |
| 11355 | c.1445G>A | Substitution | pArg482Gln | Substitution | Tail | FPLD2 | Familial partial lipodystrophy (Dunnigan type) | 151660 | # |
| 11356 | c.1445G>A | Substitution | pArg482Gln | Substitution | Tail | FPLD2 | Familial partial lipodystrophy (Dunnigan type) | 151660 | # |
| 11357 | c.1445G>A | Substitution | pArg482Gln | Substitution | Tail | FPLD2 | Familial partial lipodystrophy (Dunnigan type) | 151660 | # |
| 8763 | c.1445G>A | Substitution | pArg482Gln | Substitution | Tail | FPLD2 | Familial partial lipodystrophy (Dunnigan type) | 151660 | # |
| 8764 | c.1445G>T | Substitution | p.Arg482Leu | Substitution | Tail | FPLD2 | Familial partial lipodystrophy (Dunnigan type) | 151660 | # |
| 8768 | c.1445G>A | Substitution | pArg482Gln | Substitution | Tail | FPLD2 | Familial partial lipodystrophy (Dunnigan type) | 151660 | # |
| 11370 | c.1445G>A | Substitution | pArg482Gln | Substitution | Tail | FPLD2 | Familial partial lipodystrophy (Dunnigan type) | 151660 | # |
| 11371 | c.1445G>A | Substitution | pArg482Gln | Substitution | Tail | FPLD2 | Familial partial lipodystrophy (Dunnigan type) | 151660 | # |
| 11372 | c.1445G>A | Substitution | pArg482Gln | Substitution | Tail | FPLD2 | Familial partial lipodystrophy (Dunnigan type) | 151660 | # |
| 11373 | c.1445G>A | Substitution | pArg482Gln | Substitution | Tail | FPLD2 | Familial partial lipodystrophy (Dunnigan type) | 151660 | # |
| 8822 | c.1445G>A | Substitution | p.Arg482Gln | Substitution | Tail | FPLD2 | Familial partial lipodystrophy (Dunnigan type) | 151660 | # |
| 8868 | c.1445G>A | Substitution | p.Arg482Gln | Substitution | Tail | FPLD2 | Familial partial lipodystrophy (Dunnigan type) | 151660 | # |
| 9305 | c.1445G>T | Substitution | p.Arg482Leu | Substitution | Tail | FPLD2 | Familial partial lipodystrophy (Dunnigan type) | 151660 | # |
| 11663 | c.1445G>A | Substitution | pArg482Gln | Substitution | Tail | FPLD2 | Familial partial lipodystrophy (Dunnigan type) | 151660 | # |
| 11667 | c.1445G>A | Substitution | pArg482Gln | Substitution | Tail | FPLD2 | Familial partial lipodystrophy (Dunnigan type) | 151660 | # |
| 12098 | c.1445G>A | Substitution | pArg482Gln | Substitution | Tail | FPLD2 | Familial partial lipodystrophy (Dunnigan type) | 151660 | # |
| 12387 | c.1445G>A | Substitution | p.Arg482Gln | Substitution | Unknown | FPLD2 | Familial partial lipodystrophy (Dunnigan type) | 151660 | # |
| 12418 | c.1445G>A | Substitution | p.Arg482Gln | Substitution | Unknown | FPLD2 | Familial partial lipodystrophy (Dunnigan type) | 151660 | # |
| 12423 | c.1445G>A | Substitution | pArg482Gln | Substitution | Tail | FPLD2 | Familial partial lipodystrophy (Dunnigan type) | 151660 | # |
| 13150 | c.1445G>A | Substitution | pArg482Gln | Substitution | Tail | FPLD2 | Familial partial lipodystrophy (Dunnigan type) | 151660 | # |
| 13367 | c.1445G>A | Substitution | pArg482Gln | Substitution | Tail | FPLD2 | Familial partial lipodystrophy (Dunnigan type) | 151660 | # |
| 16285 | c.1445G>A | Substitution | pArg482Gln | Substitution | Tail | FPLD2 | Familial partial lipodystrophy (Dunnigan type) | 151660 | # |
| 16436 | c.1445G>A | Substitution | pArg482Gln | Substitution | Tail | FPLD2 | Familial partial lipodystrophy (Dunnigan type) | 151660 | # |
| 16437 | c.1445G>A | Substitution | pArg482Gln | Substitution | Tail | FPLD2 | Familial partial lipodystrophy (Dunnigan type) | 151660 | # |
| 16438 | c.1445G>A | Substitution | pArg482Gln | Substitution | Tail | FPLD2 | Familial partial lipodystrophy (Dunnigan type) | 151660 | # |
| 16439 | c.1445G>A | Substitution | pArg482Gln | Substitution | Tail | FPLD2 | Familial partial lipodystrophy (Dunnigan type) | 151660 | # |
| 16440 | c.1445G>A | Substitution | pArg482Gln | Substitution | Tail | FPLD2 | Familial partial lipodystrophy (Dunnigan type) | 151660 | # |
| 18138 | c.1445G>A | Substitution | pArg482Gln | Substitution | Tail | FPLD2 | Familial partial lipodystrophy (Dunnigan type) | 151660 | # |
| 8765 | c.1458G>C | Substitution | p.Lys486Asn | Substitution | Tail | FPLD2 | Familial partial lipodystrophy (Dunnigan type) | 151660 | # |
| 11366 | c.1458G>C | Substitution | p.Lys486Asn | Substitution | Tail | FPLD2 | Familial partial lipodystrophy (Dunnigan type) | 151660 | # |
| 8767 | c.1458G>T | Substitution | p.Lys486Asn | Substitution | Tail | FPLD2 | Familial partial lipodystrophy (Dunnigan type) | 151660 | # |
| 9319 | c.1488+5G>C | Substitution | p.Ile497_Met664delinsVal ThrGlyArgAlaLeuGlyThrL euGlyArgProTrpValAlaMe tGlyAlaLeuGlyX | Indel | Tail | FPLD2 | Familial partial lipodystrophy (Dunnigan type) | 151660 | # |
| 17455 | c.1683G>C | Substitution | p.= | Silent | Not affected | FPLD2 | Familial partial lipodystrophy (Dunnigan type) | 151660 | # |
| 11777 | c.1718C>T | Substitution | p.Ser573Leu | Substitution | Tail | FPLD2 | Familial partial lipodystrophy (Dunnigan type) | 151660 | # |
| 8780 | c.1745G>A | Substitution | p.Arg582His | Substitution | Tail | FPLD2 | Familial partial lipodystrophy (Dunnigan type) | 151660 | # |
| 16429 | c.1745G>A | Substitution | p.Arg582His | Substitution | Tail | FPLD2 | Familial partial lipodystrophy (Dunnigan type) | 151660 | # |
| 16430 | c.1745G>A | Substitution | p.Arg582His | Substitution | Tail | FPLD2 | Familial partial lipodystrophy (Dunnigan type) | 151660 | # |
| 16431 | c.1745G>A | Substitution | p.Arg582His | Substitution | Tail | FPLD2 | Familial partial lipodystrophy (Dunnigan type) | 151660 | # |
| 9452 | c.1751G>A | Substitution | p.Arg584His | Substitution | Tail | FPLD2 | Familial partial lipodystrophy (Dunnigan type) | 151660 | # |
| 11545 | c.1751G>A | Substitution | p.Arg584His | Substitution | Tail | FPLD2 | Familial partial lipodystrophy (Dunnigan type) | 151660 | # |
| 12429 | c.1772G>T | Substitution | p.Arg156Cys | Substitution | 1B | FPLD2 | Familial partial lipodystrophy (Dunnigan type) | 151660 | # |
| 17068 | c.1892G>A | Substitution | p.Gly631Asp | Substitution | Tail | FPLD2 | Familial partial lipodystrophy (Dunnigan type) | 151660 | # |
| 13575 | c.1930C>T | Substitution | p.Arg644Cys | Substitution | Tail | FPLD2 | Familial partial lipodystrophy (Dunnigan type) | 151660 | # |
| 13576 | c.1930C>T | Substitution | p.Arg644Cys | Substitution | Tail | FPLD2 | Familial partial lipodystrophy (Dunnigan type) | 151660 | # |
| 13579 | c.1930C>T | Substitution | p.Arg644Cys | Substitution | Tail | FPLD2 | Familial partial lipodystrophy (Dunnigan type) | 151660 | # |
| 17461 | c.1930C>T | Substitution | p.Arg644Cys | Substitution | Tail | FPLD2 | Familial partial lipodystrophy (Dunnigan type) | 151660 | # |
| 12601 | c.1411C>G | Substitution | p.Arg471Gly | Substitution | Tail | FPLD1 | Familial partial lipodystrophy (Köbberling) | 608600 | % |
| 11482 | c.[1583C>T; =]+[=; 1748C>T] | Substitution | p.[Thr528Met; =]+[=; Ser583Leu] | Substitution | Tail | FPLD1 | Familial partial lipodystrophy (Köbberling) | 608600 | % |
| 9183 | c.1748C>T | Substitution | p.Ser583Leu | Substitution | Tail | FPLD1 | Familial partial lipodystrophy (Köbberling) | 608600 | % |
| 11481 | c.1748C>T | Substitution | p.Ser583Leu | Substitution | Tail | FPLD1 | Familial partial lipodystrophy (Köbberling) | 608600 | % |
| 13490 | c.29C>T | Substitution | pThr10Ile | Substitution | Head | - | Generalized lipoatrophy syndrome | - | - |
| 8867 | c.398G>T | Substitution | p.Arg133Leu | Substitution | 1B | - | Generalized lipoatrophy syndrome | - | - |
| 17762 | c.1609-12T>G | Substitution | p.Glu536fsX14 | Frame shift | Tail | HSS | Hallermann-Streiff syndrome | 234100 | % |
| 17260 | c.1930C>T | Substitution | p.Arg644Cys | Substitution | Tail | HSS | Hallermann-Streiff syndrome | 234100 | % |
| 13153 | c.1609-12T>G | Substitution | p.Glu536fsX14 | Frame shift | Tail | - | Heart-hand syndrome, Slovenian Type | 610140 | % |
| 14097 | c.11C>G | Substitution | p.Pro4Arg | Substitution | Head | HGPS | Hutchinson-Gilford progeria syndrome | 176670 | # |
| 14112 | c.11C>G | Substitution | p.Pro4Arg | Substitution | Head | HGPS | Hutchinson-Gilford progeria **syndrome** | 176670 | # |
| 9172 | c.29C>T | Substitution | p.Thr10lle | Substitution | Head | HGPS | Hutchinson-Gilford progeria **syndrome** | 176670 | # |
| 14108 | c.29C>T | Substitution | p.Thr10lle | Substitution | Head | HGPS | Hutchinson-Gilford progeria **syndrome** | 176670 | # |
| 14110 | c.29C>T | Substitution | **p.Thr10Ile** | Substitution | Head | HGPS | Hutchinson-Gilford progeria **syndrome** | 176670 | # |
| 14101 | c.331G>T | Substitution | p.Glu111Lys | Substitution | 1B | HGPS | Hutchinson-Gilford progeria **syndrome** | 176670 | # |
| 14095 | c.406G>C | Substitution | p.Asp136His | Substitution | 1B | HGPS | Hutchinson-Gilford progeria **syndrome** | 176670 | # |
| 17070 | c.412G>A | Substitution | p.Glu138Lys | Substitution | 1B | HGPS | Hutchinson-Gilford progeria **syndrome** | 176670 | # |
| 9016 | c.428C>T | Substitution | p.Ser143Phe | Substitution | 1B | HGPS | Hutchinson-Gilford progeria **syndrome** | 176670 | # |
| 12609 | c.428C>T | Substitution | p.Ser143Phe | Substitution | 1B | HGPS | Hutchinson-Gilford progeria syndrome | 176670 | # |
| 8871 | c.433G>A | Substitution | p.Glu145Lys | Substitution | 1B | HGPS | Hutchinson-Gilford progeria **syndrome** | 176670 | # |
| 14106 | c.475G>A | Substitution | p.Glu159Lys | Substitution | 1B | HGPS | Hutchinson-Gilford progeria syndrome | 176670 | # |
| 17626 | c.899A>G | Substitution | p.Asp300Gly | Substitution | 2B | HGPS | Hutchinson-Gilford progeria **syndrome** | 176670 | # |
| **17903** | **c.917T>G** | **Substitution** | **p.Leu306Arg** | **Substitution** | **2B** | **HGPS** | **Hutchinson-Gilford progeria syndrome** | **176670** | **#** |
| **14114** | **c.1303C>T** | **Substitution** | **p.Arg435Cys** | **Substitution** | **Tail** | **HGPS** | **Hutchinson-Gilford progeria syndrome** | **176670** | **#** |
| **9393** | **c.1411C>T** | **Substitution** | **p.Arg471 Cys** | **Substitution** | **Tail** | **HGPS** | **Hutchinson-Gilford progeria syndrome** | **176670** | **#** |
| **12615** | **c.1411C>T** | **Substitution** | **p.Arg471 Cys** | **Substitution** | **Tail** | **HGPS** | **Hutchinson-Gilford progeria syndrome** | **176670** | **#** |
| **9394** | **c.1579C>T** | **Substitution** | **p.Arg527Cys** | **Substitution** | **Tail** | **HGPS** | **Hutchinson-Gilford progeria syndrome** | **176670** | **#** |
| **13119** | **c.1579C>T** | **Substitution** | **p.Arg527Cys** | **Substitution** | **Tail** | **HGPS** | **Hutchinson-Gilford progeria syndrome** | **176670** | **#** |
| **13651** | **c.1579C>T** | **Substitution** | **p.Arg527Cys** | **Substitution** | **Tail** | **HGPS** | **Hutchinson-Gilford progeria syndrome** | **176670** | **#** |
| **11671** | **c.[1583C>T; =]+[=; 1619T>C]** | **Substitution** | **p.[Thr528Met; =]+[=; Met540Thr]** | **Substitution** | **Tail** | **HGPS** | **Hutchinson-Gilford progeria syndrome** | **176670** | **#** |
| **9013** | **c.1626G>C** | **Substitution** | **p.Lys542Asn** | **Substitution** | **Tail** | **HGPS** | **Hutchinson-Gilford progeria syndrome** | **176670** | **#** |
| **9173** | **c.1733A>T** | **Substitution** | **p.Glu578Val** | **Substitution** | **Tail** | **HGPS** | **Hutchinson-Gilford progeria syndrome** | **176670** | **#** |
| **14099** | **c.1762T>C** | **Substitution** | **p.Cys588Arg** | **Substitution** | **Tail** | **HGPS** | **Hutchinson-Gilford progeria syndrome** | **176670** | **#** |
| **14104** | **c.1762T>C** | **Substitution** | **p.Cys588Arg** | **Substitution** | **Tail** | **HGPS** | **Hutchinson-Gilford progeria syndrome** | **176670** | **#** |
| **11785** | **c.1821G>A** | **Substitution** | **p.=** | **Silent** | **Not** affected | **HGPS** | **Hutchinson-Gilford progeria** syndrome | **176670** | **#** |
| **9527** | **c.1822G>A** | **Substitution** | **p.Gly608Ser** | **Substitution** | **Tail** | **HGPS** | **Hutchinson-Gilford progeria syndrome** | **176670** | **#** |
| **8876** | **c.1822G>A** | **Substitution** | **p.Gly608Ser** | **Substitution** | **Tail** | **HGPS** | **Hutchinson-Gilford progeria syndrome** | **176670** | **#** |
| **9396** | **c.1824C>T** | **Substitution** | **p.[=, Val607_Gln656del]** | **Silent, Deletion** | **Tail** | **HGPS** | **Hutchinson-Gilford progeria syndrome** | **176670** | **#** |
| **9398** | **c.1824C>T** | **Substitution** | **p.[=, Val607_Gln656del]** | **Silent, Deletion** | **Tail** | **HGPS** | **Hutchinson-Gilford progeria syndrome** | **176670** | **#** |
| **11428** | **c.1824C>T** | **Substitution** | **p.[=, Val607_Gln656del]** | **Silent, Deletion** | **Tail** | **HGPS** | **Hutchinson-Gilford progeria syndrome** | **176670** | **#** |
| **11429** | **c.1824C>T** | **Substitution** | **p.[=, Val607_Gln656del]** | **Silent, Deletion** | **Tail** | **HGPS** | **Hutchinson-Gilford progeria syndrome** | **176670** | **#** |
| **11430** | **c.1824C>T** | **Substitution** | **p.[=, Val607_Gln656del]** | **Silent, Deletion** | **Tail** | **HGPS** | Hutchinson-Gilford **progeria syndrome** | **176670** | **#** |
| **11431** | **c.1824C>T** | **Substitution** | **p.[=, Val607_Gln656del]** | **Silent, Deletion** | **Tail** | **HGPS** | **Hutchinson-Gilford progeria syndrome** | **176670** | **#** |
| **11432** | **c.1824C>T** | **Substitution** | **p.[=, Val607_Gln656del]** | **Silent, Deletion** | **Tail** | **HGPS** | **Hutchinson-Gilford progeria syndrome** | **176670** | **#** |
| **11433** | **c.1824C>T** | **Substitution** | **p.[=, Val607_Gln656del]** | **Silent, Deletion** | **Tail** | **HGPS** | **Hutchinson-Gilford progeria syndrome** | **176670** | **#** |
| **11434** | **c.1824C>T** | **Substitution** | **p.[=, Val607_Gln656del]** | **Silent, Deletion** | **Tail** | **HGPS** | **Hutchinson-Gilford progeria syndrome** | **176670** | **#** |
| **11435** | **c.1824C>T** | **Substitution** | **p.[=, Val607_Gln656del]** | **Silent, Deletion** | **Tail** | **HGPS** | **Hutchinson-Gilford progeria syndrome** | **176670** | **#** |
| **11436** | **c.1824C>T** | **Substitution** | **p.[=, Val607_Gln656del]** | **Silent, Deletion** | **Tail** | **HGPS** | **Hutchinson-Gilford progeria syndrome** | **176670** | **#** |
| **11437** | **c.1824C>T** | **Substitution** | **p.[=, Val607_Gln656del]** | **Silent, Deletion** | **Tail** | **HGPS** | **Hutchinson-Gilford progeria syndrome** | **176670** | **#** |
| **11438** | **c.1824C>T** | **Substitution** | **p.[=, Val607_Gln656del]** | **Silent, Deletion** | **Tail** | **HGPS** | **Hutchinson-Gilford progeria syndrome** | **176670** | **#** |
| **11439** | **c.1824C>T** | **Substitution** | **p.[=, Val607_Gln656del]** | **Silent, Deletion** | **Tail** | **HGPS** | **Hutchinson-Gilford progeria syndrome** | **176670** | **#** |
| **11440** | **c.1824C>T** | **Substitution** | **p.[=, Val607_Gln656del]** | **Silent, Deletion** | **Tail** | **HGPS** | **Hutchinson-Gilford progeria syndrome** | **176670** | **#** |
| **11441** | **c.1824C>T** | **Substitution** | **p.[=, Val607_Gln656del]** | **Silent, Deletion** | **Tail** | **HGPS** | **Hutchinson-Gilford progeria syndrome** | **176670** | **#** |
| **11442** | **c.1824C>T** | **Substitution** | **p.[=, Val607_Gln656del]** | **Silent, Deletion** | **Tail** | **HGPS** | **Hutchinson-Gilford progeria syndrome** | **176670** | **#** |
| **11443** | **c.1824C>T** | **Substitution** | **p.[=, Val607_Gln656del]** | **Silent, Deletion** | **Tail** | **HGPS** | **Hutchinson-Gilford progeria syndrome** | **176670** | **#** |
| **11444** | **c.1824C>T** | **Substitution** | **p.[=, Val607_Gln656del]** | **Silent, Deletion** | **Tail** | **HGPS** | **Hutchinson-Gilford progeria syndrome** | **176670** | **#** |
| **9395** | **c.1824C>T** | **Substitution** | **p.?** | **Unknown** | **Unknown** | **HGPS** | **Hutchinson-Gilford progeria syndrome** | **176670** | **#** |
| **11449** | **c.1824C>T** | **Substitution** | **p.?** | **Unknown** | **Unknown** | **HGPS** | **Hutchinson-Gilford progeria syndrome** | **176670** | **#** |
| **11450** | **c.1824C>T** | **Substitution** | **p.?** | **Unknown** | **Unknown** | **HGPS** | **Hutchinson-Gilford progeria syndrome** | **176670** | **#** |
| **11451** | **c.1824C>T** | **Substitution** | **p.?** | **Unknown** | **Unknown** | **HGPS** | **Hutchinson-Gilford progeria syndrome** | **176670** | **#** |
| **11452** | **c.1824C>T** | **Substitution** | **p.?** | **Unknown** | **Unknown** | **HGPS** | Hutchinson-Gilford progeria **syndrome** | **176670** | **#** |
| **9171** | **c.1824C>T** | **Substitution** | **p.[=, Val607_Gln656del]** | **Silent, Deletion** | **Tail** | **HGPS** | **Hutchinson-Gilford progeria syndrome** | **176670** | **#** |
| **9017** | **c.1824C>T** | **Substitution** | **p.[=, Val607_Gln656del]** | **Silent, Deletion** | **Tail** | **HGPS** | **Hutchinson-Gilford progeria syndrome** | **176670** | **#** |
| **9019** | **c.1824C>T** | **Substitution** | **p.[=, Val607_Gln656del]** | **Silent, Deletion** | **Tail** | **HGPS** | **Hutchinson-Gilford progeria syndrome** | **176670** | **#** |
| **12074** | **c.1824C>T** | **Substitution** | **p.[=, Val607_Gln656del]** | **Silent, Deletion** | **Not affected** | **HGPS** | **Hutchinson-Gilford progeria syndrome** | **176670** | **#** |
| **12075** | **c.1824C>T** | **Substitution** | **p.[=, Val607_Gln656del]** | **Silent, Deletion** | **Not affected** | **HGPS** | **Hutchinson-Gilford progeria syndrome** | **176670** | **#** |
| **12076** | **c.1824C>T** | **Substitution** | **p.[=, Val607_Gln656del]** | **Silent, Deletion** | **Not affected** | **HGPS** | **Hutchinson-Gilford progeria syndrome** | **176670** | **#** |
| **12085** | **c.1824C>T** | **Substitution** | **p.[=, Val607_Gln656del]** | **Silent, Deletion** | **Not affected** | **HGPS** | **Hutchinson-Gilford progeria syndrome** | **176670** | **#** |
| **12354** | **c.1824C>T** | **Substitution** | **p.=** | **Silent** | **Not affected** | **HGPS** | **Hutchinson-Gilford progeria syndrome** | **176670** | **#** |
| **13533** | **c.1824C>T** | **Substitution** | **p.[=, Val607 Gln656del]** | **Silent, Deletion** | **Tail** | **HGPS** | **Hutchinson-Gilford progeria syndrome** | **176670** | **#** |
| **17393** | **c.1824C>T** | **Substitution** | **p.=** | **Silent** | **Not affected** | **HGPS** | **Hutchinson-Gilford progeria syndrome** | **176670** | **#** |
| **17395** | **c.1824C>T** | **Substitution** | **p.=** | **Silent** | **Not affected** | **HGPS** | **Hutchinson-Gilford progeria syndrome** | **176670** | **#** |
| **17564** | **c.1824C>T** | **Substitution** | **p.=** | **Silent** | **Not affected** | **HGPS** | **Hutchinson-Gilford progeria syndrome** | **176670** | **#** |
| **17729** | **c.1824C>T** | **Substitution** | **p.=** | **Silent** | **Not affected** | **HGPS** | **Hutchinson-Gilford progeria syndrome** | **176670** | **#** |
| **17818** | **c.1824C>T** | **Substitution** | **p.=** | **Silent** | **Not affected** | **HGPS** | **Hutchinson-Gilford progeria syndrome** | **176670** | **#** |
| **9009** | **c.1868C>G** | **Substitution** | **p.[Thr623Ser, Val622 Gln656del]** | **Deletion, Substitution** | **Tail** | **HGPS** | **Hutchinson-Gilford progeria syndrome** | **176670** | **#** |
| **12457** | **c.1868C>G** | **Substitution** | **p.[Thr623Ser, Val622 Gln656del]** | **Deletion, Substitution** | **Tail** | **HGPS** | **Hutchinson-Gilford progeria syndrome** | **176670** | **#** |
| **9174** | **c.1930C>T** | **Substitution** | **p.Arg644Cys** | **Substitution** | **Tail** | **HGPS** | **Hutchinson-Gilford progeria syndrome** | **176670** | **#** |
| **11615** | **c.1960C>T** | **Substitution** | **p.Arg654X** | **Substitution** | **Tail** | **HGPS** | **Hutchinson-Gilford progeria syndrome** | **176670** | **#** |
| **17159** | **c.1968G>A** | **Substitution** | **p.=** | **Silent** | **Not affected** | **HGPS** | **Hutchinson-Gilford progeria syndrome** | **176670** | **#** |
| **11784** | **c.1968+1G>A** | **Substitution** | **p.Val607 Gin656del** | **Deletion** | **Tail** | **HGPS** | **Hutchinson-Gilford progeria syndrome** | **176670** | **#** |
| **17161** | **c.1968+5G>A** | **Substitution** | **p.Val607 Gin656del** | **Deletion** | **Tail** | **HGPS** | **Hutchinson-Gilford progeria syndrome** | **176670** | **#** |
| **12615** | **c.1411C>T** | **Substitution** | **p.Arg471Cys** | **Substitution** | **Tail** | **-** | **Lamin-related rigid spine muscular dystrophy** | **-** | **-** |
| **17426** | **c.31delC** | **Deletion** | **p.Arg11AlafsX85** | **Frame shift** | **Head** | **LGMD1B** | **Limb-girdle muscular dystrophy type 1B** | **159001** | **#** |
| **14254** | **c.73C>G** | **Substitution** | **p.Arg25Gly** | **Substitution** | **Head** | **LGMD1B** | **Limb-girdle muscular dystrophy type 1B** | **159001** | **#** |
| **17886** | **c.80C>T** | **Substitution** | **p.Thr27lle** | **Substitution** | **Head** | **LGMD1B** | **Limb-girdle muscular dystrophy type 1B** | **159001** | **#** |
| **18488** | **c.80C>T** | **Substitution** | **p.Thr27lle** | **Substitution** | **Head** | **LGMD1B** | **Limb-girdle muscular dystrophy type 1B** | **159001** | **#** |
| **17428** | **c.99G>C** | **Substitution** | **p.Glu33Asp** | **Substitution** | **1A** | **LGMD1B** | **Limb-girdle muscular dystrophy type 1B** | **159001** | **#** |
| **17429** | **c.99G>C** | **Substitution** | **p.Glu33Asp** | **Substitution** | **1A** | **LGMD1B** | **Limb-girdle muscular dystrophy type 1B** | **159001** | **#** |
| **17430** | **c.99G>C** | **Substitution** | **p.Glu33Asp** | **Substitution** | **1A** | **LGMD1B** | **Limb-girdle muscular dystrophy type 1B** | **159001** | **#** |
| **17434** | **c.194A>G** | **Substitution** | **p.Glu65Gly** | **Substitution** | **1A** | **LGMD1B** | **Limb-girdle muscular dystrophy type 1B** | **159001** | **#** |
| **17435** | **c.194A>G** | **Substitution** | **p. Glu65Gly** | **Substitution** | **1A** | **LGMD1B** | **Limb-girdle muscular dystrophy type 1B** | **159001** | **#** |
| **16283** | **c.302G>C** | **Substitution** | **p.Arg101Pro** | **Substitution** | **1B** | **LGMD1B** | **Limb-girdle muscular dystrophy type 1B** | **159001** | **#** |
| **17399** | **c.388G>T** | **Substitution** | **p.Ala130Ser** | **Substitution** | **1B** | **LGMD1B** | **Limb-girdle muscular dystrophy type 1B** | **159001** | **#** |
| **17755** | **c.388G>T** | **Substitution** | **p.Ala130Ser** | **Substitution** | **1B** | **LGMD1B** | **Limb-girdle muscular dystrophy type 1B** | **159001** | **#** |
| **17432** | **c.471G>A** | **Substitution** | **p.=** | **Silent** | **Not affected** | **LGMD1B** | **Limb-girdle muscular dystrophy type 1B** | **159001** | **#** |
| **9216** | **c.513G>A** | **Substitution** | **p.=** | **Silent** | **Not affected** | **LGMD1B** | **Limb-girdle muscular dystrophy type 1B** | **159001** | **#** |
| **17575** | **c.513+1G>A** | **Substitution** | **p.?** | **Unknown** | **Unknown** | **LGMD1B** | **Limb-girdle muscular dystrophy type 1B** | **159001** | **#** |
| **17437** | **c.565C>T** | **Substitution** | **p.Arg189Trp** | **Substitution** | **1B** | **LGMD1B** | **Limb-girdle muscular dystrophy type 1B** | **159001** | **#** |
| **9639** | **c.622_624delAAG** | **Deletion** | **p.Lys208del** | **Deletion** | **1B** | **LGMD1B** | **Limb-girdle muscular dystrophy type 1B** | **159001** | **#** |
| **12403** | **c.624_626delGAA** | **Deletion** | **p.Lys208del** | **Deletion** | **1B** | **LGMD1B** | **Limb-girdle muscular dystrophy type 1B** | **159001** | **#** |
| **16145** | **c.673C>T** | **Substitution** | **p.Arg225X** | **Substitution** | **L12** | **LGMD1B** | **Limb-girdle muscular dystrophy type 1B** | **159001** | **#** |
| **17401** | **c.673C>T** | **Substitution** | **p.Arg225X** | **Substitution** | **L12** | **LGMD1B** | **Limb-girdle muscular dystrophy type 1B** | **159001** | **#** |
| **17753** | **c.673C>T** | **Substitution** | **p.Arg225X** | **Substitution** | **L12** | **LGMD1B** | **Limb-girdle muscular dystrophy type 1B** | **159001** | **#** |
| **13555** | **c.746G>A** | **Substitution** | **p.Arg249Gln** | **Substitution** | **2A** | **LGMD1B** | **Limb-girdle muscular dystrophy type** 1B | **159001** | **#** |
| **9164** | **c.777T>A** | **Substitution** | **p.Tyr259X** | **Substitution** | **L2** | **LGMD1B** | **Limb-girdle muscular dystrophy type 1B** | **159001** | **#** |
| **12401** | **c.777T>A** | **Substitution** | **p.Tyr259X** | **Substitution** | **L2** | **LGMD1B** | **Limb-girdle muscular dystrophy type 1B** | **159001** | **#** |
| **17760** | **c.777T>A** | **Substitution** | **p.Tyr259X** | **Substitution** | **L2** | **LGMD1B** | **Limb-girdle muscular dystrophy type 1B** | **159001** | **#** |
| **11810** | **c.855delG** | **Deletion** | **p.Ala287LeufsX191** | **Frame shift** | **2B** | **LGMD1B** | **Limb-girdle muscular dystrophy type 1B** | **159001** | **#** |
| **9021** | **c.864_867delCCAC** | **Deletion** | **p.His289ArgfsX190** | **Frame shift** | **2B** | **LGMD1B** | **Limb-girdle muscular dystrophy type 1B** | **159001** | **#** |
| **11759** | **c.864_867delCCAC** | **Deletion** | **p.His289ArgfsX190** | **Frame shift** | **2B** | **LGMD1B** | **Limb-girdle muscular dystrophy type 1B** | **159001** | **#** |
| **12384** | **c.908_909delCT** | **Deletion** | **p.Ser303CysfsX26** | **Frame shift** | **2B** | **LGMD1B** | **Limb-girdle muscular dystrophy type 1B** | **159001** | **#** |
| **11811** | **c.992G>C** | **Substitution** | p.Arg331Pro | **Substitution** | **2B** | **LGMD1B** | **Limb-girdle muscular dystrophy type** 1B | **159001** | **#** |
| **18309** | **c.1001_1003delGCC** | **Deletion** | **p.Ser334del** | **Deletion** | **2B** | **LGMD1B** | **Limb-girdle muscular dystrophy type 1B** | **159001** | **#** |
| **8794** | **c.1130G>A** | **Substitution** | **p.Arg377His** | **Substitution** | **2B** | **LGMD1B** | **Limb-girdle muscular dystrophy type 1B** | **159001** | **#** |
| **8850** | **c.1130G>T** | **Substitution** | **p.Arg377Leu** | **Substitution** | **2B** | **LGMD1B** | **Limb-girdle muscular dystrophy type 1B** | **159001** | **#** |
| **9301** | **c.1130G>A** | **Substitution** | **p.Arg377His** | **Substitution** | **2B** | **LGMD1B** | **Limb-girdle muscular dystrophy type 1B** | **159001** | **#** |
| **9024** | **c.1130G>T** | **Substitution** | **p.Arg377Leu** | **Substitution** | **2B** | **LGMD1B** | **Limb-girdle muscular dystrophy type 1B** | **159001** | **#** |
| **11762** | **c.1130G>A** | **Substitution** | **p.Arg377His** | **Substitution** | **2B** | **LGMD1B** | **Limb-girdle muscular dystrophy type 1B** | **159001** | **#** |
| **13557** | **c.1130G>A** | **Substitution** | **p.Arg377His** | **Substitution** | **2B** | **LGMD1B** | **Limb-girdle muscular dystrophy type 1B** | **159001** | **#** |
| **11755** | **c.1146C>T** | **Substitution** | **p.=** | **Silent** | **Not affected** | **LGMD1B** | **Limb-girdle muscular dystrophy type 1B** | **159001** | **#** |
| **11756** | **c.1357C>T** | **Substitution** | **p.Arg453Trp** | **Substitution** | **Tail** | **LGMD1B** | **Limb-girdle muscular dystrophy type 1B** | **159001** | **#** |
| **17443** | **c.1357C>T** | **Substitution** | **p.Arg453Trp** | **Substitution** | **Tail** | **LGMD1B** | **Limb-girdle muscular dystrophy type 1B** | **159001** | **#** |
| **11812** | **c.1370delA** | **Deletion** | **p.Lys457SerfsX21** | **Frame shift** | **Tail** | **LGMD1B** | **Limb-girdle muscular dystrophy type 1B** | **159001** | **#** |
| **17441** | **c.1380+1G>A** | **Substitution** | **p.?** | **Unknown** | **Unknown** | **LGMD1B** | **Limb-girdle muscular dystrophy type 1B** | **159001** | **#** |
| **8839** | **c.1441T>C** | **Substitution** | **p.Tyr481His** | **Substitution** | **Tail** | **LGMD1B** | **Limb-girdle muscular dystrophy type 1B** | **159001** | **#** |
| **13376** | **c.1488+5G>A** | **Substitution** | **p.?** | **Unknown** | **Unknown** | **LGMD1B** | **Limb-girdle muscular dystrophy type 1B** | **159001** | **#** |
| **9181** | **c.1494G>T** | **Substitution** | **p.Trp498Cys** | **Substitution** | **Tail** | **LGMD1B** | **Limb-girdle muscular dystrophy type 1B** | **159001** | **#** |
| **9158** | **c.1494G>T** | **Substitution** | **p.Trp498Cys** | **Substitution** | **Tail** | **LGMD1B** | **Limb-girdle muscular dystrophy type 1B** | **159001** | **#** |
| **16442** | **c.1494G>T** | **Substitution** | **p.Trp498Cys** | **Substitution** | **Tail** | **LGMD1B** | **Limb-girdle muscular dystrophy type 1B** | **159001** | **#** |
| **9159** | **c.1535T>C** | **Substitution** | **p.Leu512Pro** | **Substitution** | **Tail** | **LGMD1B** | **Limb-girdle muscular dystrophy type 1B** | **159001** | **#** |
| **11763** | **c.1535T>C** | **Substitution** | **p.Leu512Pro** | **Substitution** | **Tail** | **LGMD1B** | **Limb-girdle muscular dystrophy type 1B** | **159001** | **#** |
| **17449** | **c.1535T>C** | **Substitution** | **p.Leu512Pro** | **Substitution** | **Tail** | **LGMD1B** | **Limb-girdle muscular dystrophy type 1B** | **159001** | **#** |
| **17452** | **c.1535T>C** | **Substitution** | **p.Leu512Pro** | **Substitution** | **Tail** | **LGMD1B** | **Limb-girdle muscular dystrophy type 1B** | **159001** | **#** |
| **11758** | **c.1583C>A** | **Substitution** | **p.Thr528Lys** | **Substitution** | **Tail** | **LGMD1B** | **Limb-girdle muscular dystrophy type 1B** | **159001** | **#** |
| **13446** | **c.1608+1G>A** | **Substitution** | **p.?** | **Unknown** | **Unknown** | **LGMD1B** | **Limb-girdle muscular dystrophy type 1B** | **159001** | **#** |
| **9412** | **c.1608+5G>C** | **Substitution** | **p.Glu537ValfsX36** | | **Tail** | **LGMD1B** | **Limb-girdle muscular dystrophy type 1B** | **159001** | **#** |
| **13151** | **c.1609-3C>G** | **Substitution** | **p.?** | **Unknown** | **Unknown** | **LGMD1B** | **Limb-girdle muscular dystrophy type 1B** | **159001** | **#** |
| **11764** | **c.1718C>T** | **Substitution** | **p.Ser573Leu** | **Substitution** | **Tail** | **LGMD1B** | **Limb-girdle muscular dystrophy type 1B** | **159001** | **#** |
| **13505** | **c.373G>A** | **Substitution** | **p.Gly125Ser** | **Substitution** | **1B** | **LAF** | **Lone atrial fibrillation** | **-** | **-** |
| **13509** | **c.[373G>A; =]+[=; 1243G>A]** | **Substitution** | **p.[Gly125Ser; =]+[=; Val415Ile]** | **Substitution** | **1B, Tail** | **LAF** | **Lone atrial fibrillation** | **-** | **-** |
| **13492** | **c.810+63C>A** | **Substitution** | **p.?** | **Unknown** | **Unknown** | **LAF** | **Lone atrial fibrillation** | **-** | **-** |
| **13495** | **c.937-46A>G** | **Substitution** | **p.?** | **Unknown** | **Unknown** | **LAF** | **Lone atrial fibrillation** | **-** | **-** |
| **13501** | **c.1149G>A** | **Substitution** | **p.=** | **Silent** | **Not affected** | **LAF** | **Lone atrial fibrillation** | **-** | **-** |
| **13497** | **c.1158-44C>T** | **Substitution** | **p.?** | **Unknown** | **Unknown** | **LAF** | **Lone atrial fibrillation** | **-** | **-** |
| **13499** | **c.1158-44C>T** | **Substitution** | **p.?** | **Unknown** | **Unknown** | **LAF** | **Lone atrial fibrillation** | **-** | **-** |
| **13507** | **c.1243G>A** | **Substitution** | **p.Val415Ile** | **Substitution** | **Tail** | **LAF** | **Lone atrial fibrillation** | **-** | **-** |
| **13511** | **c.1462A>C** | **Substitution** | **p.Thr488Pro** | **Substitution** | **Tail** | **LAF** | **Lone atrial fibrillation** | **-** | **-** |
| **13503** | **c.1803C>T** | **Substitution** | **p.=** | **Silent** | **Not affected** | **LAF** | **Lone atrial fibrillation** | **-** | **-** |
| 11783 | c.176T>G | Substitution | p.Leu59Arg | Substitution | 1A | MADA | Mandibuloacral dysplasia with type A lipodystrophy | 248370 | # |
| 18470 | c.683A>T | Substitution | p.Glu228Val | Substitution | L12 | MADA | Mandibuloacral dysplasia with type A lipodystrophy | 248370 | # |
| 12380 | c.[1318G>A; =]+[=; 1580G>A] | Substitution | p.[Val440Met; =]+[=; Arg527His] | Substitution | Tail | MADA | Mandibuloacral dysplasia with type A lipodystrophy | 248370 | # |
| 12615 | c.1411C>T | Substitution | p.Arg471Cys | Substitution | Tail | MADA | Mandibuloacral dysplasia with type A lipodystrophy | 248370 | # |
| 13119 | c.1579C>T | Substitution | p.Arg527Cys | Substitution | Tail | MADA | Mandibuloacral dysplasia with type A lipodystrophy | 248370 | # |
| 17878 | c.1579C>T | Substitution | p.Arg527Cys | Substitution | Tail | MADA | Mandibuloacral dysplasia with type A lipodystrophy | 248370 | # |
| 8851 | c.1580G>A | Substitution | p.Arg527His | Substitution | Tail | MADA | Mandibuloacral dysplasia with type A lipodystrophy | 248370 | # |
| 11419 | c.1580G>A | Substitution | p.Arg527His | Substitution | Tail | MADA | Mandibuloacral dysplasia with type A lipodystrophy | 248370 | # |
| 11420 | c.1580G>A | Substitution | p.Arg527His | Substitution | Tail | MADA | Mandibuloacral dysplasia with type A lipodystrophy | 248370 | # |
| 11421 | c.1580G>A | Substitution | p.Arg527His | Substitution | Tail | MADA | Mandibuloacral dysplasia with type A lipodystrophy | 248370 | # |
| 11422 | c.1580G>A | Substitution | p.Arg527His | Substitution | Tail | MADA | Mandibuloacral dysplasia with type A lipodystrophy | 248370 | # |
| 8877 | c.1580G>A | Substitution | p.Arg527His | Substitution | Tail | MADA | Mandibuloacral dysplasia with type A lipodystrophy | 248370 | # |
| 11453 | c.1580G>A | Substitution | p.Arg527His | Substitution | Tail | MADA | Mandibuloacral dysplasia with type A lipodystrophy | 248370 | # |
| 8995 | c.1580G>A | Substitution | p.Arg527His | Substitution | Tail | MADA | Mandibuloacral dysplasia with type A lipodystrophy | 248370 | # |
| 9224 | c.1580G>A | Substitution | p.Ala527His | Substitution | Tail | MADA | Mandibuloacral dysplasia with type A lipodystrophy | 248370 | # |
| 17602 | c.1580G>T | Substitution | p.Arg527Leu | Substitution | Tail | MADA | Mandibuloacral dysplasia with type A lipodystrophy | 248370 | # |
| 17603 | c.1580G>T | Substitution | p.Arg527Leu | Substitution | Tail | MADA | Mandibuloacral dysplasia with type A lipodystrophy | 248370 | # |
| 17747 | c.1580G>T | Substitution | p.Arg527Leu | Substitution | Tail | MADA | Mandibuloacral dysplasia with type A lipodystrophy | 248370 | # |
| 17748 | c.1580G>T | Substitution | p.Arg527Leu | Substitution | Tail | MADA | Mandibuloacral dysplasia with type A lipodystrophy | 248370 | # |
| 12598 | c.1585G>A | Substitution | p.Ala529Thr | Substitution | Tail | MADA | Mandibuloacral dysplasia with type A lipodystrophy | 248370 | # |
| 9317 | c.1586C>T | Substitution | p.Ala529Val | Substitution | Tail | MADA | Mandibuloacral dysplasia with type A lipodystrophy | 248370 | # |
| 18478 | c.1620G>A | Substitution | p.Met540Ile | Substitution | Tail | MADA | Mandibuloacral dysplasia with type A lipodystrophy | 248370 | # |
| 13054 | c.82C>T | Substitution | p.Arg28Trp | Substitution | Head | - | Metabolic syndrome | - | - |
| 13056 | c.274C>T | Substitution | p.Leu92Phe | Substitution | 1B | - | Metabolic syndrome | - | - |
| 13059 | c.1159C>G | Substitution | p.Leu387Val | Substitution | 2B | - | Metabolic syndrome | - | - |
| 13061 | c.1184C>T | Substitution | p.Ser395Leu | Substitution | Not affected | - | Metabolic syndrome | - | - |
| 13063 | c.1196G>A | Substitution | p.Arg399His | Substitution | Tail | - | Metabolic syndrome | - | - |
| 13065 | c.1262T>C | Substitution | p.Leu421Pro | Substitution | Tail | - | Metabolic syndrome | - | - |
| 13067 | c.1315C>T | Substitution | p.Arg439Cys | Substitution | Tail | - | Metabolic syndrome | - | - |
| 13069 | c.1516C>G | Substitution | p.His506Asp | Substitution | Tail | - | Metabolic syndrome | - | - |
| 13047 | c.1698C>T | Substitution | p.= | Silent | Not affected | - | Metabolic syndrome | - | - |
| 13071 | c.1961_1962insG | Insertion | p.Thr655AsnfsX49 | | Tail | - | Metabolic syndrome | - | - |
| **12408** | **c.73C>T** | **Substitution** | **p.Arg25Cys** | **Substitution** | **Head** | **-** | **Muscular dystrophy** | **-** | **-** |
| **12410** | **c.1130G>T** | **Substitution** | **p.Arg377Leu** | **Substitution** | **2B** | **-** | **Muscular dystrophy** | **-** | **-** |
| **12412** | **c.1622G>C** | **Substitution** | **p.Arg541Pro** | **Substitution** | **Tail** | **-** | **Muscular dystrophy** | **-** | **-** |
| **12414** | **c.1045C>T** | **Substitution** | **p.Arg349Trp** | **Substitution** | **2B** | **-** | **Muscular dystrophy and lipodystrophy** | **-** | **-** |
| **17411** | **c.1821G>A** | **Substitution** | **p.=** | **Silent** | **Not affected** | **WRS** | **Progeroid syndrome, neonatal** | **264090** | **%** |
| **18482** | **c.1940T>G** | **Substitution** | **p.Leu647Arg** | **Substitution** | **Tail** | **WRS** | **Progeroid syndrome, neonatal** | **264090** | **%** |
| 17009 | c.1303C>T | Substitution | p.Arg435Cys | Substitution | Tail | RD | Restrictive dermopathy | 275210 | # |
| 17731 | c.1303C>T | Substitution | p.Arg435Cys | Substitution | Tail | RD | Restrictive dermopathy | 275210 | # |
| 17801 | c.1303C>T | Substitution | p.Arg435Cys | Substitution | Tail | RD | Restrictive dermopathy | 275210 | # |
| 9166 | c.1824C>T | Substitution | p.[=, Val607_Gln656del] | Silent, Deletion | Tail | RD | Restrictive dermopathy | 275210 | # |
| 9208 | c.1968+1G>A | Substitution | p.Gly567_Gln656del | Deletion | Tail | RD | Restrictive dermopathy | 275210 | # |
| **17980** | **c.1057C>T** | **Substitution** | **p.Gln353X** | **Substitution** | **2B** | **-** | **Spinal muscular atrophy with cardiac involvment** | **-** | **-** |
| **18424** | **c.868G>A** | **Substitution** | **p.Glu290Lys** | **Substitution** | **2B** | **SCD** | **Sudden cardiac death** | **115080** | **#** |
| **17189** | **c.908_909deICT** | **Deletion** | **p.Ser303CysfsX26** | **Frame shift** | **2B** | **SCD** | **Sudden cardiac death** | **115080** | **#** |
| **17901** | **c.1334T>A** | **Substitution** | **p.Val445Glu** | **Substitution** | **Tail** | **SCD** | **Sudden cardiac death** | **115080** | **#** |
| 9022 | c.1804G>A | Substitution | p.Gly602Ser | Substitution | Tail | - | Type A insulin resistance syndrome | - | - |

In some embodiments the laminopathy is not Hutchinson-Gilford Progeria Syndrome (HGPS). In some embodiments, where the mutation is to *LMNA,* it is a *LMNA* mutation which does not result in an increase in the level of progerin. In some embodiments, where the mutation is to *LMNA,* it is not a HGPS-associated mutation.

Further aspects of the present invention concern the treatment/prevention of diseases characterised by hyperlipidemia.

Further aspects of the present invention concern the treatment/prevention of diseases associated with LDL receptor deficiency (i.e. a reduced level of LDL receptor protein and/or function).

Hyperlipidemia refers to an elevated level of lipid or lipoprotein in the blood. Hyperlipidemia includes hypertriglyceridemia, hypercholesterolemia and combined hyperlipidemia (combination of hypertriglyceridemia and hypercholesterolemia). Hyperlipidemia is associated e.g. with atherosclerosis, hypertension and cardiovascular disease.

Hypercholesterolemia is described e.g. in Bhatnagar et al., BMJ (2008) 337:a993. The UK NHS defines hypercholesterolemia as blood total cholesterol level of ≥5 mmol/L or blood low-density lipoprotein (LDL) level of ≥3 mmol/L. The US NIH defines hypercholesterolemia as blood total cholesterol level of ≥240 mg/dL. Hypertriglyceridemia is described e.g. in Berglund et al., J. Clin. Endocrinol. Metab. (2012) 97(9):2969-89, and is defined by blood triglyceride level ≥150 mg/dL ≥1.7 mmol/L).

In some embodiments, the disease characterised by hyperlipidemia may be a familial hyperlipidemia or an acquired (secondary) hyperlipidemia.

In some embodiments a familial hyperlipidemia is selected from Buerger-Gruetz syndrome, familial apoprotein CII deficiency, type Ic hyperlipoproteinemia, familial hypercholesterolemia, familial combined hyperlipidemia, familial dysbetalipoproteinemia, familial hypertriglyceridemia and type V hyperlipoproteinemia. In some embodiments a familial hyperlipidemia is familial hypercholesterolemia.

LDL receptor deficiency may arise e.g. as a result of mutation to *LDLR.* Accordingly, aspects of the present invention concern the treatment/prevention of diseases associated with mutation to *LDLR.* In some embodiments the mutation is known or predicted to reduce the level of one or more LDL receptor isoforms encoded by the wildtype *LDLR* allele and/or increase the level of one or more disease-associated LDL receptor variants. In some embodiments the disease is associated with mutation to *LDLR* is familial hypercholesterolemia.

In some embodiments, the disease to be treated/prevented in accordance with the present invention is characterised by one or more of hyperlipidemia, hypercholesterolemia, atherosclerosis, stenosis or hypertension. In some embodiments, the disease to be treated/prevented in accordance with the present invention is characterised by atherosclerosis.

In some embodiments, the disease to be treated/prevented is selected from atherosclerosis, cardiovascular disease, stroke and a familial hyperlipidemia.

In some embodiments, the methods comprise determining whether a subject has a disease described herein. In some embodiments the methods comprise diagnosing a disease described herein. Determining a whether a subject has a disease described herein may comprise analysing a subject for one or more symptoms/correlates of the disease.

In some embodiments the subject may be suspected of having or suffering from a disease, e.g. based on the presence of other symptoms indicative of the disease in the subject or in a cell/tissue/organ of the subject. In some embodiments the subject may be considered at risk of developing the disease, e.g. because of genetic predisposition or other risk factors for the disease.

In some embodiments, the methods comprise determining whether a subject has a mutation to a gene described herein. In some embodiments the methods comprise detecting a mutation to a gene described herein.

Determination of mutation to a gene described herein may confirm a diagnosis or suspected diagnosis, or may confirm that the subject is at risk of developing the disease. The determination may diagnose a disease, or predisposition to a disease, for treatment/prevention with a LINC complex inhibitor.

Genetic factors may be assayed by methods known to those of ordinary skill in the art, including PCR based and sequencing assays. By determining the presence of genetic factors, e.g. in a sample obtained from a subject, a diagnosis may be confirmed, and/or a subject may be classified as being at risk of developing a disease described herein, and/or a subject may be identified as being suitable for treatment with a LINC complex inhibitor.

Assays may be performed *in vitro* on a sample obtained from a subject, or following processing of a sample obtained from a subject. The sample obtained from a subject may be of any kind. A biological sample may be taken from any tissue or bodily fluid, e.g. a blood sample, blood-derived sample, serum sample, lymph sample, semen sample, saliva sample, synovial fluid sample. A blood-derived sample may be a selected fraction of a patient's blood, e.g. a selected cell-containing fraction or a plasma or serum fraction. A sample may comprise a tissue sample or biopsy; or cells isolated from a subject.

In some embodiments, the methods comprise determining whether a subject comprises mutation to an allele of one or more of *LMNA, LMNB1, LMNB2, EMD, LAP2, LBR, ZMPSTE24, SYN*E-1 and *NUP62.* In some embodiments, the methods comprise determining whether a subject comprises mutation to an allele of one or more of *LMNA, LMNB1* and *LMNB2.* In some embodiments, the methods comprise determining whether a subject comprises mutation to an allele of *LMNA.* In some embodiments, the methods comprise determining whether a subject comprises mutation to an allele of *LDLR.*

In such embodiments, detection of mutation may identify a subject as a subject to be administered a LING complex inhibitor according to the present disclosure. Accordingly, in some embodiments the methods comprise selecting a subject determined to comprise mutation to one or more of *LMNA, LMNB1, LMNB2, EMD, LAP2, LBR, ZMPSTE24, SYNE*-1 and *NUP62* for administration with a LING complex inhibitor. In some embodiments the methods comprise selecting a subject determined to comprise mutation to one or more of *LMNA, LMNB1* and *LMNB2* for administration with a LING complex inhibitor. In some embodiments the methods comprise selecting a subject determined to comprise mutation to *LMNA* for administration with a LING complex inhibitor. In some embodiments the methods comprise selecting a subject determined to comprise mutation to *LDLR* for administration with a LING complex inhibitor.

In some embodiments, the methods comprise testing a sample obtained from a subject suspected of having a disease for the presence or absence of at least one *LMNA* mutation; wherein the presence of at least one *LMNA* mutation indicates that the subject is to be administered a LING complex inhibitor according to the present disclosure.

In accordance with various aspects of the present invention, a method of treating and/or preventing a disease according to the present invention may comprise one or more of the following:
Increasing survival of a subject having the disease;
Increasing the lifespan of a subject having the disease;
Increasing cardiac function;
Delaying the onset of decline of cardiac function;
Increasing myocardial contractility;
Increasing ejection fraction and/or fractional shortening;
Reducing atherosclerosis.

### Subjects

A subject in accordance with the present disclosure may be any animal. In some embodiments a subject may be mammalian. In some embodiments a subject may be human. In some embodiments a subject may be a non-human animal, e.g. a non-human mammal. The subject may be male or female.

The subject may be a patient. The patient may have a disease described herein. A subject may have been diagnosed with a disease described herein, may be suspected of having a disease described herein, or may be at risk from developing a disease described herein.

In embodiments according to the present invention, a subject/patient may be selected for therapy/prophylaxis according to the methods described herein based on characterisation for markers of a disease described herein.

### Numbered paragraphs

The following numbered paragraphs (paras) provide further statements of features and combinations of features which are contemplated in connection with the present invention.

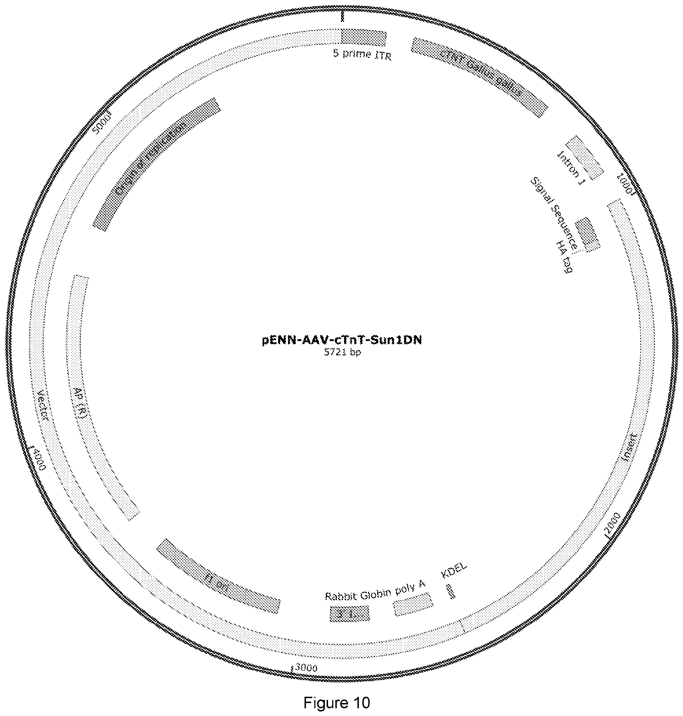

In accordance with some of the various aspects and embodiments of the present invention, subject-matter according to the following numbered paras may be specifically disclaimed.
1. An isolated nucleic acid molecule, wherein the nucleic acid molecule comprises an expression vector and a transgene, whereby the transgene is operably linked to the expression vector, wherein expression of the transgene in a transfected cell results in disruption of a Linker of Nucleoskeleton and Cytoskeleton (LINC) complex in the transfected cell.
2. The nucleic acid molecule of para 1, wherein the expression vector is a cardiac- or cardiomyocyte-specific expression vector.
3. The nucleic acid molecule of para 1 or para 2, wherein the expression vector comprises a cardiac- or cardiomyocyte-specific promoter.
4. The nucleic acid molecule of para 3, wherein the expression vector comprises a cardiac- or cardiomyocyte-specific promoter selected from the group comprising a cardiac troponin T promoter (cTnT), a α-myosin heavy chain (α-MHC) promoter and a myosin light chain (MLC2v) promoter.
5. The nucleic acid molecule of para 3 or 4, wherein the cardiomyocyte-specific promoter is chicken cardiac troponin T (cTnT) promoter.
6. The nucleic acid molecule of any one of the preceding paras, wherein the expression vector is a virus expression vector.
7. The nucleic acid molecule of para 6, wherein the virus expression vector is selected from the group comprising Lentivirus, Adenovirus and Adeno-associated virus (AAV).
8. The nucleic acid molecule of any one of the preceding paras, wherein the adeno-associated virus expression vector (AAV) has cardiac tropism/is cardiotropic.
9. The nucleic acid molecule of any one of the preceding paras, wherein in the AAV vector is selected from the group consisting of AAV9 (serotype 9), AAV1 (serotype 1), AAV6 (serotype 6), AAV8 (serotype 8), AAV2i8, and AAV9.45.
10. The nucleic acid molecule of any one of the preceding paras, wherein in the AAV vector is AAV9 (serotype 9).
11. The nucleic acid molecule of any one of the preceding paras, wherein transgene comprises nucleic acid sequences for expressing a lumenal domain of a SUN domain-containing protein, an N-terminal signal sequence, a signal peptidase cleavage site, and a C-terminal targeting peptide sequence.
12. The nucleic acid molecule of para 11, wherein the lumenal domain of the SUN domain-containing protein comprises a coiled coil domain and a SUN domain.
13. The nucleic acid molecule of para 11, wherein the coiled coil domain is upstream of the SUN domain.
14. The nucleic acid molecule of any one of the preceding paras, wherein the transgene further comprises nucleic acid sequences for expressing an N-terminal signal sequence, a signal peptidase cleavage site, and a C-terminal targeting peptide sequence.
15. The nucleic acid molecule of any one of the preceding paras, wherein the transgene comprises nucleic acid sequences for expressing an N-terminal signal sequence, a signal peptidase cleavage site, and a C-terminal targeting peptide sequence, and either the lumenal domain of the SUN domain-containing protein or the SUN domain.
16. The nucleic acid molecule of any one of paras 11 to 15, wherein the SUN domain protein is SUN1 or SUN2.
17. The nucleic acid molecule of any one of paras 11 to 16, wherein the N-terminal signal sequence is derived from a secretory protein or a Type I transmembrane protein.
18. The nucleic acid molecule of para 17, wherein the secretory protein or Type I transmembrane protein is selected from the group consisting of human serum albumin, proinsulin, transferrin receptor, EGF receptor, pre-pro-opiomelanocortin, pancreatic digestive enzymes (for example, proteases, amylases and lipases), endoplasmic reticulum lumenal proteins, for example protein disulphide isomerases, GRP94 and combinations thereof.
19. The nucleic acid molecule of para 18, wherein the N-terminal signal sequence is derived from human serum albumin.
20. The nucleic acid molecule of any one of paras 11 to 19, wherein the N-terminal signal sequence is not preceded at its N-terminus by any other tags.
21. The nucleic acid molecule of any one of paras 11 to 20, wherein the signal peptidase cleavage site is a signal peptidase cleavage site derived from or is one of the group consisting of human serum albumin, proinsulin, transferrin receptor, EGF receptor, pre-pro-opiomelanocortin, carboxypeptidases, complement proteins, fibrinogen, cytokines, chemokines, fibrinogen, pancreatic digestive enzymes (for example, proteases, amylases and lipases), endoplasmic reticulum lumenal proteins, such as protein disulphide isomerases, GRP94 and combinations thereof.
22. The nucleic acid molecule of para 21, wherein the signal peptidase cleavage site is a signal peptidase cleavage site derived from human serum albumin.
23. The nucleic acid molecule of any one of paras 11 to 22, wherein the C-terminal targeting peptide sequence prevents secretion of a peptide expressed from the transgene according to any one of paras 1 to 19.
24. The nucleic acid molecule of any one of paras 11 to 23, wherein the C-terminal targeting peptide sequence is a KDEL sequence.
25. The nucleic acid molecule of any one of paras 1 to 24, wherein the transgene further comprises an epitope tag.
26. The nucleic acid molecule of para 25, wherein the optional epitope tag is N-terminal, or located anywhere in the nucleic acid molecule except downstream of (after) the C-terminal targeting peptide sequence [for example KDEL], or located anywhere in the nucleic acid molecule except upstream of (before) the N-terminal signal sequence.
27. The nucleic acid molecule of para 26, wherein the optional epitope tag is selected from the group consisting of cellulose binding domain (CBD), chloramphenicol acetyl transferase (CAT), dihydrofolate reductase (DHFR), one or more FLAG tags, glutathione S-transferase (GST), green fluorescent protein (GFP), haemagglutinin A (HA), histidine (His), Herpes simplex virus (HSV), luciferase, maltose-binding protein (MBP), c-Myc, Protein A, Protein G, streptavidin, T7, thioredoxin, V5, vesicular stomatitis virus glycoprotein (VSV-G), and combinations thereof.
28. The nucleic acid molecule of para 27, wherein the epitope tag is haemagglutinin A (HA).
29. The nucleic acid molecule of any one of the preceding paras, wherein the vector is the adeno-associated virus vector (AAV) comprising the chicken cardiac troponin T promoter (cTnT), and the transgene according to any one of paras 1 to 28, wherein the lumenal domain of the SUN domain-containing protein is derived from SUN1, wherein the N-terminal signal sequence and the signal peptidase cleavage site are each derived from human serum albumin, wherein the C-terminal targeting peptide sequence is the KDEL sequence, and wherein the transgene further comprises haemagglutinin (HA) as the N-terminal epitope tag;
   optionally wherein the vector comprises the nucleic acid sequence shown in SEQ ID NO:3 (see e.g. Figure 10).
30. The nucleic acid molecule of any one of the preceding paras, wherein the vector is the adeno-associated virus vector (AAV) comprising the chicken cardiac troponin T promoter (cTnT), and the transgene according to any one of paras 1 to 28, wherein the lumenal domain of the SUN domain-containing protein is derived from SUN2, wherein the an N-terminal signal sequence and the signal peptidase cleavage site are each derived from human serum albumin, and wherein the C-terminal targeting peptide sequence is the KDEL sequence, and wherein the transgene further comprises haemagglutinin (HA) as the N-terminal epitope tag;
   optionally wherein the vector comprises the nucleic acid sequence shown in SEQ ID NO:5 (see e.g. Figure 10).
31. The nucleic acid molecule of any one of paras 1 to 10, wherein the transgene comprises nucleic acid sequences for expressing a KASH domain, and an N-terminal stabiliser polypeptide sequence.
32. The nucleic acid molecule of para 31, wherein the KASH domain comprises a transmembrane domain and a SUN-interacting peptide.
33. The nucleic acid molecule of any one of paras 31 to 32, wherein the KASH domain is selected from the group consisting of KASH1 (derived from Nesprin-1 (SYNE1 gene)), KASH2 (derived from Nesprin-2 (SYNE2 gene)), KASH3 (derived from Nesprin-3 (SYNE3 gene)), KASH4 (derived from Nesprin-4 (SYNE4 gene)), and KASH5 (derived from KASH5/CCDC155 (KASH5 gene)).
34. The nucleic acid molecule of any one of paras 1 to 10, wherein the transgene comprises nucleic acid sequences for expressing a CRISPR-Cas or other synthetic nuclease system to modify nucleic acid that encodes the SUN domain or KASH domain of endogenous Sun or Nesprin protein, respectively.
35. The nucleic acid molecule of para 34, wherein the transgene comprises nucleic acid sequences for expressing a CRISPR-Cas.
36. The nucleic acid molecule of any one of paras 1 to 33, wherein the transgene is a dominant negative construct.
37. The nucleic acid molecule of any one of the preceding paras, wherein the transgene is a humanised or human transgene.
38. The nucleic acid molecule of any one of the preceding paras, wherein expression of the transgene results in the disruption of the protein-protein interaction between SUN and KASH of the LINC complex.
39. The nucleic acid molecule of para 38, wherein the disruption of the protein-protein interaction between SUN and KASH of the LINC complex occurs between the protein interactions selected from the group consisting of Sun1+Nesprin-1, Sun2+Nesprin-1, Sun1+Nesprin-2, Sun1+Nesprin-3, Sun2+Nesprin-2, and Sun2+Nesprin-3.
40. The nucleic acid molecule of para 39, wherein the disruption of the protein-protein interaction between SUN and KASH of the LINC complex occurs between Sun1 and Nesprin-1.
41. The nucleic acid molecule of any one of the preceding paras, wherein the AAV vector is formulated for delivery into the myocardium of a subject.
42. The nucleic acid molecule of any one of the preceding paras for use in treating a disease caused by one or more Lmna mutations in a subject.
43. The nucleic acid molecule of para 42, wherein the disease is selected from the group consisting of restrictive dermopathy, familial partial lipodystrophy (for example, Dunnigan type), mandibuloacral dysplasia with type A lipodystrophy, metabolic syndrome, Charcot-Marie-Tooth disease type 2, Charcot-Marie-Tooth disease type 2B1 and diseases presented in normal font in Table 1.
44. The nucleic acid molecule of any one of the preceding paras for use in treating cardiovascular disease in a subject.
45. The nucleic acid molecule of para 42 or 43, wherein the disease or the cardiovascular disease is characterised by the presence of at least one Lmna mutation.
46. The nucleic acid molecule of any one of paras 44 to 45, wherein the cardiovascular disease is selected from the group consisting of laminopathy, cardiomyopathy, such as dilated cardiomyopathy (DCM), dilated cardiomyopathy 1A, dilated cardiomyopathy with conduction system defects, cardiomyopathy with advanced AV block and arrhythmia, lone atrial fibrillation; muscular dystrophy (often associated with cardiomyopathy), such as cardiomyopathy associated with Emery-Dreifuss muscular dystrophy (autosomal dominant), cardiomyopathy associated with Emery-Dreifuss muscular dystrophy (autosomal recessive), cardiomyopathy associated with Limb-girdle muscular dystrophy type 1B, cardiomyopathy associated with congenital muscular dystrophy; premature aging syndromes (thought to be primarily vascular, but may have cardiac involvement) such as cardiomyopathy associated with Atypical Werner syndrome, cardiomyopathy associated with Hutchinson-Gilford progeria syndrome and the like, as well as diseases presented in bold font in Table 1.
47. An adeno-associated virus vector (AAV) comprising the cardiac troponin T promoter (cTnT), and the transgene according to any one of paras 11 to 30 or paras 34 to 38.
48. A pharmaceutical composition comprising the nucleic acid molecule according to any one of paras 1 to 41.
49. A method of treating a disease in a subject, the method comprising administration of a pharmaceutically effective amount of the nucleic acid molecule according to any one of paras 1 to 41, or the pharmaceutical composition of para 48.
50. The method of para 49, wherein the disease is characterised by the presence of at least one *Lmna* mutation.
51. The method of any one of paras 49 to 50, wherein the *Lmna* mutation(s) affect(s) lamin A isoform, or lamin C isoform of the Lmna gene, or both lamin A/C isoforms.
52. The method of any one of paras 49 to 51, wherein the disease is selected from the group consisting of restrictive dermopathy, familial partial lipodystrophy (for example, Dunnigan type), mandibuloacral dysplasia with type A lipodystrophy, metabolic syndrome, Charcot-Marie-Tooth disease type 2, Charcot-Marie-Tooth disease type 2B1 and diseases present in normal font in Table 1.
53. The method according to any one of paras 49 to 51, wherein the disease is a cardiovascular disease selected from the group consisting of laminopathy, cardiomyopathy, such as dilated cardiomyopathy (DCM), dilated cardiomyopathy 1A, dilated cardiomyopathy with conduction system defects, cardiomyopathy with advanced AV block and arrhythmia, lone atrial fibrillation; muscular dystrophy (often associated with cardiomyopathy), such as cardiomyopathy associated with Emery-Dreifuss muscular dystrophy (autosomal dominant), cardiomyopathy associated with Emery-Dreifuss muscular dystrophy (autosomal recessive), cardiomyopathy associated with Limb-girdle muscular dystrophy type 1B, cardiomyopathy associated with congenital muscular dystrophy; premature aging syndromes (thought to be primarily vascular, but may have cardiac involvement) such as cardiomyopathy associated with Atypical Werner syndrome, cardiomyopathy associated with Hutchinson-Gilford progeria syndrome; and diseases presented in bold font in Table 1.
54. The method of any one of paras 49 to 53, wherein the subject is a non-human mammal or a human;
   optionally wherein the non-human mammal is a mouse;
   optionally wherein the mouse is an N195K mouse (*Lmna*^{N195K/N195K}), or a *Lmna* conditional knockout (*Lmna*^{*flox*/flox}).
55. Use of the pharmaceutical composition according to para 48 or the nucleic acid molecule according to any one of paras 1 to 41 in the manufacture of a medicament for treating a disease caused by one or more Lmna mutations or cardiovascular disease.
56. The use of para 55, wherein the disease is selected from the group consisting of restrictive dermopathy, familial partial lipodystrophy (for example, Dunnigan type), mandibuloacral dysplasia with type A lipodystrophy, metabolic syndrome, Charcot-Marie-Tooth disease type 2, Charcot-Marie-Tooth disease type 2B1 and diseases presented in normal font in Table 1.
57. The use of para 55, wherein the cardiovascular disease is selected from the group consisting of laminopathy, cardiomyopathy, such as dilated cardiomyopathy (DCM), dilated cardiomyopathy 1A, dilated cardiomyopathy with conduction system defects, cardiomyopathy with advanced AV block and arrhythmia, lone atrial fibrillation; muscular dystrophy (often associated with cardiomyopathy), such as cardiomyopathy associated with Emery-Dreifuss muscular dystrophy (autosomal dominant), cardiomyopathy associated with Emery-Dreifuss muscular dystrophy (autosomal recessive), cardiomyopathy associated with Limb-girdle muscular dystrophy type 1B, cardiomyopathy associated with congenital muscular dystrophy; premature aging syndromes (thought to be primarily vascular, but may have cardiac involvement) such as cardiomyopathy associated with Atypical Werner syndrome, cardiomyopathy associated with Hutchinson-Gilford progeria syndrome and the like, as well as diseases presented in bold font in Table 1.
58. The pharmaceutical composition according to para 48 for use in therapy.
59. A method of screening for drug candidates capable of inhibiting the interaction of the proteins of a LINC complex in a cell, which comprises:
   (a) combining the proteins of said LINC complex in the presence of a drug to form a first complex;
   (b) combining the proteins in the absence of said drug to form a second complex;
   (c) measuring the amount of said first complex and said second complex; and
   (d) comparing the amount of said first complex with the amount of said second complex,
   wherein if the amount of said first complex is less than the amount of said second complex, then the drug is a drug candidate for inhibiting the interaction of the proteins of said LINC complex in a cell.
60. The method of para 59, wherein the drug candidate disrupts the protein-protein interaction between SUN and KASH of the LINC complex.
61. The method of para 60, wherein the drug candidate disrupts the interaction between Sun1 and Nesprin-1 proteins.
62. The method of any one of paras 59 to 61, wherein the screening is an in vitro screening.
63. The method of any one of paras 59 to 62, wherein the complex is measured by an ELISA method or by a fluorescence anisotropy method.
64. The method of any one of paras 59 to 63, wherein if the amount of said first complex is less than the amount of said second complex, then said drug is a drug candidate for inhibiting the interaction of said proteins.
65. The method of any one of paras 59 to 64, wherein recombinant SUN and KASH domains are used.
65. The method of any one of paras 59 to 65, wherein recombinant SUN and KASH domains are used;
   optionally wherein recombinant SUN domain is immobilized on a solid surface and recombinant KASH domain is labelled with an enzyme that can generate a colorimetric or chemiluminescent readout (compounds that fail to inhibit the SUN-KASH interaction will result in a well in the plate where the recombinant SUN would bind to the enzyme-linked KASH domain. Following wash steps and incubation with colorimetric or chemiluminescent enzyme substrates, the presence of the SUN-KASH interaction can be detected in standard plate readers. If the compound can inhibit SUN-KASH interaction, then following the wash step, the KASH domain would be removed, and there would be reduced or no enzymatic reaction in the well).
   optionally wherein the KASH domain is fluorescently labelled with a fluorescein moiety and fluorescence anisotropy of the KASH domain interacting with SUN domain may be measured using standard equipment such as a plate reader incorporating a fluorescence spectrometer function;
   optionally wherein if the amount of said first complex is less than the amount of said second complex there will be a difference in the fluorescence anisotropy of the fluorescent KASH and said drug is a drug candidate for inhibiting the interaction of said proteins.

### Sequence identity

Pairwise and multiple sequence alignment for the purposes of determining percent identity between two or more amino acid or nucleic acid sequences can be achieved in various ways known to a person of skill in the art, for instance, using publicly available computer software such as ClustalOmega (Söding, J. 2005, Bioinformatics 21, 951-960), T-coffee (Notredame et al. 2000, J. Mol. Biol. (2000) 302, 205-217), Kalign (Lassmann and Sonnhammer 2005, BMC Bioinformatics, 6(298)) and MAFFT (Katoh and Standley 2013, Molecular Biology and Evolution, 30(4) 772-780 software). When using such software, the default parameters, e.g. for gap penalty and extension penalty, are preferably used.

### Sequences

| **SEQ ID** NO: | **DESCRIPTION** | **SEQUENCE** |
|---|---|---|
| 1 | pAdDeltaF6 | TCGACGGTATCGATAAGCTTGATATCGAATTCCTGCAGCCCGGGGGATCCACTAGTTCTAGAG CGGCCGCCACCGCGGTGGAGCTCCAGCTTTTGTTCCCTTTAGTGAGGGTTAATTTCGAGCTTG GCGTAATCATGGTCATAGCTGTTTCCTGTGTGAAATTGTTATCCGCTCACAATTCCACACAACAT ACGAGCCGGAAGCATAAAGTGTAAAGCCTGGGGTGCCTAATGAGTGAGCTAACTCACATTAATT GCGTTGCGCTCACTGCCCGCTTTCCAGTCGGGAAACCTGTCGTGCCAGCTGCATTAATGAATC GGCCAACGCGCGGGGAGAGGCGGTTTGCGTATTGGGCGCTCTTCCGCTTCCTCGCTCACTGA CTCGCTGCGCTCGGTCGTTCGGCTGCGGCGAGCGGTATCAGCTCACTCAAAGGCGGTAATAC GGTTATCCACAGAATCAGGGGATAACGCAGGAAAGAACATGTGAGCAAAAGGCCAGCAAAAGG CCAGGAACCGTAAAAAGGCCGCGTTGCTGGCGTTTTTCCATAGGCTCCGCCCCCCTGACGAGC ATCACAAAAATCGACGCTCAAGTCAGAGGTGGCGAAACCCGACAGGACTATAAAGATACCAGG CGTTTCCCCCTGGAAGCTCCCTCGTGCGCTCTCCTGTTCCGACCCTGCCGCTTACCGGATACC TGTCCGCCTTTCTCCCTTCGGGAAGCGTGGCGCTTTCTCATAGCTCACGCTGTAGGTATCTCAG TTCGGTGTAGGTCGTTCGCTCCAAGCTGGGCTGTGTGCACGAACCCCCCGTTCAGCCCGACC GCTGCGCCTTATCCGGTAACTATCGTCTTGAGTCCAACCCGGTAAGACACGACTTATCGCCACT GGCAGCAGCCACTGGTAACAGGATTAGCAGAGCGAGGTATGTAGGCGGTGCTACAGAGTTCTT GAAGTGGTGGCCTAACTACGGCTACACTAGAAGGACAGTATTTGGTATCTGCGCTCTGCTGAA GCCAGTTACCTTCGGAAAAAGAGTTGGTAGCTCTTGATCCGGCAAACAAACCACCGCTGGTAG CGGTGGTTTTTTTGTTTGCAAGCAGCAGATTACGCGCAGAAAAAAAGGATCTCAAGAAGATCCT TTGATCTTTTCTACGGGGTCTGACGCTCAGTGGAACGAAAACTCACGTTAAGGGATTTTGGTCA TGAGATTATCAAAAAGGATCTTCACCTAGATCCTTTTAAATTAAAAATGAAGTTTTAAATCAATCT AAAGTATATATGAGTAAACTTGGTCTGACAGTTACCAATGCTTAATCAGTGAGGCACCTATCTCA GCGATCTGTCTATTTCGTTCATCCATAGTTGCCTGACTCCCCGTCGTGTAGATAACTACGATAC GGGAGGGCTTACCATCTGGCCCCAGTGCTGCAATGATACCGCGAGACCCACGCTCACCGGCT CCAGATTTATCAGCAATAAACCAGCCAGCCGGAAGGGCCGAGCGCAGAAGTGGTCCTGCAACT TTATCCGCCTCCATCCAGTCTATTAATTGTTGCCGGGAAGCTAGAGTAAGTAGTTCGCCAGTTA ATAGTTTGCGCAACGTTGTTGCCATTGCTACAGGCATCGTGGTGTCACGCTCGTCGTTTGGTAT GGCTTCATTCAGCTCCGGTTCCCAACGATCAAGGCGAGTTACATGATCCCCCATGTTGTGCAAA AAAGCGGTTAGCTCCTTCGGTCCTCCGATCGTTGTCAGAAGTAAGTTGGCCGCAGTGTTATCAC TCATGGTTATGGCAGCACTGCATAATTCTCTTACTGTCATGCCATCCGTAAGATGCTTTTCTGTG ACTGGTGAGTACTCAACCAAGTCATTCTGAGAATAGTGTATGCGGCGACCGAGTTGCTCTTGCC CGGCGTCAATACGGGATAATACCGCGCCACATAGCAGAACTTTAAAAGTGCTCATCATTGGAAA ACGTTCTTCGGGGCGAAAACTCTCAAGGATCTTACCGCTGTTGAGATCCAGTTCGATGTAACCC ACTCGTGCACCCAACTGATCTTCAGCATCTTTTACTTTCACCAGCGTTTCTGGGTGAGCAAAAA CAGGAAGGCAAAATGCCGCAAAAAAGGGAATAAGGGCGACACGGAAATGTTGAATACTCATAC TCTTCCTTTTTCAATATTATTGAAGCATTTATCAGGGTTATTGTCTCATGAGCGGATACATATTTG AATGTATTTAGAAAAATAAACAAATAGGGGTTCCGCGCACATTTCCCCGAAAAGTGCCACCTGA CGTCTAAGAAACCATTATTATCATGACATTAACCTATAAAAATAGGCGTATCACGAGGCCCTTTC GTCTTCAAGAATTCTCATGTTTGACAGCTTATCATCGATAAGCTTTAATGCGGTAGTTTATCACA GTTAAATTGCTAACGCAGTCAGGCACCGTGTATGAAATCTAACAATGCGCTCATCGTCATCCTC GGCACCGTCACCCTGGATGCTGTAGGCATAGGCTTGGTTATGCCGGTACTGCCGGGCCTCTTG CGGGATATCGTCCATTCCGACAGCATCGCCAGTCACTATGGCGTGCTGCTAGCGCTATATGCG TTGATGCAATTTCTATGCGCACCCGTTCTCGGAGCACTGTCCGACCGCTTTGGCCGCCGCCCA GTCCTGCTCGCTTCGCTACTTGGAGCCACTATCGACTACGCGATCATGGCGACCACACCCGTC CTGTGGATCCGGCGCACACCAAAAACGTCACTTTTGCCACATCCGTCGCTTACATGTGTTCCGC CACACTTGCAACATCACACTTCCGCCACACTACTACGTCACCCGCCCCGTTCCCACGCCCCGC |
| | | GCCACGTCACAAACTCCACCCCCTCATTATCATATTGGCTTCAATCCAAAATAAATCATCAATAA TATACCTTATTTTGGATTGAAGCCAATATGATAATGAGGGGGTGGAGTTTGTGACGTGGCGCGG GGCGTGGGAACGGGGCGGGTGACGTAGGTTTTAGGGCGGAGTAACTTGTATGTGTTGGGAAT TGTAGTTTTCTTAAAATGGGAAGTTACGTAACGTGGGAAAACGGAAGTGACGATTTGAGGAAGT TGTGGGTTTTTTGGCTTTCGTTTCTGGGCGTAGGTTCGCGTGCGGTTTTCTGGGTGTTTTTTGT GGACTTTAACCGTTACGTCATTTTTTAGTCCTATATATACTCGCTCTGCACTTGGCCCTTTTTTAC ACTGTGACTGATTGAGCTGGTGCCGTGTCGAGTGGTGTTTTTTTAATAGGTTTTCTTTTTTACTG GTAAGGCTGACTGTTAGGCTGCCGCTGTGAAGCGCTGTATGTTGTTCTGGAGCGGGAGGGTG CTATTTTGCCTAGGCAGGAGGGTTTTTCAGGTGTTTATGTGTTTTTCTCTCCTATTAATTTTGTTA TACCTCCTATGGGGGCTGTAATGTTGTCTCTACGCCTGCGGGTATGTATTCCCCCGGGCTATTT CGGTCGCTTTTTAGCACTGACCGATGAATCAACCTGATGTGTTTACCGAGTCTTACATTATGACT CCGGACATGACCGAGGAGCTGTCGGTGGTGCTTTTTAATCACGGTGACCAGTTTTTTTACGGTC ACGCCGGCATGGCCGTAGTCCGTCTTATGCTTATAAGGGTTGTTTTTCCTGTTGTAAGACAGGC TTCTAATGTTTAAATGTTTTTTTTGTTATTTTATTTTGTGTTTATGCAGAAACCCGCAGACATGTTTG AGAGAAAAATGGTGTCTTTTTCTGTGGTGGTTCCGGAGCTTACCTGCCTTTATCTGCATGAGCA TGACTACGATGTGCTTTCTTTTTTGCGCGAGGCTTTGCCTGATTTTTTGAGCAGCACCTTGCATT TTATATCGCCGCCCATGCAACAAAGCTTACATCGGGGCTACGCTGGTTAGCATAGCTCCGAGT ATGCGTGTCATAATCAGTGTGGGTTCTTTTGTCAAGGTTCCTGGCGGGGAAGTGGCCGCGCTG GTCCGTGCAGACCTGCACGATTATGTTCAGCTGGCCCTGCGAAGGGACCTACGGGATCGCGG TATTTTTGTTAATGTTCCGCTTTTGAATCTTATACAGGTCTGTGAGGAACCTGAATTTTTGCAATC ATGATTCGCTGCTTGAGGCTGAAGGTGGAGGGCGCTCTGGAGCAGATTTTTACAATGGCCGGA CTTAATATTCGGGATTTGCTTAGAGATATATTGAGAAGGTGGCGAGATGAGAATTATTTGGGCA TGGTTGAAGGTGCTGGAATGTTTATAGAGGAGATTCACCCTGAAGGGTTTAGCCTTTACGTCCA CTTGGACGTGAGGGCCGTTTGCCTTTTGGAAGCCATTGTGCAACATCTTACAAATGCCATTATC TGTTCTTTGGCTGTAGAGTTTGACCACGCCACCGGAGGGGAGCGCGTTCACTTAATAGATCTTC ATTTTGAGGTTTTGGATAATCTTTTGGAATAAAAAAAAAAACATGGTTCTTCCAGCTCTTCCCGC TCCTCCCGTGTGTGACTCGCAGAACGAATGTGTAGGTTGGCTGGGTGTGGCTTATTCTGCGGT GGTGGATGTTATCAGGGCAGCGGCGCATGAAGGAGTTTACATAGAACCCGAAGCCAGGGGGC GCCTGGATGCTTTGAGAGAGTGGATATACTACAACTACTACACAGAGCGATCTAAGCGGCGAG ACCGGAGACGCAGATCTGTTTGTCACGCCCGCACCTGGTTTTGCTTCAGGAAATATGACTACGT CCGGCGTTCCATTTGGCATGACACTACGACCAACACGATCTCGGTTGTCTCGGCGCACTCCGT ACAGTAGGGATCGTCTACCTCCTTTTGAGACAGAAACCCGCGCTACCATACTGGAGGATCATC CGCTGCTGCCCGAATGTAACACTTTGACAATGCACAACGTGAGTTACGTGCGAGGTCTTCCCT GCAGTGTGGGATTTACGCTGATTCAGGAATGGGTTGTTCCCTGGGATATGGTTCTAACGCGGG AGGAGCTTGTAATCCTGAGGAAGTGTATGCACGTGTGCCTGTGTTGTGCCAACATTGATATCAT GACGAGCATGATGATCCATGGTTACGAGTCCTGGGCTCTCCACTGTCATTGTTCCAGTCCCGG TTCCCTGCAGTGTATAGCCGGCGGGCAGGTTTTGGCCAGCTGGTTTAGGATGGTGGTGGATG GCGCCATGTTTAATCAGAGGTTTATATGGTACCGGGAGGTGGTGAATTACAACATGCCAAAAGA GGTAATGTTTATGTCCAGCGTGTTTATGAGGGGTCGCCACTTAATCTACCTGCGCTTGTGGTAT GATGGCCACGTGGGTTCTGTGGTCCCCGCCATGAGCTTTGGATACAGCGCCTTGCACTGTGG GATTTTGAACAATATTGTGGTGCTGTGCTGCAGTTACTGTGCTGATTTAAGTGAGATCAGGGTG CGCTGCTGTGCCCGGAGGACAAGGCGCCTTATGCTGCGGGCGGTGCGAATCATCGCTGAGGA GACCACTGCCATGTTGTATTCCTGCAGGACGGAGCGGCGGCGGCAGCAGTTTATTCGCGCGC TGCTGCAGCACCACCGCCCTATCCTGATGCACGATTATGACTCTACCCCCATGTAGGCGTGGA CTTCTCCTTCGCCGCCCGTTAAGCAACCGCAAGTTGGACAGCAGCCTGTGGCTCAGCAGCTGG ACAGCGACATGAACTTAAGTGAGCTGCCCGGGGAGTTTATTAATATCACTGATGAGCGTTTGGC TCGACAGGAAACCGTGTGGAATATAACACCTAAGAATATGTCTGTTACCCATGATATGATGCTTT TTAAGGCCAGCCGGGGAGAAAGGACTGTGTACTCTGTGTGTTGGGAGGGAGGTGGCAGGTTG AATACTAGGGTTCTGTGAGTTTGATTAAGGTACGGTGATCTGTATAAGCTATGTGGTGGTGGGG CTATACTACTGAATGAAAAATGACTTGAAATTTTCTGCAATTGAAAAATAAACACGTTGAAACATA ACACAAACGATTCTTTATTCTTGGGCAATGTATGAAAAAGTGTAAGAGGATGTGGCAAATATTTC ATTAATGTAGTTGTGGCCAGACCAGTCCCATGAAAATGACATAGAGTATGCACTTGGAGTTGTG TCTCCTGTTTCCTGTGTACCGTTTAGTGTAATGGTTAGTGTTACAGGTTTAGTTTTGTCTCCGTT TAAGTAAACTTGACTGACAATGTTACTTTTGGCAGTTTTACCGTGAGATTTTGGATAAGCTGATA GGTTAGGCATAAATCCAACAGCGTTTGTATAGGCTGTGCCTTCAGTAAGATCTCCATTTCTAAA GTTCCAATATTCTGGGTCCAGGAAGGAATTGTTTAGTAGCACTCCATTTTCGTCAAATCTTATAA TAAGATGAGCACTTTGAACTGTTCCAGATATTGGAGCCAAACTGCCTTTAACAGCCAAAACTGA AACTGTAGCAAGTATTTGACTGCCACATTTTGTTAAGACCAAAGTGAGTTTAGCATCTTTCTCTG CATTTAGTCTACAGTTAGGAGATGGAGCTGGTGTGGTCCACAAAGTTAGCTTATCATTATTTTTG TTTCCTACTGTAATGGCACCTGTGCTGTCAAAACTAAGGCCAGTTCCTAGTTTAGGAACCATAG CCTTGTTTGAATCAAATTCTAGGCCATGGCCAATTTTTGTTTTGAGGGGATTTGTGTTTGGTGCA TTAGGTGAACCAAATTCAAGCCCATCTCCTGCATTAATGGCTATGGCTGTAGCGTCAAACATCA ACCCCTTGGCAGTGCTTAGGTTAACCTCAAGCTTTTTGGAATTGTTTGAAGCTGTAAACAAGTAA AGGCCTTTGTTGTAGTTAATATCCAAGTTGTGGGCTGAGTTTATAAAAAGAGGGCCCTGTCCTA GTCTTAGATTTAGTTGGTTTTGAGCATCAAACGGATAACTAACATCAAGTATAAGGCGTCTGTTT TGAGAATCAATCCTTAGTCCTCCTGCTACATTAAGTTGCATATTGCCTTGTGAATCAAAACCCAA GGCTCCAGTAACTTTAGTTTGCAAGGAAGTATTATTAATAGTCACACCTGGACCAGTTGCTACG |
| | | GTCAAAGTGTTTAGGTCGTCTGTTACATGCAAAGGAGCCCCGTACTTTAGTCCTAGTTTTCCATT TTGTGTATAAATGGGCTCTTTCAAGTCAATGCCCAAGCTACCAGTGGCAGTAGTTAGAGGGGGT GAGGCAGTGATAGTAAGGGTACTGCTATCGGTGGTGGTGAGGGGGCCTGATGTTTGCAGGGC TAGCTTTCCTTCTGACACTGTGAGGGGTCCTTGGGTGGCAATGCTAAGTTTGGAGTCGTGCAC GGTTAGCGGGGCCTGTGATTGCATGGTGAGTGTGTTGCCCGCGACCATTAGAGGTGCGGCGG CAGCCACAGTTAGGGCTTCTGAGGTAACTGTGAGGGGTGCAGATATTTCCAGGTTTATGTTTGA CTTGGTTTTTTTGAGAGGTGGGCTCACAGTGGTTACATTTTGGGAGGTAAGGTTGCCGGCCTC GTCCAGAGAGAGAGGCCGTTGCCCATTTTGAGCGCAAGCATGCCATTGGAGGTAACTAGAGGTTC GGATAGGCGCAAAGAGAGTACCCCAGGGGGACTCTCTTGAAACCCATTGGGGATACAAAGG GAGGAGTAAGAAAAGGCACAGTTGGAGGACCGGTTTCCGTGTCATATGGATACACGGGGTTGA AGGTATCTTCAGACGGTCTTGCGCGCTTCATCTGCAACAACATGAAGATAGTGGGTGCGGATG GACAGGAACAGGAGGAAACTGACATTCCATTTAGATTGTGGAGAAAGTTTGCAGCCAGGAGGA AGCTGCAATACCAGAGCTGGGAGGAGGGCAAGGAGGTGCTGCTGAATAAACTGGACAGAAATT TGCTAACTGATTTTAAGTAAGTGATGCTTTATTATTTTTTTTTATTAGTTAAAGGGAATAAGATCC CCGGGTACTCTAGTTAATTAACTAGAGGATCTTGATGTAATCCAAGGTTAGGACAGTTGCAAAT CACAGTGAGAACACAGGGTCCCCTGTCCCGCTCAACTAGCAGGGGGCGCTGGGTAAACTCCC GAATCAGGCTACGGGCAAGCTCTCCCTGGGCGGTAAGCCGGACGCCGTGCGCCGGGCCCTC GATATGATCCTCGGGCAATTCAAAGTAGCAAAACTCACCGGAGTCGCGGGCAAAGCACTTGTG GCGGCGACAGTGGACCAGGTGTTTCAGGCGCAGTTGCTCTGCCTCTCCACTTAACATTCAGTC GTAGCCGTCCGCCGAGTCCTTTACCGCGTCAAAGTTAGGAATAAATTGATCCGGATAGTGGCC GGGAGGTCCCGAGAAGGGGTTAAAGTAGACCGATGGCACAAACTCCTCAATAAATTGCAGAGT TCCAATGCCTCCAGAGCGCGGCTCAGAGGACGAGGTCTGCAGAGTTAGGATTGCCTGACGAG GCGTGAATGAAGGACGGCCGGCGCCGCCGATCTGAAATGTCCCGTCCGGACGGAGACCAAGC GAGGAGCTCACCGACTCGTCGTTGAGCTGAATACCTCGCCCTCTGATTGTCAGGTGAGTTATA CCCTGCCCGGGCGACCGCACCCTGTGACGAAAGCCGCCCGCAAGCTGCGCCCCTGAGTTAGT CATCTGAACTTCGGCCTGGGCGTCTCTGGGAAGTACCACAGTGGTGGGAGCGGGACTTTCCT GGTACACCAGGGCAGCGGGCCAACTACGGGGATTAAGGTTATTACGAGGTGTGGTGGTAATA GCCGCCTGTTCCAAGAGAATTCGGTTTCGGTGGGCGCGGATTCCGTTGACCCGGGATATCATG TGGGGTCCCGCGCTCATGTAGTTTATTCGGGTTGAGTAGTCTTGGGCAGCTCCAGCCGCAAGT CCCATTTGTGGCTGGTAACTCCACATGTAGGGCGTGGGAATTTCCTTGCTCATAATGGCGCTGA CGACAGGTGCTGGCGCCGGGTGTGGCCGCTGGAGATGACGTAGTTTTCGCGCTTAAATTTGA GAAAGGGCGCGAAACTAGTCCTTAAGAGTCAGCGCGCAGTATTTACTGAAGAGAGCCTCCGCG TCTTCCAGCGTGCGCCGAAGCTGATCTTCGCTTTTGTGATACAGGCAGCTGCGGGTGAGGGAT CGCAGAGACCTGTTTTTTATTTTCAGCTCTTGTTCTTGGCCCCTGCTCTGTTGAAATATAGCATA CAGAGTGGGAAAAATCCTGTTTCTAAGCTCGCGGGTCGATACGGGTTCGTTGGGCGCCAGACG CAGCGCTCCTCCTCCTGCTGCTGCCGCCGCTGTGGATTTCTTGGGCTTTGTCAGAGTCTTGCT ATCCGGTCGCCTTTGCTTCTGTGTGGCCGCTGCTGTTGCTGCCGCTGCCGCTGCCGCCGGTG CAGTATGGGCTGTAGAGATGACGGTAGTAATGCAGGATGTTACGGGGGAAGGCCACGCCGTG ATGGTAGAGAAGAAAGCGGCGGGCGAAGGAGATGTTGCCCCCACAGTCTTGCAAGCAAGCAA CTATGGCGTTCTTGTGCCCGCGCCATGAGCGGTAGCCTTGGCGCTGTTGTTGCTCTTGGGCTA ACGGCGGCGGCTGCTTGGACTTACCGGCCCTGGTTCCAGTGGTGTCCCATCTACGGTTGGGT CGGCGAACGGGCAGTGCCGGCGGCGCCTGAGGAGCGGAGGTTGTAGCCATGCTGGAACCGG TTGCCGATTTCTGGGGCGCCGGCGAGGGGAATGCGACCGAGGGTGACGGTGTTTCGTCTGAC ACCTCTTCGACCTCGGAAGCTTCCTCGTCTAGGCTCTCCCAGTCTTCCATCATGTCCTCCTCCT CCTCGTCCAAAACCTCCTCTGCCTGACTGTCCCAGTATTCCTCCTCGTCCGTGGGTGGCGGCG GCAGCTGCAGCTTCTTTTTGGGTGCCATCCTGGGAAGCAAGGGCCCGCGGCTGCTGCTGATA GGGCTGCGGCGGCGGGGGGATTGGGTTGAGCTCCTCGCCGGACTGGGGTCCAAGTAAACC CCCCGTCCCTTTCGTAGCAGAAACTCTTGGCGGGCTTTGTTGATGGCTTGCAATTGGCCAAGA ATGTGGCCCTGGGTAATGACGCAGGCGGTAAGCTCCGCATTTGGCGGGCGGGATTGGTCTTC GTAGAACCTAATCTCGTGGGCGTGGTAGTCCTCAGGTACAAATTTGCGAAGGTAAGCCGACGT CCACAGCCCCGGAGTGAGTTTCAACCCCGGAGCCGCGGACTTTTCGTCAGGCGAGGGACCCT GCAGCTCAAAGGTACCGATAATTTGACTTTCGTTAAGCAGCTGCGAATTGCAAACCAGGGAGC GGTGCGGGGTGCATAGGTTGCAGCGACAGTGACACTCCAGTAGACCGTCACCGCTCACGTCT TCCATTATGTCAGAGTGGTAGGCAAGGTAGTTGGCTAGCTGCAGAAGGTAGCAGTGGCCCCAA AGCGGCGGAGGGCATTCGCGGTACTTAATGGGCACAAAGTCGCTAGGAAGTGCACAGCAGGT GGCGGGCAAGATTCCTGAGCGCTCTAGGATAAAGTTCCTAAAGTTCTGCAACATGCTTTGACTG GTGAAGTCTGGCAGACCCTGTTGCAGGGTTTTAAGCAGGCGTTCGGGGAAAATGATGTCCGCC AGGTGCGCGGCCACGGAGCGCTCGTTGAAGGCCGTCCATAGGTCCTTCAAGTTTTGCTTTAGC AGTTTCTGCAGCTCCTTGAGGTTGCACTCCTCCAAGCACTGCTGCCAAACGCCCATGGCCGTC TGCCAGGTGTAGCATAGAAATAAGTAAACGCAGTCGCGGACGTAGTCGCGGCGCGCCTCGCC CTTGAGCGTGGAATGAAGCACGTTTTGCCCAAGGCGGTTTTCGTGCAAAATTCCAAGGTAGGA GACCAGGTTGCAGAGCTCCACGTTGGAGATCTTGCAGGCCTGGCGTACGTAGCCCTGTCGAAA GGTGTAGTGCAATGTTTCCTCTAGCTTGCGCTGCATCTCCGGGTCAGCAAAGAACCGCTGCAT GCACTCAAGCTCCACGGTAACGAGCACTGCGGCCATCATTAGTTTGCGTCGCTCCTCCAAGTC GGCAGGCTCGCGCGTTTGAAGCCAGCGCGCTAGCTGCTCGTCGCCAACTGCGGGTAGGCCCT CCTCTGTTTGTTCTTGCAAATTTGCATCCCTCTCCAGGGGCTGCGCACGGCGCACGATCAGCT CACTCATGACTGTGCTCATGACCTTGGGGGGTAGGTTAAGTGCCGGGTAGGCAAAGTGGGTGA |
| | | CCTCGATGCTGCGTTTTAGTACGGCTAGGCGCGCGTTGTCACCCTCGAGTTCCACCAACACTC CAGAGTGACTTTCATTTTCGCTGTTTTCCTGTTGCAGAGCGTTTGCCGCGCGCTTCTCGTCGCG TCCAAGACCCTCAAAGATTTTTGGCACTTCGTTGAGCGAGGCGATATCAGGTATGACAGCGCC CTGCCGCAAGGCCAGCTGCTTGTCCGCTCGGCTGCGGTTGGCACGGCAGGATAGGGGTATCT TGCAGTTTTGGAAAAAGATGTGATAGGTGGCAAGCACCTCTGGCACGGCAAATACGGGGTAGA AGTTGAGGCGCGGGTTGGGCTCGCATGTGCCGTTTTCTTGGCGTTTGGGGGGTACGCGCGGT GAGAATAGGTGGCGTTCGTAGGCAAGGCTGACATCCGCTATGGCGAGGGGCACATCGCTGCG CTCTTGCAACGCGTCGCAGATAATGGCGCACTGGCGCTGCAGATGCTTCAACAGCACGTCGTC TCCCACATCTAGGTAGTCGCCATGCCTTTCGTCCCCCCGCCCGACTTGTTCCTCGTTTGCCTCT GCGTTGTCCTGGTCTTGCTTTTTATCCTCTGTTGGTACTGAGCGGTCCTCGTCGTCTTCGCTTA CAAAACCTGGGTCCTGCTCGATAATCACTTCCTCCTCCTCAAGCGGGGGTGCCTCGACGGGGA AGGTGGTAGGCGCGTTGGCGGCATCGGTGGAGGCGGTGGTGGCGAACTCAGAGGGGGCGGT TAGGCTGTCCTTCTTCTCGACTGACTCCATGATCTTTTTCTGCCTATAGGAGAAGGAAATGGCC AGTCGGGAAGAGGAGCAGCGCGAAACCACCCCCGAGCGCGGACGCGGTGCGGCGCGACGTC CCCCAACCATGGAGGACGTGTCGTCCCCGTCCCCGTCGCCGCCGCCTCCCCGGGCGCCCCC AAAAAAGCGGATGAGGCGGCGTATCGAGTCCGAGGACGAGGAAGACTCATCACAAGACGCGC TGGTGCCGCGCACACCCAGCCCGCGGCCATCGACCTCGGCGGCGGATTTGGCCATTGCGCC CAAGAAGAAAAAGAAGCGCCCTTCTCCCAAGCCCGAGCGCCCGCCATCACCAGAGGTAATCGT GGACAGCGAGGAAGAAAGAGAAGATGTGGCGCTACAAATGGTGGGTTTCAGCAACCCACCGG TGCTAATCAAGCATGGCAAAGGAGGTAAGCGCACAGTGCGGCGGCTGAATGAAGACGACCCA GTGGCGCGTGGTATGCGGACGCAAGAGGAAGAGGAAGAGCCCAGCGAAGCGGAAAGTGAAAT TACGGTGATGAACCCGCTGAGTGTGCCGATCGTGTCTGCGTGGGAGAAGGGCATGGAGGCTG CGCGCGCGCTGATGGACAAGTACCACGTGGATAACGATCTAAAGGCGAACTTCAAACTACTGC CTGACCAAGTGGAAGCTCTGGCGGCCGTATGCAAGACCTGGCTGAACGAGGAGCACCGCGGG TTGCAGCTGACCTTCACCAGCAACAAGACCTTTGTGACGATGATGGGGCGATTCCTGCAGGCG TACCTGCAGTCGTTTGCAGAGGTGACCTACAAGCATCACGAGCCCACGGGCTGCGCGTTGTG GCTGCACCGCTGCGCTGAGATCGAAGGCGAGCTTAAGTGTCTACACGGAAGCATTATGATAAA TAAGGAGCACGTGATTGAAATGGATGTGACGAGCGAAAACGGGCAGCGCGCGCTGAAGGAGC AGTCTAGCAAGGCCAAGATCGTGAAGAACCGGTGGGGCCGAAATGTGGTGCAGATCTCCAACA CCGACGCAAGGTGCTGCGTGCACGACGCGGCCTGTCCGGCCAATCAGTTTTCCGGCAAGTCT TGCGGCATGTTCTTCTCTGAAGGCGCAAAGGCTCAGGTGGCTTTTAAGCAGATCAAGGCTTTTA TGCAGGCGCTGTATCCTAACGCCCAGACCGGGCACGGTCACCTTTTGATGCCACTACGGTGCG AGTGCAACTCAAAGCCTGGGCACGCGCCCTTTTTGGGAAGGCAGCTACCAAAGTTGACTCCGT TCGCCCTGAGCAACGCGGAGGACCTGGACGCGGATCTGATCTCCGACAAGAGCGTGCTGGCC AGCGTGCACCACCCGGCGCTGATAGTGTTCCAGTGCTGCAACCCTGTGTATCGCAACTCGCGC GCGCAGGGCGGAGGCCCCAACTGCGACTTCAAGATATCGGCGCCCGACCTGCTAAACGCGTT GGTGATGGTGCGCAGCCTGTGGAGTGAAAACTTCACCGAGCTGCCGCGGATGGTTGTGCCTG AGTTTAAGTGGAGCACTAAACACCAGTATCGCAACGTGTCCCTGCCAGTGGCGCATAGCGATG CGCGGCAGAACCCCTTTGATTTTTAAACGGCGCAGACGGCAAGGGTGGGGGTAAATAATCACC CGAGAGTGTACAAATAAAAGCATTTGCCTTTATTGAAAGTGTCTCTAGTACATTATTTTTACATGT TTTTCAAGTGACAAAAAGAAGTGGCGCTCCTAATCTGCGCACTGTGGCTGCGGAAGTAGGGCG AGTGGCGCTCCAGGAAGCTGTAGAGCTGTTCCTGGTTGCGACGCAGGGTGGGCTGTACCTGG GGACTGTTGAGCATGGAGTTGGGTACCCCGGTAATAAGGTTCATGGTGGGGTTGTGATCCATG GGAGTTTGGGGCCAGTTGGCAAAGGCGTGGAGAAACATGCAGCAGAATAGTCCACAGGCGGC CGAGTTGGGCCCCTGTACGCTTTGGGTGGACTTTTCCAGCGTTATACAGCGGTCGGGGGAAGA AGCAATGGCGCTACGGCGCAGGAGTGACTCGTACTCAAACTGGTAAACCTGCTTGAGTCGCTG GTCAGAAAAGCCAAAGGGCTCAAAGAGGTAGCATGTTTTTGAGTGCGGGTTCCAGGCAAAGGC CATCCAGTGTACGCCCCCAGTCTCGGTCCGAGACTCGAACCGGGGGTCCCGCGACTCAACCC TTGGAAAATAACCCTCCGGCTACAGGGAGCGAGCCACTTAATGCTTTCGCTTTCCAGCCTAACC GCTTACGCTGCGCGCGGCCAGTGGCCAAAAAAGCTAGCGCAGCAGCCGCCGCGCCTGGAAG GAAGCCAAAAGGAGCACTCCCCCGTTGTCTGACGTCGCACACCTGGGTTCGACACGCGGGCG GTAACCGCATGGATCACGGCGGACGGCCGGATACGGGGCTCGAACCCCGGTCGTCCGCCAT GATACCCTTGCGAATTTATCCACCAGACCACGGAAGAGTGCCCGCTTACAGGCTCTCCTTTTGC ACGGTAGAGCGTCAACGATTGCGCGCGCCTGACCGGCCAGAGCGTCCCGACCATGGAGCACT TTTTGCCGCTGCGCAACATCTGGAACCGCGTCCGCGACTTTCCGCGCGCCTCCACCACCGCC GCCGGCATCACCTGGATGTCCAGGTACATCTACGGATATCATCGCCTTATGTTGGAAGATCTCG CCCCCGGAGCCCCGGCCACCCTACGCTGGCCCCTCTACCGCCAGCCGCCGCCGCACTTTTTG GTGGGATACCAGTACCTGGTGCGGACTTGCAACGACTACGTATTTGACTCGAGGGCTTACTCG CGTCTCAGGTACACCGAGCTCTCGCAGCCGGGTCACCAGACCGTTAACTGGTCCGTTATGGCC AACTGCACTTACACCATCAACACGGGCGCATACCACCGCTTTGTGGACATGGATGACTTCCAGT CTACCCTCACGCAGGTGCAGCAGGCCATATTAGCCGAGCGCGTTGTCGCCGACCTAGCCCTG CTTCAGCCGATGAGGGGCTTCGGGGTCACACGCATGGAGGAAGAGGGCGCCACCTACGGC CAAACTCCGCCGCCGCCGCAGCGATAGATGCAAGAGATGCAGGACAAGAGGAAGGAGAAGAA GAAGTGCCGGTAGAAAGGCTCATGCAAGACTACTACAAAGACCTGCGCCGATGTCAAAACGAA GCCTGGGGCATGGCCGACCGCCTGCGCATTCAGCAGGCCGGACCCAAGGACATGGTGCTTCT G |
| 2 | pAAV2_9 | GTCGACGGTATCGGGGGAGCTCGCAGGGTCTCCATTTTGAAGCGGGAGGTTTGAACGCGCAG |
| | | CCGCCATGCCGGGGTTTTACGAGATTGTGATTAAGGTCCCCAGCGACCTTGACGAGCATCTGC CCGGCATTTCTGACAGCTTTGTGAACTGGGTGGCCGAGAAGGAATGGGAGTTGCCGCCAGATT CTGACATGGATCTGAATCTGATTGAGCAGGCACCCCTGACCGTGGCCGAGAAGCTGCAGCGC GACTTTCTGACGGAATGGCGCCGTGTGAGTAAGGCCCCGGAGGCTCTTTTCTTTGTGCAATTT GAGAAGGGAGAGAGCTACTTCCACATGCACGTGCTCGTGGAAACCACCGGGGTGAAATCCAT GGTTTTGGGACGTTTCCTGAGTCAGATTCGCGAAAAACTGATTCAGAGAATTTACCGCGGGATC GAGCCGACTTTGCCAAACTGGTTCGCGGTCACAAAGACCAGAAATGGCGCCGGAGGCGGGAA CAAGGTGGTGGATGAGTGCTACATCCCCAATTACTTGCTCCCCAAAACCCAGCCTGAGCTCCA GTGGGCGTGGACTAATATGGAACAGTATTTAAGCGCCTGTTTGAATCTCACGGAGCGTAAACG GTTGGTGGCGCAGCATCTGACGCACGTGTCGCAGACGCAGGAGCAGAACAAAGAGAATCAGA ATCCCAATTCTGATGCGCCGGTGATCAGATCAAAAACTTCAGCCAGGTACATGGAGCTGGTCG GGTGGCTCGTGGACAAGGGGATTACCTCGGAGAAGCAGTGGATCCAGGAGGACCAGGCCTCA TACATCTCCTTCAATGCGGCCTCCAACTCGCGGTCCCAAATCAAGGCTGCCTTGGACAATGCG GGAAAGATTATGAGCCTGACTAAAACCGCCCCCGACTACCTGGTGGGCCAGCAGCCCGTGGA GGACATTTCCAGCAATCGGATTTATAAAATTTTGGAACTAAACGGGTACGATCCCCAATATGCG GCTTCCGTCTTTCTGGGATGGGCCACGAAAAAGTTCGGCAAGAGGAACACCATCTGGCTGTTT GGGCCTGCAACTACCGGGAAGACCAACATCGCGGAGGCCATAGCCCACACTGTGCCCTTCTA CGGGTGCGTAAACTGGACCAATGAGAACTTTCCCTTCAACGACTGTGTCGACAAGATGGTGAT CTGGTGGGAGGAGGGGAAGATGACCGCCAAGGTCGTGGAGTCGGCCAAAGCCATTCTCGGAG GAAGCAAGGTGCGCGTGGACCAGAAATGCAAGTCCTCGGCCCAGATAGACCCGACTCCCGTG ATCGTCACCTCCAACACCAACATGTGCGCCGTGATTGACGGGAACTCAACGACCTTCGAACAC CAGCAGCCGTTGCAAGACCGGATGTTCAAATTTGAACTCACCCGCCGTCTGGATCATGACTTTG GGAAGGTCACCAAGCAGGAAGTCAAAGACTTTTTCCGGTGGGCAAAGGATCACGTGGTTGAGG TGGAGCATGAATTCTACGTCAAAAAGGGTGGAGCCAAGAAAAGACCCGCCCCCAGTGACGCAG ATATAAGTGAGCCCAAACGGGTGCGCGAGTCAGTTGCGCAGCCATCGACGTCAGACGCGGAA GCTTCGATCAACTACGCGGACAGGTACCAAAACAAATGTTCTCGTCACGTGGGCATGAATCTGA TGCTGTTTCCCTGCAGACAATGCGAGAGACTGAATCAGAATTCAAATATCTGCTTCACTCACGG TGTCAAAGACTGTTTAGAGTGCTTTCCCGTGTCAGAATCTCAACCCGTTTCTGTCGTCAAAAAG GCGTATCAGAAACTGTGCTACATTCATCACATCATGGGAAAGGTGCCAGACGCTTGCACTGCTT GCGACCTGGTCAATGTGGACTTGGATGACTGTGTTTCTGAACAATAAATGACTTAAACCAGGTA TGGCTGCCGATGGTTATCTTCCAGATTGGCTCGAGGACAACCTTAGTGAAGGAATTCGCGAGT GGTGGGCTTTGAAACCTGGAGCCCCTCAACCCAAGGCAAATCAACAACATCAAGACAACGCTC GAGGTCTTGTGCTTCCGGGTTACAAATACCTTGGACCCGGCAACGGACTCGACAAGGGGGAG CCGGTCAACGCAGCAGACGCGGCGGCCCTCGAGCACGACAAGGCCTACGACCAGCAGCTCAA GGCCGGAGACAACCCGTACCTCAAGTACAACCACGCCGACGCCGAGTTCCAGGAGCGGCTCA AAGAAGATACGTCTTTTGGGGGCAACCTCGGGCGAGCAGTCTTCCAGGCCAAAAAGAGGCTTC TTGAACCTCTTGGTCTGGTTGAGGAAGCGGCTAAGACGGCTCCTGGAAAGAAGAGGCCTGTAG AGCAGTCTCCTCAGGAACCGGACTCCTCCGCGGGTATTGGCAAATCGGGTGCACAGCCCGCT AAAAAGAGACTCAATTTCGGTCAGACTGGCGACACAGAGTCAGTCCCAGACCCTCAACCAATC GGAGAACCTCCCGCAGCCCCCTCAGGTGTGGGATCTCTTACAATGGCTTCAGGTGGTGGCGC ACCAGTGGCAGACAATAACGAAGGTGCCGATGGAGTGGGTAGTTCCTCGGGAAATTGGCATTG CGATTCCCAATGGCTGGGGGACAGAGTCATCACCACCAGCACCCGAACCTGGGCCCTGCCCA CCTACAACAATCACCTCTACAAGCAAATCTCCAACAGCACATCTGGAGGATCTTCAAATGACAA CGCCTACTTCGGCTACAGCACCCCCTGGGGGTATTTTGACTTCAACAGATTCCACTGCCACTTC TCACCACGTGACTGGCAGCGACTCATCAACAACAACTGGGGATTCCGGCCTAAGCGACTCAAC TTCAAGCTCTTCAACATTCAGGTCAAAGAGGTTACGGACAACAATGGAGTCAAGACCATCGCCA ATAACCTTACCAGCACGGTCCAGGTCTTCACGGACTCAGACTATCAGCTCCCGTACGTGCTCG GGTCGGCTCACGAGGGCTGCCTCCCGCCGTTCCCAGCGGACGTTTTCATGATTCCTCAGTACG GGTATCTGACGCTTAATGATGGAAGCCAGGCCGTGGGTCGTTCGTCCTTTTACTGCCTGGAAT ATTTCCCGTCGCAAATGCTAAGAACGGGTAACAACTTCCAGTTCAGCTACGAGTTTGAGAACGT ACCTTTCCATAGCAGCTACGCTCACAGCCAAAGCCTGGACCGACTAATGAATCCACTCATCGAC CAATACTTGTACTATCTCTCAAAGACTATTAACGGTTCTGGACAGAATCAACAAACGCTAAAATT CAGTGTGGCCGGACCCAGCAACATGGCTGTCCAGGGAAGAAACTACATACCTGGACCCAGCTA CCGACAACAACGTGTCTCAACCACTGTGACTCAAAACAACAACAGCGAATTTGCTTGGCCTGGA GCTTCTTCTTGGGCTCTCAATGGACGTAATAGCTTGATGAATCCTGGACCTGCTATGGCCAGCC ACAAAGAAGGAGAGGACCGTTTCTTTCCTTTGTCTGGATCTTTAATTTTTGGCAAACAAGGAACT GGAAGAGACAACGTGGATGCGGACAAAGTCATGATAACCAACGAAGAAGAAATTAAAACTACTA ACCCGGTAGCAACGGAGTCCTATGGACAAGTGGCCACAAACCACCAGAGTGCCCAAGCACAG GCGCAGACCGGCTGGGTTCAAAACCAAGGAATACTTCCGGGTATGGTTTGGCAGGACAGAGAT GTGTACCTGCAAGGACCCATTTGGGCCAAAATTCCTCACACGGACGGCAACTTTCACCCTTCTC CGCTGATGGGAGGGTTTGGAATGAAGCACCCGCCTCCTCAGATCCTCATCAAAAACACACCTG TACCTGCGGATCCTCCAACGGCCTTCAACAAGGACAAGCTGAACTCTTTCATCACCCAGTATTC TACTGGCCAAGTCAGCGTGGAGATCGAGTGGGAGCTGCAGAAGGAAAACAGCAAGCGCTGGA ACCCGGAGATCCAGTACACTTCCAACTATTACAAGTCTAATAATGTTGAATTTGCTGTTAATACT GAAGGTGTATATAGTGAACCCCGCCCCATTGGCACCAGATACCTGACTCGTAATCTGTAATTGC TTGTTAATCAATAAACCGTTTAATTCGTTTCAGTTGAACTTTGGTCTCTGCGAAGGGCGAATTCG TTTAAACCTGCAGGACTAGAGGTCCTGTATTAGAGGTCACGTGAGTGTTTTGCGACATTTTGCG |
| | | ACACCATGTGGTCACGCTGGGTATTTAAGCCCGAGTGAGCACGCAGGGTCTCCATTTTGAAGC GGGAGGTTTGAACGCGCAGCCGCCAAGCCGAATTCTGCAGATATCCATCACACTGGCGGCCG CTCGACTAGAGCGGCCGCCACCGCGGTGGAGCTCCAGCTTTTGTTCCCTTTAGTGAGGGTTAA TTGCGCGCTTGGCGTAATCATGGTCATAGCTGTTTCCTGTGTGAAATTGTTATCCGCTCACAATT CCACACAACATACGAGCCGGAAGCATAAAGTGTAAAGCCTGGGGTGCCTAATGAGTGAGCTAA CTCACATTAATTGCGTTGCGCTCACTGCCCGCTTTCCAGTCGGGAAACCTGTCGTGCCAGCTG CATTAATGAATCGGCCAACGCGCGGGGAGAGGCGGTTTGCGTATTGGGCGCTCTTCCGCTTCC TCGCTCACTGACTCGCTGCGCTCGGTCGTTCGGCTGCGGCGAGCGGTATCAGCTCACTCAAA GGCGGTAATACGGTTATCCACAGAATCAGGGGATAACGCAGGAAAGAACATGTGAGCAAAAGG CCAGCAAAAGGCCAGGAACCGTAAAAAGGCCGCGTTGCTGGCGTTTTTCCATAGGCTCCGCCC CCCTGACGAGCATCACAAAAATCGACGCTCAAGTCAGAGGTGGCGAAACCCGACAGGACTATA AAGATACCAGGCGTTTCCCCCTGGAAGCTCCCTCGTGCGCTCTCCTGTTCCGACCCTGCCGCT TACCGGATACCTGTCCGCCTTTCTCCCTTCGGGAAGCGTGGCGCTTTCTCATAGCTCACGCTG TAGGTATCTCAGTTCGGTGTAGGTCGTTCGCTCCAAGCTGGGCTGTGTGCACGAACCCCCCGT TCAGCCCGACCGCTGCGCCTTATCCGGTAACTATCGTCTTGAGTCCAACCCGGTAAGACACGA CTTATCGCCACTGGCAGCAGCCACTGGTAACAGGATTAGCAGAGCGAGGTATGTAGGCGGTG CTACAGAGTTCTTGAAGTGGTGGCCTAACTACGGCTACACTAGAAGAACAGTATTTGGTATCTG CGCTCTGCTGAAGCCAGTTACCTTCGGAAAAAGAGTTGGTAGCTCTTGATCCGGCAAACAAAC CACCGCTGGTAGCGGTGGTTTTTTTGTTTGCAAGCAGCAGATTACGCGCAGAAAAAAAGGATCT CAAGAAGATCCTTTGATCTTTTCTACGGGGTCTGACGCTCAGTGGAACGAAAACTCACGTTAAG GGATTTTGGTCATGAGATTATCAAAAAGGATCTTCACCTAGATCCTTTTAAATTAAAAATGAAGTT TTAAATCAATCTAAAGTATATATGAGTAAACTTGGTCTGACAGTTACCAATGCTTAATCAGTGAG GCACCTATCTCAGCGATCTGTCTATTTCGTTCATCCATAGTTGCCTGACTCCCCGTCGTGTAGA TAACTACGATACGGGAGGGCTTACCATCTGGCCCCAGTGCTGCAATGATACCGCGAGACCCAC GCTCACCGGCTCCAGATTTATCAGCAATAAACCAGCCAGCCGGAAGGGCCGAGCGCAGAAGT GGTCCTGCAACTTTATCCGCCTCCATCCAGTCTATTAATTGTTGCCGGGAAGCTAGAGTAAGTA GTTCGCCAGTTAATAGTTTGCGCAACGTTGTTGCCATTGCTACAGGCATCGTGGTGTCACGCTC GTCGTTTGGTATGGCTTCATTCAGCTCCGGTTCCCAACGATCAAGGCGAGTTACATGATCCCCC ATGTTGTGCAAAAAAGCGGTTAGCTCCTTCGGTCCTCCGATCGTTGTCAGAAGTAAGTTGGCCG CAGTGTTATCACTCATGGTTATGGCAGCACTGCATAATTCTCTTACTGTCATGCCATCCGTAAGA TGCTTTTCTGTGACTGGTGAGTACTCAACCAAGTCATTCTGAGAATAGTGTATGCGGCGACCGA GTTGCTCTTGCCCGGCGTCAATACGGGATAATACCGCGCCACATAGCAGAACTTTAAAAGTGCT CATCATTGGAAAACGTTCTTCGGGGCGAAAACTCTCAAGGATCTTACCGCTGTTGAGATCCAGT TCGATGTAACCCACTCGTGCACCCAACTGATCTTCAGCATCTTTTACTTTCACCAGCGTTTCTGG GTGAGCAAAAACAGGAAGGCAAAATGCCGCAAAAAAGGGAATAAGGGCGACACGGAAATGTTG AATACTCATACTCTTCCTTTTTCAATATTATTGAAGCATTTATCAGGGTTATTGTCTCATGAGCGG ATACATATTTGAATGTATTTAGAAAAATAAACAAATAGGGGTTCCGCGCACATTTCCCCGAAAAG TGCCACCTAAATTGTAAGCGTTAATATTTTGTTAAAATTCGCGTTAAATTTTTGTTAAATCAGCTC ATTTTTTAACCAATAGGCCGAAATCGGCAAAATCCCTTATAAATCAAAAGAATAGACCGAGATAG GGTTGAGTGTTGTTCCAGTTTGGAACAAGAGTCCACTATTAAAGAACGTGGACTCCAACGTCAA AGGGCGAAAAACCGTCTATCAGGGCGATGGCCCACTACGTGAACCATCACCCTAATCAAGTTT TTTGGGGTCGAGGTGCCGTAAAGCACTAAATCGGAACCCTAAAGGGAGCCCCCGATTTAGAGC TTGACGGGGAAAGCCGGCGAACGTGGCGAGAAAGGAAGGGAAGAAAGCGAAAGGAGCGGGC GCTAGGGCGCTGGCAAGTGTAGCGGTCACGCTGCGCGTAACCACCACACCCGCCGCGCTTAA TGCGCCGCTACAGGGCGCGTCCCATTCGCCATTCAGGCTGCGCAACTGTTGGGAAGGGCGAT CGGTGCGGGCCTCTTCGCTATTACGCCAGCTGGCGAAAGGGGGATGTGCTGCAAGGCGATTA AGTTGGGTAACGCCAGGGTTTTCCCAGTCACGACGTTGTAAAACGACGGCCAGTGAGCGCGC GTAATACGACTCACTATAGGGCGAATTGGGTACCGGGCCCCCCCTCGATCGAG |
| 3 | pENN.AAV.cTnT.PI.S un1-dom-neg | CTGCGCGCTCGCTCGCTCACTGAGGCCGCCCGGGCAAAGCCCGGGCGTCGGGCGACCTTTG GTCGCCCGGCCTCAGTGAGCGAGCGAGCGCGCAGAGAGGGAGTGGCCAACTCCATCACTAG GGGTTCCTTGTAGTTAATGATTAACCCGCCATGCTACTTATCTACCAGGGTAATGGGGATCCTC TAGAACTATAGCTAGAATTCGCCCTTACGGCCCCCCCTCGAGGTCGGGATAAAAGCAGTCTG GGCTTTCACATGACAGCATCTGGGGCTGCGGCAGAGGGTCGGGTCCGAAGCGCTGCCTTATC AGCGCCCCAGCCCTGGGAGGTGACAGCTGGCTGGCTTGTGTCAGCCCCTCGGGCACTCACGT ATCTCCGTCCGACGGGTTTAAAATAGCAAAACTCTGAGGCCACACAATAGCTTGGGCTTATATG GGCTCCTGTGGGGGAAGGGGGAGCACGGAGGGGGCCGGGGCCGCTGCTGCCAAAATAGCAG CTCACAAGTGTTGCATTCCTCTCTGGGCGCCGGGCACATTCCTGCTGGCTCTGCCCGCCCCGG GGTGGGCGCCGGGGGGACCTTAAAGCCTCTGCCCCCCAAGGAGCCCTTCCCAGACAGCCGC CGGCACCCACCGCTCCGTGGGACGATCCCCGAAGCTCTAGAGCTTTATTGCGGTAGTTTATCA CAGTTAAATTGCTAACGCAGTCAGTGCTTCTGACACAACAGTCTCGAACTTAAGCTGCAGAAGT TGGTCGTGAGGCACTGGGCAGGTAAGTATCAAGGTTACAAGACAGGTTTAAGGAGACCAATAG AAACTGGGCTTGTCGAGACAGAGAAGACTCTTGCGTTTCTGATAGGCACCTATTGGTCTTACTG ACATCCACTTTGCCTTTCTCTCCACAGGTGTCCACTCCCAGTTCAATTACAGCTCTTAAGGCTAG AGTACTTAATACGACTCACTATAGGCTAGCCTCGAGAATTCACGCGGGCCGCCATGAAGTGGG TAACCTTTATTTCCCTTCTTTTTCTCTTTAGCTCGGCCTATTCCAGGGGTGTGTTTCGTCGAGAT GGGCCCGCTTACCCTTACGATGTACCGGATTACGCACTCGACGATTCCAAGGGCATGCATAGA CCTGGCCCTCTTCCCCCGAGCCCACCTCCAAAGGTTGATCACAAGGCTTCCCAGTGGCCTCAG |
| | | GAGAGTGACATGGGGCAGAAGGTAGCTTCTTTGAGTGCGCAGTGCCACAACCATGATGAGAGA CTTGCAGAGCTGACAGTCCTGCTTCAGAAACTACAGATACGGGTAGACCAAGTGGATGACGGC AGGGAAGGGCTGTCACTGTGGGTCAAGAATGTGGTTGGACAGCACCTGCAGGAGATGGGCAC CATAGAACCACCTGATGCTAAGACTGACTTCATGACTTTCCACCATGACCATGAAGTGCGTCTC TCCAACTTGGAAGATGTTCTTAGAAAACTGACAGAAAAATCTGAGGCTATCCAGAAGGAGCTGG AAGAAACCAAGCTGAAAGCAGGCAGCAGGGATGAAGAGCAGCCCCTCCTTGACCGTGTGCAG CACCTAGAACTGGAACTGAACCTGTTGAAGTCACAGCTGTCAGACTGGCAGCATCTGAAGACC AGCTGTGAGCAGGCTGGGGCCCGCATCCAGGAGACTGTGCAGCTCATGTTCTCTGAGGATCA GCAGGGCGGTTCCCTCGAGTGGCTATTAGAGAAGCTTTCTTCTCGGTTCGTGAGCAAGGATGA GCTGCAGGTGCTCTTACATGACCTTGAGCTGAAACTGCTGCAGAATATCACACACCACATCACC GTGACAGGACAGGCCCCGACATCCGAGGCTATTGTGTCTGCCGTGAATCAGGCAGGGATTTCA GGAATCACAGAAGCGCAAGCACATATCATTGTGAACAATGCTCTGAAGCTGTACTCCCAAGACA AGACGGGGATGGTGGACTTTGCTCTGGAGTCTGGAGGTGGCAGCATCCTAAGCACTCGGTGC TCTGAGACCTATGAGACCAAGACGGCACTGCTGAGCCTGTTTGGGGTCCCACTGTGGTACTTC TCACAGTCACCTCGAGTGGTGATCCAGCCCGACATCTACCCAGGGAATTGCTGGGCGTTCAAA GGTTCCCAGGGGTACCTGGTGGTGCGGTTGTCCATGAAGATCTACCCAACCACATTCACCATG GAACACATTCCAAAGACACTATCACCCACTGGTAACATCTCCAGTGCCCCCAAAGACTTTGCAG TCTATGGACTGGAAACGGAGTATCAAGAAGAGGGGCAGCCTCTGGGACGGTTCACCTATGACC AGGAAGGAGACTCACTCCAGATGTTCCACACACTGGAAAGACCTGACCAAGCCTTCCAGATAG TAGAGCTCCGGGTCCTGTCCAACTGGGGCCACCCTGAGTACACTTGCCTCTACCGGTTCCGAG TCCACGGAGAGCCCATCCAGAAAGATGAGTTGTAGTAAGGTACCTCTAGAGTCGACCCGGGCG GCCTCGAGGACGGGGTGAACTACGCCTGAGGATCCGATCTTTTTCCCTCTGCCAAAAATTATG GGGACATCATGAAGCCCCTTGAGCATCTGACTTCTGGCTAATAAAGGAAATTTATTTTCATTGCA ATAGTGTGTTGGAATTTTTTGTGTCTCTCACTCGGAAGCAATTCGTTGATCTGAATTTCGACCAC CCATAATACCCATTACCCTGGTAGATAAGTAGCATGGCGGGTTAATCATTAACTACAAGGAACC CCTAGTGATGGAGTTGGCCACTCCCTCTCTGCGCGCTCGCTCGCTCACTGAGGCCGGGCGAC CAAAGGTCGCCCGACGCCCGGGCTTTGCCCGGGCGGCCTCAGTGAGCGAGCGAGCGCGCAG CCTTAATTAACCTAATTCACTGGCCGTCGTTTTACAACGTCGTGACTGGGAAAACCCTGGCGTT ACCCAACTTAATCGCCTTGCAGCACATCCCCCTTTCGCCAGCTGGCGTAATAGCGAAGAGGCC CGCACCGATCGCCCTTCCCAACAGTTGCGCAGCCTGAATGGCGAATGGACGCGCCCTGTAG CGGCGCATTAAGCGCGGCGGGTGTGGTGGTTACGCGCAGCGTGACCGCTACACTTGCCAGCG CCCTAGCGCCCGCTCCTTTCGCTTTCTTCCCTTCCTTTCTCGCCACGTTCGCCGGCTTTCCCCG TCAAGCTCTAAATCGGGGGCTCCCTTTAGGGTTCCGATTTAGTGCTTTACGGCACCTCGACCCC AAAAAACTTGATTAGGGTGATGGTTCACGTAGTGGGCCATCGCCCTGATAGACGGTTTTTCGCC CTTTGACGTTGGAGTCCACGTTCTTTAATAGTGGACTCTTGTTCCAAACTGGAACAACACTCAAC CCTATCTCGGTCTATTCTTTTGATTTATAAGGGATTTTGCCGATTTCGGCCTATTGGTTAAAAAAT GAGCTGATTTAACAAAAATTTAACGCGAATTTTAACAAAATATTAACGCTTACAATTTAGGTGGC ACTTTTCGGGGAAATGTGCGCGGAACCCCTATTTGTTTATTTTTCTAAATACATTCAAATATGTAT CCGCTCATGAGACAATAACCCTGATAAATGCTTCAATAATATTGAAAAAGGAAGAGTATGAGTAT TCAACATTTCCGTGTCGCCCTTATTCCCTTTTTTGCGGCATTTTGCCTTCCTGTTTTTGCTCACC CAGAAACGCTGGTGAAAGTAAAAGATGCTGAAGATCAGTTGGGTGCACGAGTGGGTTACATCG AACTGGATCTCAACAGCGGTAAGATCCTTGAGAGTTTTCGCCCCGAAGAACGTTTTCCAATGAT GAGCACTTTTAAAGTTCTGCTATGTGGCGCGGTATTATCCCGTATTGACGCCGGGCAAGAGCA ACTCGGTCGCCGCATACACTATTCTCAGAATGACTTGGTTGAGTACTCACCAGTCACAGAAAAG CATCTTACGGATGGCATGACAGTAAGAGAATTATGCAGTGCTGCCATAACCATGAGTGATAACA CTGCGGCCAACTTACTTCTGACAACGATCGGAGGACCGAAGGAGCTAACCGCTTTTTTGCACA ACATGGGGGATCATGTAACTCGCCTTGATCGTTGGGAACCGGAGCTGAATGAAGCCATACCAA ACGACGAGCGTGACACCACGATGCCTGTAGCAATGGCAACAACGTTGCGCAAACTATTAACTG GCGAACTACTTACTCTAGCTTCCCGGCAACAATTAATAGACTGGATGGAGGCGGATAAAGTTGC AGGACCACTTCTGCGCTCGGCCCTTCCGGCTGGCTGGTTTATTGCTGATAAATCTGGAGCCGG TGAGCGTGGGTCTCGCGGTATCATTGCAGCACTGGGGCCAGATGGTAAGCCCTCCCGTATCGT AGTTATCTACACGACGGGGAGTCAGGCAACTATGGATGAACGAAATAGACAGATCGCTGAGAT AGGTGCCTCACTGATTAAGCATTGGTAACTGTCAGACCAAGTTTACTCATATATACTTTAGATTG ATTTAAAACTTCATTTTTAATTTAAAAGGATCTAGGTGAAGATCCTTTTTGATAATCTCATGACCA AAATCCCTTAACGTGAGTTTTCGTTCCACTGAGCGTCAGACCCCGTAGAAAAGATCAAAGGATC TTCTTGAGATCCTTTTTTTCTGCGCGTAATCTGCTGCTTGCAAACAAAAAAACCACCGCTACCAG CGGTGGTTTGTTTGCCGGATCAAGAGCTACCAACTCTTTTTCCGAAGGTAACTGGCTTCAGCAG AGCGCAGATACCAAATACTGTTCTTCTAGTGTAGCCGTAGTTAGGCCACCACTTCAAGAACTCT GTAGCACCGCCTACATACCTCGCTCTGCTAATCCTGTTACCAGTGGCTGCTGCCAGTGGCGAT AAGTCGTGTCTTACCGGGTTGGACTCAAGACGATAGTTACCGGATAAGGCGCAGCGGTCGGG CTGAACGGGGGGTTCGTGCACACAGCCCAGCTTGGAGCGAACGACCTACACCGAACTGAGAT ACCTACAGCGTGAGCTATGAGAAAGCGCCACGCTTCCCGAAGGGAGAAAGGCGGACAGGTAT CCGGTAAGCGGCAGGGTCGGAACAGGAGAGCGCACGAGGGAGCTTCCAGGGGGAAACGCCT GGTATCTTTATAGTCCTGTCGGGTTTCGCCACCTCTGACTTGAGCGTCGATTTTTGTGATGCTC GTCAGGGGGGCGGAGCCTATGGAAAAACGCCAGCAACGCGGCCTTTTTACGGTTCCTGGCCT TTTGCTGGCCTTTTGCTCACATGTTCTTTCCTGCGTTATCCCCTGATTCTGTGGATAACCGTATT ACCGCCTTTGAGTGAGCTGATACCGCTCGCCGCAGCCGAACGACCGAGCGCAGCGAGTCAGT |
| | | GAGCGAGGAAGCGGAAGAGCGCCCAATACGCAAACCGCCTCTCCCCGCGCGTTGGCCGATTC ATTAATGCAGCTGGCACGACAGGTTTCCCGACTGGAAAGCGGGCAGTGAGCGCAACGCAATTA ATGTGAGTTAGCTCACTCATTAGGCACCCCAGGCTTTACACTTTATGCTTCCGGCTCGTATGTT GTGTGGAATTGTGAGCGGATAACAATTTCACACAGGAAACAGCTATGACCATGATTACGCCAGA TTTAATTAAGGCCTTAATTAGG |
| 4 | Humanized Sun1DNpositions 1-3 = start codon; positions 4-84 = signal sequence; positions 88 to 1446 = Sun1; positions 1447-1458 = KDEL; positions 1459-1461 = stop codon | ATGAAGTGGGTAACCTTTATTTCCCTTCTTTTTCTCTTTAGCTCGGCCTATTCCAGGGGTGTGTT TCGTCGAGATGGGCCCGCTCTCGATGACCCCCAGGACGTGTTTAAACCCACGACTTCTCGCCT GAAGCAGCCTCTGCAGGGTGACAGTGAGGCTTTTCCGTGGCATTGGATGAGTGGCGTGGAGC AGCAGGTGGCCTCTCTGTCTGGACAGTGCCACCACCATGGTGAGAATCTCCGAGAGCTGACCA CTTTGCTACAGAAGCTGCAGGCTCGGGTGGACCAGATGGAAGGCGGCGCTGCCGGGCCGTCA GCTTCGGTCAGAGACGCTGTGGGACAGCCCCCGAGGGAGACTGACTTTATGGCCTTTCACCAA GAACATGAAGTGCGTATGTCACACTTGGAAGATATTCTGGGAAAACTGAGAGAAAAATCTGAGG CCATCCAGAAGGAACTAGAACAGACCAAGCAAAAAACAATCAGTGCGGTTGGTGAGCAGCTCC TGCCCACAGTCGAGCACCTCCAGCTGGAGCTGGATCAGCTAAAGTCAGAGCTGTCCAGCTGG CGACACGTGAAGACCGGCTGTGAGACAGTGGATGCCGTACAAGAAAGAGTGGACGTGCAAGT CAGAGAAATGGTGAAACTCCTGTTTTCCGAAGATCAGCAAGGCGGTTCTCTGGAACAGCTGCT GCAGAGGTTCTCATCACAGTTTGTGAGCAAAGGCGACTTGCAGACGATGCTGCGAGACCTGCA GCTGCAGATCCTGCGGAACGTCACCCACCACGTTTCCGTGACCAAGCAGCTCCCAACCTCAGA AGCCGTGGTGTCTGCTGTGAGCGAGGCGGGGGCGTCTGGAATAACAGAGGCGCAAGCACGT GCCATCGTGAACAGCGCCTTGAAGCTGTATTCCCAAGATAAGACCGGGATGGTGGACTTTGCT CTGGAATCTGGTGGTGGCAGCATCTTGAGTACTCGCTGTTCTGAAACTTACGAAACCAAAACGG CGCTGATGAGTCTGTTTGGGATCCCGCTGTGGTACTTCTCGCAGTCCCCGCGCGTGGTCATCC AGCCTGACATTTACCCCGGTAACTGCTGGGCATTTAAAGGCTCCCAGGGGTACCTGGTGGTGA GGCTCTCCATGATGATCCACCCAGCCGCCTTCACTCTGGAGCACATCCCTAAGACGCTGTCGC CAACAGGCAACATCAGCAGCGCCCCCAAGGACTTCGCCGTCTATGGATTAGAAAATGAGTATC AGGAAGAAGGGCAGCTTCTGGGACAGTTCACGTATGATCAGGATGGGGAGTCGCTCCAGATGT TCCAGGCCCTGAAAAGACCCGACGACACAGCTTTCCAAATAGTGGAACTTCGGATTTTTTCTAA CTGGGGCCATCCTGAGTATACCTGTCTGTATCGGTTCAGAGTTCATGGCGAACCTGTCAAGAAA GATGAGTTGTGA |
| 5 | SUN2positions 1-3 = start codon; positions 4-84 = signal sequence; positions 88 to 1467 = Sun2; positions 1468-1479 = KDEL; positions 1480-1482 = stop codon | ATGAAGTGGGTAACCTTTATTTCCCTTCTTTTTCTCTTTAGCTCGGCCTATTCCAGGGGTGTGTT TCGTCGAGATGGGCCCGCTCTCGATGAGGGCTGGGAAGCCAGAGACTCATCGCCACATTTCC AGGCTGAGCAGCGTGTTATGTCCCGGGTACACTCTCTGGAGCGGCGTCTGGAAGCTCTTGCTG CTGAATTTTCCTCCAACTGGCAGAAGGAGGCCATGCGGCTGGAACGTCTGGAGCTGCGGCAA GGGGCTCCTGGCCAGGGAGGTGGTGGTGGCCTGAGCCACGAGGACACCCTGGCGCTGCTGG AGGGGCTAGTGAGCCGCCGTGAAGCTGCCCTGAAGGAGGATTTCCGCAGGGAAACTGCTGCT CGCATCCAGGAAGAACTGTCTGCCCTGAGAGCAGAGCATCAGCAAGACTCAGAAGACCTCTTC AAGAAGATCGTCCGGGCCTCCCAGGAGTCCGAGGCTCGCATCCAGCAGCTGAAGTCAGAGTG GCAAAGCATGACCCAGGAGTCCTTCCAGGAGAGCTCTGTGAAGGAGCTGAGGCGGCTGGAGG ACCAGCTGGCCGGCCTGCAGCAGGAGCTGGCGGCTCTGGCACTGAAGCAGAGCTCGGTGGC GGAAGAAGTGGGCCTGCTGCCCCAGCAGATCCAGGCCGTGCGGGACGACGTGGAATCTCAGT TCCCGGCCTGGATCAGTCAGTTCCTTGCCCGAGGTGGAGGGGGCCGCGTGGGGCTCCTTCAG AGAGAGGAGATGCAAGCTCAGCTGCGAGAGCTGGAGAGCAAGATCCTCACCCATGTGGCAGA GATGCAGGGCAAGTCGGCCCAGGGAAGCCGCGGCCTCCCTGAGCCTGACGCTGCAGAAAGAA GGTGTGATTGGAGTGACAGAGGAGCAGGTGCACCACATCGTGAAGCAGGCCCTGCAGCGCTA CAGTGAGGACCGCATCGGGCTGGCAGACTACGCCCTGGAGTCAGGAGGGGCCAGCGTCATCA GCACCCGATGTTCTGAGACCTACGAGACCAAGACGGCCCTCCTCAGCCTCTTCGGCATCCCCC TGTGGTACCACTCCCAGTCACCCCGAGTCATCCTCCAGCCAGATGTGCACCCAGGCAACTGCT GGGCCTTCCAGGGGCCACAAGGCTTCGCCGTGGTCCGCCTCTCTGCCCGCATCCGCCCCACA GCCGTTACCTTAGAGCATGTGCCCAAGGCCTTGTCACCCAACAGCACTATCTCCAGTGCCCCC AAGGACTTCGCCATCTTTGGGTTTGACGAAGACCTGCAGCAGGAGGGGACACTCCTTGGCAAG TTCACTTACGATCAGGACGGCGAGCCTATTCAGACGTTTCACTTTCAGGCCCCTACGATGGCCA CGTACCAGGTGGTGGAGCTGCGGATCCTGACTAACTGGGGCCACCCCGAGTACACCTGCATC TACCGCTTCAGAGTGCATGGGGAGCCCGCCCACAAAGATGAGTTGTAG |
| 6 | KASH1, derived from Nesprin-1 | CGCGGCTTCCTGTTCAGAGTCCTCCGAGCAGCTCTTCCCCTTCAGCTTCTCCTGCTCCTCCTCA TCGGGCTTGCCTGCCTTGTACCAATGTCAGAGGAAGACTACAGCTGTGCCCTCTCCAACAACTT TGCCCGGTCATTCCACCCCATGCTCAGATACACGAATGGCCCTCCTCCACTC |
| 7 | KASH1 amino acid sequence | RGFLFRVLRAALPLQLLLLLLIGLACLVPMSEEDYSCALSNNFARSFHPMLRYTNGPPPL |
| 8 | KASH2, derived from Nesprin-2 | CGCTCCTTCCTCTCAAGGGTGGTCCGGGCAGCCCTACCCCTGCAGCTGCTCCTCCTGCTGCTG CTGCTCCTGGCCTGCCTGCTGCCCTCCTCCGAAGAAGACTACAGCTGCACTCAGGCCAACAAC TTTGCCCGGTCCTTTTACCCCATGCTGAGGTACACCAATGGGCCACCCCCCACA |
| 9 | KASH2 amino acid sequence | RSFLSRVVRAALPLQLLLLLLLLLACLLPSSEEDYSCTQANNFARSFYPMLRYTNGPPPT |
| 10 | KASH3, derived from Nesprin-3 | GGCTCCCTCTTCCGGAGGGCGTGCTGTGTGGCGCTCCCACTGCAGCTGCTTCTGCTGCTGTTC CTCCTCCTGCTGTTCCTGCTCCCAATCAGGGAAGAGGACCGCAGCTGCACCCTGGCCAACAAC TTCGCCCGCTCCTTCACGCTCATGCTGCGCTACAATGGCCCACCACCCACC |
| 11 | KASH3 amino acid sequence | GSLFRRACCVALPLQLLLLLFLLLLFLLPIREEDRSCTLANNFARSFTLMLRYNGPPPT |
| 12 | KASH4, derived from Nesprin-4 | GATCCTGCATCCAGGCAGCCTCTGACCTTCCTCCTTATCCTCTTCCTCCTCTTCCTCCTCCTGG TGGGTGCCATGTTTCTCCTGCCCGCGTCAGGAGGCCCCTGCTGCTCTCATGCCCGAATACCCA GGACACCCTACCTGGTGCTCAGCTATGTCAATGGTCTTCCCCCAGTC |
| 13 | KASH4 amino acid sequence | DPASRQPLTFLLILFLLFLLLVGAMFLLPASGGPCCSHARIPRTPYLVLSYVNGLPPV |
| 14 | KASH5, derived from CCDC155/KASH5 | CTCAGAGTCACTCGACATCCACTGATCCCAGCTCCTGTCCTGGGCCTGCTGCTGCTGCTGCTG CTCTCTGTCCTGCTGCTTGGCCCGTCCCCACCTCCCACCTGGCCCCACCTCCAGCTCTGCTAC CTCCAGCCCCCTCCAGTG |
| 15 | KASH5 amino acid sequence | LRVTRHPLIPAPVLGLLLLLLLSVLLLGPSPPPTWPHLQLCYLQPPPV |
| 16 | FLX/FLX-F1 primer | CCAGCTTACAGAGCACCGAGCT |
| 17 | FLX/FLX-F2 primer | TCCTTGCAGTCCCTCTTGCATC |
| 18 | FLX/FLX-R1 primer | AGGCACCATTGTCACAGGGTC |
| 19 | Sun1-F primer | GGCAAGTGGATCTCTTGTGAATTCTTGAC |
| 20 | Sun1-R primer | GTAGCACCCACCTTGGTGAGCTGGTAC |
| 21 | Sun1-E8 primer | AGCCACATAACCACCTGGAG |
| 22 | MyHC-tF primer | ATGACAGACAGATCCCTCCTATCTCC |
| 23 | MyHC-tR primer | CTCATCACTCGTTGCATCATCGAC |
| 24 | MyHC-F primer | CAAATGTTGCTTGTCTGGTG |
| 25 | MyHC-R primer | GTCAGTCGAGTGCACAGTTT |
| 26 | mcm-3798t primer | AGGTGGACCTGATCATGGAG |
| 27 | mcm-8346t primer | ATACCGGAGATCATGCAAGC |
| 28 | mcm-7338 primer | CTAGGCCACAGAATTGAAAGATCT |
| 29 | mcm-7339 primer | GTAGGTGGAAATTCTAGCATCATCC |
| 30 | aav Sun1 F primer | CGAGAATTCACGCGGGCCGCCATGAAGTGGGTAACCTTTATTTC |
| 31 | aav Sun1 R primer | CGGGTCGACTCTAGAGGTACCTTACTACAACTCATCTTTCTGGATG |
| 32 | aav GFP Sun R primer | CGGGTCGACTCTAGAGGTACTTACTACAACTCATCTTTGGATCC |
| 33 | Sun1 delSUN RNA guide sequence | GCACAATAGCCTCGGATGTCG |
| 34 | Syne1-stop guide RNA sequence | CCGTTGGTATATCTGAGCAT |
| 35 | Tyrosinase4a guide RNA sequence | GGTTATGGCCGATAGGTGCAT |
| 36 | Common reverse primer for in vitro transcription | AAAAGCACCGACTCGGTGCC |
| 37 | SunldelSUN gRNA-specific forward primer | TTAATACGACTCACTATAGCACAATAGCCTCGGATGTCG |
| 38 | Syne1-stop gRNA-specific forward primer | TTAATACGACTCACTATAGCCGTTGGTATATCTGAGCAT |
| 39 | Tyrosinase4a gRNA-specific forward primer | TTAATACGACTCACTATAGGTTATGGCCGATAGGTGCAT |
| 40 | Syne1CT' del8 forward primer | TGCTCCTGCTGCTGCTTATT |
| 41 | Syne1CT' del8 reverse primer | ACATGGTGGAGCATTTGTCTCC |
| 42 | Sun1 CRISPR forward primer | TGACCTTGAGCTGAAACTGC |
| 43 | Sun1 CRISPR reverse primer | TCAGAACACTGGCACACACA |
| 44 | Potential guide RNA sequence, SYNE1 | TCGTGTATCTGAGCATGGGG |
| 45 | Potential guide RNA sequence, SYNE1 | GCCATTCGTGTATCTGAGCA |
| 46 | Potential guide RNA sequence, SYNE1 | TCCACCCCATGCTCAGATAC |
| 47 | Potential guide RNA sequence, SYNE1 | GAGCATGGGGTGGAATGACC |
| 48 | Potential guide RNA sequence, SYNE1 | TGAGCATGGGGTGGAATGAC |
| 49 | Potential guide RNA sequence, SYNE1 | TCGTGTATCTGAGCATGGGG |
| 50 | Potential guide RNA sequence, SYNE1 | CATTCGTGTATCTGAGCATG |
| 51 | Potential guide RNA sequence, SYNE1 | CCATTCGTGTATCTGAGCAT |
| 52 | Potential guide RNA sequence, SYNE1 | GCCATTCGTGTATCTGAGCA |
| 53 | Potential guide RNA sequence, SYNE1 | CCCATGCTCAGATACACGAA |
| 54 | Potential guide RNA sequence, SYNE1 | CCCGGTCATTCCACCCCATG |
| 55 | Potential guide RNA sequence, SUN1 | TTTTTCTAACTGGGGCCATC |
| 56 | Potential guide RNA sequence, SUN1 | CCGATACAGACAGGTATACT |
| 57 | Potential guide RNA sequence, SUN1 | AACTTCGGATTTTTTCTAAC |
| 58 | Potential guide RNA sequence, SUN1 | ACTTCGGATTTTTTCTAACT |
| 59 | Potential guide RNA sequence, SUN1 | CTTCGGAITTTTTTCIAACTG |
| 60 | Potential guide RNA sequence, SUN1 | ACAGACAGGTATACTCAGGA |
| 61 | Potential guide RNA sequence, SUN1 | CTGAGTATACCTGTCTGTAT |
| 62 | Potential guide RNA sequence, SUN1 | TTCTAACTGGGGCCATCCTG |
| 63 | Potential guide RNA sequence, SUN1 | TCTAACTGGGGCCATCCTGA |
| 64 | Potential guide RNA sequence, SUN1 | CTAACTGGGGCCATCCTGAG |
| 65 | Potential guide RNA sequence, SUN1 | TAACTGGGGCCATCCTGAGT |
| 66 | Cas9 mRNA and gRNA targeting SUN1 domain | GCACAATAGCCTCGGATGTCG |
| 67 | Cas9 mRNA and gRNA targeting the KASH1 domain | CCGTTGGTATATCTGAGCAT |
| 68 | gRNA targeting tyrosinase | GGTTATGGCCGATAGGTGCAT |
| 69 | wildtype Sun1 DNA | CATGACCTTGAGCTGAAACTGCTGCAGAATATCACACACCACATCACCGTGACAGGACAGGCC CCGACATCCGAGGCTATTGTGTCTGCCGTGAATCAGGCAGGGATTTCAGGAATCACAGAAGCG CAAGCACATATCATTGTGAACAATGCTCTGAAGCTGTACTCCCAAGACAA |
| 70 | Sun1_plus4 | CATGACCTTGAGCTGAAACTGCTGCAGAATATCACACACCACATCACCGTGACAGGACAGGCC CCGAGACACATCCGAGGCTATTGTGTCTGCCGTGAATCAGGCAGGGATTTCAGGAATCACAGA AGCGCAAGCACATATCATTGTGAACAATGCTCTGAAGCTGTACTCCCAAGACAA |
| 71 | Sun1_del7 | CATGACCTTGAGCTGAAACTGCTGCAGAATATCACACACCACATCACCGTGACAGGACAGGCC CCGAGGCTATTGTGTCTGCCGTGAATCAGGCAGGGATTTCAGGAATCACAGAAGCGCAAGCAC ATATCATTGTGAACAATGCTCTGAAGCTGTACTCCCAAGACAA |
| 72 | wildtype Sun1 | WLLEKLSSRFVSKDELQVLLHDLELKLLQNITHHITVTGQAPTSEAIVSAVNQAGISGITEAQAHIIVNN ALKLYSQDKTGMVDFALESGGGSILSTRCSETYETKTALLSLFGVPLWYFSQSPRVVIQPDIYPGNC WAFKGSQGYLVVRLSMKIYPTTFTMEHIPKTLSPTGNISSAPKDFAVYGLETEYQEEGQPLGRFTYD QEGDSLQMFHTLERPDQAFQIVELRVLSNWGHPEYTCLYRFRVHGEPIQ |
| 73 | Sun1_plus4 protein | WLLEKLSSRFVSKDELQVLLHDLELKLLQNITHHITVTGQAPRHIRGYCVCRESGRDFRNHRSASTY HCEQCSEAVLPRQDGDGGLCSGVWRWQHPKHSVL |
| 74 | Sun1 del7 protein | WLLWKLSSRFVSKDELQVLLHDLELKLLQNITHHITVTGQAPRLLCLP |
| 75 | wildtype Nesprin-1 DNA | TATTGGACTCACCTGCCTTGTACCCATGTCAGAGAAAGACTACAGCTGTGCCCTCTCCAACAAC TTTGCCCGATCCTTCCATCCGATGCTCAGATATACCAACGGTCCTCCTCCACTCTGAAGCAAGC AGACATCCCCACACAAGTGCAGGCAGTAAGAGGAGAAGGAATATCAAATGGC |
| 76 | Syne1_CTdel8 | TATTGGACTCACCTGCCTTGTACCCATGTCAGAGAAAGACTACAGCTGTGCCCTCTCCAACAAC TTTGCCCGATCCTTCCATCCGATATACCAACGGTCCTCCTCCACTCTGAAGCAAGCAGACATCC CCACACAAGTGCAGGCAGTAAGAGGAGAAGGAATATCAAATGGC |
| 77 | wildtype Nesprin-1 | STRDGSDSSRSDPRPERVGRAFLFRILRAALPFQLLLLLLIGLTCLVPMSEKDYSCALSNNFARSFHP MLRYTNGPPPL |
| 78 | Nesprin1_CTdel8 | STRDGSDSSRSDPRPERVGRAFLFRILRAALPFQLLLLLLIGLTCLVPMSEKDYSCALSNNFARSFHP IYQRSSSTLKQADIPTQVQAVRGEGISNGREAPKEKFNILNHQGNANQNNPEMETNAPPCS |
| 79 | | CGCGGCTTCCTGTTCAGAGTCCTCCGAGCAGCTCTTCCCCTTCAGCTTCTCCTGCTCCTCCTCA TCGGGCTTGCCTGCCTTGTACCAATGTCAGAGGAAGACTACAGCTGTGCCCTCTCCAACAACTT TGCCCGGTCATTCCACCCCATGCTCAGATACACGAATGGCCCTCCTCCACTCTGA |
| 80 | Human Sun1 SUN domain nucleic acid sequence | GATAAGACCGGGATGGTGGACTTTGCTCTGGAATCTGGTGGTGGCAGCATCTTGAGTACTCGC TGTTCTGAAACTTACGAAACCAAAACGGCGCTGATGAGTCTGTTTGGGATCCCGCTGTGGTACT TCTCGCAGTCCCCGCGCGTGGTCATCCAGCCTGACATTTACCCCGGTAACTGCTGGGCATTTA AAGGCTCCCAGGGGTACCTGGTGGTGAGGCTCTCCATGATGATCCACCCAGCCGCCTTCACTC TGGAGCACATCCCTAAGACGCTGTCGCCAACAGGCAACATCAGCAGCGCCCCCAAGGACTTC GCCGTCTATGGATTAGAAAATGAGTATCAGGAAGAAGGGCAGCTTCTGGGACAGTTCACGTAT GATCAGGATGGGGAGTCGCTCCAGATGTTCCAGGCCCTGAAAAGACCCGACGACACAGCTTTC CAAATAGTGGAACTTCGGATTTTTTCTAACTGGGGCCATCCTGAGTATACCTGTCTGTATCGGTT CAGAGTTCATGGCGAACCTGTCAAGTGA |
| 81 | Human Sun1 exon 20 end sequence | AAAAGACCCGACGACACAGCTTTCCAAATAGTGGAACTTCGGATTTTTCTAACTGGGGCCATC CTGAGTATACCTGTCTGTATCGGTTCAGAGTTCATGGCGAACCTGTCAAGTGA |
| 82 | SUN domain of human SUN1 | GGSILSTRCSETYETKTALMSLFGIPLWYFSQSPRVVIQPDIYPGNCWAFKGSQGYLVVRLSMMIHP AAFTLEHIPKTLSPTGNISSAPKDFAVYGLENEYQEEGQLLGQFTYDQDGESLQMFQALKRPDDTAF QIVELRIFSNWGHPEYTCLYRFRVHGEPV |
| 83 | SUN domain of human SUN2 | GASVISTRCSETYETKTALLSLFGIPLWYHSQSPRVILQPDVHPGNCWAFQGPQGFAVVRLSARIRP TAVTLEHVPKALSPNSTISSAPKDFAIFGFDEDLQQEGTLLGKFTYDQDGEPIQTFHFQAPTMATYQ VVELRILTNWGHPEYTCIYRFRVHGEPA |
| 84 | SUN domain of human SUNS | GASIIEAGTSESYKNNKAKLYWHGIGFLNHEMPPDIILQPDVYPGKCWAFPGSQGHTLIKLATKIIPTA VTMEHISEKVSPSGNISSAPKEFSVYGITKKCEGEEIFLGQFIYNKTGTTVQTFELQHAVSEYLLCVKL NIFSNWGHPKYTCLYRFRVHGTPG |
| 85 | SUN domain of human SUNS | GASIDFEHTSVTYNHEKAHSYWNWIQLWNYAQPPDVILEPNVTPGNCWAFEGDRGQVTIQLAQKV YLSNLTLQHIPKTISLSGSLDTAPKDFVIYGMEGSPKEEVFLGAFQFQPENIIQMFPLQNQPARAFSA VKVKISSNWGNPGFTCLYRVRVHGSVA |
| 86 | SUN domain of human SPAG4 | GASIDLQKTSHDYADRNTAYFWNRFSFWNYARPPTVILEPHVFPGNCWAFEGDQGQVVIQLPGRV QLSDITLQHPPPSVEHTGGANSAPRDFAVFGLQVYDETEVSLGKFTFDVEKSEIQTFHLQNDPPAAF PKVKIQILSNWGHPRFTCLYRVRAHGVRT |
| 87 | SUN domain of human SUCO | EKSQSMHASSNGGSHATKKVQKNRNNYASVECGAKILAANPEAKSTSAILIENMDLYMLNPCSTKI WFVIELCEPIQVKQLDIANYELFSSTPKDFLVSISDRYPTNKWIKLGTFHGRDERNVQSFPLDEQMYA KYVKMFIKYIKVELLSHFGSEHFCPLSLIRVFGTSM |
| 88 | Human SUN1 (UniProtKB 094901-1) | MDFSRLHMYSPPQCVPENTGYTYALSSSYSSDALDFETEHKLDPVFDSPRMSRRSLRLATTACTLG DGEAVGADSGTSSAVSLKNRAARTTKQRRSTNKSAFSINHVSRQVTSSGVSHGGTVSLQDAVTRR PPVLDESWIREQTTVDHFWGLDDDGDLKGGNKAAIQGNGDVGAAAATAHNGFSCSNCSMLSERK DVLTAHPAAPGPVSRVYSRDRNQKCDDCKGKRHLDAHPGRAGTLWHIWACAGYFLLQILRRIGAV GQAVSRTAWSALWLAVVAPGKAASGVFWWLGIGWYQFVTLISWLNVFLLTRCLRNICKFLVLLIPLF LLLAGLSLRGQGNFFSFLPVLNWASMHRTQRVDDPQDVFKPTTSRLKQPLQGDSEAFPWHWMSG VEQQVASLSGQCHHHGENLRELTTLLQKLQARVDQMEGGAAGPSASVRDAVGQPPRETDFMAFH QEHEVRMSHLEDILGKLREKSEAIQKELEQTKQKTISAVGEQLLPTVEHLQLELDQLKSELSSWRHV KTGCETVDAVQERVDVQVREMVKLLFSEDQQGGSLEQLLQRFSSQFVSKGDLQTMLRDLQLQILR NVTHHVSVTKQLPTSEAVVSAVSEAGASGITEAQARAIVNSALKLYSQDKTGMVDFALESGGGSILS TRCSETYETKTALMSLFGIPLWYFSQSPRVVIQPDIYPGNCWAFKGSQGYLVVRLSMMIHPAAFTLE HIPKTLSPTGNISSAPKDFAVYGLENEYQEEGQLLGQFTYDQDGESLQMFQALKRPDDTAFQIVELR IFSNWGHPEYTCLYRFRVHGEPVK |
| 89 | Human SUN2 (UniProtKB Q9UH99-1) | MSRRSQRLTRYSQGDDDGSSSSGGSSVAGSQSTLFKDSPLRTLKRKSSNMKRLSPAPQLGPSSD AHTSYYSESLVHESWFPPRSSLEELHGDANWGEDLRVRRRRGTGGSESSRASGLVGRKATEDFL GSSSGYSSEDDYVGYSDVDQQSSSSRLRSAVSRAGSLLWMVATSPGRLFRLLYWWAGTTWYRLT TAASLLDVFVLTRRFSSLKTFLWFLLPLLLTCLTYGAWYFYPYGLQTFHPALVSWWAAKDSRRPDE GWEARDSSPHFQAEQRVMSRVHSLERRLEALAAEFSSNWQKEAMRLERLELRQGAPGQGGGGG LSHEDTLALLEGLVSRREAALKEDFRRETAARIQEELSALRAEHQQDSEDLFKKIVRASQESEARIQ QLKSEWQSMTQESFQESSVKELRRLEDQLAGLQQELAALALKQSSVAEEVGLLPQQIQAVRDDVE SQFPAWISQFLARGGGGRVGLLQREEMQAQLRELESKILTHVAEMQGKSAREAAASLSLTLQKEG VIGVTEEQVHHIVKQALQRYSEDRIGLADYALESGGASVISTRCSETYETKTALLSLFGIPLWYHSQS PRVILQPDVHPGNCWAFQGPQGFAVVRLSARIRPTAVTLEHVPKALSPNSTISSAPKDFAIFGFDED LQQEGTLLGKFTYDQDGEPIQTFHFQAPTMATYQVVELRILTNWGHPEYTCIYRFRVHGEPAH |
| 90 | Human SUNS (UniProtKB Q8TAQ9-1) | MSGKTKARRAAMFFRRCSEDASGSASGNALLSEDENPDANGVTRSWKIILSTMLTLTFLLVGLLNH QWLKETDVPQKSRQLYAIIAEYGSRLYKYQARLRMPKEQLELLKKESQNLENNFRQILFLIEQIDVLK ALLRDMKDGMDNNHNWNTHGDPVEDPDHTEEVSNLVNYVLKKLREDQVEMADYALKSAGASIIEA GTSESYKNNKAKLYWHGIGFLNHEMPPDIILQPDVYPGKCWAFPGSQGHTLIKLATKIIPTAVTMEHI SEKVSPSGNISSAPKEFSVYGITKKCEGEEIFLGQFIYNKTGTTVQTFELQHAVSEYLLCVKLNIFSN WGHPKYTCLYRFRVHGTPGKHI |
| 91 | Human SUNS (UniProtKB A9Z1W8-1) | MPRSSRSPGDPGALLEDVAHNPRPRRIAQRGRNTSRMAEDTSPNMTWFTCFACSLRTQAQQVLF NTCRCKLLCQKLMEKTGILLLCAFGFWMFSIHLPSKMKVWQDDSINGPLQSLRLYQEKVRHHSGEI QDLRGSMNQLIAKLQEMEAMSDEQKMAQKIMKMIHGDYIEKPDFALKSIGASIDFEHTSVTYNHEKA HSYWNWIQLWNYAQPPDVILEPNVTPGNCWAFEGDRGQVTIQLAQKVYLSNLTLQHIPKTISLSGSL DTAPKDFVIYGMEGSPKEEVFLGAFQFQPENIIQMFPLQNQPARAFSAVKVKISSNWGNPGFTCLY RVRVHGSVAPPREQPHQNPYPKRD |
| 92 | Human SPAG4 (UniProtKB Q9NPE6-1) | MRRSSRPGSASSSRKHTPNFFSENSSMSITSEDSKGLRSAEPGPGEPEGRRARGPSCGEPALSAG VPGGTTWAGSSQQKPAPRSHNWQTACGAATVRGGASEPTGSPVVSEEPLDLLPTLDLRQEMPPP RVFKSFLSLLFQGLSVLLSLAGDVLVSMYREVCSIRFLFTAVSLLSLFLSAFWLGLLYLVSPLENEPKE MLTLSEYHERVRSQGQQLQQLQAELDKLHKEVSTVRAANSERVAKLVFQRLNEDFVRKPDYALSS VGASIDLQKTSHDYADRNTAYFWNRFSFWNYARPPTVILEPHVFPGNCWAFEGDQGQVVIQLPGR VQLSDITLQHPPPSVEHTGGANSAPRDFAVFGLQVYDETEVSLGKFTFDVEKSEIQTFHLQNDPPAA FPKVKIQILSNWGHPRFTCLYRVRAHGVRTSEGAEGSAQGPH |
| 93 | Human SUCO (UniProtKB Q9UBS9-1) | MKKHRRALALVSCLFLCSLVWLPSWRVCCKESSSASASSYYSQDDNCALENEDVQFQKKDEREG PINAESLGKSGSNLPISPKEHKLKDDSIVDVQNTESKKLSPPVVETLPTVDLHEESSNAVVDSETVEN ISSSSTSEITPISKLDEIEKSGTIPIAKPSETEQSETDCDVGEALDASAPIEQPSFVSPPDSLVGQHIEN VSSSHGKGKITKSEFESKVSASEQGGGDPKSALNASDNLKNESSDYTKPGDIDPTSVASPKDPEDIP TFDEWKKKVMEVEKEKSQSMHASSNGGSHATKKVQKNRNNYASVECGAKILAANPEAKSTSAILIE NMDLYMLNPCSTKIWFVIELCEPIQVKQLDIANYELFSSTPKDFLVSISDRYPTNKWIKLGTFHGRDE RNVQSFPLDEQMYAKYVKMFIKYIKVELLSHFGSEHFCPLSLIRVFGTSMVEEYEEIADSQYHSERQ ELFDEDYDYPLDYNTGEDKSSKNLLGSATNAILNMVNIAANILGAKTEDLTEGNKSISENATATAAPK MPESTPVSTPVPSPEYVTTEVHTHDMEPSTPDTPKESPIVQLVQEEEEEASPSTVTLLGSGEQEDE SSPWFESETQIFCSELTTICCISSFSEYIYKWCSVRVALYRQRSRTALSKGKDYLVLAQPPLLLPAES VDVSVLQPLSGELENTNIEREAETVVLGDLSSSMHQDDLVNHTVDAVELEPSHSQTLSQSLLLDITP EINPLPKIEVSESVEYEAGHIPSPVIPQESSVEIDNETEQKSESFSSIEKPSITYETNKVNELMDNIIKED VNSMQIFTKLSETIVPPINTATVPDNEDGEAKMNIADTAKQTLISVVDSSSLPEVKEEEQSPEDALLR GLQRTATDFYAELQNSTDLGYANGNLVHGSNQKESVFMRLNNRIKALEVNMSLSGRYLEELSQRY RKQMEEMQKAFNKTIVKLQNTSRIAEEQDQRQTEAIQLLQAQLTNMTQLVSNLSATVAELKREVSD RQSYLVISLVLCVVLGLMLCMQRCRNTSQFDGDYISKLPKSNQYPSPKRCFSSYDDMNLKRRTSFP LMRSKSLQLTGKEVDPNDLYIVEPLKFSPEKKKKRCKYKIEKIETIKPEEPLHPIANGDIKGRKPFTNQ RDFSNMGEVYHSSYKGPPSEGSSETSSQSEESYFCGISACTSLCNGQSQKTKTEKRALKRRRSKV QDQGKLIKTLIQTKSGSLPSLHDIIKGNKEITVGTFGVTAVSGHI |
| 94 | LMNA binding domain of human SUN1 | MDFSRLHMYSPPQCVPENTGYTYALSSSYSSDALDFETEHKLDPVFDSPRMSRRSLRLATTACTLG DGEAVGADSGTSSAVSLKNRAARTTKQRRSTNKSAFSINHVSRQVTSSGVSHGGTVSLQDAVTRR PPVLDES |
| 95 | EMD binding domain of human SUN1 | CKGKRHLDAHPGRAGTLWHIWACAGYFLLQILRRIGAVGQAVSRTAWSALWLAVVAPGKAASGVF WWLGIGWYQFVTLIS |
| 96 | LMNA binding domain of human SUN2 | MSRRSQRLTRYSQGDDDGSSSSGGSSVAGSQSTLFKDSPLRTLKRKSSNMKRLSPAPQLGPSSD AHTSYYSESLVHESWFPPRSSLEELHGDANWGEDLRVRRRRGTGGSESSRASGLVGRKATEDFL GSSSGYSSEDD |
| 97 | KASH domain of human Nesprin-1 | RGFLFRVLRAALPLQLLLLLLIGLACLVPMSEEDYSCALSNNFARSFHPMLRYTNGPPPL |
| 98 | KASH domain of human Nesprin-2 | RSFLSRVVRAALPLQLLLLLLLLLACLLPSSEEDYSCTQANNFARSFYPMLRYTNGPPPT |
| 99 | KASH domain of human Nesprin-3 | GSLFRRACCVALPLQLLLLLFLLLLFLLPIREEDRSCTLANNFARSFTLMLRYNGPPPT |
| 100 | KASH domain of human Nesprin-4 | DPASRQPLTFLLILFLLFLLLVGAMFLLPASGGPCCSHARIPRTPYLVLSYVNGLPPV |
| 101 | KASH domain of human KASH5 | LIPAPVLGLLLLLLLSVLLLGPSPPPTWPHLQLCYLQPPPV |
| 102 | Human Nesprin-1(UniProtKB Q8NF91-1) | MATSRGASRCPRDIANVMQRLQDEQEIVQKRTFTKWINSHLAKRKPPMVVDDLFEDMKDGVKLLAL LEVLSGQKLPCEQGRRMKRIHAVANIGTALKFLEGRKIKLVNINSTDIADGRPSIVLGLMWTIILYFQIE ELTSNLPQLQSLSSSASSVDSIVSSETPSPPSKRKVTTKIQGNAKKALLKWVQYTAGKQTGIEVKDF GKSWRSGVAFHSVIHAIRPELVDLETVKGRSNRENLEDAFTIAETELGIPRLLDPEDVDVDKPDEKSI MTYVAQFLKHYPDIHNASTDGQEDDEILPGFPSFANSVQNFKREDRVIFKEMKVWIEQFERDLTRA QMVESNLQDKYQSFKHFRVQYEMKRKQIEHLIQPLHRDGKLSLDQALVKQSWDRVTSRLFDWHIQ LDKSLPAPLGTIGAWLYRAEVALREEITVQQVHEETANTIQRKLEQHKDLLQNTDAHKRAFHEIYRTR SVNGIPVPPDQLEDMAERFHFVSSTSELHLMKMEFLELKYRLLSLLVLAESKLKSWIIKYGRRESVEQ LLQNYVSFIENSKFFEQYEVTYQILKQTAEMYVKADGSVEEAENVMKFMNETTAQWRNLSVEVRSV RSMLEEVISNWDRYGNTVASLQAWLEDAEKMLNQSENAKKDFFRNLPHWIQQHTAMNDAGNFLIE TCDEMVSRDLKQQLLLLNGRWRELFMEVKQYAQADEMDRMKKEYTDCVVTLSAFATEAHKKLSEP LEVSFMNVKLLIQDLEDIEQRVPVMDAQYKIITKTAHLITKESPQEEGKEMFATMSKLKEQLTKVKEC YSPLLYESQQLLIPLEELEKQMTSFYDSLGKINEIITVLEREAQSSALFKQKHQELLACQENCKKTLTLI EKGSQSVQKFVTLSNVLKHFDQTRLQRQIADIHVAFQSMVKKTGDWKKHVETNSRLMKKFEESRA ELEKVLRIAQEGLEEKGDPEELLRRHTEFFSQLDQRVLNAFLKACDELTDILPEQEQQGLQEAVRKL HKQWKDLQGEAPYHLLHLKIDVEKNRFLASVEECRTELDRETKLMPQEGSEKIIKEHRVFFSDKGPH |
| | | HLCEKRLQLIEELCVKLPVRDPVRDTPGTCHVTLKELRAAIDSTYRKLMEDPDKWKDYTSRFSEFSS WISTNETQLKGIKGEAIDTANHGEVKRAVEEIRNGVTKRGETLSWLKSRLKVLTEVSSENEAQKQGD ELAKLSSSFKALVTLLSEVEKMLSNFGDCVQYKEIVKNSLEELISGSKEVQEQAEKILDTENLFEAQQ LLLHHQQKTKRISAKKRDVQQQIAQAQQGEGGLPDRGHEELRKLESTLDGLERSRERQERRIQVTL RKWERFETNKETVVRYLFQTGSSHERFLSFSSLESLSSELEQTKEFSKRTESIAVQAENLVKEASEI PLGPQNKQLLQQQAKSIKEQVKKLEDTLEEDIKTMEMVKTKWDHFGSNFETLSVWITEKEKELNALE TSSSAMDMQISQIKVTIQEIESKLSSIVGLEEEAQSFAQFVTTGESARIKAKLTQIRRYGEELREHAQC LEGTILGHLSQQQKFEENLRKIQQSVSEFEDKLAVPIKICSSATETYKVLQEHMDLCQALESLSSAITA FSASARKVVNRDSCVQEAAALQQQYEDILRRAKERQTALENLLAHWQRLEKELSSFLTWLERGEAK ASSPEMDISADRVKVEGELQLIQALQNEVVSQASFYSKLLQLKESLFSVASKDDVKMMKLHLEQLDE RWRDLPQIINKRINFLQSVVAEHQQFDELLLSFSVWIKLFLSELQTTSEISIMDHQVALTRHKDHAAEV ESKKGELQSLQGHLAKLGSLGRAEDLHLLQGKAEDCFQLFEEASQVVERRQLALSHLAEFLQSHAS LSGILRQLRQTVEATNSMNKNESDLIEKDLNDALQNAKALESAAVSLDGILSKAQYHLKIGSSEQRTS CRATADQLCGEVERIQNLLGTKQSEADALAVLKKAFQDQKEELLKSIEDIEERTDKERLKEPTRQAL QQRLRVFNQLEDELNSHEHELCWLKDKAKQIAQKDVAFAPEVDREINRLEVTWDDTKRLIHENQGQ CCGLIDLMREYQNLKSAVSKVLENASSVIVTRTTIKDQEDLKWAFSKHETAKNKMNYKQKDLDNFTS KGKHLLSELKKIHSSDFSLVKTDMESTVDKWLDVSEKLEENMDRLRVSLSIWDDVLSTRDEIEGWS NNCVPQMAENISNLDNHLRAEELLKEFESEVKNKALRLEELHSKVNDLKELTKNLETPPDLQFIEADL MQKLEHAKEITEVAKGTLKDFTAQSTQVEKFINDITTWFTKVEESLMNCAQNETCEALKKVKDIQKEL QSQQSNISSTQENLNSLCRKYHSAELESLGRAMTGLIKKHEAVSQLCSKTQASLQESLEKHFSESM QEFQEWFLGAKAAAKESSDRTGDSKVLEAKLHDLQNILDSVSDGQSKLDAVTQEGQTLYAHLSKQI VSSIQEQITKANEEFQAFLKQCLKDKQALQDCASELGSFEDQHRKLNLWIHEMEERFNTENLGESK QHIPEKKNEVHKVEMFLGELLAARESLDKLSQRGQLLSEEGHGAGQEGRLCSQLLTSHQNLLRMTK EKLRSCQVALQEHEALEEALQSMWFWVKAIQDRLACAESTLGSKDTLEKRLSQIQDILLMKGEGEV KLNMAIGKGEQALRSSNKEGQRVIQTQLETLKEVWADIMSSSVHAQSTLESVISQWNDYVERKNQL EQWMESVDQKIEHPLQPQPGLKEKFVLLDHLQSILSEAEDHTRALHRLIAKSRELYEKTEDESFKDT AQEELKTQFNDIMTVAKEKMRKVEEIVKDHLMYLDAVHEFTDWLHSAKEELHRWSDMSGDSSATQ KKLSKIKELIDSREIGASRLSRVESLAPEVKQNTTASGCELMHTEMQALRADWKQWEDSVFQTQSC LENLVSQMALSEQEFSGQVAQLEQALEQFSALLKTWAQQLTLLEGKNTDEEIVECWHKGQEILDAL QKAEPRTEDLKSQLNELCRFSRDLSTYSGKVSGLIKEYNCLCLQASKGCQNKEQILQQRFRKAFRD FQQWLVNAKITTAKCFDIPQNISEVSTSLQKIQEFLSESENGQHKLNMMLSKGELLSTLLTKEKAKGI QAKVTAAKEDWKNFHSNLHQKESALENLKIQMKDFEVSAEPIQDWLSKTEKMVHESSNRLYDLPAK RREQQKLQSVLEEIHCYEPQLNRLKEKAQQLWEGQAASKSFRHRVSQLSSQYLALSNLTKEKVSRL DRIVAEHNQFSLGIKELQDWMTDAIHMLDSYCHPTSDKSVLDSRTLKLEALLSVKQEKEIQMKMIVT RGESVLQNTSPEGIPTIQQQLQSVKDMWASLLSAGIRCKSQLEGALSKWTSYQDGVRQFSGWMDS MEANLNESERQHAELRDKTTMLGKAKLLNEEVLSYSSLLETIEVKGAGMTEHYVTQLELQDLQERY RAIQERAKEAVTKSEKLVRLHQEYQRDLKAFEVWLGQEQEKLDQYSVLEGDAHTHETTLRDLQELQ VHCAEGQALLNSVLHTREDVIPSGIPQAEDRALESLRQDWQAYQHRLSETRTQFNNVVNKLRLME QKFQQVDEWLKTAEEKVSPRTRRQSNRATKEIQLHQMKKWHEEVTAYRDEVEEVGARAQEILDES HVNSRMGCQATQLTSRYQALLLQVLEQIKFLEEEIQSLEESESSLSSYSDWYGSTHKNFKNVATKID KVDTVMMGKKLKTLEVLLKDMEKGHSLLKSAREKGERAVKYLEEGEAERLRKEIHDHMEQLKELTS TVRKEHMTLEKGLHLAKEFSDKCKALTQWIAEYQEILHVPEEPKMELYEKKAQLSKYKSLQQTVLSH EPSVKSVREKGEALLELVQDVTLKDKIDQLQSDYQDLCSIGKEHVFSLEAKVKDHEDYNSELQEVEK WLLQMSGRLVAPDLLETSSLETITQQLAHHKAMMEEIAGFEDRLNNLQMKGDTLIGQCADHLQAKL KQNVHAHLQGTKDSYSAICSTAQRMYQSLEHELQKHVSRQDTLQQCQAWLSAVQPDLEPSPQPPL SRAEAIKQVKHFRALQEQARTYLDLLCSMCDLSNASVKTTAKDIQQTEQTIEQKLVQAQNLTQGWE EIKHLKSELWIYLQDADQQLQNMKRRHSELELNIAQNMVSQVKDFVKKLQSKQASVNTIIEKVNKLT KKEESPEHKEINHLNDQWLDLCRQSNNLCLQREEDLQRTRDYHDCMNVVEVFLEKFTTEWDNLAR SDAESTAVHLEALKKLALALQERKYAIEDLKDQKQKMIEHLNLDDKELVKEQTSHLEQRWFQLEDLIK RKIQVSVTNLEELNVVQSRFQELMEWAEEQQPNIAEALKQSPPPDMAQNLLMDHLAICSELEAKQM LLKSLIKDADRVMADLGLNERQVIQKALSDAQSHVNCLSDLVGQRRKYLNKALSEKTQFLMAVFQAT SQIQQHERKIMFREHICLLPDDVSKQVKTCKSAQASLKTYQNEVTGLWAQGRELMKEVTEQEKSEV LGKLQELQSVYDSVLQKCSHRLQELEKNLVSRKHFKEDFDKACHWLKQADIVTFPEINLMNESSEL HTQLAKYQNILEQSPEYENLLLTLQRTGQTILPSLNEVDHSYLSEKLNALPRQFNVIVALAKDKFYKV QEAILARKEYASLIELTTQSLSELEAQFLRMSKVPTDLAVEEALSLQDGCRAILDEVAGLGEAVDELN QKKEGFRSTGQPWQPDKMLHLVTLYHRLKRQTEQRVSLLEDTTSAYQEHEKMCQQLERQLKSVK EEQSKVNEETLPAEEKLKMYHSLAGSLQDSGIVLKRVTIHLEDLAPHLDPLAYEKARHQIQSWQGEL KLLTSAIGETVTECESRMVQSIDFQTEMSRSLDWLRRVKAELSGPVYLDLNLQDIQEEIRKIQIHQEE VQSSLRIMNALSHKEKEKFTKAKELISADLEHSLAELSELDGDIQEALRTRQATLTEIYSQCQRYYQV FQAANDWLEDAQELLQLAGNGLDVESAEENLKSHMEFFSTEDQFHSNLEELHSLVATLDPLIKPTG KEDLEQKVASLELRSQRMSRDSGAQVDLLQRCTAQWHDYQKAREEVIELMNDTEKKLSEFSLLKT SSSHEAEEKLSEHKALVSVVNSFHEKIVALEEKASQLEKTGNDASKATLSRSMTTVWQRWTRLRAV AQDQEKILEDAVDEWTGFNNKVKKATEMIDQLQDKLPGSSAEKASKAELLTLLEYHDTFVLELEQQ QSALGMLRQQTLSMLQDGAAPTPGEEPPLMQEITAMQDRCLNMQEKVKTNGKLVKQELKDREMV ETQINSVKCWVQETKEYLGNPTIEIDAQLEELQILLTEATNHRQNIEKMAEEQKEKYLGLYTILPSELS LQLAEVALDLKIRDQIQDKIKEVEQSKATSQELSRQIQKLAKDLTTILTKLKAKTDNVVQAKTDQKVLG EELDGCNSKLMELDAAVQKFLEQNGQLGKPLAKKIGKLTELHQQTIRQAENRLSKLNQAASHLEEY |
| | | NEMLELILKWIEKAKVLAHGTIAWNSASQLREQYILHQTLLEESKEIDSELEAMTEKLQYLTSVYCTEK MSQQVAELGRETEELRQMIKIRLQNLQDAAKDMKKFEAELKKLQAALEQAQATLTSPEVGRLSLKE QLSHRQHLLSEMESLKPKVQAVQLCQSALRIPEDVVASLPLCHAALRLQEEASRLQHTAIQQCNIMQ EAVVQYEQYEQEMKHLQQLIEGAHREIEDKPVATSNIQELQAQISRHEELAQKIKGYQEQIASLNSK CKMLTMKAKHATMLLTVTEVEGLAEGTEDLDGELLPTPSAHPSVVMMTAGRCHTLLSPVTEESGEE GTNSEISSPPACRSPSPVANTDASVNQDIAYYQALSAERLQTDAAKIHPSTSASQEFYEPGLEPSAT AKLGDLQRSWETLKNVISEKQRTLYEALERQQKYQDSLQSISTKMEAIELKLSESPEPGRSPESQMA EHQALMDEILMLQDEINELQSSLAEELVSESCEADPAEQLALQSTLTVLAERMSTIRMKASGKRQLL EEKLNDQLEEQRQEQALQRYRCEADELDSWLLSTKATLDTALSPPKEPMDMEAQLMDCQNMLVEI EQKVVALSELSVHNENLLLEGKAHTKDEAEQLAGKLRRLKGSLLELQRALHDKQLNMQGTAQEKEE SDVDLTATQSPGVQEWLAQARTTWTQQRQSSLQQQKELEQELAEQKSLLRSVASRGEEILIQHSA AETSGDAGEKPDVLSQELGMEGEKSSAEDQMRMKWESLHQEFSTKQKLLQNVLEQEQEQVLYSR PNRLLSGVPLYKGDVPTQDKSAVTSLLDGLNQAFEEVSSQSGGAKRQSIHLEQKLYDGVSATSTWL DDVEERLFVATALLPEETETCLFNQEILAKDIKEMSEEMDKNKNLFSQAFPENGDNRDVIEDTLGCLL GRLSLLDSVVNQRCHQMKERLQQILNFQNDLKVLFTSLADNKYIILQKLANVFEQPVAEQIEAIQQAE DGLKEFDAGIIELKRRGDKLQVEQPSMQELSKLQDMYDELMMIIGSRRSGLNQNLTLKSQYERALQ DLADLLETGQEKMAGDQKIIVSSKEEIQQLLDKHKEYFQGLESHMILTETLFRKIISFAVQKETQFHTE LMAQASAVLKRAHKRGVELEYILETWSHLDEDQQELSRQLEVVESSIPSVGLVEENEDRLIDRITLYQ HLKSSLNEYQPKLYQVLDDGKRLLISISCSDLESQLNQLGECWLSNTNKMSKELHRLETILKHWTRY QSESADLIHWLQSAKDRLEFWTQQSVTVPQELEMVRDHLNAFLEFSKEVDAQSSLKSSVLSTGNQL LRLKKVDTATLRSELSRIDSQWTDLLTNIPAVQEKLHQLQMDKLPSRHAISEVMSWISLMENVIQKDE DNIKNSIGYKAIHEYLQKYKGFKIDINCKQLTVDFVNQSVLQISSQDVESKRSDKTDFAEQLGAMNKS WQILQGLVTEKIQLLEGLLESWSEYENNVQCLKTWFETQEKRLKQQHRIGDQASVQNALKDCQDLE DLIKAKEKEVEKIEQNGLALIQNKKEDVSSIVMSTLRELGQTWANLDHMVGQLKILLKSVLDQWSSH KVAFDKINSYLMEARYSLSRFRLLTGSLEAVQVQVDNLQNLQDDLEKQERSLQKFGSITNQLLKECH PPVTETLTNTLKEVNMRWNNLLEEIAEQLQSSKALLQLWQRYKDYSKQCASTVQQQEDRTNELLKA ATNKDIADDEVATWIQDCNDLLKGLGTVKDSLFFLHELGEQLKQQVDASAASAIQSDQLSLSQHLCA LEQALCKQQTSLQAGVLDYETFAKSLEALEAWIVEAEEILQGQDPSHSSDLSTIQERMEELKGQMLK FSSMAPDLDRLNELGYRLPLNDKEIKRMQNLNRHWSLISSQTTERFSKLQSFLLQHQTFLEKCETW MEFLVQTEQKLAVEISGNYQHLLEQQRAHELFQAEMFSRQQILHSIIIDGQRLLEQGQVDDRDEFNL KLTLLSNQWQGVIRRAQQRRGIIDSQIRQWQRYREMAEKLRKWLVEVSYLPMSGLGSVPIPLQQAR TLFDEVQFKEKVFLRQQGSYILTVEAGKQLLLSADSGAEAALQAELAEIQEKWKSASMRLEEQKKKL AFLLKDWEKCEKGIADSLEKLRTFKKKLSQSLPDHHEELHAEQMRCKELENAVGSWTDDLTQLSLL KDTLSAYISADDISILNERVELLQRQWEELCHQLSLRRQQIGERLNEWAVFSEKNKELCEWLTQMES KVSQNGDILIEEMIEKLKKDYQEEIAIAQENKIQLQQMGERLAKASHESKASEIEYKLGKVNDRWQHL LDLIAARVKKLKETLVAVQQLDKNMSSLRTWLAHIESELAKPIVYDSCNSEEIQRKLNEQQELQRDIE KHSTGVASVLNLCEVLLHDCDACATDAECDSIQQATRNLDRRWRNICAMSMERRLKIEETWRLWQ KFLDDYSRFEDWLKSSERTAAFPSSSGVIYTVAKEELKKFEAFQRQVHECLTQLELINKQYRRLARE NRTDSACSLKQMVHEGNQRWDNLQKRVTSILRRLKHFIGQREEFETARDSILVWLTEMDLQLTNIE HFSECDVQAKIKQLKAFQQEISLNHNKIEQIIAQGEQLIEKSEPLDAAIIEEELDELRRYCQEVFGRVE RYHKKLIRLPLPDDEHDLSDRELELEDSAALSDLHWHDRSADSLLSPQPSSNLSLSLAQPLRSERSG RDTPASVDSIPLEWDHDYDLSRDLESAMSRALPSEDEEGQDDKDFYLRGAVGLSGDHSALESQIR QLGKALDDSRFQIQQTENIIRSKTPTGPELDTSYKGYMKLLGECSSSIDSVKRLEHKLKEEEESLPGF VNLHSTETQTAGVIDRWELLQAQALSKELRMKQNLQKWQQFNSDLNSIWAWLGDTEEELEQLQRL ELSTDIQTIELQIKKLKELQKAVDHRKAIILSINLCSPEFTQADSKESRDLQDRLSQMNGRWDRVCSLL EEWRGLLQDALMQCQGFHEMSHGLLLMLENIDRRKNEIVPIDSNLDAEILQDHHKQLMQIKHELLES QLRVASLQDMSCQLLVNAEGTDCLEAKEKVHVIGNRLKLLLKEVSRHIKELEKLLDVSSSQQDLSSW SSADELDTSGSVSPTSGRSTPNRQKTPRGKCSLSQPGPSVSSPHSRSTKGGSDSSLSEPGPGRS GRGFLFRVLRAALPLQLLLLLLLIGLACLVPMSEEDYSCALSNNFARSFHPMLRYTNGPPPL |
| 103 | Human Nesprin-2(UniProtKB Q8WXH0-1) | MASSPELPTEDEQGSWGIDDLHISLQAEQEDTQKKAFTCWINSQLARHTSPSVISDLFTDIKKGHVL LDLLEVLSGQQLPRDKGSNTFQCRINIEHALTFLRNRSIKLINIHVTDIIDGNPSIILGLIWTIILHFHIEKL AQTLSCNYNQPSLDDVSVVDSSPASSPPAKKCSKVQARWQMSARKALLLWAQEQCATYESVNVT DFKSSWRNGMAFLAIIHALRPDLIDMKSVKHRSNKDNLREAFRIAEQELKIPRLLEPEDVDVVDPDEK SIMTYVAQFLQYSKDAPGTGEEAQGKVKDAMGWLTLQKEKLQKLLKDSENDTYFKKYNSLLSFMES FNEEKKSFLDVLSIKRDLDELDKDHLQLREAWDGLDHQINAWKIKLNYALPPPLHQTEAWLQEVEEL MDEDLSASQDHSQAVTLIQEKMTLFKSLMDRFEHHSNILLTFENKDENHLPLVPPNKLEEMKRRINNI LEKKFILLLEFHYYKCLVLGLVDEVKSKLDIWNIKYGSRESVELLLEDWHKFIEEKEFLARLDTSFQKC GEIYKNLAGECQNINKQYMMVKSDVCMYRKNIYNVKSTLQKVLACWATYVENLRLLRACFEETKKE EIKEVPFETLAQWNLEHATLNEAGNFLVEVSNDVVGSSISKELRRLNKRWRKLVSKTQLEMNLPLMI KKQDQPTFDNSGNILSKEEKATVEFSTDMSVELPENYNQNIKAGEKHEKENEEFTGQLKVAKDVEK LIGQVEIWEAEAKSVLDQDDVDTSMEESLKHLIAKGSMFDELMARSEDMLQMDIQNISSQESFQHVL TTGLQAKIQEAKEKVQINVVKLIAALKNLTDVSPDLDIRLKMEESQKELESYMMRAQQLLGQRESPG ELISKHKEALIISNTKSLAKYLKAVEELKNNVTEDIKMSLEEKSRDVCAKWESLHHELSLYVQQLKIDIE KGKLSDNILKLEKQINKEKKLIRRGRTKGLIKEHEACFSEEGCLYQLNHHMEVLRELCEELPSQKSQ QEVKRLLKDYEQKIERLLKCASEIHMTLQPTAGGTSKNEGTITTSENRGGDPHSEAPFAKSDNQPST EKAMEPTMKFSLASVLRPLQEESIMEKDYSASINSLLERYDTYRDILEHHLQNNKFRITSDFSSEEDR SSSCLQAKLTDLQVIKNETDARWKEFEIISLKLENHVNDIKKPFVIKERDTLKERERELQMTLNTRME |
| | | SLETALRLVLPVEKASLLLCGSDLPLHKMAIQGFHLIDADRIYQHLRNIQDSIAKQIEICNRLEEPGNFV LKELHPFDLHAMQNIILKYKTQFEGMNHRVQRSEDTLKALEDFLASLRTAKLSAEPVTDLSASDTQV AQENTLTVKNKEGEIHLMKDKAKHLDKCLKMLDMSFKDAERGDDTSCENLLDAFSIKLSETHGYGV QEEFTEENKLLEACIFKNNELLKNIQDVQSQISKIGLKDPTVPAVKHRKKSLIRLDKVLDEYEEEKRHL QEMANSLPHFKDGREKTVNQQCQNTVVLWENTKALVTECLEQCGRVLELLKQYQNFKSILTTLIQK EESVISLQASYMGKENLKKRIAEIEIVKEEFNEHLEVVDKINQVCKNLQFYLNKMKTFEEPPFEKEANI VDRWLDINEKTEDYYENLGRALALWDKLFNLKNVIDEWTEKALQKMELHQLTEEDRERLKEELQVH EQKTSEFSRRVAEIQFLLQSSEIPLELQVMESSILNKMEHVQKCLTGESNCHALSGSTAELREDLDQ AKTQIGMTESLLKALSPSDSLEIFTKLEEIQQQILQQKHSMILLENQIGCLTPELSELKKQYESVSDLFN TKKSVLQDHFSKLLNDQCKNFNDWFSNIKVNLKECFESSETKKSVEQKLQKLSDFLTLEGRNSKIKQ VDSVLKHVKKHLPKAHVKELISWLVGQEFELEKMESICQARAKELEDSLQQLLRLQDDHRNLRKWL TNQEEKWKGMEEPGEKTELFCQALARKREQFESVAQLNNSLKEYGFTEEEEIIMEATCLMDRYQTL LRQLSEIEEEDKLLPTEDQSFNDLAHDVIHWIKEIKESLMVLNSSEGKMPLEERIQKIKEIILLKPEGDA RIETIMKQAESSEAPLVQKTLTDISNQWDNTLHLASTYLSHQEKLLLEGEKYLQSKEDLRLMLIELKK KQEAGFALQHGLQEKKAQLKIYKKFLKKAQDLTSLLKELKSQGNYLLECTKNPSFSEEPWLEIKHLH ESLLQQLQDSVQNLDGHVREHDSYQVCVTDLNTTLDNFSKEFVSFSDKPVDQIAVEEKLQKLQELE NRLSLQDGTLKKILALAKSVKQNTSSVGQKIIKDDIKSLQCKQKDLENRLASAKQEMECCLNSILKSK RSTEKKGKFTLPGREKQATSDVQESTQESAAVEKLEEDWEINKDSAVEMAMSKQLSLNAQESMKN TEDERKVNELQNQPLELDTMLRNEQLEEIEKLYTQLEAKKAAIKPLEQTECLNKTETGALVLHNIGYS AQHLDNLLQALITLKKNKESQYCVLRDFQEYLAAVESSMKALLTDKESLKVGPLDSVTYLDKIKKFIA SIEKEKDSLGNLKIKWENLSNHVTDMDKKLLESQIKQLEHGWEQVEQQIQKKYSQQVVEYDEFTTL MNKVQDTEISLQQQQQHLQLRLKSPEERAGNQSMIALTTDLQATKHGFSVLKGQAELQMKRIWGE KEKKNLEDGINNLKKQWETLEPLHLEAENQIKKCDIRNKMKETILWAKNLLGELNPSIPLLPDDILSQI RKCKVTHDGILARQQSVESLAEEVKDKVPSLTTYEGSDLNNTLEDLRNQYQMLVLKSTQRSQQLEF KLEERSNFFAIIRKFQLMVQESETLIIPRVETAATEAELKHHHVTLEASQKELQEIDSGISTHLQELTNI YEELNVFERLFLEDQLKNLKIRTNRIQRFIQNTCNEVEHKIKFCRQFHEKTSALQEEADSIQRNELLLN QEVNKGVKEEIYNLKDRLTAIKCCILQVLKLKKVFDYIGLNWDFSQLDQLQTQVFEKEKELEEKIKQL DTFEEEHGKYQALLSKMRAIDLQIKKMTEVVLKAPDSSPESRRLNAQILSQRIEKAKCLCDEIIKKLNE NKTFDDSFKEKEILQIKLNAEENDKLYKVLQNMVLELSPKELDEKNCQDKLETSLHVLNQIKSQLQQP LLINLEIKHIQNEKDNCEAFQEQVWAEMCSIKAVTAIEKQREENSSEASDVETKLREFEDLQMQLNTS IDLRTNVLNDAYENLTRYKEAVTRAVESITSLEAIIIPYRVDVGNPEESLEMPLRKQEELESTVAHIQDL TEKLGMISSPEAKLQLQYTLQELVSKNSAMKEAFKAQETEAERYLENYKCYRKMEEDIYTNLSKMET VLGQSMSSLPLSYREALERLEQSKALVSNLISTKEELMKLRQILRLLRLRCTENDGICLLKIVSALWEK WLSLLEAAKEWEMWCEELKQEWKFVSEEIEREAIILDNLQEELPEISKTKEAATTEELSELLDCLCQY GENVEKQQLLLTLLLQRIRSIQNVPESSGAVETVPAFQEITSMKERCNKLLQKVQKNKELVQTEIQER HSFTKEIIALKNFFQQTTTSFQNMAFQDHPEKSEQFEELQSILKKGKLTFENIMEKLRIKYSEMYTIVP AEIESQVEECRKALEDIDEKISNEVLKSSPSYAMRRKIEEINNGLHNVEKMLQQKSKNIEKAQEIQKK MWDELDLWHSKLNELDSEVQDIVEQDPGQAQEWMDNLMIPFQQYQQVSQRAECRTSQLNKATVK MEEYSDLLKSTEAWIENTSHLLANPADYDSLRTLSHHASTVQMALEDSEQKHNLLHSIFMDLEDLSII FETDELTQSIQELSNQVTALQQKIMESLPQIQRMADDVVAIESEVKSMEKRVSKIKTILLSKEIFDFSP EEHLKHGEVILENIRPMKKTIAEIVSYQVELRLPQTGMKPLPVFQRTNQLLQDIKLLENVTQEQNELLK VVIKQTNEWDEEIENLKQILNNYSAQFSLEHMSPDQADKLPQLQGEIERMEKQILSLNQRKEDLLVD LKATVLNLHQHLKQEQEGVERDRLPAVTSEEGGVAERDASERKLNRRGSMSYLAAVEEEVESSV KSDNGDEKAEPSPQSWSSLWKHDKDMEEDRASSSSGTIVQEAYGKISTSDNSMAQILTPDSLNTE QGPECSLRPNQTEEGTTPPIEADTLDSSDAQGGLEPRVEKTRPEPTEVLHACKTQVAELELWLQQA NVAVEPETLNADMQQVLEQQLVGCQAMLTEIEHKVAFLLETCKDQGLGDNGATQHEAEALSLKLKT VKCNLEKVQMMLQEKHSEDQHPTILKKSSEPEHQEALQPVNLSELESIVTERPQFSRQKDFQQQQV LELKPMEQKDFIKFIEFNAKKMWPQYCQHDNDTTQESSASNQASSPENDVPDSILSPQGGQNGDKW QYLHHELSSKIKLPLPQLVEPQVSTNMGILPSVTMYNFRYPTTEELKTYTTQLEDLRQEASNLQTQE NMTEEAYINLDKKLFELFLTLSQCLSSVEEMLEMPRLYREDGSGQQVHYETLALELKKLYLALSDKK GDLLKAMTWPGENTNLLLECFDNLQVCLEHTQAAAVCRSKSLKAGLDYNRSYQNEIKRLYHQLIKS KTSLQQSLNEISGQSVAEQLQKADAYTVELENAESRVAKLRDEGERLHLPYALLQEVYKLEDVLDS MWGMLRARYTELSSPFVTESQQDALLQGMVELVKIGKEKLAHGHLKQTKSKVALQAQIENHKVFFQ KLVADMLLIQAYSAKILPSLLQNRETFWAEQVTEVKILEEKSRQCGMKLQSLLQKWEEFDENYASLE KDLEILISTLPSVSLVEETEERLVERISFYQQIKRNIGGKHARLYQTLNEGKQLVASVSCPELEGQIAK LEEQWLSLNKKIDHELHRLQALLKHLLSYNRDSDQLTKWLESSQHTLNYWKEQSLNVSQDLDTIRS NINNFFEFSKEVDEKSSLKTAVISIGNQLLHLKETDTATLRASLAQFEQKWTMLITQLPDIQEKLHQLQ MEKLPSRKAITEMISWMNNVEHQTSDEDSVHSPSSASQVKHLLQKHKEFRMEMDYKQWIVDFVNQ SLLQLSTCDVESKRYERTEFAEHLGEMNRQWHRVHGMLNRKIQHLEQLLESITESENKIQILNNWLE AQEERLKTLQKPESVISVQKLLLDCQDIENQLAIKSKALDELKQSYLTLESGAVPLLEDTASRIDELFQ KRSSVLTQVNQLKTSMQSVLQEWKIYDQLYDEVNMMTIRFWYCMEHSKPVVLSLETLRCQVENLQ SLQDEAESSEGSWEKLQEVIGKLKGLCPSVAEIIEEKCQNTHKRWTQVNQAIADQLQKAQSLLQLW KAYSNAHGEAAARLKQQEAKFQQLANISMSGNNLAEILPPALQDIKELQHDVQKTKEAFLQNSSVLD RLPQPAESSTHMLLPGPLHSLQRAAYLEKMLLVKANEFEFVLSQFKDFGVRLESLKGLIMHEEENLD RLHQQEKENPDSFLNHVLALTAQSPDIEHLNEVSLKLPLSDVAVKTLQNMNRQWIRATATALERCSE LQGIGLNEKFLYCCEKWIQLLEKIEEALKVDVANSLPELLEQQKTYKMLEAEVSINQTIADSYVTQSLQ LLDTTEIENRPEFITEFSKLTDRWQNAVQGVRQRKGDVDGLVRQWQDFTTSVENLFRFLTDTSHLL |
| | | SAVKGQERFSLYQTRSLIHELKNKEIHFQRRRTTCALTLEAGEKLLLTTDLKTKESVGRRISQLQDSW KDMEPQLAEMIKQFQSTVETWDQCEKKIKELKSRLQVLKAQSEDPLPELHEDLHNEKELIKELEQSL ASWTQNLKELQTMKADLTRHVLVEDVMVLKEQIEHLHRQWEDLCLRVAIRKQEIEDRLNTWVVFNE KNKELCAWLVQMENKVLQTADISIEEMIEKLQKDCMEEINLFSENKLQLKQMGDQLIKASNKSRAAEI DDKLNKINDRWQHLFDVIGSRVKKLKETFAFIQQLDKNMSNLRTWLARIESELSKPVVYDVCDDQEI QKRLAEQQDLQRDIEQHSAGVESVFNICDVLLHDSDACANETECDSIQQTTRSLDRRWRNICAMSM ERRMKIEETWRLWQKFLDDYSRFEDWLKSAERTAACPNSSEVLYTSAKEELKRFEAFQRQIHERLT QLELINKQYRRLARENRTDTASRLKQMVHEGNQRWDNLQRRVTAVLRRLRHFTNQREEFEGTRES ILVWLTEMDLQLTNVEHFSESDADDKMRQLNGFQQEITLNTNKIDQLIVFGEQLIQKSEPLDAVLIED ELEELHRYCQEVFGRVSRFHRRLTSCTPGLEDEKEASENETDMEDPREIQTDSWRKRGESEEPSS PQSLCHLVAPGHERSGCETPVSVDSIPLEWDHTGDVGGSSSHEEDEEGPYYSALSGKSISDGHSW HVPDSPSCPEHHYKQMEGDRNVPPVPPASSTPYKPPYGKLLLPPGTDGGKEGPRVLNGNPQQED GGLAGITEQQSGAFDRWEMIQAQELHNKLKIKQNLQQLNSDISAITTWLKKTEAELEMLKMAKPPSD IQEIELRVKRLQEILKAFDTYKALVVSVNVSSKEFLQTESPESTELQSRLRQLSLLWEAAQGAVDSW RGGLRQSLMQCQDFHQLSQNLLLWLASAKNRRQKAHVTDPKADPRALLECRRELMQLEKELVER QPQVDMLQEISNSLLIKGHGEDCIEAEEKVHVIEKKLKQLREQVSQDLMALQGTQNPASPLPSFDEV DSGDQPPATSVPAPRAKQFRAVRTTEGEEETESRVPGSTRPQRSFLSRVVRAALPLQLLLLLLLLLA CLLPSSEEDYSCTQANNFARSFYPMLRYTNGPPPT |
| 104 | Human Nesprin-3 (UniProtKB Q6ZMZ3-1) | MTQQPQDDFDRSVEDAQAWMKAVQDQLQVNDNTQGPRAALEARLWETEKICQLEPEGRVRVDLV LRMAEALLACCPGDQKPGILARLKDIKAQWEETVTYMTHCHSRIEWVWLHWSEYLLARDEFYRWF QKMMVTLEPHIELQLGLKEKQWQLSHAQVLLHNVDNQAVLLDRLLEEAASLFNRIGDPSVDEDAQK RMKAEYDAVKAKAQKRVDLLEQVAREHEEYQAGVDEFQLWLKAVVEKVNGCLGRNCKLPITQRLS TLQDIAKDFPRGEESLETLEEQSAGVIRNTSPLGAEKITGELEEMRKVLEKLRALWEEEEERLRGLLR SRGAWEQQIKQLEAELSEFRMVLQRLAQEGLQPAAKAGTEDELVAHWRRYSATRAALASEEPRVD RLQAQLKELIVFPHNLKPLSDSVIATIQEYQSLKVKSARLRNAAAVELWQHFQRPLQDLQLWKALAQ RLLEVTASLPDLPSLHTFLPQIEAALMESSRLKELLTMLQLKKDLLIGIFGQERATALLEQVAGSMRD RDLLHNSLLQRKSKLQSLLAQHKDFGAAFEPLQRKLLDLQVRVQAEKGLQRDLPGKQAQLSRLQGL QEEGLDLGAQMEAARPLVQENPNHQHKMDQLSSDFQALQRSLEDLVDRCRQSVQEHCTFSHQLL ELRQWIVVTTQKLEAHRGEAGPGDAESQEAEFERLVAEFPEKEAQLSLVEAQGWLVMEKSSPEGA AVVQEELRELAESWRALRLLEESLLSLIRNWHLQRMEVDSGKKMVFTNNIPKSGFLINPMDPIPRHR RRANLLQEEEGSHEDFSQLLRNFGQWLQVENSKLVRIIAMRTSTAEDLRTRKSKLQELEARVPEGQ HLFENLLRLGPARGTSDELEDLRYQWMLYKSKLKDSGHLLTQSSPGEPTGFQKTRRWRGLGSLFR RACCVALPLQLLLLLFLLLLFLLPIREEDRSCTLANNFARSFTLMLRYNGPPPT |
| 105 | Human Nesprin-4 (UniProtKB Q8N205-1) | MALSLPLGPRLGSEPLNHPPGAPREADIVGCTVCPASGEESTSPEQAQTLGQDSLGPPEHFQGGP RGNEPAAHPPRWSTPSSYEDPAGGKHCEHPISGLEVLEAEQNSLHLCLLGLGRRLQDLEQGLGHW ALAQSGMVQLQALQVDLRGAAERVEALLAFGEGLAQRSEPRAWAALEQILRALGAYRDSIFRRLW QLQAQLVSYSLVFEEANTLDQDLEVEGDSDWPGPGGVWGPWAPSSLPTSTELEWDPAGDIGGLG PLGQKTARTLGVPCELCGQRGPQGRGQGLEEADTSHSRQDMLESGLGHQKRLARHQRHSLLRKP QDKKRQASPHLQDVRLEGNPGAPDPASRQPLTFLLILFLLFLLLVGAMFLLPASGGPCCSHARIPRT PYLVLSYVNGLPPV |
| 106 | Human KASH5 (UniProtKB Q8N6L0-1) | MDLPEGPVGGPTAEMYLRERPEEARLGMPVSLEEQILNSTFEACDPQRTGTVAVAQVLAYLEAVTG QGPQDARLQTLANSLDPNGEGPKATVDLDTFLVVMRDWIAACQLHGGLELEEETAFQGALTSRQL PSGCPEAEEPANLESFGGEDPRPELQATADLLSSLEDLELSNRRLVGENAKLQRSMETAEEGSARL GEEILALRKQLHSTQQALQFAKAMDEELEDLKTLARSLEEQNRSLLAQARQAEKEQQHLVAEMETL QEENGKLLAERDGVKKRSQELAMEKDTLKRQLFECEHLICQRDTILSERTRDVESLAQTLEEYRVTT QELRLEISRLEEQLSQTYEGPDELPEGAQLRRVGWTELLPPSLGLEIEAIRQKQEVATADLSNPLCG VWQWEEVIHETSEETEFPSEAPAGGQRNFQGEPAHPEEGRKEPSMWLTRREEEEDAESQVTADL PVPLGAPRPGDIPENPPERPARRELQQALVPVMKKLVPVRRRAWGQLCLPPQRLRVTRHPLIPAPV LGLLLLLLLSVLLLGPSPPPTWPHLQLCYLQPPPV |
| 107 | Actin binding domain of human Nesprin-1 | MATSRGASRCPRDIANVMQRLQDEQEIVQKRTFTKWINSHLAKRKPPMVVDDLFEDMKDGVKLLAL LEVLSGQKLPCEQGRRMKRIHAVANIGTALKFLEGRKIKLVNINSTDIADGRPSIVLGLMWTIILYFQIE ELTSNLPQLQSLSSSASSVDSIVSSETPSPPSKRKVTTKIQGNAKKALLKWVQYTAGKQTGIEVKDF GKSWRSGVAFHSVIHAIRPELVDLETVKGRSNRENLEDAFTIAETELGIPRLLDPEDVDVDKPDEKSI MTYVAQFLKHYPDIHNAS |
| 108 | Actin binding domain of human Nesprin-2 | MASSPELPTEDEQGSWGIDDLHISLQAEQEDTQKKAFTCWINSQLARHTSPSVISDLFTDIKKGHVL LDLLEVLSGQQLPRDKGSNTFQCRINIEHALTFLRNRSIKLINIHVTDIIDGNPSIILGLIWTIILHFHIEKL AQTLSCNYNQPSLDDVSVVDSSPASSPPAKKCSKVQARWQMSARKALLLWAQEQCATYESVNVT DFKSSWRNGMAFLAIIHALRPDLIDMKSVKHRSNKDNLREAFRIAEQELKIPRLLEPEDVDVVDPDEK SIMTYVAQFLQYSK |
| 109 | LMNA-targeted guide RNA | AGGAGATGGATCCGCCCACC |
| 110 | Lmna-G609G progeria mutation repair oligo | GTGCTGTGCGGGACGTGTGGGCAGCCTGCTGACAAGGCTGCCGGTGGAGCGGGAGCCCAGG TGGGTGGATCCATCTCCTCTGGCTCTTCTGCCTCCAGTGTCACAGTCACTCGAAGCTTCCGCA GTGTGGG |
| 111 | Lmna GT up | CCACAGGTCTCCCAAGTCCCCATC |
| 112 | Lmna GT down | TCCTCTCCCTCCCTGACCCCAAA |
| 113 | Human LRMP (UniProtKB Q12912-1) | MESTPFSGVANQIHTLCERPTYGEVKDGALDVKRQHKCPGPTSGPSPGTNLSGCIRMNDDPSMEE NGVERVCPESLLQSREYSSLPLPRHTSSTDGTITSSDPGLEILNMASCDLDRNSLCKKEEDTRSASP TIEAQGTSPAHDNIAFQDSTSKDKTILNLEAKEEPETIEEHKKEHASGDSVVSPLPVTTVKSVNLRQS ENTSANEKEVEAEFLRLSLGFKCDWFTLEKRVKLEERSRDLAEENLKKEITNCLKLLESLTPLCEDD NQAQEIIKKLEKSIKFLSQCAARVASRAEMLGAINQESRVSKAVEVMIQHVENLKRMYAKEHAELEEL KQVLLQNERSFNPLEDDDDCQIKKRSASLNSKPSSLRRVTIASLPRNIGNAGMVAGMENNDRFSRR SSSWRILGSKQSEHRPSLPRFISTYSWADAEEEKCELKTKDDSEPSGEETVERTRKPSLSEKKNNP SKWDVSSVYDTIASWATNLKSSIRKANKALWLSIAFIVLFAALMSFLTGQLFQKSVDAAPTQQEDSW TSLEHILWPFTRLRHNGPPPV |
| 114 | KASH domain of human LRMP | QLFQKSVDAAPTQQEDSWTSLEHILWPFTRLRHNGPPPV |
| 115 | SUN1 SUN domain plus upstream α3 region | GITEAQARAIVNSALKLYSQDKTGMVDFALESGGGSILSTRCSETYETKTALMSLFGIPLWYFSQSP RVVIQPDIYPGNCWAFKGSQGYLVVRLSMMIHPAAFTLEHIPKTLSPTGNISSAPKDFAVYGLENEY QEEGQLLGQFTYDQDGESLQMFQALKRPDDTAFQIVELRIFSNWGHPEYTCLYRFRVHGEPVK |
| 116 | SUN2 SUN domain plus upstream α3 region | GVTEEQVHHIVKQALQRYSEDRIGLADYALESGGASVISTRCSETYETKTALLSLFGIPLWYHSQSP RVILQPDVHPGNCWAFQGPQGFAVVRLSARIRPTAVTLEHVPKALSPNSTISSAPKDFAIFGFDEDL QQEGTLLGKFTYDQDGEPIQTFHFQAPTMATYQVVELRILTNWGHPEYTCIYRFRVHGEPAH |
| 117 | gBlock humanized SUN1 dominant negative megaprimer | TATAGGCTAGCGGCACAATGAAGTGGGTAACCTTTATTTCCCTTCTTTTTCTCTTTAGCTCGGCC TATTCCAGGGGTGTGTTTCGTCGAGATGGGCCCTCCGGAGGTGAACAAAAACTCATCTCAGAA GAGGATCTCACCGGTGGAGATGAGGGCTGGGAAGCCAGAG |
| 118 | gBlock amiplification F primer | TATAGGCTAGCGGCACAATG |
| 119 | gBIock amiplification R primer | CTCTGGCTTCCCAGCCCTCA |
| 120 | SUN2 lumenal domain R primer | CCGGGTCGACCTACAACTCATCTTTGTGGGCGGGCTCCCCATGCAC |
| 121 | SUN1 lumenal domain F primer | TCTCACCGGTGGAGATGACCCCCAGGACGTGTTTAAAC |
| 122 | SUN1 lumenal domain R primer | CCGGGTCGACCTACAACTCATCTTTCTTGACAGGTTCGCCATGAACTC |
| 123 | qRT-PCR F primer for viral genome quantification | ACAGTCTCGAACTTAAGCTGCA |
| 124 | qRT-PCR R primer for viral genome quantification | GTCTCGACAAGCCCAGTTTCTA |
| 125 | pAAV-cTnT-MYC-SUN1DN | CTGCGCGCTCGCTCGCTCACTGAGGCCGCCCGGGCAAAGCCCGGGCGTCGGGCGACCTTTG GTCGCCCGGCCTCAGTGAGCGAGCGAGCGCGCAGAGAGGGAGTGGCCAACTCCATCACTAG GGGTTCCTTGTAGTTAATGATTAACCCGCCATGCTACTTATCTACCAGGGTAATGGGGATCCTC TAGAACTATAGCTAGAATTCGCCCTTACGGGCCCCCCCTCGAGGTCGGGATAAAAGCAGTCTG GGCTTTCACATGACAGCATCTGGGGCTGCGGCAGAGGGTCGGGTCCGAAGCGCTGCCTTATC AGCGTCCCCAGCCCTGGGAGGTGACAGCTGGCTGGCTTGTGTCAGCCCCTCGGGCACTCACG TATCTCCGTCCGACGGGTTTAAAATAGCAAAACTCTGAGGCCACACAATAGCTTGGGCTTATAT GGGCTCCTGTGGGGGAAGGGGGAGCACGGAGGGGGCCGGGGCCGCTGCTGCCAAAATAGCA GCTCACAAGTGTTGCATTCCTCTCTGGGCGCCGGGCACATTCCTGCTGGCTCTGCCCGCCCCG GGGTGGGCGCCGGGGGGACCTTAAAGCCTCTGCCCCCCAAGGAGCCCTTCCCAGACAGCCG CCGGCACCCACCGCTCCGTGGGACGATCCCCGAAGCTCTAGAGCTTTATTGCGGTAGTTTATC ACAGTTAAATTGCTAACGCAGTCAGTGCTTCTGACACAACAGTCTCGAACTTAAGCTGCAGAAG TTGGTCGTGAGGCACTGGGCAGGTAAGTATCAAGGTTACAAGACAGGTTTAAGGAGACCAATA GAAACTGGGCTTGTCGAGACAGAGAAGACTCTTGCGTTTCTGATAGGCACCTATTGGTCTTACT GACATCCACTTTGCCTTTCTCTCCACAGGTGTCCACTCCCAGTTCAATTACAGCTCTTAAGGCTA GAGTACTTAATACGACTCACTATAGGCTAGCGGCACAATGAAGTGGGTAACCTTTATTTCCCTT CTTTTTCTCTTTAGCTCGGCCTATTCCAGGGGTGTGTTTCGTCGAGATGGGCCCTCCGGAGGT GAACAAAAACTCATCTCAGAAGAGGATCTCACCGGTGGAGATGACCCCCAGGACGTGTTTAAA CCCACGACTTCTCGCCTGAAGCAGCCTCTGCAGGGTGACAGTGAGGCTTTTCCGTGGCATTGG ATGAGTGGCGTGGAGCAGCAGGTGGCCTCTCTGTCTGGACAGTGCCACCACCATGGTGAGAA TCTCCGAGAGCTGACCACTTTGCTACAGAAGCTGCAGGCTCGGGTGGACCAGATGGAAGGCG GCGCTGCCGGGCCGTCAGCTTCGGTCAGAGACGCTGTGGGACAGCCCCCGAGGGAGACTGA CTTTATGGCCTTTCACCAAGAACATGAAGTGCGTATGTCACACTTGGAAGATATTCTGGGAAAA CTGAGAGAAAAATCTGAGGCCATCCAGAAGGAACTAGAACAGACCAAGCAAAAAACAATCAGT GCGGTTGGTGAGCAGCTCCTGCCCACAGTCGAGCACCTCCAGCTGGAGCTGGATCAGCTAAA GTCAGAGCTGTCCAGCTGGCGACACGTGAAGACCGGCTGTGAGACAGTGGATGCCGTACAAG |
| | | AAAGAGTGGACGTGCAAGTCAGAGAAATGGTGAAACTCCTGTTTTCCGAAGATCAGCAAGGCG GTTCTCTGGAACAGCTGCTGCAGAGGTTCTCATCACAGTTTGTGAGCAAAGGCGACTTGCAGA CGATGCTGCGAGACCTGCAGCTGCAGATCCTGCGGAACGTCACCCACCACGTTTCCGTGACCA AGCAGCTCCCAACCTCAGAAGCCGTGGTGTCTGCTGTGAGCGAGGCGGGGGCGTCTGGAATA ACAGAGGCGCAAGCACGTGCCATCGTGAACAGCGCCTTGAAGCTGTATTCCCAAGATAAGACC GGGATGGTGGACTTTGCTCTGGAATCTGGTGGTGGCAGCATCTTGAGTACTCGCTGTTCTGAA ACTTACGAAACCAAAACGGCGCTGATGAGTCTGTTTGGGATCCCGCTGTGGTACTTCTCGCAG TCCCCGCGCGTGGTCATCCAGCCTGACATTTACCCCGGTAACTGCTGGGCATTTAAAGGCTCC CAGGGGTACCTGGTGGTGAGGCTCTCCATGATGATCCACCCAGCCGCCTTCACTCTGGAGCAC ATCCCTAAGACGCTGTCGCCAACAGGCAACATCAGCAGCGCCCCCAAGGACTTCGCCGTCTAT GGATTAGAAAATGAGTATCAGGAAGAAGGGCAGCTTCTGGGACAGTTCACGTATGATCAGGAT GGGGAGTCGCTCCAGATGTTCCAGGCCCTGAAAAGACCCGACGACACAGCTTTCCAAATAGTG GAACTTCGGATTTTTTCTAACTGGGGCCATCCTGAGTATACCTGTCTGTATCGGTTCAGAGTTC ATGGCGAACCTGTCAAGAAAGATGAGTTGTAGGTCGACCCGGGCGGCCTCGAGGACGGGGTG AACTACGCCTGAGGATCCGATCTTTTTCCCTCTGCCAAAAATTATGGGGACATCATGAAGCCCC TTGAGCATCTGACTTCTGGCTAATAAAGGAAATTTATTTTCATTGCAATAGTGTGTTGGAATTTTT TGTGTCTCTCACTCGGAAGCAATTCGTTGATCTGAATTTCGACCACCCATAATACCCATTACCCT GGTAGATAAGTAGCATGGCGGGTTAATCATTAACTACAAGGAACCCCTAGTGATGGAGTTGGC CACTCCCTCTCTGCGCGCTCGCTCGCTCACTGAGGCCGGGCGACCAAAGGTCGCCCGACGCC CGGGCTTTGCCCGGGCGGCCTCAGTGAGCGAGCGAGCGCGCAGCCTTAATTAACCTAATTCA CTGGCCGTCGTTTTACAACGTCGTGACTGGGAAAACCCTGGCGTTACCCAACTTAATCGCCTTG CAGCACATCCCCCTTTCGCCAGCTGGCGTAATAGCGAAGAGGCCCGCACCGATCGCCCTTCC CAACAGTTGCGCAGCCTGAATGGCGAATGGACGCGCCCTGTAGCGGCGCATTAAGCGCGGC GGGTGTGGTGGTTACGCGCAGCGTGACCGCTACACTTGCCAGCGCCCTAGCGCCCGCTCCTT TCGCTTTCTTCCCTTCCTTTCTCGCCACGTTCGCCGGCTTTCCCCGTCAAGCTCTAAATCGGGG GCTCCCTTTAGGGTTCCGATTTAGTGCTTTACGGCACCTCGACCCCAAAAAACTTGATTAGGGT GATGGTTCACGTAGTGGGCCATCGCCCTGATAGACGGTTTTTCGCCCTTTGACGTTGGAGTCC ACGTTCTTTAATAGTGGACTCTTGTTCCAAACTGGAACAACACTCAACCCTATCTCGGTCTATTC TTTTGATTTATAAGGGATTTTGCCGATTTCGGCCTATTGGTTAAAAAATGAGCTGATTTAACAAAA ATTTAACGCGAATTTTAACAAAATATTAACGCTTACAATTTAGGTGGCACTTTTCGGGGAAATGT GCGCGGAACCCCTATTTGTTTATTTTTCTAAATACATTCAAATATGTATCCGCTCATGAGACAAT AACCCTGATAAATGCTTCAATAATATTGAAAAAGGAAGAGTATGAGTATTCAACATTTCCGTGTC GCCCTTATTCCCTTTTTTGCGGCATTTTGCCTTCCTGTTTTTGCTCACCCAGAAACGCTGGTGAA AGTAAAAGATGCTGAAGATCAGTTGGGTGCACGAGTGGGTTACATCGAACTGGATCTCAACAG CGGTAAGATCCTTGAGAGTTTTCGCCCCGAAGAACGTTTTCCAATGATGAGCACTTTTAAAGTT CTGCTATGTGGCGCGGTATTATCCCGTATTGACGCCGGGCAAGAGCAACTCGGTCGCCGCATA CACTATTCTCAGAATGACTTGGTTGAGTACTCACCAGTCACAGAAAAGCATCTTACGGATGGCA TGACAGTAAGAGAATTATGCAGTGCTGCCATAACCATGAGTGATAACACTGCGGCCAACTTACT TCTGACAACGATCGGAGGACCGAAGGAGCTAACCGCTTTTTTGCACAACATGGGGGATCATGT AACTCGCCTTGATCGTTGGGAACCGGAGCTGAATGAAGCCATACCAAACGACGAGCGTGACAC CACGATGCCTGTAGCAATGGCAACAACGTTGCGCAAACTATTAACTGGCGAACTACTTACTCTA GCTTCCCGGCAACAATTAATAGACTGGATGGAGGCGGATAAAGTTGCAGGACCACTTCTGCGC TCGGCCCTTCCGGCTGGCTGGTTTATTGCTGATAAATCTGGAGCCGGTGAGCGTGGGTCTCGC GGTATCATTGCAGCACTGGGGCCAGATGGTAAGCCCTCCCGTATCGTAGTTATCTACACGACG GGGAGTCAGGCAACTATGGATGAACGAAATAGACAGATCGCTGAGATAGGTGCCTCACTGATT AAGCATTGGTAACTGTCAGACCAAGTTTACTCATATATACTTTAGATTGATTTAAAACTTCATTTT TAATTTAAAAGGATCTAGGTGAAGATCCTTTTTGATAATCTCATGACCAAAATCCCTTAACGTGA GTTTTCGTTCCACTGAGCGTCAGACCCCGTAGAAAAGATCAAAGGATCTTCTTGAGATCCTTTTT TTCTGCGCGTAATCTGCTGCTTGCAAACAAAAAAACCACCGCTACCAGCGGTGGTTTGTTTGCC GGATCAAGAGCTACCAACTCTTTTTCCGAAGGTAACTGGCTTCAGCAGAGCGCAGATACCAAAT ACTGTTCTTCTAGTGTAGCCGTAGTTAGGCCACCACTTCAAGAACTCTGTAGCACCGCCTACAT ACCTCGCTCTGCTAATCCTGTTACCAGTGGCTGCTGCCAGTGGCGATAAGTCGTGTCTTACCG GGTTGGACTCAAGACGATAGTTACCGGATAAGGCGCAGCGGTCGGGCTGAACGGGGGGTTCG TGCACACAGCCCAGCTTGGAGCGAACGACCTACACCGAACTGAGATACCTACAGCGTGAGCTA TGAGAAAGCGCCACGCTTCCCGAAGGGAGAAAGGCGGACAGGTATCCGGTAAGCGGCAGGGT CGGAACAGGAGAGCGCACGAGGGAGCTTCCAGGGGGAAACGCCTGGTATCTTTATAGTCCTG TCGGGTTTCGCCACCTCTGACTTGAGCGTCGATTTTTGTGATGCTCGTCAGGGGGGCGGAGCC TATGGAAAAACGCCAGCAACGCGGCCTTTTTACGGTTCCTGGCCTTTTGCTGGCCTTTTGCTCA CATGTTCTTTCCTGCGTTATCCCCTGATTCTGTGGATAACCGTATTACCGCCTTTGAGTGAGCT GATACCGCTCGCCGCAGCCGAACGACCGAGCGCAGCGAGTCAGTGAGCGAGGAAGCGGAAG AGCGCCCAATACGCAAACCGCCTCTCCCCGCGCGTTGGCCGATTCATTAATGCAGCTGGCACG ACAGGTTTCCCGACTGGAAAGCGGGCAGTGAGCGCAACGCAATTAATGTGAGTTAGCTCACTC ATTAGGCACCCCAGGCTTTACACTTTATGCTTCCGGCTCGTATGTTGTGTGGAATTGTGAGCGG ATAACAATTTCACACAGGAAACAGCTATGACCATGATTACGCCAGATTTAATTAAGGCCTTAATT AGG |
| 126 | KASH2 C-term 1 | FARSFYPMLRYTNGPPPT |
| 127 | KASH2 C-term 2 | FYPMLRYTNGPPPT |
| 128 | KASH2 C-term 3 | FARSFYPMLRYTNG |
| 129 | KASH2 C-term 4 | FARSFYPMLRYTNGPPPTA |

The invention includes the combination of the aspects and preferred features described except where such a combination is clearly impermissible or expressly avoided.

The features disclosed in the foregoing description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the invention in diverse forms thereof.

For the avoidance of any doubt, any theoretical explanations provided herein are provided for the purposes of improving the understanding of a reader. The inventors do not wish to be bound by any of these theoretical explanations.

Any section headings used herein are for organizational purposes only and are not to be construed as limiting the subject matter described.

Throughout this specification, including the claims which follow, unless the context requires otherwise, the word "comprise" and "include", and variations such as "comprises", "comprising", and "including" will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means for example +/- 10%.

Methods disclosed herein may be performed, or products may be present, *in vitro, ex vivo,* or *in vivo.* The term "*in vitro*" is intended to encompass experiments with materials, biological substances, cells and/or tissues in laboratory conditions or in culture whereas the term "*in vivo*" is intended to encompass experiments and procedures with intact multi-cellular organisms. "*Ex vivo*" refers to something present or taking place outside an organism, e.g. outside the human or animal body, which may be on tissue (e.g. whole organs) or cells taken from the organism.

Where a nucleic acid sequence is disclosed herein, the reverse complement thereof is also expressly contemplated.

For standard molecular biology techniques, see Sambrook, J., Russel, D.W. Molecular Cloning, A Laboratory Manual. 3 ed. 2001, Cold Spring Harbor, New York: Cold Spring Harbor Laboratory Press

Aspects and embodiments of the present invention will now be discussed with reference to the accompanying figures. Further aspects and embodiments will be apparent to those skilled in the art. All documents mentioned in this text are incorporated herein by reference. While the invention has been described in conjunction with the exemplary embodiments described below, many equivalent modifications and variations will be apparent to those skilled in the art when given this disclosure. Accordingly, the exemplary embodiments of the invention set forth above are considered to be illustrative and not limiting. Various changes to the described embodiments may be made without departing from the spirit and scope of the invention.

### Brief Description of the Figures

Embodiments and experiments illustrating the principles of the invention will now be discussed with reference to the accompanying figures.

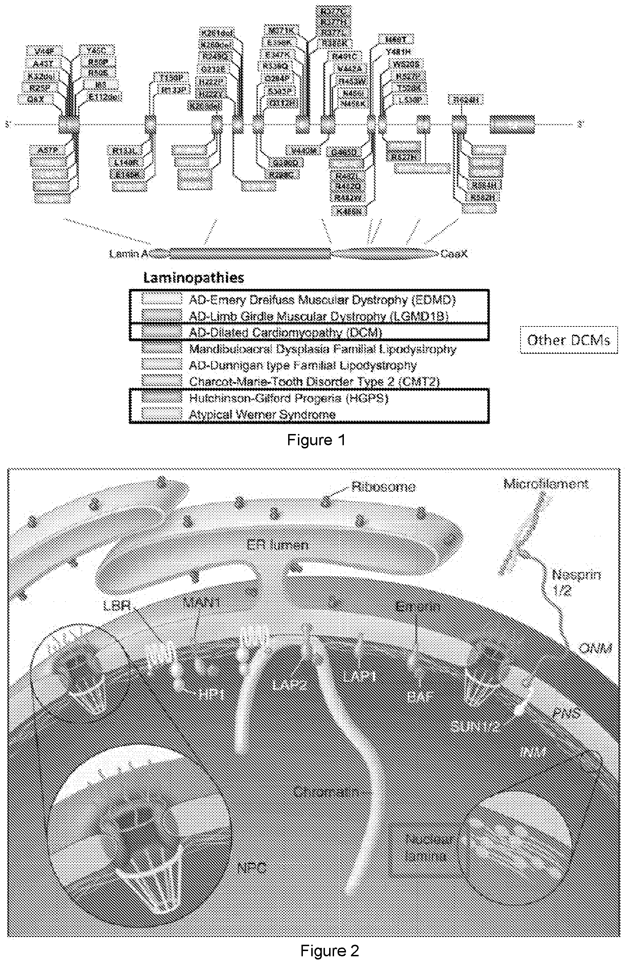

**Figure 1** shows a schematic of the mutations in the lamin A/C gene *LMNA* and the laminopathies resulting from the mutations.
**Figure 2** shows a schematic of the positioning of components of the nuclear envelope membrane and lamina.

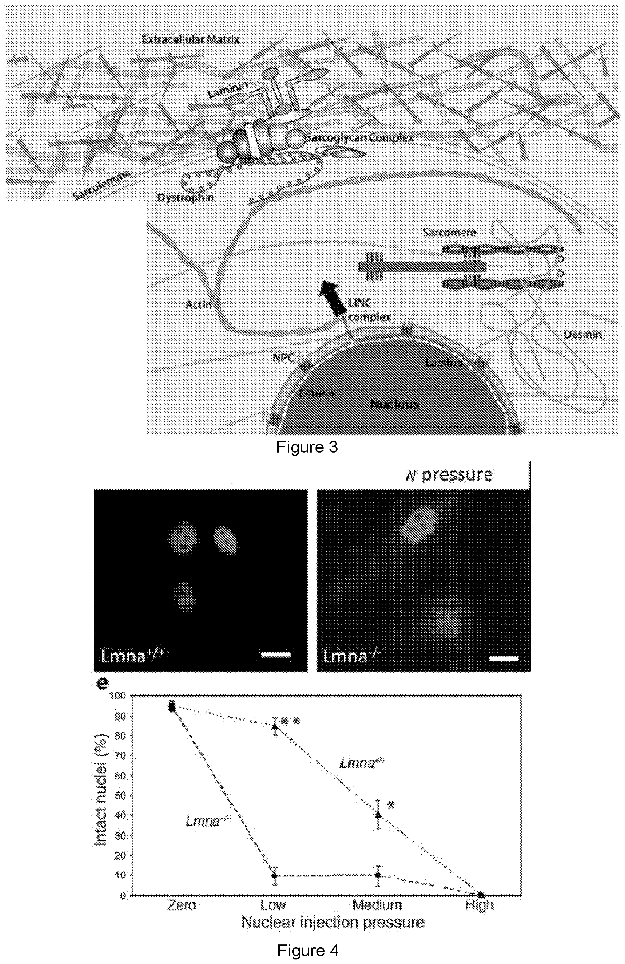

**Figure 3** shows a schematic of the connections between the nucleus and the extracellular matrix via the LINC complex and how mutations in lamin A/C might result in DCM. The plasma membrane, cytoskeleton and nucleus form a mechanically and physically linked entity. In Lmna mutants, the nucleus is structurally weak. It is much more susceptible to mechanical stress from cytoskeletal forces. This leads to severe damage to the myocyte nuclei that in turn leads to a cascade of events such as apoptosis and fibrosis that results finally in DCM.
**Figure 4** shows the effect of microinjection of dextran into the nucleus of *Lmna*^{+/+} and *Lmna*^{-/-} mice under low pressure. In the wildtype cells, the dextran stays in the nucleus, while in the Lmna mutant cells the dextran leaks out of the nucleus into the cytoplasm.

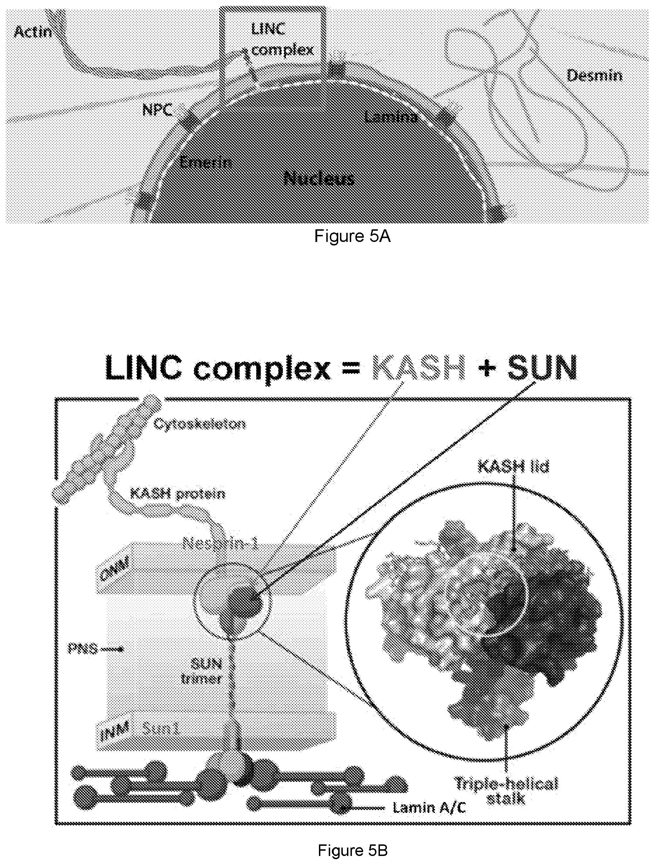

**Figures 5A-5B** show schematics of a LINC complex (**Fig. 5A**) and interaction between KASH and SUN (**Fig. 5B**).

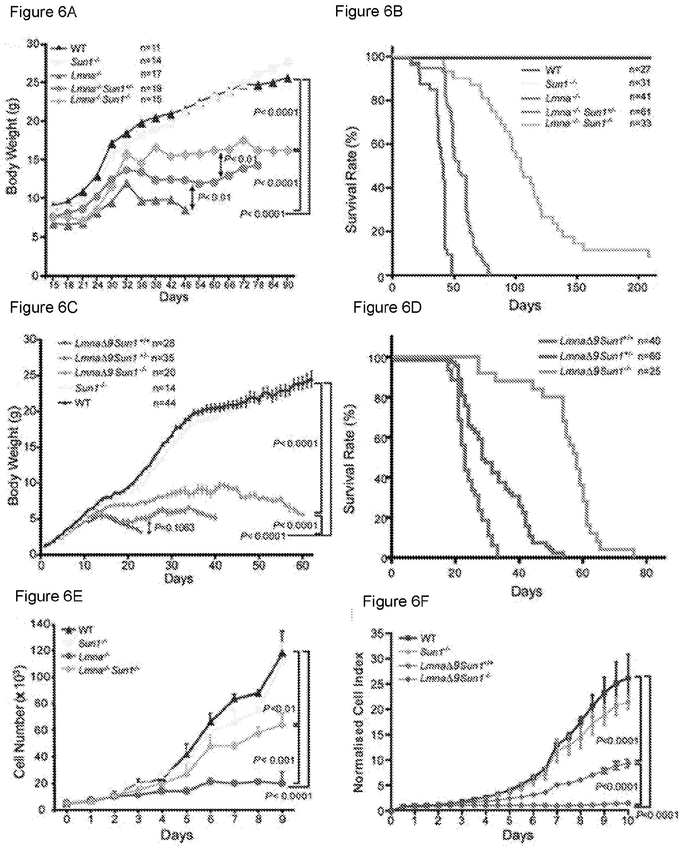

**Figure 6** shows defects in body weight and longevity in *Lmna*^{-/-} and *Lmna*Δ9 mice are ameliorated in homozygous Sun1 knockout *Lmna*^{-/-}*Sun1*^{-/-} and *Lmna*Δ9*Sun1*^{-/-} animals. (**A**) Body weights are averages from mice with the indicated genotypes. The number (n) of animals used is indicated. (**B**) Kaplan-Meier graph showing increased life span of *Lmna*^{-/-}*Sun1*^{-/-} compared to *Lmna*^{-/-} mice. Median survival of wild-type or *Sun1*^{-/-} is > 210 days in a 7 month follow up; *Lmna*^{-/-} mice have median survival of 41 days; *Lmna*^{-/-}*Sun1*^{+/-} mice have a median survival of 54 days; *Lmna*^{-/-}*Sun1*^{-/-}mice have a median survival of 104 days (p < 0.01 comparing *Lmna*^{-/-} and *Lmna*^{-/-}*Sun1*^{-/-})*.* (**C**) Body weights of *Lmna*Δ9 mice that are wild-type, heterozygous, or homozygous for *Sun1* deficiency. Wild-type and *Sun1*^{-/-} cohorts are graphed for comparison. Values are averages ± SEM from animals in each cohort. Number (n) of animals is indicated (p < 0.0001 comparing *Lmna*Δ9*Sun1*^{+/+} and *Lmna*Δ9*Sun1*^{-/-}). (**D**) Kaplan-Meier graph showing increased life span of *Lmna*Δ9*Sun1*^{-/-} compared to *Lmna*Δ9*Sun1*^{+/+} mice. *Lmna*Δ9*Sun1*^{+/-} mice are also graphed. (p < 0.0001 comparing *Lmna*Δ9*Sun1*^{+/+} and *Lmna*Δ9*Sun1*^{-/-}). **(E)** Cell proliferation of the indicated MEFs. Curves are averages ± SD, representative of > 3 independent isolates from embryos of the indicated genotypes. (**F**) Proliferation curves of MAFs (mouse adult fibroblasts) from WT, *Sun1*^{-/-}, *Lmna*Δ9*Sun1*^{+/+} and *Lmna*Δ9*Sun1*^{-/-}mice. MAFs were seeded at a density of 1000 cells per well. Growth was measured, and normalized cell indexes (averages ± SD) are presented.
**Figure 7** shows a schematic of the features of the Sun1 protein and the components used to generate a dominant negative Sun1 protein, including a signal sequence, coiled-coil sequence, SUN domain sequence and KDEL sequence.
**Figure 8** shows a schematic of a plasmid (SEQ ID NO:1) used for AAV production.

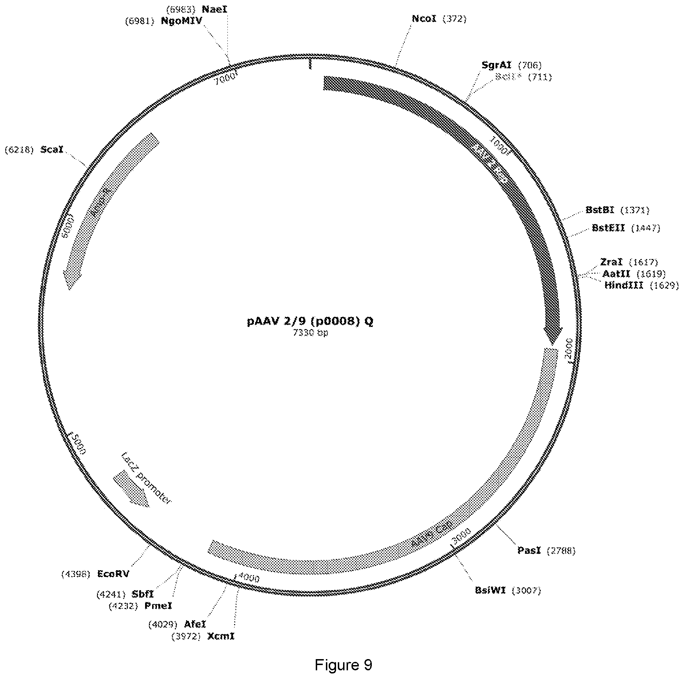

**Figure 9** shows a schematic of a plasmid (SEQ ID NO:2) comprising sequences from AAV2 and AAV9 for AAV production.
**Figure 10** shows a schematic of an AAV expression construct (SEQ ID NO:3) comprising cardiac-specific promoter and Sun1 dominant negative sequence.
**Figure 11** shows a schematic of the features of the dominant negative Sun1 protein, including a signal sequence, coiled-coil sequence, SUN domain sequence and KDEL sequence (SEQ ID NO:4).
**Figure 12** shows a schematic of the features of a dominant negative Sun2 protein, including a signal sequence, lumenal domain sequence and KDEL sequence (SEQ ID NO:5).
**Figure 13** shows a schematic of the region of Sun1 protein used in dominant negative constructs.
**Figure 14** shows an alignment of KASH1-KASH5 domain amino acid sequences with conserved residues (SEQ ID NOs:7, 9, 11, 13 and 15, respectively).

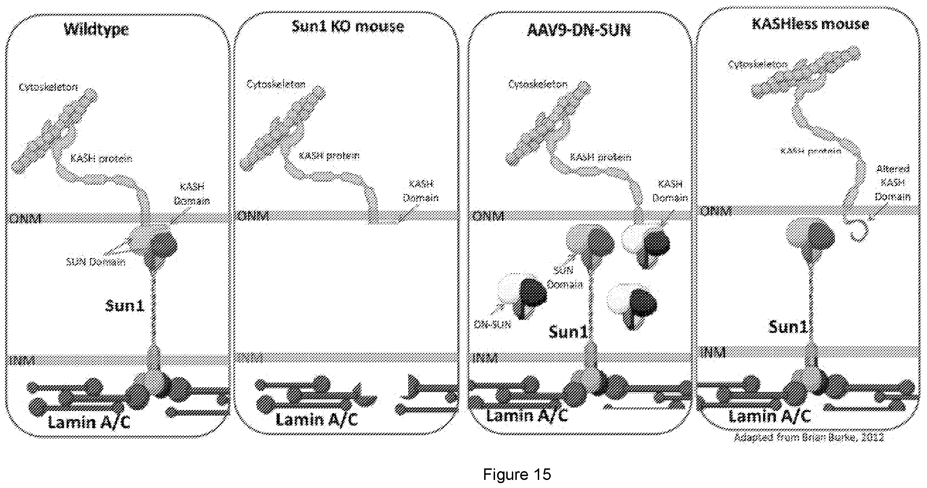

**Figure 15** shows a schematic of the LINC complex in wildtype mice, Sun1 KO mice, AAV dominant negative SUN mice and mice with altered KASH domain. The schematic for wildtype mice is obtained from Brian Burke, 2012. The schematic for Sun1 KO mice represents the results from Chen et al., 2012. The AAV dominant negative SUN and the altered KASH domain schematics represent inventor proposals at the priority date on methods for LINC complex disruption to ameliorate laminopathies, based on data obtained at that time.

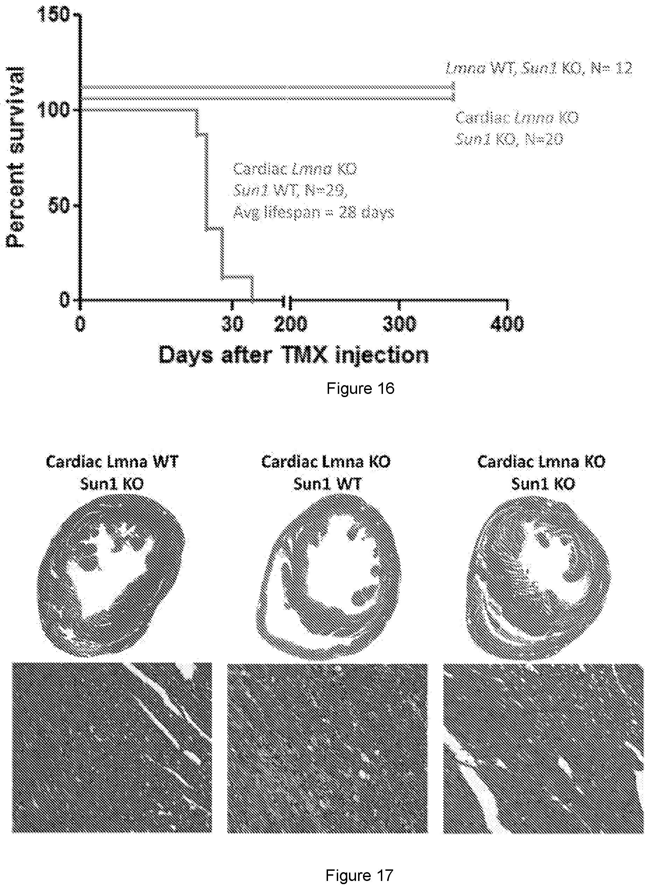

**Figure 16** shows a Kaplan Meier curve of *Lmna* KO mice surviving for an average of 28 days, *Sun1* KO mice living beyond 300 days and cardiac *Lmna* KO/*Sun1* KO mice living beyond 300 days.
**Figure 17** shows H&E stained sections of hearts from *Sun1* KO mice, cardiac *Lmna* KO mice and cardiac *Lmna* KO/*Sun1* KO mice, with LmnaKO/Sun1WT hearts showing enlargement of the left ventricle (DCM) compared to WT and LmnaKo/Sun1KO hearts.

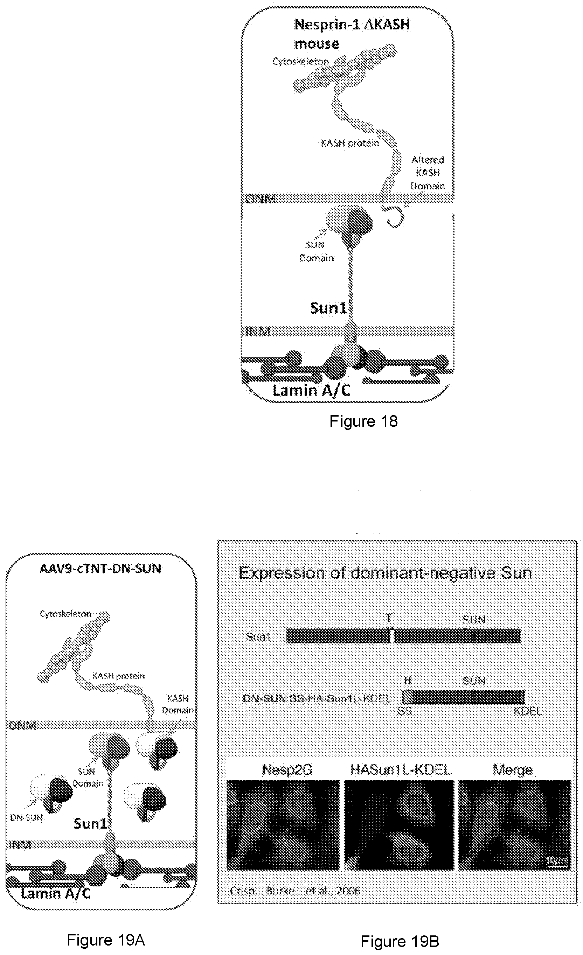

**Figure 18** shows a schematic of disruption of a LINC complex in a Nesprin-1 ΔKASH mouse. *Lmna*KO Nesprin-1WT mice have a lifespan of about 20 days. *Lmna*KO Nesprin-1-ΔKASH survive about 40 days, which is similar to *Lmna*KO*Sun1*KO mice.
**Figures 19A-19B** show a schematic of anticipated AAV-cTNT-DN-SUN expression and competition between exogenous DN-SUN and native Sun1 for binding to the KASH domain (**Fig. 19A**) with the DN-SUN shown in 19B (upper panel) and the effect of transfected DN-SUN on native Nesprin2G positioning in cells where the 2 nuclei in the middle panel express the DN-SUN and in the merge panel both show loss of Nesprin2 from the nuclear membranes(**Fig. 19B**).

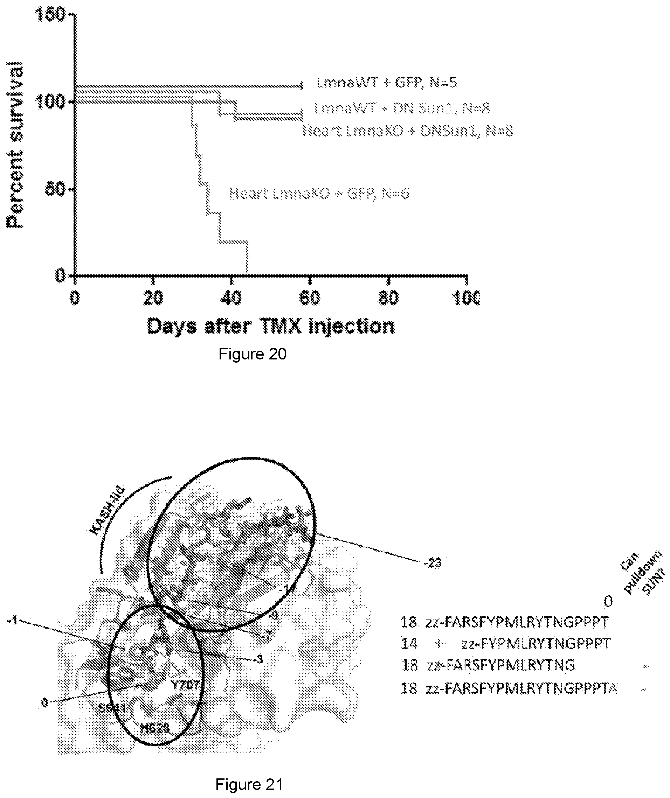

**Figure 20** is a Kaplan Meier curve showing disruption of SUN-KASH interaction in vivo, using AAV9-cTNT-dominant negative Sun1 (DNSun1), extends the longevity of the heart-specific *Lmna* KO in male and female mice.
**Figure 21** shows C-terminal amino acids of the KASH domain of Nesprin-2 (KASH2). The 14 or 18 amino acid sequence from KASH2 C-terminus are able to physically interact with the SUN domain of SUN2. Loss of the last 4 amino acids from KASH2 or addition of a single alanine amino acid at the C-terminus of KASH2 is sufficient to disrupt interaction of the KASH2 domain with the SUN domain.

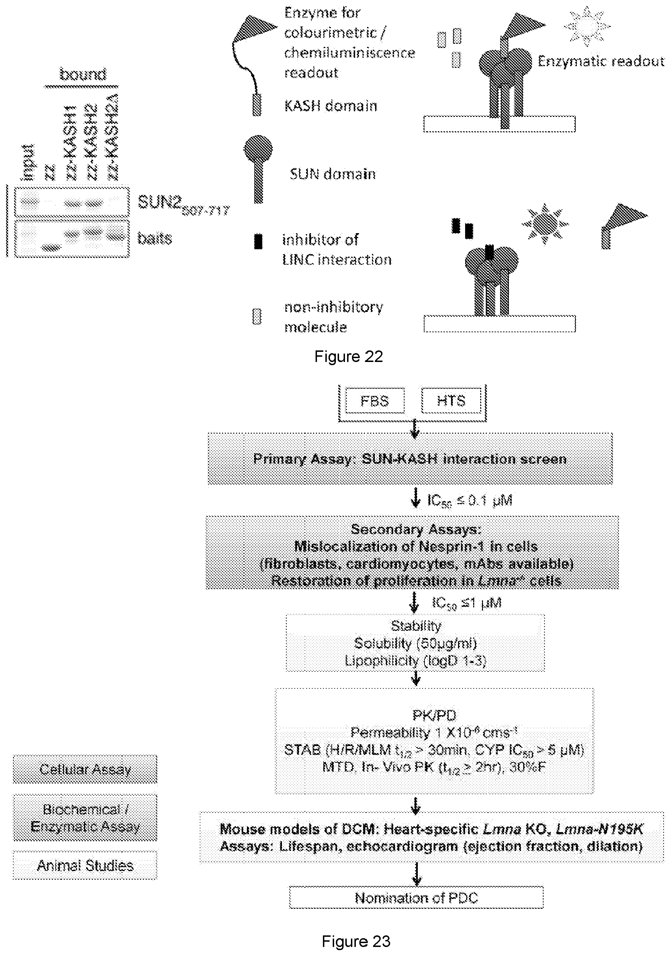

**Figure 22** shows a schematic of a screening method for detecting agents that disrupt the LINC complex.
**Figure 23** shows a flowchart showing a more detailed screening method for identifying a small molecule to disrupt the LINC complex.

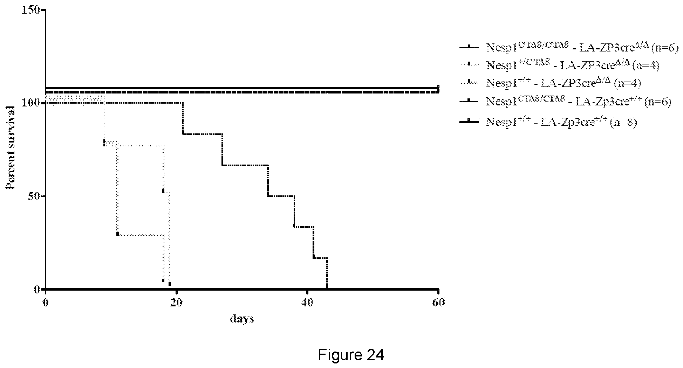

**Figure 24** is a Kaplan Meier curve showing that wild type (C57/BI6) mice with or without a Nesprin-1 KASH-disrupting (C'TΔ8) mutation have a normal lifespan. Mice with a Lmna null/KO mutation (LA-ZP3cre^{Δ/Δ}) and wildtype (Nesp1^{+/+}) or heterozygous (Nesp1^{+/C'TΔ8}) for Nesp1-C'TΔ8 have a median lifespan of 15 or 18 days, which is increased to 38 days in Lmna KO / homozygous Nesp1 mutant (LA-ZP3cre^{Δ/Δ}; Nesp1 ^{C'TΔ8/C'TΔ8}) mice.

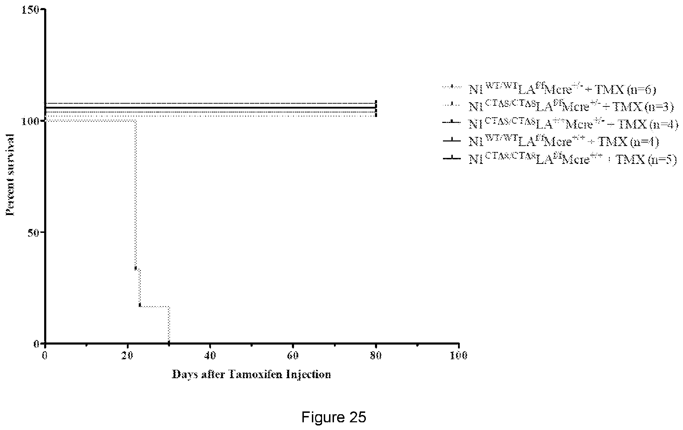

**Figure 25** is a Kaplan Meier curve showing that mice with wildtype Lmna (N1^{CTΔ8/CTΔ8}LA^{+/+}MCre^{+/-}), or floxed alleles of Lmna but lacking a cardiac-specific Cre driver (N1^{CTΔ8/CTΔ8}LA^{f/f}MCre^{+/+} and N1^{WT/WT}LA^{f/f}MCre^{+/+}), live for the length of the experiment (~ 80 days at priority filing, which extended to 120 days unchanged). Mice with a cardiomyocyte-specific deletion of Lmna (N1^{WT/WT}LA^{f/f}MCre^{+/-}) have a lifespan of 22-24 days following induction of the Cre/loxP-mediated deletion by tamoxifen (TMX) delivery, which is increased to the length of the experiment in mice with a cardiomyocyte-specific deletion of Lmna induced by TMX and also homozygous mutant for Nesprin-1 (N1^{CTΔ8/ CTΔ8}LA^{f/f}MCre^{+/-}).

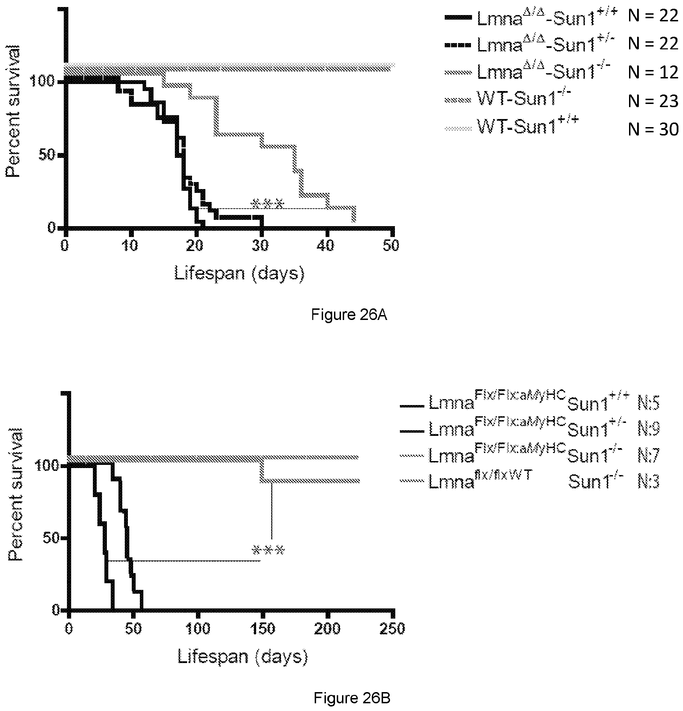

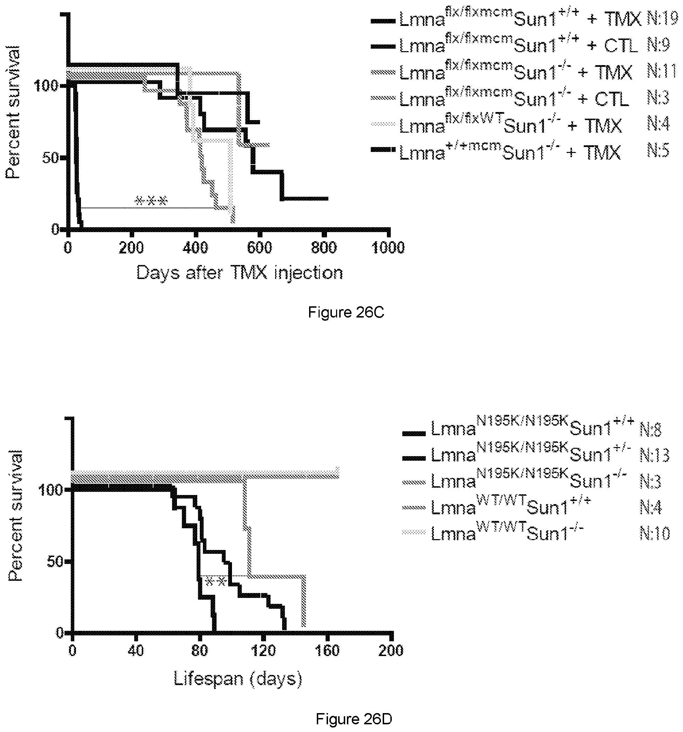

**Figures 26A-26D** show Kaplan Meier curves of Sun1 loss extending the longevity of *Lmna* mutant mice. (**Fig. 26A**) Wild type (C57/BI6) mice with or without Sun1 have a normal lifespan, whereas the average postnatal lifespan of the *Lmna*^{*Flx*/*Flx:Zp3*} mice in which LaminA is deleted in all tissues was 17.5 days (***P=<0.0001; Log-rank test). On a *Sun1*^{-/-} background longevity is increased to 32.5 days. **(****Fig. 26B****)** When *Lmna*^{*Flx*/*Flx*} was deleted specifically and constitutively in hearts by crossing the mice with the *Cre^{αMyHC}* line, the *Lmna^{FlxlFlx:αMyHC}* mice lived on average 26.5 days. On a *Sun1*^{-/-} background these mice lived for longer than 6 months. (**Fig. 26C**) 3-5 month old *Lmna*^{*Flx*/*Flx*} were crossed with the Tmx inducible cardiomyocyte specific Cre *Tg*(*Myh6-cre*/*Esr1*), (abbreviated to *mcm*), after a single injection of Tmx the mice die within 3-4 weeks. On a *Sun1*^{-/-} background these mice lived for more than 1 year. **(****Fig. 26D****)** *Lmna*^{*N195K*/*N195K*} mice lived for an average of 78 days compared to *Lmna*^{*N195K*/}*^{N195K}Sun1*^{-/-} mice which had an average lifespan of 111 days. (***P=<0.0001, **P=0.0073 Log-rank test).

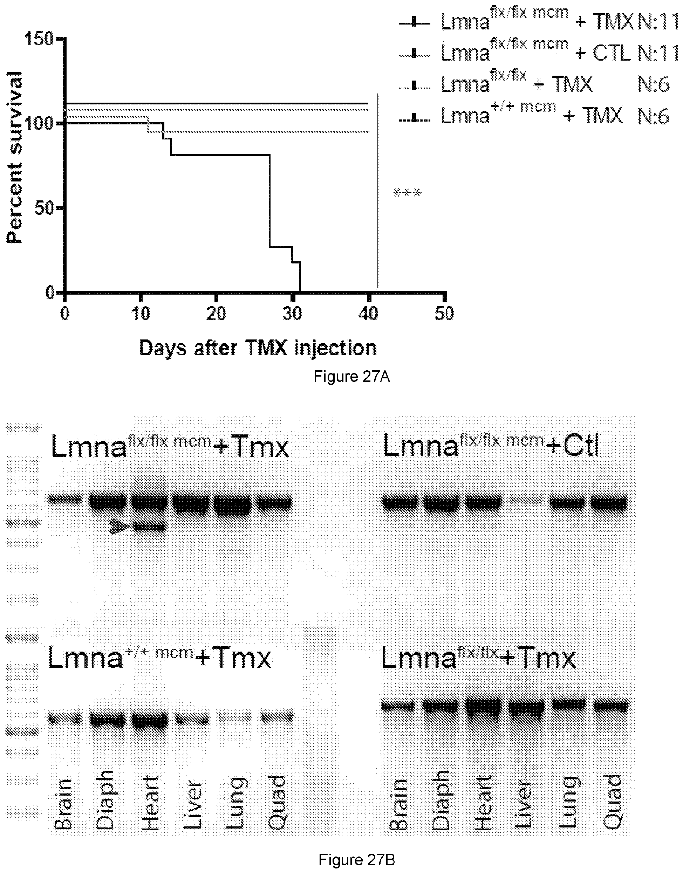

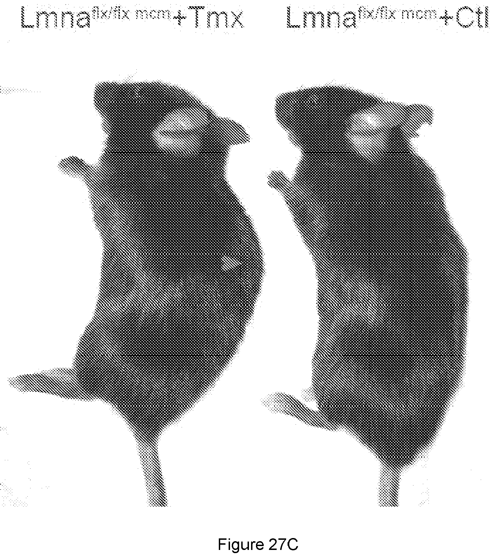

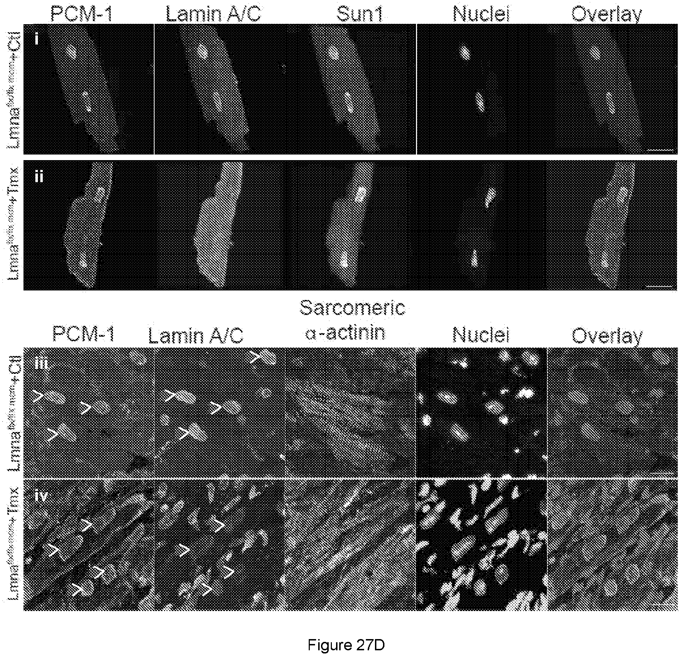

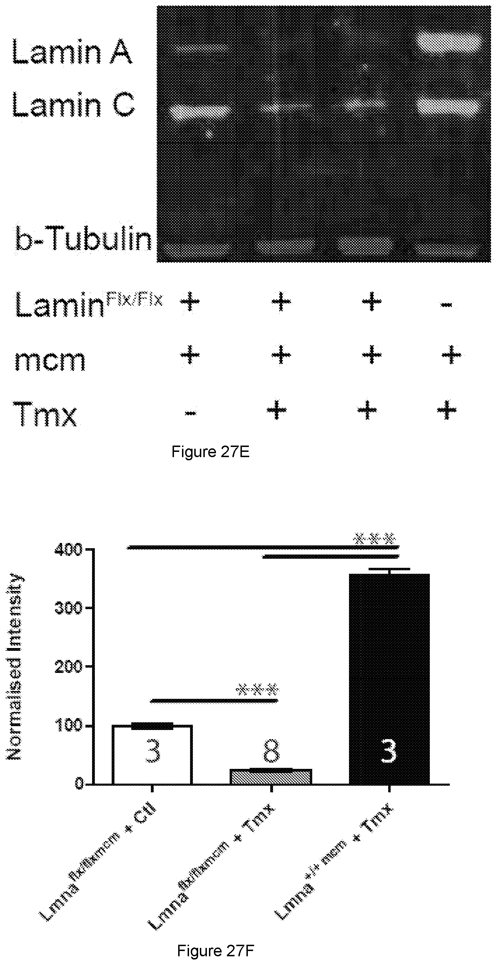

**Figures 27A-27E** show the lifespan and phenotype of *Lmna^{FlxlFlx:mcm}* + Tmx mice. (**Fig. 27A**) The average lifespan of the *Lmna*^{*Flx*/*Flx:mcm*} mice was 27 days after a single Tmx injection (***P=<0.0001 ; Log-rank test). (**Fig. 27B**) PCR detected the floxed (deleted) *Lmna* gene (arrow head) only in heart tissue after Tmx injection and not in other tissues or when Tmx was not injected (**Fig. 27C**) *Lmna*^{*Flx*/*Flx:mcm*}+Tmx mice developed kyphosis (arrow head) by 21 days after injection. (**Fig. 27D**) LaminA/C protein, detected by immunofluorescence, were present in control (i, iii), but reduced/absent in cardiomyocyte (CM) nuclei in both isolated CMs (ii second panel) and heart sections (iv) (white arrowheads) with CM nuclei being detected by PCM-1 staining, 21 days after Tmx. (**Fig. 27E**) LaminA/C levels were quantified by Western analysis of whole heart lysates 21 days after injection. A significant reduction (***P=<0.0001; T-test) in A-type Lamin protein was detected, although Lamin C levels were not reduced as much in the *Lmna*^{*Flx*/*Flx:mcm*}+Tmx mice compared to *Lmna*^{*Flx*/*Flx:mcm*}+CTL. (**Fig. 27F**) Quantitative analysis was performed at 21 days post Tmx. The presence of the LoxP sites in the WT-*Lmna* gene (*Lmna*^{*Flx*/*Flx*}) results in a reduction in *Lmna* transcript levels compared to *Lmna*^{*Wt*/*Wt*} levels, although this had no overt effect on longevity or growth/viability.

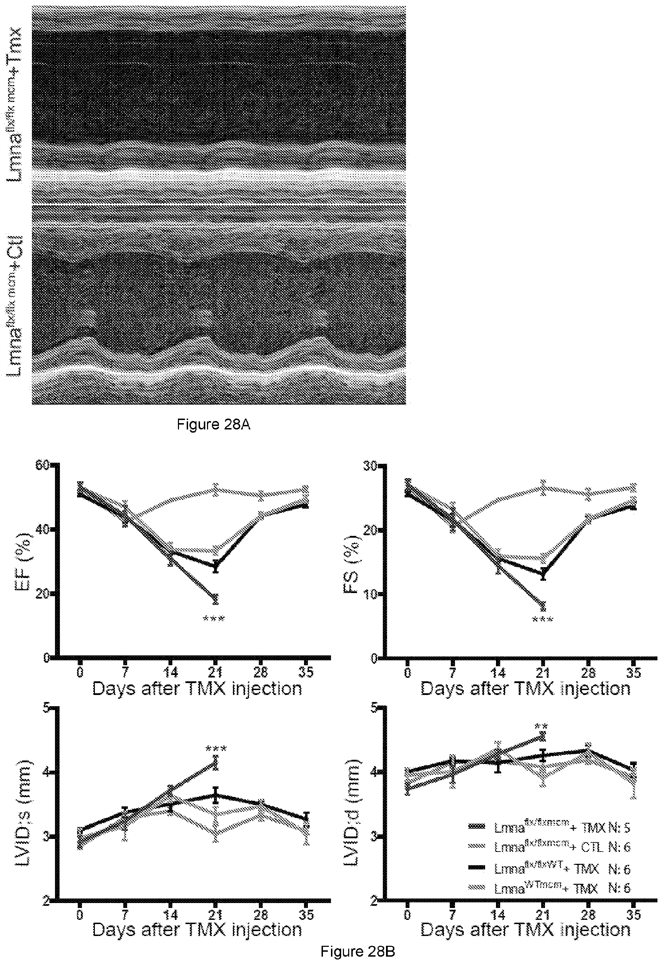

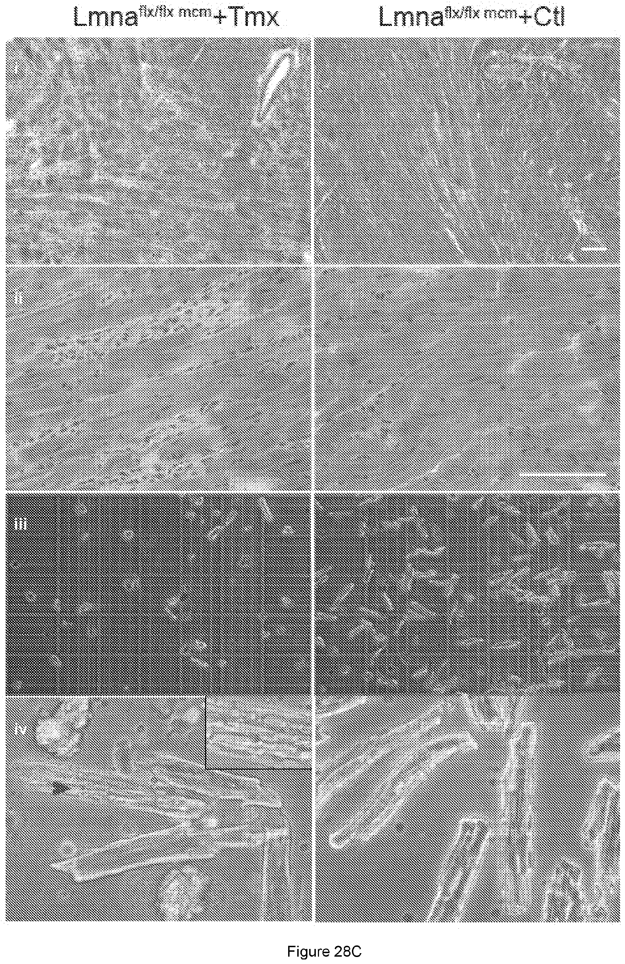

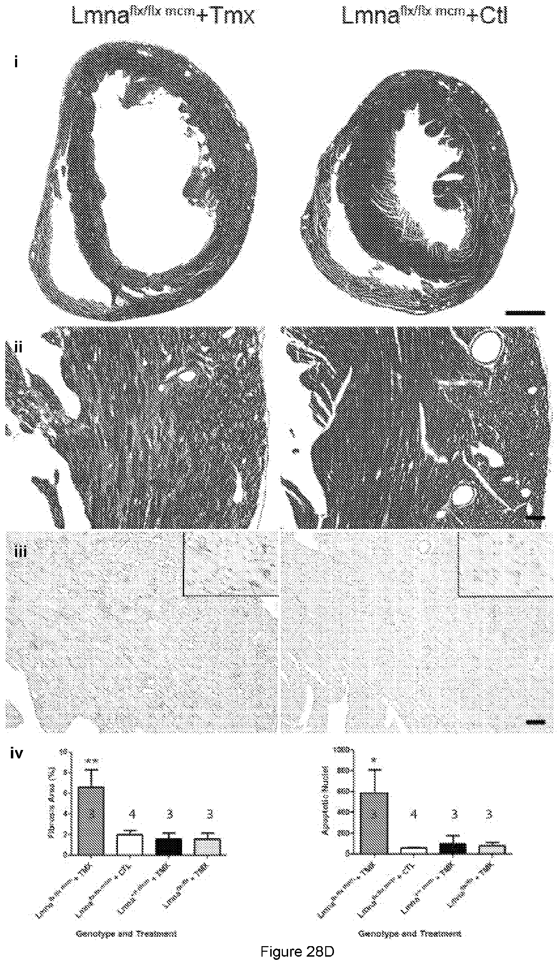

**Figures 28A-28D** show echocardiograms, heart function and histology of *Lmna*^{*Flx*/*Flxmcm*} + Tmx mice. **(****Fig. 28A****)** *Lmna*^{*Flx*/*Flx:mcm*} + Tmx mice show reduced cardiac contractile function. (**Fig. 28B**) *Lmna^{FlxlFlx:mcm}* hearts show reduced EF% and FS%, and increased LVID (***P=<0.0001, **P=0.0010; Two way ANOVA). (**Fig. 28C**) Histological analysis of the hearts revealed increased infiltration of nucleated cells and intercellular spaces in *Lmna^{FlxlFlxmcm}* hearts (i and ii). Significantly fewer viable (brick-like) CMs were isolated from *Lmna*^{*Flx*/*Flx:mcm*} hearts compared to *Lmna*^{*Flx*/*Flx:mcm*} controls (iii). With higher magnification, the isolated cardiomyocytes from *Lmna^{FlxlFlx:mcm}* hearts contained large intracellular vacuoles (arrow head, iv). (**Fig. 28D**) The left ventricular lumen in *Lmna*^{*Flx*/*Flx:mcm*} hearts was enlarged (i) together with increased fibrosis (ii) (**P=0.0007 visible as lighter grey areas in the 28D ii, middle panels and iv left panel) and apoptotic nuclei revealed by TUNEL staining (*P=0.0220; One way ANOVA) (iii and iv right panel). All samples and analyses were performed on hearts 21 days post Tmx injection.

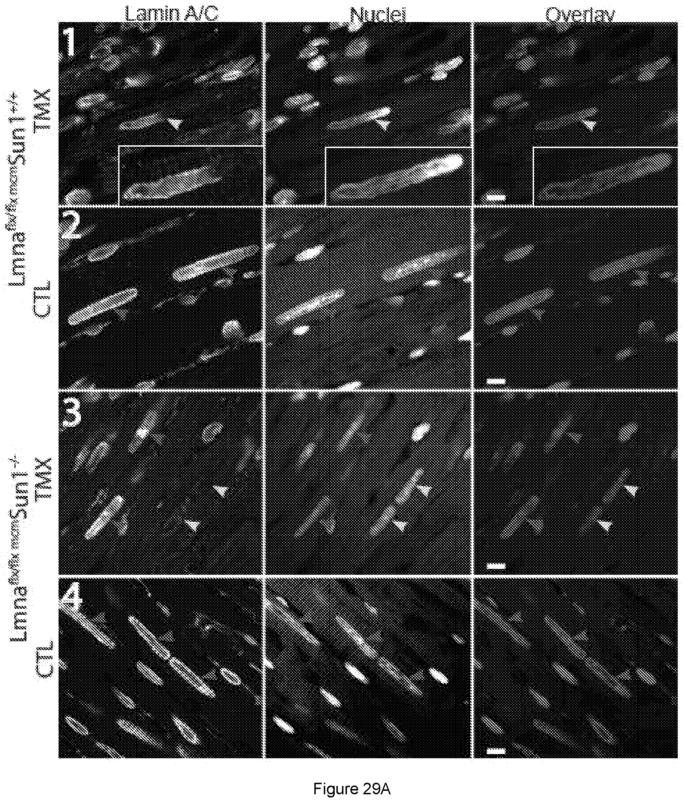

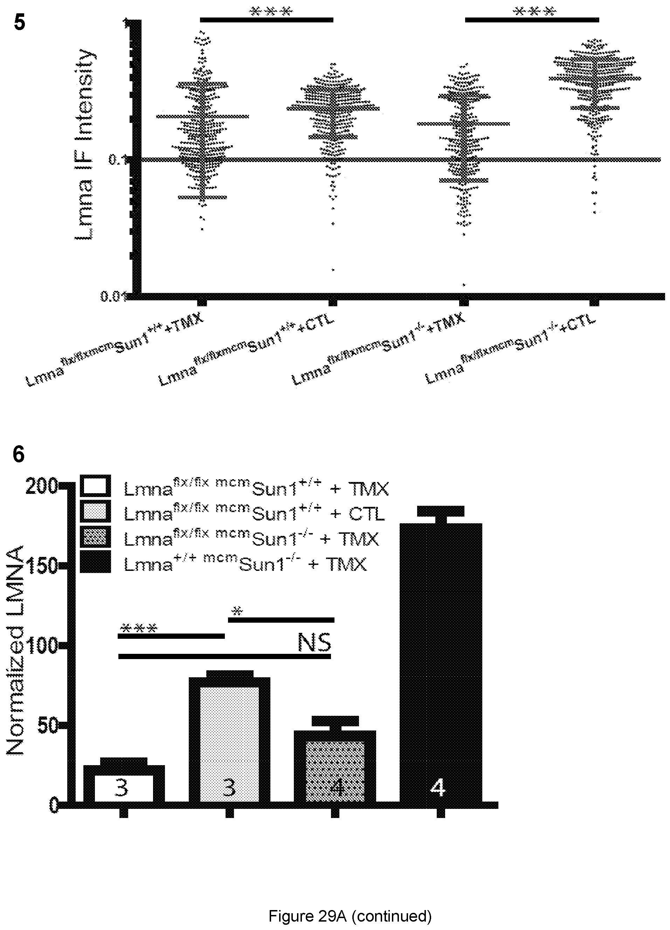

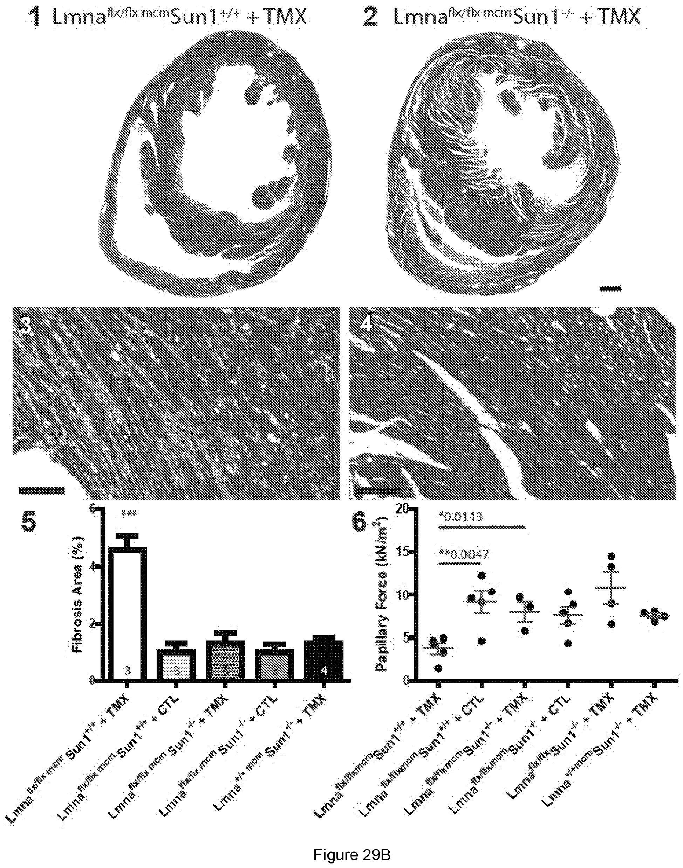

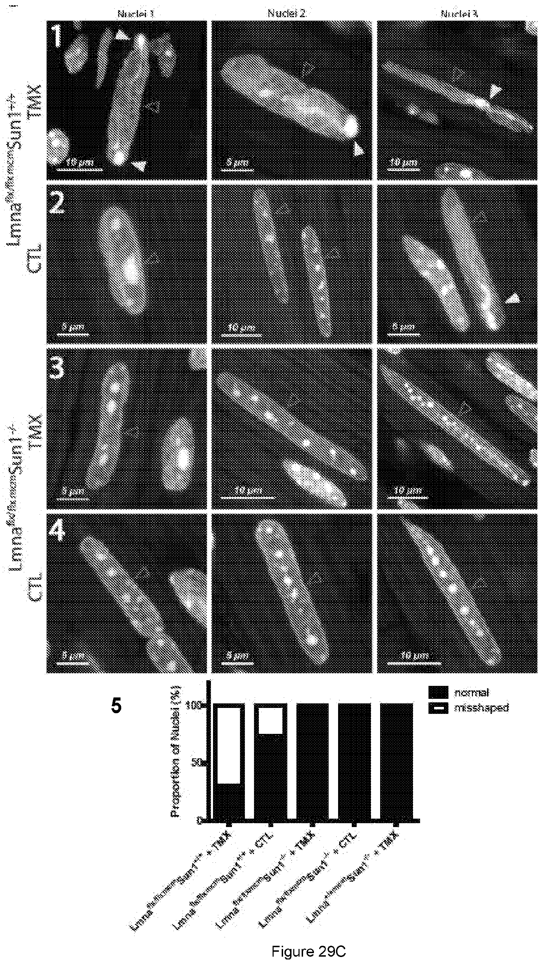

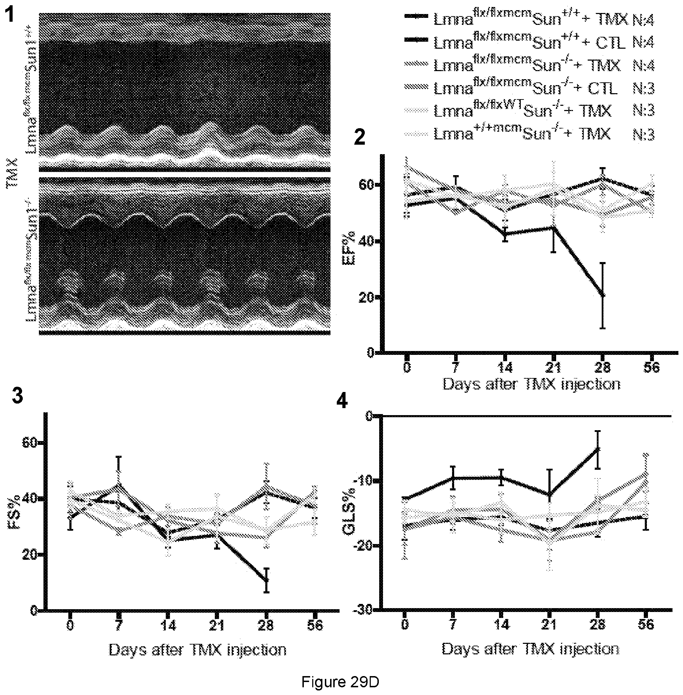

**Figures 29A-29D** shows changes in nuclear morphologies and heart structure with and without Sun1 in the *Lmna*^{*Flx*/*Flx:mcm*} after Tmx injection. (**Fig. 29A**) CM nuclei with reduced or absent Lamin A/C expression are indicated by white arrow heads (1, 3). CM nuclei (2, 4) with normal Lamin A/C levels are indicated by grey arrowheads. LMNA protein levels, measured by both fluorescence intensity (5) and Western blot (6), were significantly reduced in *Lmna^{FlxlFlx:mcm} Sun1*^{+/+}+Tmx (***P=0.0009; T-test) and *Lmna*^{*Flx*/}*^{Flx:mcm} Sun1*^{-/-}+Tmx (*P= 0.0359; T-test) compared to *Lmna*^{*Flx*/*Flxmcm*} Sun1^{+/+} controls (lower graph, 6) (**Fig. 29B**). Left ventricular (LV) enlargement was apparent in the *Lmna^{FlxlFlx:mcm} Sun1*^{+/+}+Tmx hearts (panel 1) but not in the LV of the *Lmna^{FlxlFlx:mcm} Sun1*^{*-*/*-*}+Tmx hearts (panel 2). The *Lmna*^{*Flx*/}*^{Flx:mcm} Sun1*^{+/+}+Tmx mice had significantly increased fibrosis (panel 3, fibrosis in grey) compared to controls, but there was no significant increase in fibrosis in the *Lmna*^{*Fx*/}*^{lFlxmcm} Sun1*^{-/-}+Tmx hearts (panel 3) compared to controls (panel 4, quantified in panel 5, ***P=0.0001; One way ANOVA). Cardiac papillary muscle active force measurements were significantly reduced from the *Lmna*^{*Flx*/}*^{Flx:mcm} Sun1*^{+/+}+Tmx mice compared to *Lmna*^{*Flx*/}*^{Flx:mcm} Sun1*^{+/+} controls (**P=0.0047; T-test) and *Lmna^{FlxlFlx:mcm} Sun1*^{-/-} + Tmx (*P=0.0113; T-test) (panel 6). (**Fig. 29C**) CM nuclear morphologies were significantly altered in *Lmna*^{*Flx*/}*^{Flx:mcm} Sun1*^{+/+}+Tmx mice (Panel 1, solid arrow heads). In the absence of TMX, control heart sections (CTL, panel 2) display few nuclear abnormalities. In the absence of Sun1, *Lmna*^{*Flx*/}*^{Flx:mcm} Sun1*^{*-*/*-*}+Tmx cardiomyocytes showed no nuclear abnormalities (Panels 3 and 4). In summary Figure 29C panel 5 reveals that, 70% of CM in *Lmna*^{*Flx*/}*^{Flx:mcm} Sun1*^{+/+}+Tmx mice had NE ruptures/distortions or misshapen nuclei compared to less than 1% of CM nuclei in *Lmna^{FlxlFlx:mcm} Sun1*^{-/-}+Tmx mice. (**Fig. 29D**) Echo analyses on TMX-treated and control mice were performed following Tmx induction. Echocardiograms (ECGs) performed at 28 days after Tmx injection on 3-5 month old mice (panel 1). ECGs performed before and after Cre induction revealed a progressive worsening of cardiac contractility in *Lmna*^{*Flx*/}*^{Flx:mcm} Sun1*^{+/+} +Tmx mice (solid black line) compared to *Lmna^{FlxlFlx:mcm} Sun1*^{-/-} + Tmx mice (panels 2-4). The loss of SUN1 preserved EF (panel 2), FS (panel 3) and Global Longitudinal Strain (GLS, panel 4) in *Lmna^{FlxlFlx:mcm} Sun1*^{-/-} + Tmx mice compared to *Lmna*^{*Flx*/}*^{Flx:mcm} Sun1*^{+/+} +Tmx mice.

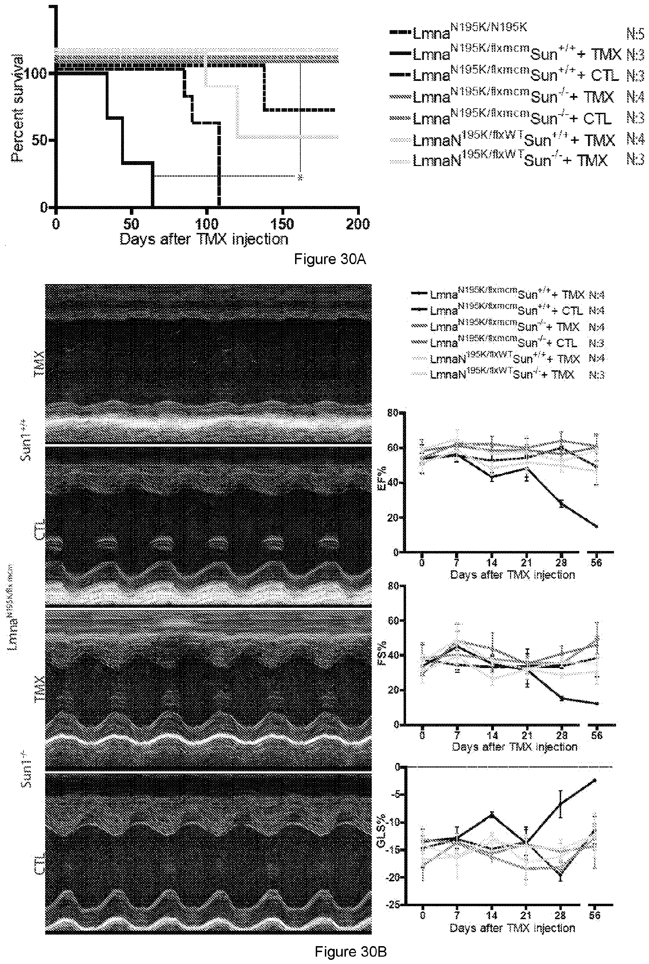

**Figures 30A-30B** show Kaplan Meier graph and heart function effects of deletion of SUN1 on cardiac pathology induced by a missense mutation in the *Lmna* gene (N195K). (**Fig. 30A**) The absence of Sun1 significantly increases the lifespan of *Lmna*^{*N195K*/}*^{Flx:mcm}Sun1*^{-/-}+ Tmx mice compared *to Lmna*^{*N195K*/}*^{Flx:mcm}Sun1*^{+/+} + Tmx mice (*P=0.0101; Log-rank test). Mice with only one copy of the N195K mutation (*Lmna*^{*N195K*/}*^{-:mcm}Sun1*^{+/+} + Tmx) had an average lifespan of 47 days, approximately half the lifespan of mice homozygous i.e. with two copies of the N195K allele. (**Fig. 30B**) Echocardiograms (ECGs) performed before and after Cre induction revealed progressive worsening of cardiac contractility in *Lmna*^{*N195K*/}*^{-:mcm}Sun1*^{+/+} +Tmx mice compared to *Lmna*^{*N195K*/}*^{-:mcm}Sun1*^{-/-} + Tmx mice over time. ECGs images were recorded at 28 days after Tmx injection (left-hand side panels). The loss of SUN1 preserved EF, FS and GLS in *Lmna*^{*N195K*/}*^{Flx:mcm} Sun1*^{-/-} + Tmx mice compared to *Lmna*^{*N195K*/}*^{Flx:mcm} Sun1*^{+/+} +Tmx mice (right-hand side bottom 3 panels).

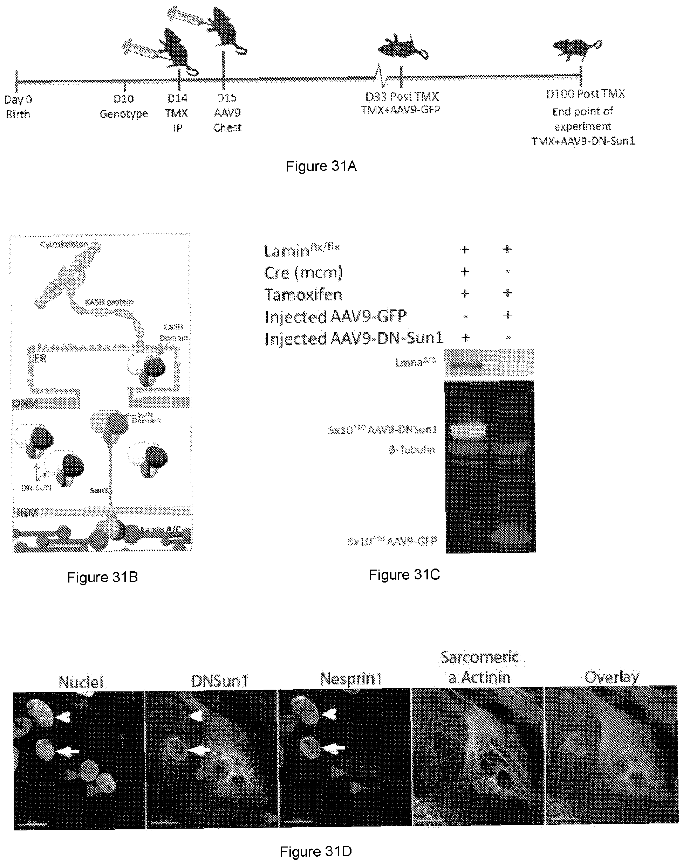

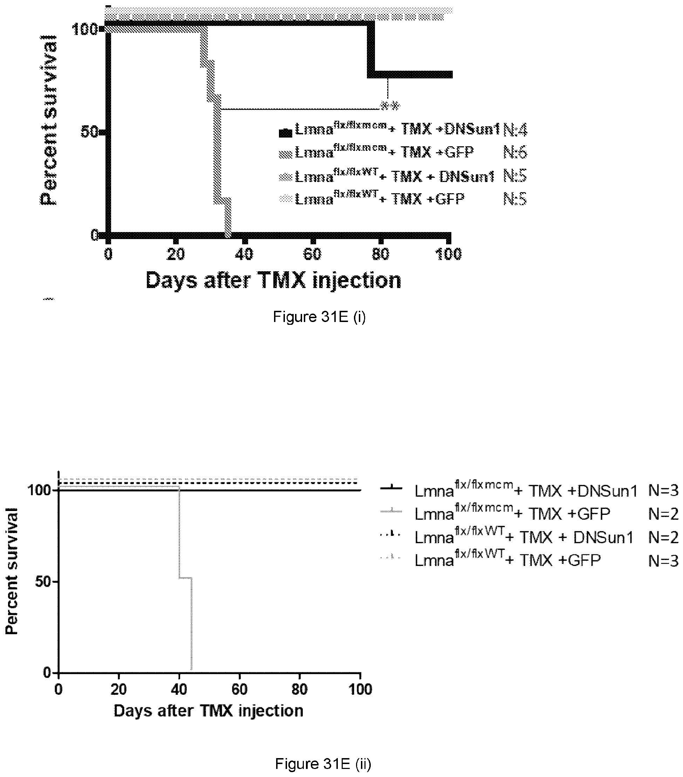

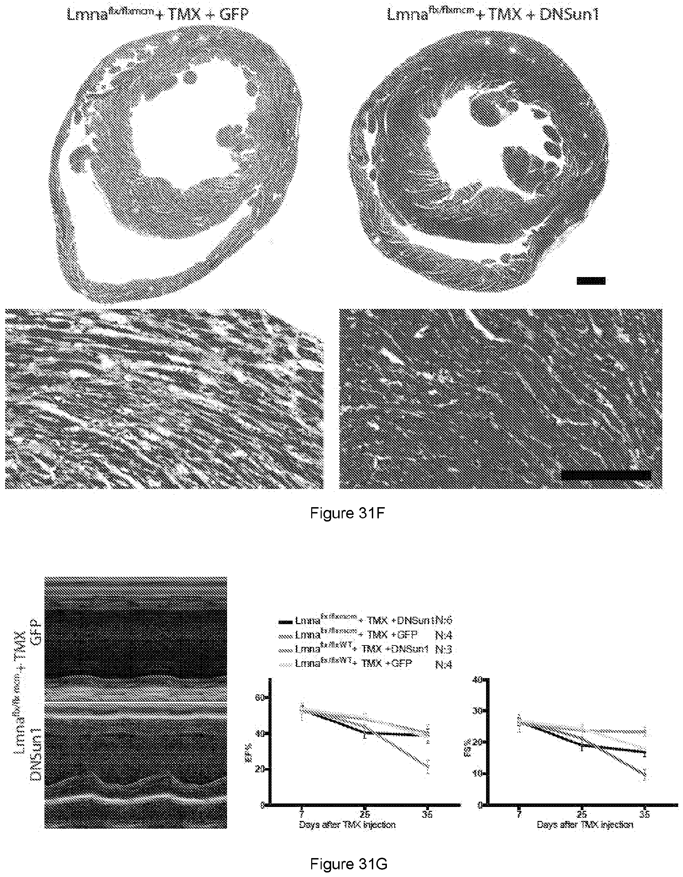

**Figures 31A-31G** show *Lmna*^{*Flxx*/*Flx:mcm*}+Tmx mice expressing an AAV transduced DNSun1 exhibit improved cardiac function and increased longevity. (**Fig. 31A**) Protocol for AAV-mediated transduction of the DN-Sun1 miniprotein into *Lmna*^{*Flxx*/*Flx:mcm*} +Tmx mice. A single Tmx (IP) injection is given at D14 postnatally to induce *Lmna* deletion. AAV9-DNSun1 or AAV9-GFP viral particles are then injected into the chest cavity on D15 postnatally. The experimental endpoint was set at 100 days after Tmx. (**Fig. 31B**) The DNSun1 miniprotein competes with endogenous Sun1 for binding to the KASH domain of the Nesprins (in CMs this is Nesprin1). The miniprotein competes with endogenous SUN1 in binding to the KASH domain of the Nesprins. As the DNSun1 miniprotein is not anchored in the INM this effectively disconnects the endogenous SUN proteins from binding to the KASH domains so breaking the LINC. (**Fig. 31C**) The presence of the recombined *Lmna* gene following Tmx injection was confirmed by PCR of the heart tissues (upper panel). Robust expression of both AAV9-DNSun1 and AAV9-GFP protein (Dosage: 5x10^10vg/g of mouse) was detected in extracts from whole hearts 99 days post AAV injection (lower panel). (**Fig. 31D**) CMs derived from human iPS stem cells were transduced with the DNSun1 using AVV-DJ as the vector. In CMs expressing high levels of DNSun1, indicated by grey arrows, Nesprin1 localization to the NE is reduced or absent. Nesprin1 localization to the NE is maintained in CMs either not expressing the AVV-DJ-DNSun1 or when expressed at lower levels (white arrow heads) . (**Fig. 31E**) The *Lmna*^{*Flx*/*Flx:mcm*}+Tmx+AAV9-GFP mice lived for an average of 34.5d after Tmx induction, whereas *Lmna^{FlxlFlx:mcm}* +Tmx mice injected with AA9-DNSun1 (5x10^10vg/g/mouse) lived significantly longer (**P=0.0038; Log-rank test) to at least 100D post Tmx, after which the mice were sacrificed for analysis. This set of data was derived from that shown in Fig. 20, adjusted by removing mice that were female and those with a different dose of virus. Fig. E(i) represents male mice and Fig. E(ii) represents female mice. (**Fig. 31F**) At 35d after Tmx, extensive fibrosis (blue in original image, grey here) and ventricular enlargement was detected in *Lmna*^{*F*/*x*/*Flx:mcm*}+Tmx+AAV9-GFP hearts compared to *Lmna*^{*Flx*/*Flx:mcm*} +Tmx + AAV9-DNSun1 hearts. (**Fig. 31G**) ECG analysis confirmed *Lmna^{FlxlFlx:mcm}* +Tmx+AAV9-DNSun1 hearts had better cardiac function compared to the *Lmna^{FlxlFlx:mcm}* +Tmx +AAV9-GFP hearts at 35d days after Tmx injection.

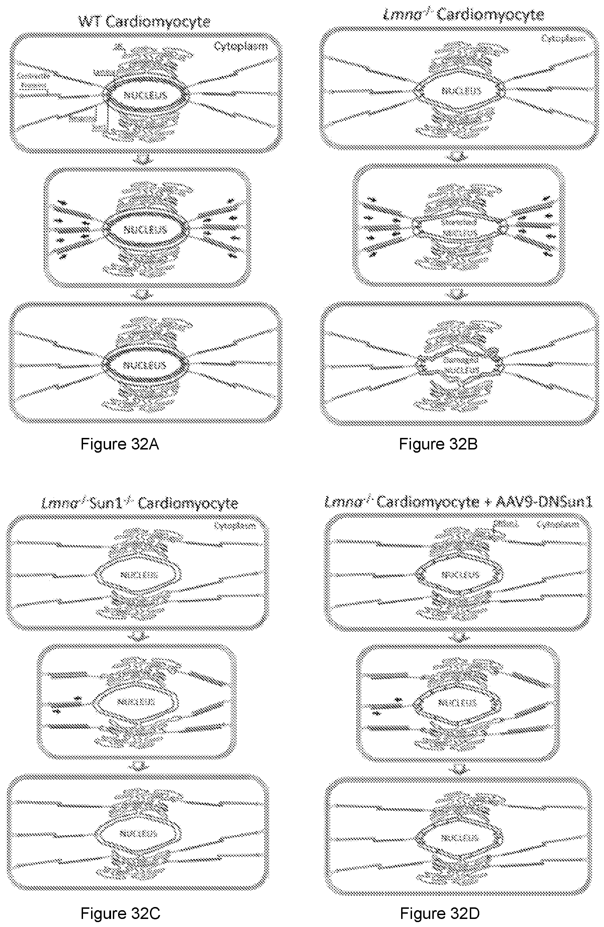

**Figures 32A-32D** shows models of how breaking the LINC by disrupting Sun1 protects cardiomyocytes from contraction induced stress (**Fig. 32A**) Cardiomyocyte nuclei expressing LmnaA/C, are able to withstand mechanical stress and tension forces transmitted via the LINC complex from the cytoplasm to the NE. (**Fig. 32B**) The loss of or introduction of a mutation within the *Lmna* gene results in loss/or incorrect assembly of the nuclear lamina, which weakens the Lamina/NE. The weakened nuclei are damaged due to the tension/stress forces exerted via the LINC complex from the contractile sarcomeres of the cardiomyocytes. (**Figs. 32C****, D**). In the absence of SUN1 or by disrupting its binding to the KASH domains by expression of DNSun1, the untethered LINC complexes exert less tensional force on the cardiomyocyte nuclei, enabling survival of the *Lmna* mutant cardiomyocytes.

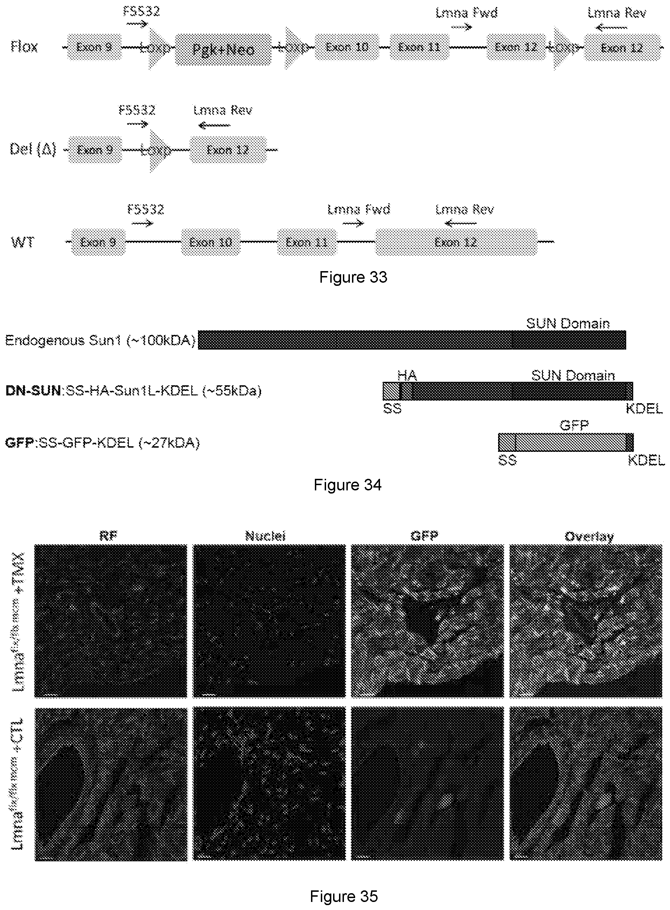

**Figure 33****:** shows the structure of the *Lmna*^{Flx/Flx} conditional allele. Primer locations for genotyping the *Lmna* gene both before and after Cre recombination are indicated for the *Lmna*^{Flx} allele (Flox), the *Lmna* deleted allele (Δ) and the wildtype allele [A. S. Wang, et al., Differentiation 89: 11-21 (2015)].
**Figure 34** shows a diagram of the recombinant AAV9-DNSun1 and AAV9-GFP miniproteins. The DN-Sun1 includes the Sun domain, an HA tag, a Signal Sequence (SS, for targeting the protein to the ER), and the KDEL (ER retention signal) [M. Crisp et al., J Cell Biol. 172: 41-53 (2006)]. The AAV9-GFP includes the SS and KDEL sequences. GFP was used as a control in place of the Sun1L-KDEL.
**Figure 35** shows photomicrographs of cardiomyocyte specific expression of Cre recombinase after Tmx injection. The *Lmna*^{*Flx*/*Flx:mcm*} mice were crossed with the mT/mG (JAX:
   Gt(ROSA)26Sortm4(ACTB-tdTomato,-EGFP)Luo/J) reporter mice. In the absence of Cre, RFP is expressed. When Cre is induced, GFP is expressed. Only CMs of *Lmna*^{*Flx*//*Flx:mcm*} mice express GFP upon TMX injection. Heart tissues were analyzed 7 days after Tmx injection.

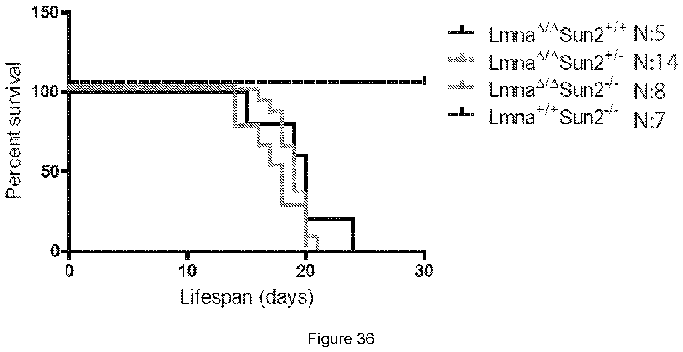

**Figures 36** shows that loss of Sun2 does not rescue loss of *Lmna.* Loss of Sun2 does not extend the lifespan of *Lmna*^{*Δ*/}*^{Δ}Sun2*^{*-*/*-*} mice.

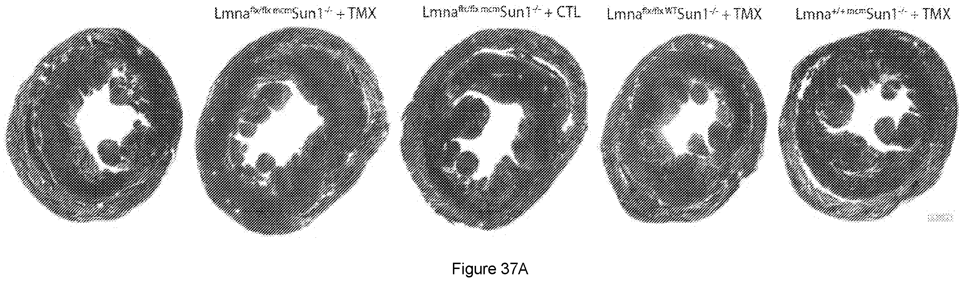

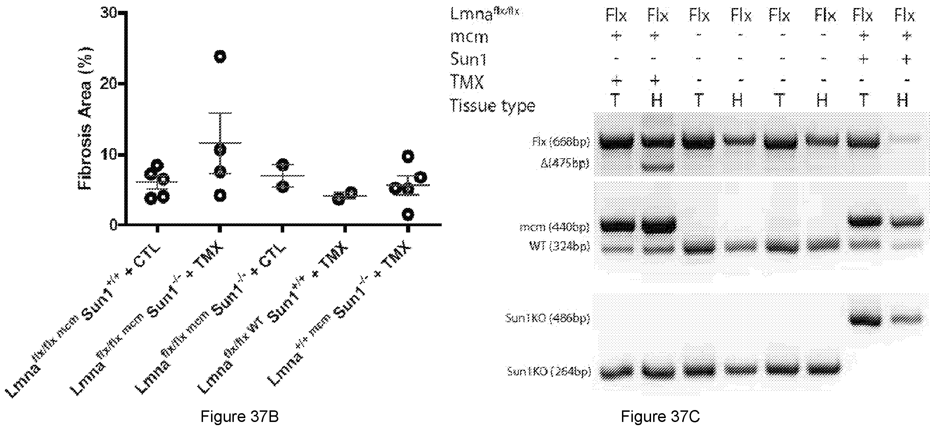

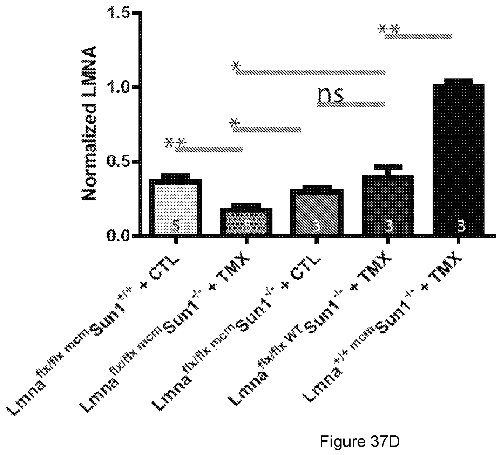

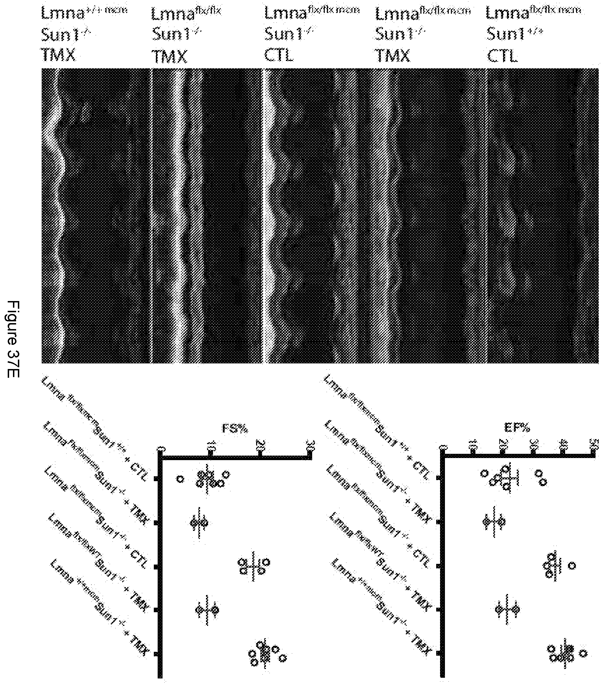

**Figures 37A-37E** show the phenotypes of *Lmna^{FlxlFlxmcm} Sun1*^{+/+} and *Lmna^{FlxlFlxmcm} Sun1*^{*-*/*-*} hearts at 12-14 months after Tmx injection. (**Fig. 37A**) Histological analysis of the aged *Lmna*^{*Flx*/*Flx*/}*^{:mcm}Sun1*^{+/+} hearts, 12-14 months after the Tmx injection, revealing no significant morphological changes e.g. LV enlargement or (**Fig. 37B**) in fibrosis compared to the controls. (**Fig. 37C**) PCR analysis confirmed the sustained deletion of *Lmna* gene. (**Fig. 37D**) Protein quantification revealed a significant reduction of LMNA levels in *Lmna^{FlxlFlx:mcm}Sun*^{*1-*/*-*}+Tmx hearts at 14months after TMX. (**Fig. 37E**) Echocardiograms (left-hand side panel) from the aged mice showed reduced EF and FS (right-hand side panels) in both *Lmna*^{*Flx*/*Flx:mcm*}Sun1^{+/+} +CTL and *Lmna^{FlxlFlx:mcm}Sun1*^{*-*/*-*} + Tmx aged mice.

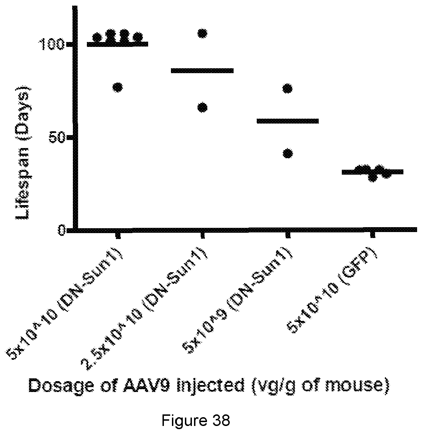

**Figure 38** shows the rescue by AAV9-DNSun1 depends on the dosage of viral particles injected. Lifespan of *Lmna*^{*Flx*/*Flx:mcm*} +TMX mice depends of the dosage of AAV9-DNSun1 with, with a lower concentrations resulting in shorter lifespans. Each dot represents a mouse, horizontal lines indicate mean.

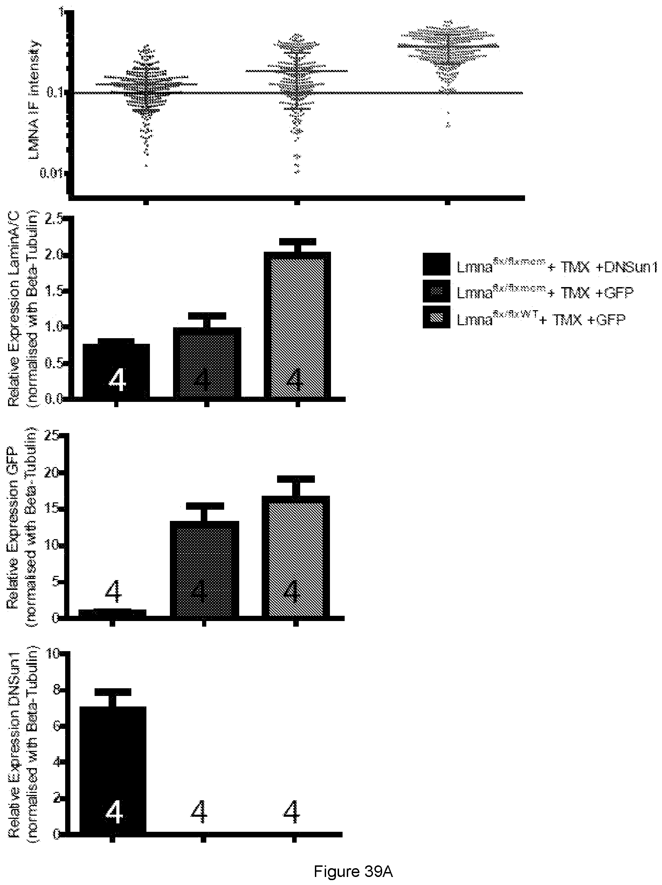

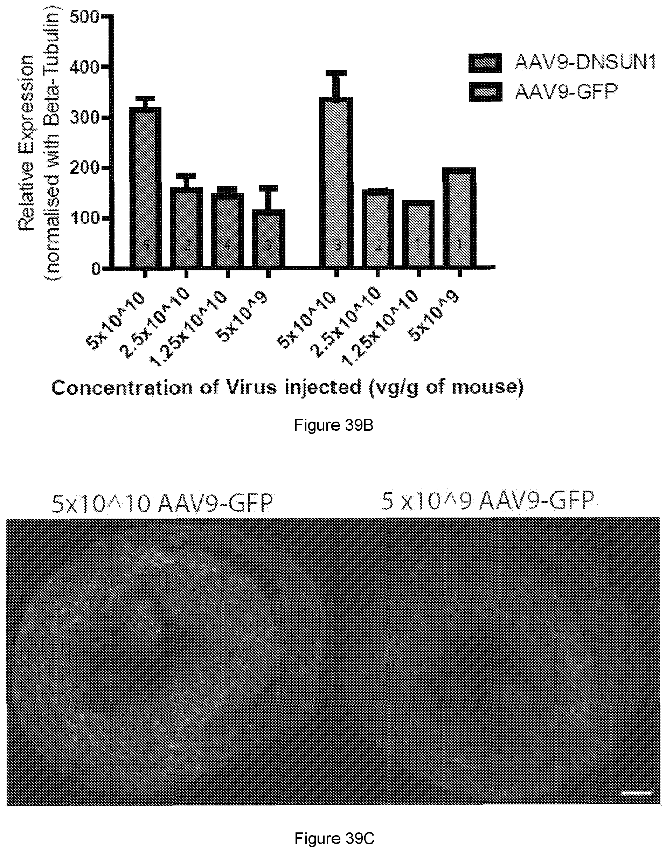

**Figures 39A-39C** show levels of LaminA/C following Tmx induction and expression of AAV-expressed proteins. (**Fig. 39A**) LaminA/C levels were significantly reduced following Tmx induction, and the presence of either AAV9-DNSun1 or AAV9-GFP protein did not alter LMNA protein levels (Quantification of LaminA/C immunofluorescence intensity). The amount of LaminA/C, DNSun1 and GFP protein in whole hearts were also quantified by Western analysis (lower 3 graphs). (Analysis performed 35 days after Tmx). (**Fig. 39B**) The expression of both DNSun1 and GFP proteins were dependent on the concentration of viral particles injected. (**Fig. 39C**) Immunofluorescence revealed the majority of CMs were successfully infected and expressed GFP with 5×10^10vg/g of AAV9-GFP (left image) compared to infection with a 10-fold lower (5×10^9 AAV9-GFP, right image) concentration of viral particles.

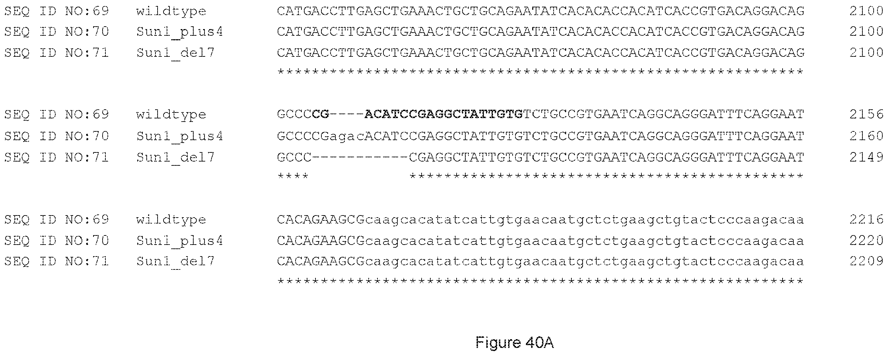

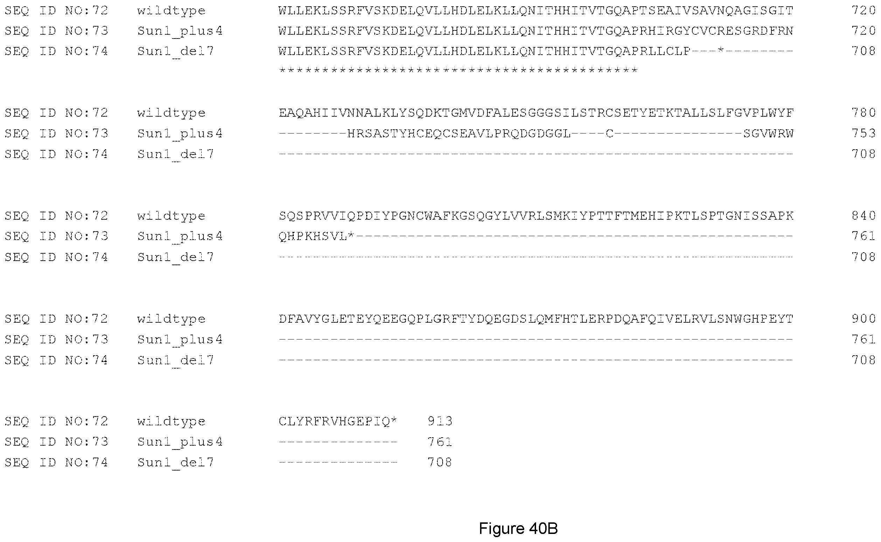

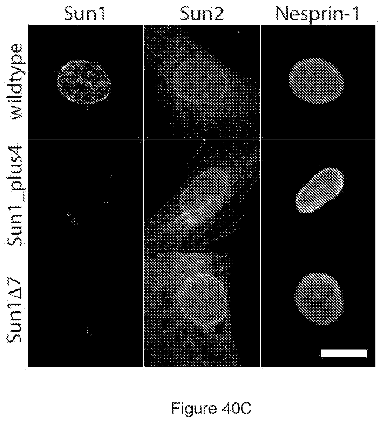

**Figures 40A-40C** show CRISPR targeting of Sun1 SUN domain results in loss of Sun1 protein. Clustal alignment of Sun1 DNA (**Fig. 40A**) and amino acid (**Fig. 40B**) sequence (SEQ ID NOs:69 and 72, respectively) adjacent to CRISPR-induced mutation in wildtype Sun1, Sun1 with 4 bp insertion (Sun1_plus4; SEQ ID NOs:70 and 73, respectively) and Sun1 with 7 bp deletion (Sun1_del7; SEQ ID NOs:71 and 74, respectively). Numbering is of Sun1 coding sequence (A) and protein sequence (B). Bold letters in (B) indicate SUN domain. (**Fig. 40C**) Immunofluorescence staining of mouse adult fibroblasts derived from wildtype and Sun1 mutant mice. Sun1 expression is lost in mutant mice, but Sun2 and Nesprin-1 expression is similar in all 3 genotypes. Scale bar = 10 µm.

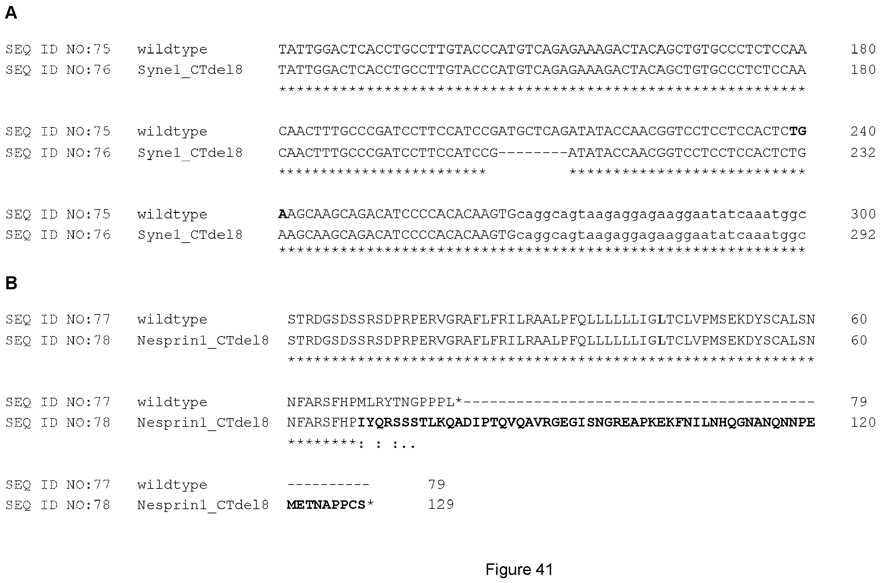

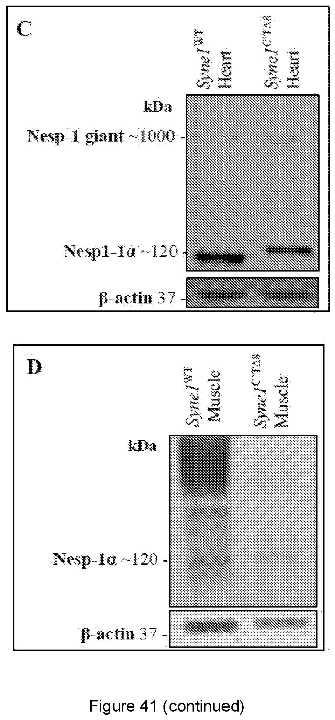

**Figures 41A-41D** shows CRISPR targeting of Syne1 C-terminus results in expression of a mutant Nesprin-1 protein. (**Fig. 41A, Fig. 41B**) Clustal alignment of wildtype Nesprin-1 DNA (SEQ ID NO; 75) and Nesprin-1C'TΔ8 (Nesprin1_CTdel8) (SEQ ID NO:76) (**Fig. 41A**) and amino acid sequence adjacent to CRISPR-induced mutation in wildtype Nesprin-1 (SEQ ID NO:77) and Nesprin-1C'TΔ8 (Nesprin1_CTdel8) (SEQ ID NO:78) (**Fig. 41B**). TGA in bold indicates stop codon of Syne1 / Nesprin-1 gene. (**Fig. 41C, Fig. 41D**) Immunoblots of Nesprin-1 from Syne1 / Nesprin-1 wildtype and Syne1 / Nesprin-1C'TΔ8 mutant heart and muscle tissue.

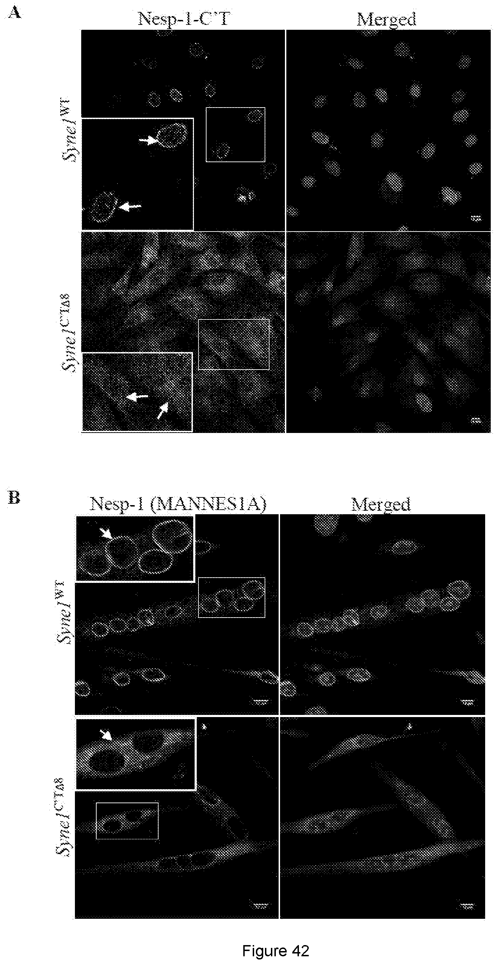

**Figures 42A-42B** are photomicrographs showing CRISPR-induced Syne1 mutation results in mislocalized, "KASH-less" Nesprin-1 protein. Immunofluorescence staining of mouse adult fibroblasts **(****Fig. 42A****)** and primary myotubes (**Fig. 42B**) derived from wildtype (WT) and Syne1C'TΔ8 mutant mice. Nesprin-1 is mislocalized from the nuclear envelope in the mutant samples. Merged images shows Nesprin-1 and DNA staining. Scale bar = 10 µm.

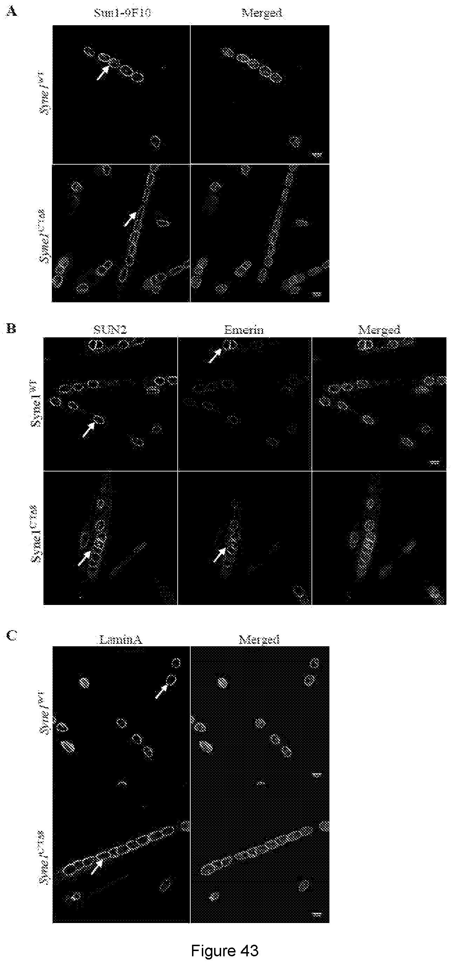

**Figures 43A-43C** are photomicrographs showing Syne1 mutation does not disrupt localization of certain nuclear envelope proteins. Immunofluorescence staining of mouse primary myotubes derived from wildtype (WT) and Syne1C'TΔ8 mutant mice. Sun1 (**Fig. 43A**), Sun2 and emerin (**Fig. 43B**) and lamin A/C (**Fig. 43A**) localize normally to the nuclear envelope. Merged images show protein and DNA staining. Arrows indicate examples of normally localized nuclear envelope proteins. Scale bar = 10 µm.

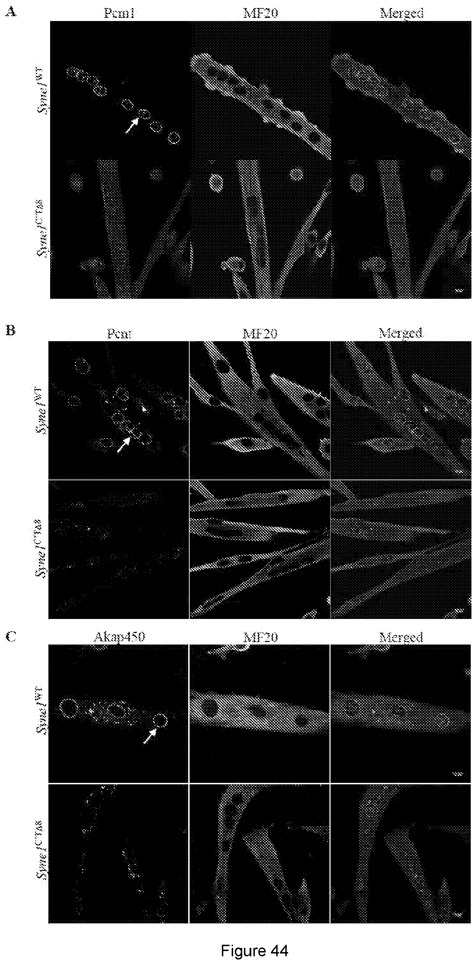

**Figures 44A-44C** are photomicrographs showing Syne1 mutation disrupts localization of nuclear-envelope-localized centrosomal proteins. (**Fig. 44A-44C**) Immunofluorescence staining of mouse primary myotubes derived from wildtype (WT) and Syne1C'TΔ8 mutant mice. Pcm1, Pericentrin (Pent), and Akap450, which normally localize to the nuclear envelope in myotubes, are displaced from the nuclear envelope in Syne1C'TΔ8 mutant myotubes. MF20 is an antibody for myosin heavy chain, a myotube marker. Merged images show protein and DNA staining. Arrows indicate typical nuclear envelope staining for these centrosomal proteins. Scale bar = 10 µm.

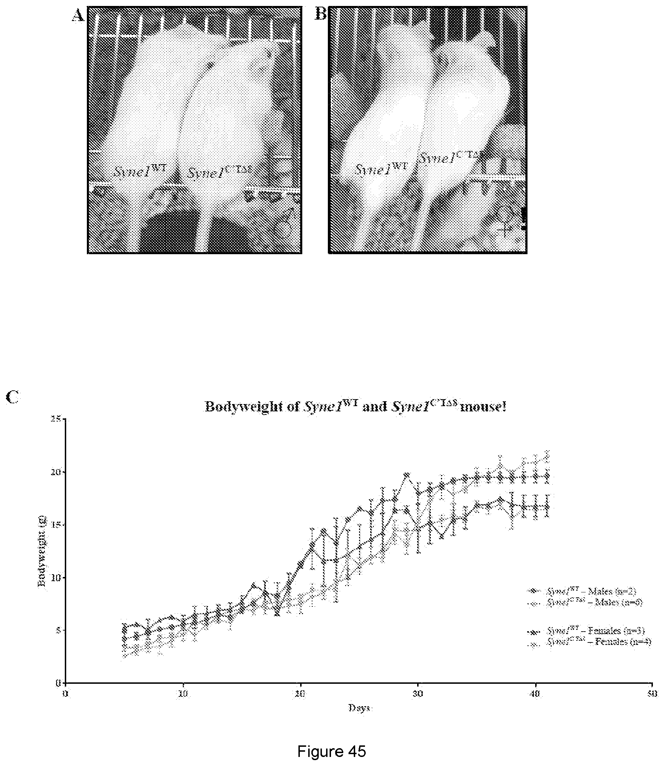

**Figure 45A-C** shows Syne1 mutation does not affect mouse phenotype. (A-B) Representative images of 12-week-old male (A) and female (B) mice. (C) Bodyweight of male and female, wildtype (WT) and Syne1C'TΔ8 mutant, mice over 6 weeks.

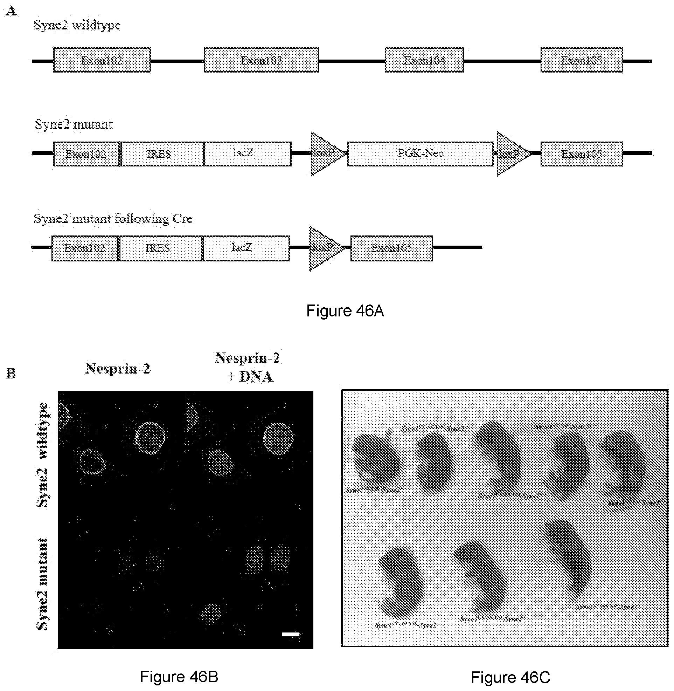

**Figures 46A-46C** show Syne2 constructs and Syne1/Syne2 double mutant mice experience perinatal lethality. (**Fig. 46A**) Design of IRES-βgal PGK-Neo targeting construct for generating Syne2 mutation. (**Fig. 46B**) Immunofluorescence staining of mouse adult fibroblasts derived from wildtype (WT) and Syne2 mutant mice showing loss of Nesprin-2. (**Fig. 46C**) Images of newborn pups. Top row are of mice with at least 1 wildtype Syne1 or Syne2 allele that appear a healthy pink. Bottom row shows cyanotic double mutant Syne1 ^{C'TΔ8/C'TA8}:Syne^{2-/-} pups which appear blue and die at birth.

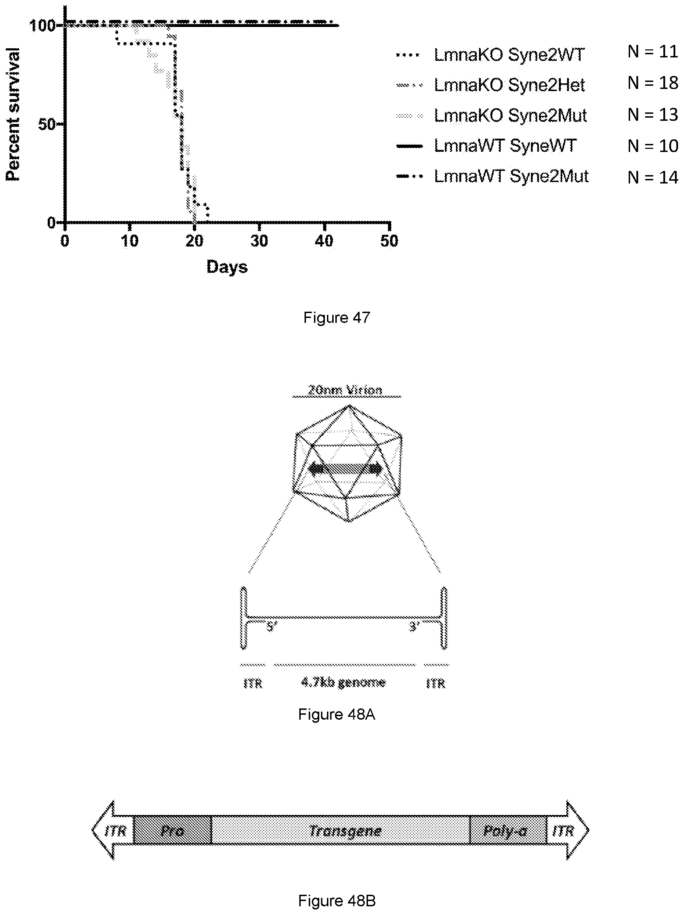

**Figure 47** is a Kaplan Meier graph showing a Syne2 mutation does not ameliorate Lmna pathology. Kaplan-Meier survival curve showing that regardless of their Syne2 mutation status (wildtype, heterozygous or mutant), Lmna^{Δ/Δ} mice die within 3 weeks of birth.
**Figures 48A and 48B****.** Schematic representation of an AAV viral capsid and its genomic payload (derived from Figures 1B and 1C of Lipinski et al., Prog Retin Eye Res. (2013) 32:22-47). (**Fig. 48A**) 20 nm icosahedral capsid of the AAV virion containing a single-stranded DNA AAV genome. (**Fig. 48B**) The genome can be engineered to comprise an expression cassette with a maximum size of 4.7 kb which are bounded by inverted terminal repeats (ITR). The transgene minimally comprises a promoter, a transgene and poly-a tail.

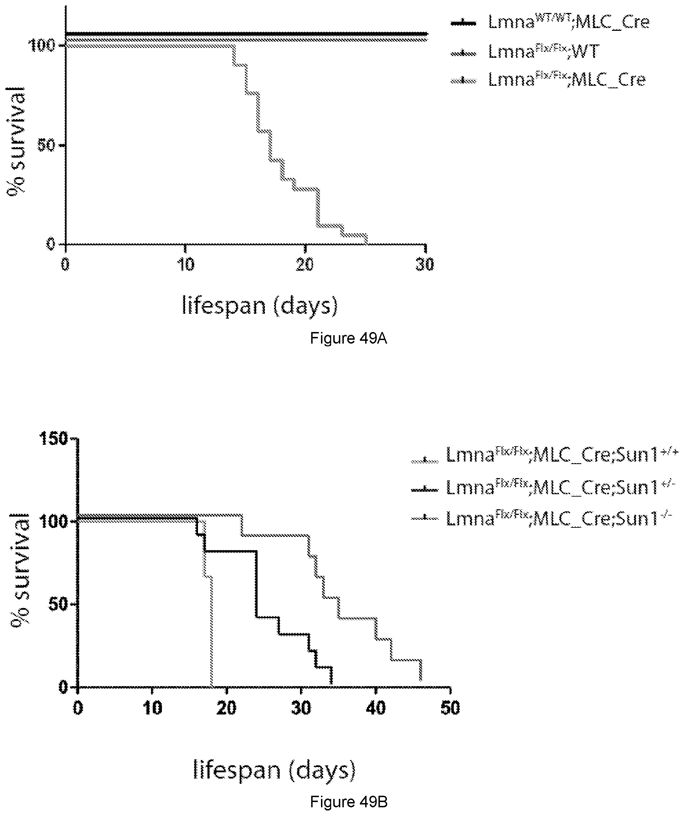

**Figures 49A and 49B****.** Lethality of skeletal muscle-specific knockout of *Lmna* is rescued by loss of Sun1. (**Fig. 49A**) Mice harbouring floxed alleles of *Lmna* and a transgene containing the myosin light chain regulatory sequence driving Cre recombinanse (*Lmna*^{*Flx*/}*^{Flx};MLC_Cre*, N = 21, survive an average of 18 days after birth. However, (**Fig. 49B**) the absence of one copy of *Sun1* in these mice extends their lifespan from 18 to ~24 days (*Lmna*^{*Flx*/}*^{Flx};MLC_Cre;Sun1^{+l-}*, N = 10), and loss of both copies of *Sun1* (*Lmna^{FlxlFlx};MLC_Cre;Sun*^{*1-*/*-*}, N = 8) doubles their lifespan from 18 to 35 days.

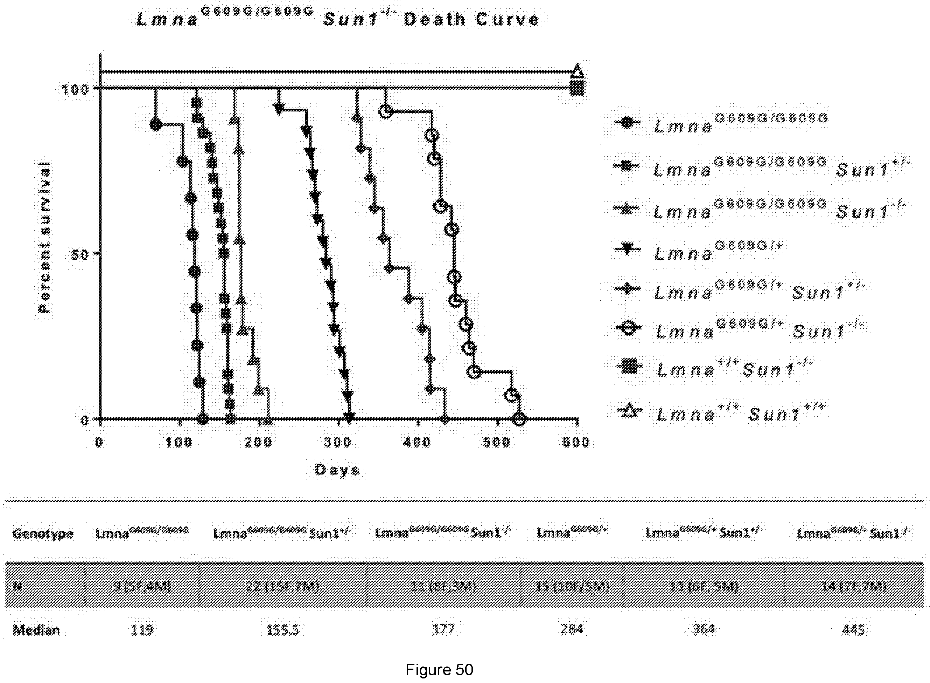

**Figure 50****.** Mortality of progerin-expressing mice is rescued by loss of Sun1. Mice homozygous for the *Lmna-G609G* progerin splice mutant allele (*Lmna*^{*G609G*/*G609G*}) have a median lifespan of 116 days, which increases to 152 days following loss of one copy of *Sun1* (*Lmna*^{*G609G-CR*/}*^{G609G-CR};Sun1*^{*+*/*-*}) and to 174 days with loss of both copies of *Sun1* (*Lmna*^{*G609G-CR*/}*^{G609G-CR};Sun1*^{*-*/*-*}). Mice heterozygous for the *Lmna-G609G* progerin splice mutant allele (*Lmna*^{*G609G-CR*/+}) live for a median of 290 days, and loss of Sun1 (*Lmna*^{*G609G-CR*/+}*;Sun1*^{*-*/}*⁻)* extends lifespan to more than 1 year. Total number (N) of mice and number of mice for each sex (M - Male, F - Female) for 4 of the genotypes are at the bottom of the figure.

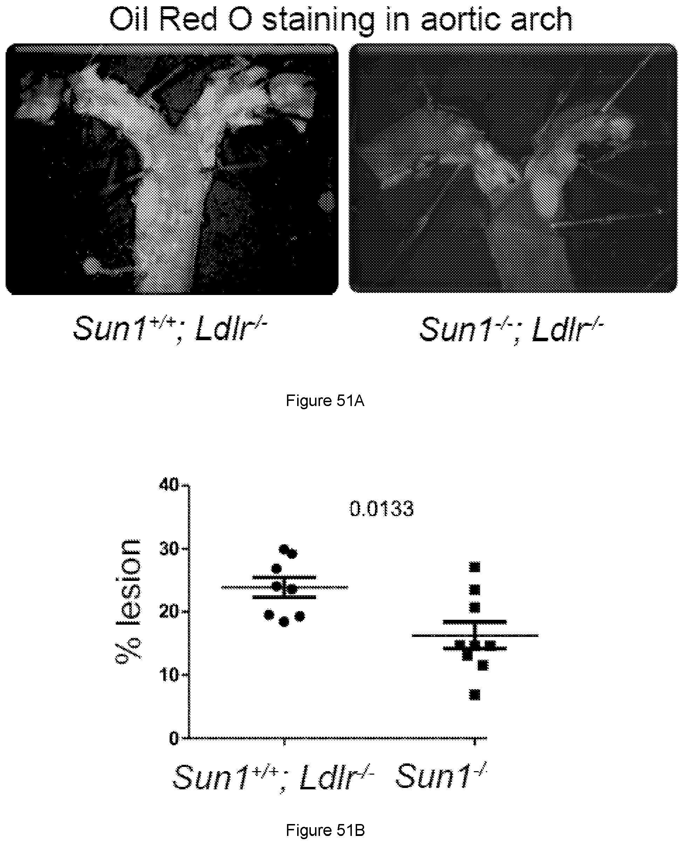

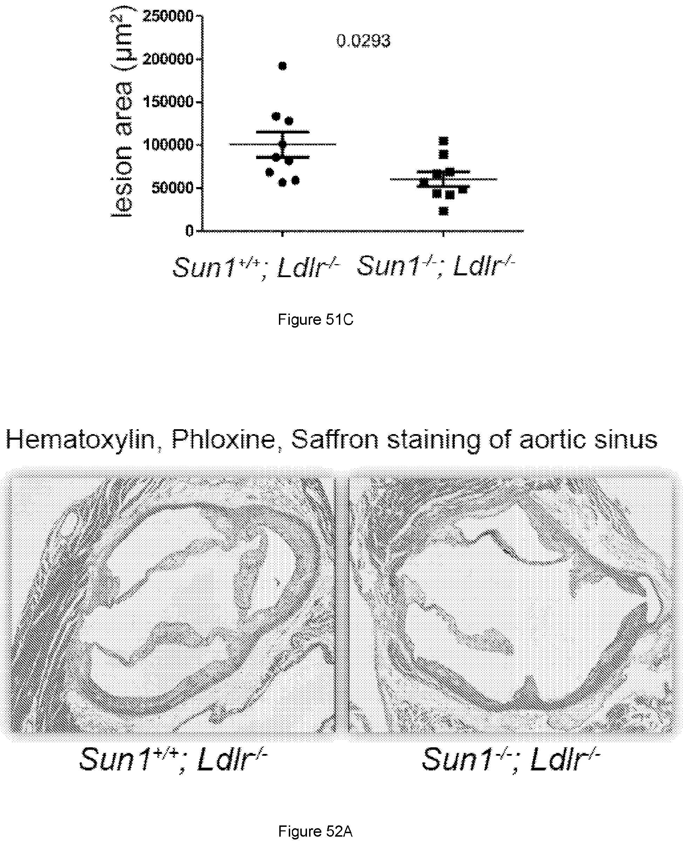

**Figures 51A to 51C****.** Loss of Sun1 reduces atherosclerotic lesion area in the aortic arch. To assess atherosclerosis in mice with deleted *Sun1,* wild-type (*Sun1*^{+/+}) and *Sun1*^{-/-} mice were generated on the atherogenic *Ldlr*^{*-*/*-*} background. Mice were fed a Western-type diet for 15 weeks. (**Fig. 51A**) Representative images of Oil Red O stained atherosclerotic lesions in the aortic arch of *Sun1*^{+/+}*;Ldlr*^{*-*/*-*}and *Sun1*^{*-*/}*⁻;Ldlr*^{*-*/*-*} mice. (**Fig. 51B**) Significantly decreased lesion area was observed upon quantification of area occupied by atherosclerotic lesions, shown as percentage of total surface area and (**Fig. 51C**) shown as the actual lesion area in square microns. N = 8-9 each.

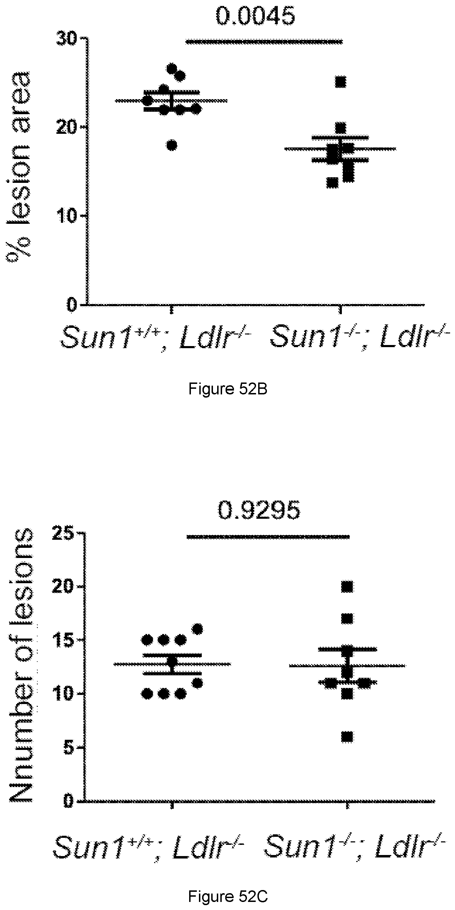

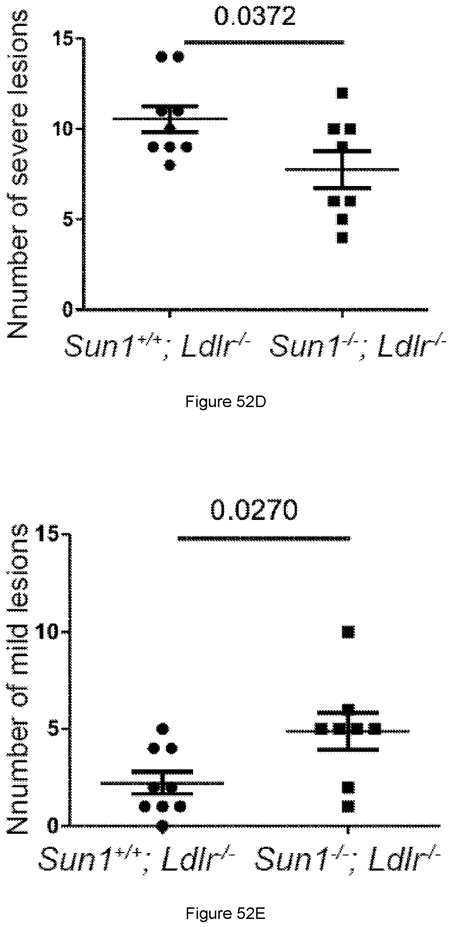

**Figures 52A to 52E****.** Loss of Sun1 significantly reduces atherosclerotic lesion area in the aortic sinus. Atherogenic *Ldlr*^{*-*/*-*} mice either wildtype (*Sun1*^{+/+}*;Ldlr*^{*-*/*-*}) or mutant for Sun1 (*Sun1*^{+/+}*;Ldlr*^{*-*/*-*}) were fed with a Western-type diet for 15 weeks. (**Fig. 52A**) Representative images of Hematoxylin, Phloxine, Saffron (HPS) staining of the aortic sinus. (**Fig. 52B**) Significantly decreased atherosclerotic lesion area, and (**Fig. 52C**) unchanged total lesion number, resulting from (**Fig. 52D**), significantly decreased number of severe lesions, and (**Fig. 52E**) significantly increased number of mild lesions, as classified by American Heart Association criteria, were observed in the absence of Sun1 in the *Sun1*^{-/-}; *Ldlr*^{*-*/*-*} mice.

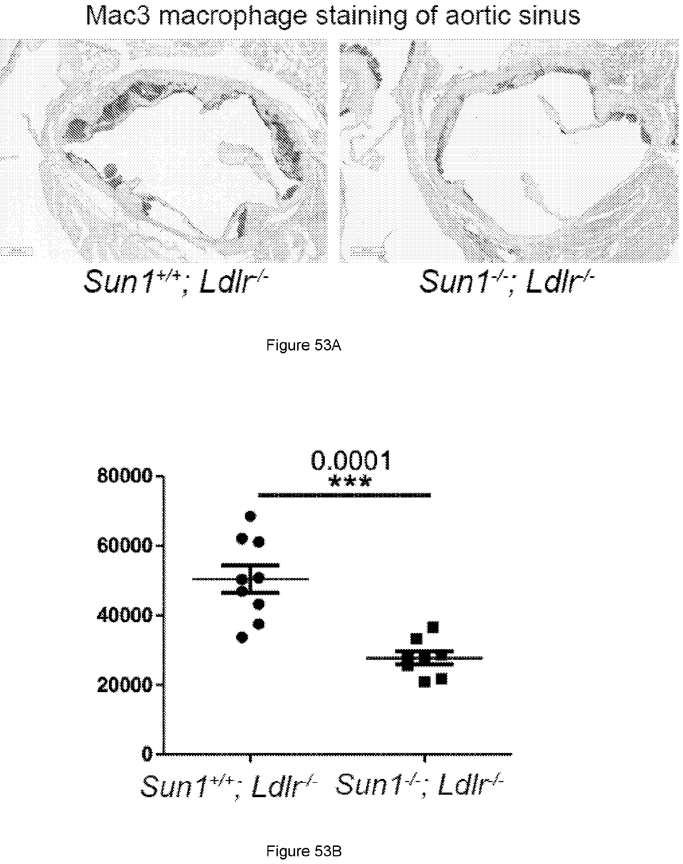

**Figures 53A and 53B****.** Loss of Sun1 significantly reduces lesional macrophage area in the aortic sinus. Mice mutant for the low density lipoprotein receptor gene and either wildtype (*Sun1*^{+/+}*;Ldlr*^{*-*/*-*}) or mutant for Sun1 (*Sun1*^{+/+}*;Ldlr*^{*-*/*-*}) were fed with a Western-type diet for 15 weeks. (**Fig. 53A**) Representative images of the aortic sinus stained with an antibody to the macrophage marker Mac-3, and (**Fig. 53B**) significantly reduced macrophage positive area in the *Sun1*^{*-*/}*⁻;Ldlr*^{*-*/*-*} mice.

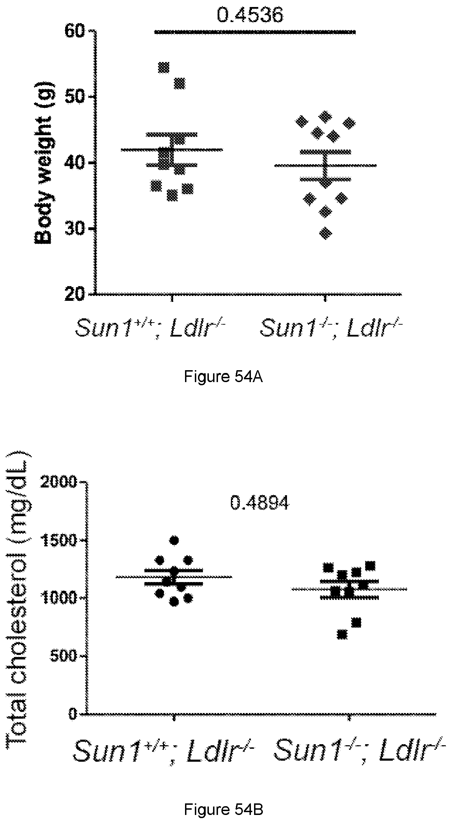

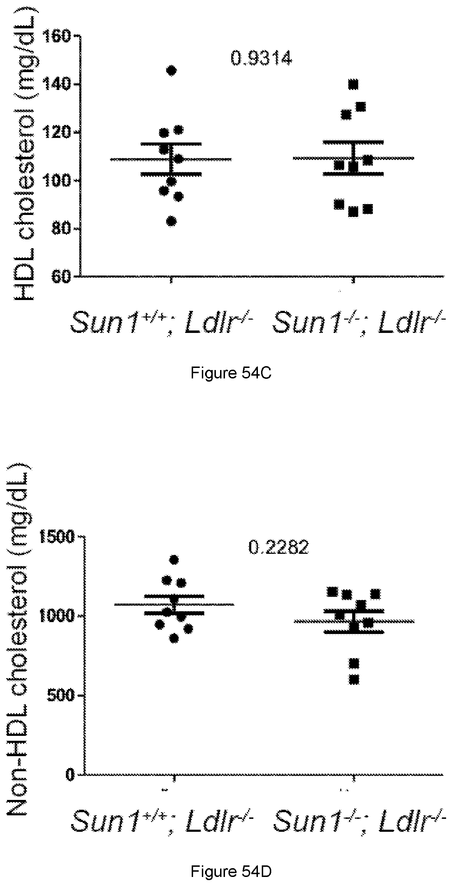

**Figures 54A to 54D****.** Loss of Sun1 in an accelerated atherosclerosis model does not affect body weight or lipid levels. Mice mutant for the low density lipoprotein receptor gene and either wildtype (*Sun1*^{+/+}*;Ldlr*^{*-*/*-*}) or mutant for Sun1 (*Sun1*^{*-*/}*⁻,Ldlr*^{*-*/*-*}) were fed with a Western-type diet for 15 weeks. (**Fig. 54A**) No changes in body weights of the mice were found. (**Fig. 54B**) Plasma levels of total cholesterol, high density lipoprotein (HDL) cholesterol (**Fig. 54C**), and non-HDL cholesterol (**Fig. 54D**) were unchanged in the *Sun1*^{*-*/}*⁻;Ldlr*^{*-*/*-*} mice.

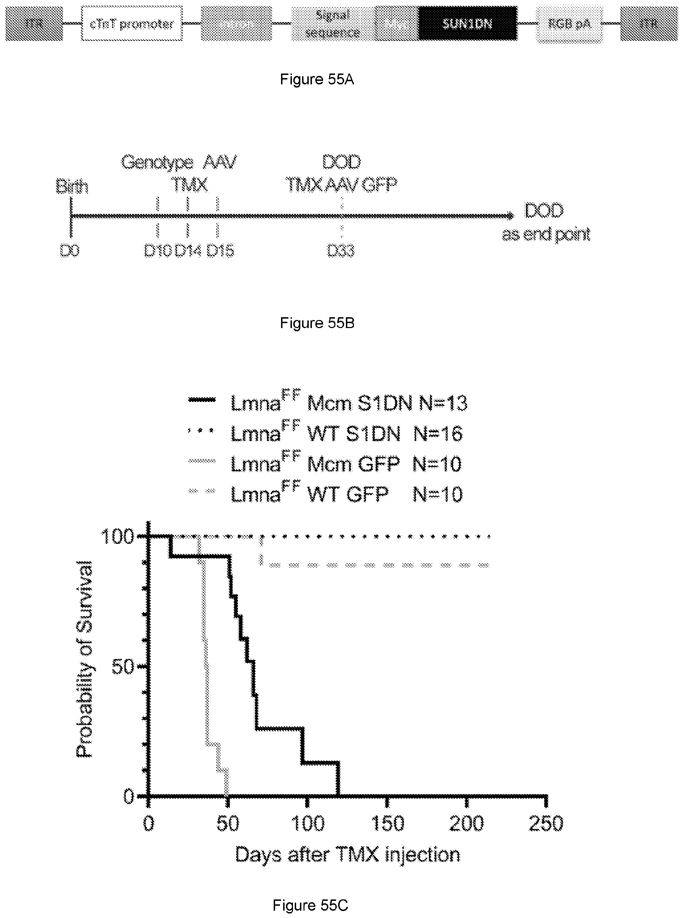

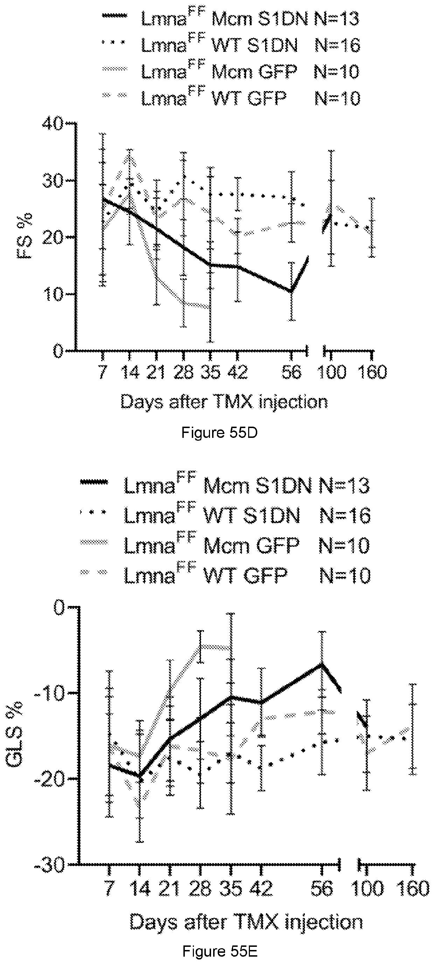

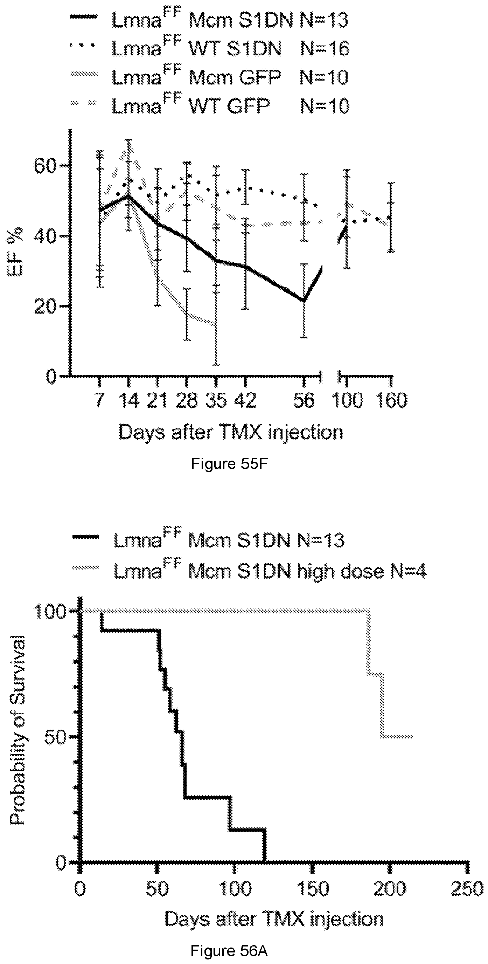

**Figures 55A to 55F.** Delivery of human Sun1 dominant-negative construct via AAV9 improves the cardiac function and life span in the *Lmna*^{*Flx*/}*^{Flx} Mem* mouse model of DCM. (**Fig.55A**) Schematic of the AAV9 construct encoding dominant-negative human Sun1 (AAV9-huSUN1DN). The dominant negative SUN1 sequence encompasses parts of the lumenal domain including the Sun domain. A MYC-tag sequence is fused to the NH2 terminus. ITR = AAV2 inverted terminal repeat; cTNT = Chicken cardiac troponin promotor; intron =β-globin/IgG chimeric intron; Signal sequence = 1-25 aa of human serum albumin (Uniprot P02768 signal peptide + propeptide); Myc = Myc epitope tag; SUN1DN = 1046-2404 nt of NM 001130965; KDEL = Golgi-to-ER retrieval sequence; RGB pA = Rabbit globin polyA tail. (**Fig. 55B**) Schematic of experimental set-up. Tamoxifen (TMX) was injected intra-peritoneally at postnatal day 14, followed on day 15 by retro-orbital injection of AAV9 huSUN1DN or AAV9 GFP as control (AAV). The expected lifespan of *Lmna*^{*Flx*/}*^{Flx} Mem* + TMX injected with AAV9 GFP is 33 days after TMX injection. The end point of this study is date of death of the *Lmna*^{*Flx*/}*^{Flx} Mem* animals (DOD). (**Fig. 55C**) Transduction of AAV9 huSUN1DN (S1 DN) extends the lifespan of *Lmna*^{*Flx*/}*^{Flx} Mem* + TMX animals. *Lmna*^{*Flx*/}*^{Flx} Mem* + TMX + huSUN1DN mice live on average 66 days post TMX injection, whereas the *Lmna*^{*Flx*/}*^{Flx} Mem* + TMX + GFP have a shorter lifespan (36 days). (P < 0.0001) (**Figs. 55D to 55F**) Cardiac function after transduction with AAV9 huSUNIDN is improved in *Lmna*^{*Flx*/}*^{Flx} Mem* + TMX animals. Echocardiogram analysis showed an improvement of Fractional Shortening (FS; **Fig. 55D**), Global Longitudinal Strain (GLS; **Fig. 55E**) and Ejection Fraction (EF; **Fig. 55F**) in *Lmna*^{*Flx*/}*^{Flx} Mem* + TMX + huSUN1DN animals compared to *Lmna*^{*Flx*/}*^{Flx} Mem* + TMX + GFP control animals. (day 28: FS P < 0.002; GLS P < 0.0006; EF P < 0.0001).

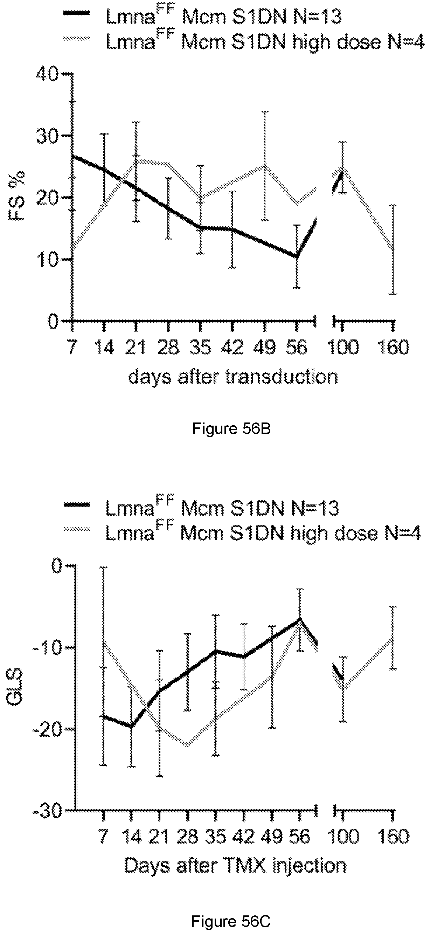

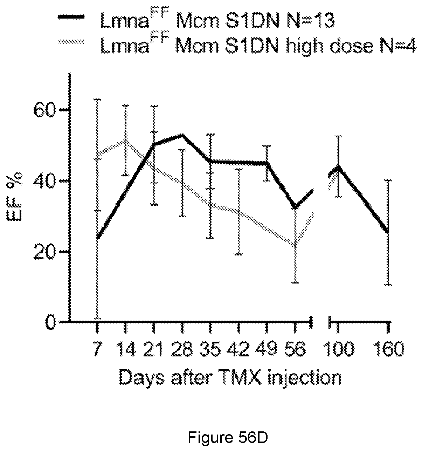

**Figures 56A to 56D****.** Delivery of human Sun1 dominant-negative construct via AAV9 improves the cardiac function and life span in the *Lmna*^{*Flx*/}*^{Flx} Mem* mouse model of DCM in a dose-dependent manner. (**Fig. 56A**) Transduction of a higher dose (5 × 10^10 viral genome/g bodyweight) of AAV9 huSUN1DN further prolongs the lifespan of *Lmna*^{*Flx*/}*^{Flx} Mem* + TMX animals. Lmnaflx/flx Mcm + TMX injected with a higher dose (5 × 10^10 viral genome/g bodyweight) of huSUN1DN live on average to day 205 post TMX injection, whereas the *Lmna*^{*Flx*/}*^{Flx} Mem* + TMX injected with the standard dose (2 × 10^10 viral genome/g bodyweight) huSUN1DN have a shorter lifespan (66 days). (P < 0.0001) (**Fig. 56B to 56D**) Cardiac function is improved in a dose-dependent manner. Echocardiogram analysis showed an improvement of Fractional Shortening (FS; **Fig. 56B**), Global Longitudinal Strain (GLS; **Fig. 56C**) and Ejection Fraction (EF; **Fig. 56D**) in *Lmna*^{*Flx*/}*^{Flx} Mem* + TMX + high dose of huSUN1DN animals compared to animals transduced with the standard dose.

### Examples

In the following examples, the inventors demonstrate that LINC complex disruption ameliorates the laminopathy associated with mutations to the gene encoding lamins A/C, including knockout mutations, missense mutations, and progerin-associated mutations. The inventors also show that LINC complex inhibition can ameliorate the symptoms of diseases characterised by hyperlipidemia. The inventors show that LINC complex disruption reduces the symptoms of atherosclerosis.

### Example 1: Materials and Methods

Mice were maintained at the A*STAR Biological Resource Centre facility and the NUS Animal Facility in accordance with the guidelines of the Institutional Animal Care and Use Committee for each facility. The *Lmna*^{*Flx*/*Flx*} mice were generated and characterized as previously described [A. S. Wang, et al., Differentiation; research in biological diversity, (2015); I. Solovei et al., Cell 152: 584-598 (2013)] (Figure 33). To derive mice with a global deletion *Lmna* (*Lmna*^{*Δ*/*Δ*}), we crossed the floxed allele (*Lmna*^{*Flx*/*Flx*}) to mice in which Cre recombinase is driven by the regulatory sequences of the mouse zona pellucida 3 gene (Zp3;Tg(Zp3-cre)93Knw, JAX stock 003651) [W. N. de Vries et al., Genesis 26: 110-112 (2000)]. To obtain cardiomyocyte-specific deletion of *Lmna* (*Lmna*^{*Flx*/*Flx*/*NIMhc*}), we first crossed the *Lmna*^{*Flx*/*Flx*} mice to mice in which Cre expression was driven by the cardiac-specific murine alpha myosin-heavy chain (Myh6, myosin, heavy polypeptide 6, cardiac muscle, alpha) promoter (MyHC;Tg(Myhca-cre)2182Mds, JAX stock 011038). To obtain a tamoxifen inducible cardiomyocyte-specific deletion of *Lmna* (*LmnaFlx*/*Flx:mcm*), we crossed the *Lmna*^{*Flx*/*Flx*} with mice in which Cre expression was driven by the mouse cardiac-specific alpha-myosin heavy chain promoter (αMHC or alpha-MHC;Myh6) that expressed a tamoxifen-inducible Cre recombinase (MerCreMer) specifically in juvenile and adult cardiac myocytes (mcm;Tg(Myh6-cre/Esr1*)1Jmk, JAX stock 005657). The specificity of mcm Cre expression to cardiomyocytes was confirmed by crossing Cre lines to the mT/mG reporter mice [M. D. Muzumdar, et al., Genesis 45: 593-605 (2007)] (Figure 35). Generation of the *Sun1*^{-/-}mice was previously described [Y. H. Chi et al., Development 136: 965-973 (2009)] as were the *Lmna*^{*N195K*/*N195K*}mice [L. C. Mounkes, et al., Hum Mol Genet 14: 2167-2180 (2005)]. The *Lmna*^{*Δ*/}*^{Δ}:Sunt1*^{*-*/*-*} and *Lmna*^{*Flx*/}*Flxmcm:Sun1^{-l-}* mice were obtained by crossing the respective Lamin-Cre mice strains with *Sun1*^{+/-} mice as *Sun1*^{-/-} mice are infertile.

To test for the insertion of IoxP sites and conditional deleted allele, genotyping was performed with a duplex PCR protocol with the following primers were used:

| | |
|---|---|
| FLX/FLX-F1: 5'-CCAGCTTACAGAGCACCGAGCT-3' | (SEQ ID NO:16) |
| FLX/FLX-F2: 5'-TCCTTGCAGTCCCTCTTGCATC-3' | (SEQ ID NO:17) |
| FLX/FLX-R1: 5'-AGGCACCATTGTCACAGGGTC-3' | (SEQ ID NO:18) |

To test for Sun1 deletion, the following primers were used:

| | |
|---|---|
| Sun1-F: 5'-GGC AAG TGG ATC TCT TGT GAA TTC TTG AC-3' | (SEQ ID NO:19) |
| Sun1-R: 5'-GTA GCA CCC ACC TTG GTG AGC TGG TAC-3' | (SEQ ID NO:20) |
| Sun1-E8: 5'-AGC CAC ATA ACC ACC TGG AG-3' | (SEQ ID NO:21) |

To test for the MyHC transgene, the following primers were used:

| | |
|---|---|
| MyHC-tF: 5'-ATG ACA GAC AGA TCC CTC CTA TCT CC-3' | (SEQ ID NO:22) |
| MyHC-tR: 5'-CTC ATC ACT CGT TGC ATC ATC GAC-3' | (SEQ ID NO:23) |
| MyHC-F: 5'-CAA ATG TTG CTT GTC TGG TG-3' | (SEQ ID NO:24) |
| MyHC-R: 5'-GTC AGT CGA GTG CAC AGT TT-3' | (SEQ ID NO:25) |

To test for the presence of mcm transgene, the following primers were used:

| | |
|---|---|
| mcm-3798t: 5'-AGG TGG ACC TGA TCA TGG AG-3' | (SEQ ID NO:26) |
| mcm-8346t: 5'-ATA CCG GAG ATC ATG CAA GC-3' | (SEQ ID NO:27) |
| mcm-7338: 5'-CTA GGC CAC AGA ATT GAA AGA TCT-3' | (SEQ ID NO:28) |
| mcm-7339: 5'-GTA GGT GGA AAT TCT AGC ATC ATC C-3' | (SEQ ID NO:29) |

### 1.1 Tamoxifen injection and tissue collection

Young mice (14 days old) and adult mice (3-5 months old) were injected once with 40mg/kg of Tamoxifen (Sigma) dissolved in Corn Oil (Sigma). Mice were sacrificed by CO₂ euthanasia or anesthetised with a gaseous mixture of 1.5% Isoflurane (BioMac) and 1.5LO₂ at various time points after tamoxifen injection. Cardiac arrest was induced by injection of 15% KCI, followed by flushing with PBS to remove blood. Hearts for paraffin embedding were additionally flushed with 4% paraformaldehyde (PFA), left in 4% paraformaldehyde (PFA) overnight, dehydrated in 70% ethanol for at least 24hr and embedded in paraffin. Hearts for cryosection were embedded in tragacanth gum (Sigma), frozen in isopentane (BDH-AnalaR) cooled in liquid N₂, cut 9µm sections by cryostat (Leica CM3050), collected onto charged slides and stored at -20°C for histological and immunofluorescence staining. Hearts for protein and RNA extraction were snap frozen in liquid N2 and stored for further processing.

### 1.2 Cardiomyocyte isolation

Cardiomyocyte isolation was carried out as per standard protocol [M. Ackers-Johnson et al., Circulation Research 119: 909 (2016)]. Briefly, mice were anaesthetised with isoflurane (100% O₂ at 0.5L/min, isoflurane atomiser dial at 4%). Mice hearts were stopped with 15% KCI, descending aorta was cut and hearts were flushed with 7mL of EDTA buffer into the right ventricle. Ascending aorta was clamped using Reynolds forceps, the entire heart removed and placed in a 60mm dish containing fresh EDTA buffer. Hearts were digested by sequential injections of 10mL EDTA buffer, 3mL Perfusion buffer and 30-50mL Collagenase buffer into the left ventricle. Forceps were used to gently pull the digested heart into smaller pieces ~1mm and gentle trituration. Enzymatic activity was inhibited by addition of 5ml of Stop buffer. Cell suspension was passed through a 100um filter, and four sequential rounds of gravity settling to enrich for myocytes, ultimately obtaining a highly pure myocyte fraction. The myocyte pellet was snap frozen in liquid N₂ and stored a -80°C until further processing.

### 1.3 Histological and immunofluorescence microscopy

For histological studies, sections (9 µm) were stained with standard Hematoxylin and Eosin for cell morphology, Masson's trichrome stain to detect collagen and TUNEL assay to detect apoptotic nuclei. Images were obtained on a Zeiss Axio Imager Microscope. For immunofluorescence on frozen heart sections, sections were warmed to room temperature, rehydrated with PBS, blocked with M.O.M block (Vector Shields) and donkey serum (Sigma-Aldrich), incubated with primary antibodies overnight at 4°C. The slides were then washed in PBS and incubated with secondary antibodies and Hoechst dye (Sigma-Aldrich) for 60mins, washed with PBS and mounted in Prolong-Gold Anti-fade reagent (Invitrogen). Primary antibodies: LMNA/C N-18 (goat, 1:50, Santa Cruz), Sun1 monoclonal (mouse, neat, from B. Burke), PCM-1 (rabbit, 1:200, Sigma) and sarcomere-α-actinin (mouse, 1:100, abcam); Secondary antibodies were: Alexa Fluor 488, 568 and 647 (1:250, Invitrogen). For isolated cardiomyocyte immunofluorescence, myocytes were stained in suspension and spun down gently for each solution change then plated on glass slides for imaging with a Zeiss LSM510 inverted confocal microscope.

### 1.4 Western analysis for LMNA, SUN1, Ha-tag and GFP.

Whole Hearts and Quadriceps muscles were homogenized in RIPA lysis buffer and spun at 13,200 g, 10 min, 4 °C. Total cell lysates were electrophoresed and transferred to PVDF membrane and blocked with Odyssey Blocking Buffer (Li-Cor Biosciences). The membrane was incubated with primary antibodies for 2 h at room temperature. After which, membrane was washed in TBST washing solution and incubated in Odyssey IRDye secondary antibodies for 1 h before visualization on the Odyssey Infrared Imaging System (Li-Cor Biosciences). The primary antibodies used for detection of LMNA/C (Rabbit, Cell Signalling) that is specific to an epitope in the first 50 amino acids in LMNA, Sun1 monoclonal (mouse, 1:500, Burke) and control beta-tubulin (rabbit, 1:1000, Abcam).

### 1.5 Active force measurement of cardiac papillary muscle.

Mouse papillary muscle from mouse left ventricle was prepared according to the methods described before [C. N. Toepfer, et al., J Physiol 594: 5237-5254 (2016)]. Briefly, explanted mouse heart was immediately rinsed with oxygenated ice-cold Krebs-Henseleit solution with 12 unit/mL heparin sodium (EDQM) and 30mM 2,3-Butanedione monoxime_(BDM, Sigma) and excess blood was removed. After that, the heart was transferred to ice-cold Krebs-Henseleit solution in a glass petri-dish under a dissection microscope with a cooling stage. Cylindrical papillary (200-300 µm in diameter and 1.5-2mm in length) were excised from the left ventricle. T-shaped aluminium clips with a hole were crimped onto the ends of a papillary preparation and the prepared papillary chunks were fixed using pins onto a glass petri-dish with a layer of PDMS sylgard 184 (Dow Corning). Papillary preparations were immersed in a 2% Triton X-100 solution at 4 °C overnight.

Force measurement was performed as previously described [C. Toepfer et al., J Biol Chem 288: 13446-13454 (2013)]. The T-shaped aluminium clips at the ends of the papillary preparations were attached to the hooks of a force transducer (AE801, HJK Sensoren+Systeme) and servo-motor in the experimental rig and were glued with shellac in ethanol (Sigma) to minimize the movement during the experiment. Papillary contraction force was measured at 20 °C. The max contraction force was measured in activing solution (100mM TES, 6.5Mm MgCl2, 25mM Ca-EGTA, 5.7mM Na2ATP, 20mM Glutathione, 21.5mM sodium creatine phosphate, pH=7.1, Ionic strength is 150 mmol/L) with 32 µmol/L free Ca2+. The data were collected and processed from the force transducer and DAQ data acquisition device (National Instrument) using a customized software programmed by LabVIEW2013 (National instrument). At least 5 fibres were tested in each mouse, and at least 3 mice were tested for each experimental group.

### 1.6 AA V9-DN-Sun1 and AAV9-GFP virus

The DN-Sun1 (SS-HA-Sun1L-KDEL) and GFP (SS-GFP-KDEL) vectors were as described [M. Crisp et al., J Cell Biol. 172: 41-53 (2006)]. Briefly, almost the entire lumenal domain of Sun1 was tagged at its NH₂ terminus with HA (HA-Sun1L). To introduce the HA-Sun1L as a soluble form into the lumen of the ER and PNS, signal sequence and signal peptidase cleavage site of human serum albumin was fused onto the NH₂ terminus of HA-Sun1 L to yield SS-HA-Sun1L. To prevent its secretion, a KDEL tetrapeptide was fused to the COOH terminus of SS-HA-Sun1L to form the final SS-HA-Sun1L-KDEL. The HA-Sun1L region was replaced with GFP sequence to generate the SS-GFP-KDEL. The DN-Sun1 and GFP fragments were amplified with the primers listed below (same forward primer was used for both fragments) and ligated into pENN-AAV-cTnT-PI-eGFP plasmid (kind gift from Dr J. Jian), digested with Ncol and Kpnl, to produce Penn-AAV-cTnT-Sun1DN (Figure 10; SEQ ID NO:3).

| | |
|---|---|
| aav Sun1 F: 5'-CgagaattcacgcgggccgccATGAAGTGGGTAACCTTTATTTC-3' | (SEQ ID NO:30) |
| aav Sun1 R: 5'-CgggtcgactctagaggtaccttaCTACAACTCATCTTTCTGGATG-3' | (SEQ ID NO:31) |
| aav GFP Sun R: 5'-CgggtcgactctagaggtacttaCTACAACTCATCTTTGGATCC-3' | (SEQ ID NO:32) |

All restriction enzymes were purchased from NEB. PCR reactions were conducted using Q5^{®} Hot Start High-Fidelity 2X Master Mix (NEB, M0494L). Ligations were conducted using isothermal assembly with NEBuilder^{®} HiFi DNA Assembly Master Mix (NEB, E2621L). Primers used for constructing the plasmids were ordered from IDT.

AAV Viruses were produced as per standard protocol [H. Wakimoto, et al., in Current Protocols in Molecular Biology. (John Wiley & Sons, Inc., 2001)]. Materials supplied by R. Foo: pAAV2/9- the trans-plasmid encoding AAV replicase and capsid gene (SEQ ID NO:2; available from University of Pennsylvania Penn Vector Core); pAdDeltaF6 - the adenoviral helper plasmid (SEQ ID NO:1) (available from University of Pennsylvania Penn Vector Core); QIAGEN Plasmid Maxi Kit; HEK293T cells (ATCC); Transfection reagent (polyethylenimine e.g., Polysciences). AAV-DJ capsid was obtained from Cell Biolabs, Inc. The pAAV2/9, AAV-DJ, pAdDeltaF6, DN-Sun1 and GFP plasmids were purified using a QIAGEN Plasmid Maxi Kit. HEK293T cells were transfected with the virus combination of pAAV2/9, pAdDeltaF6 and either DN-Sun1 or GFP plasmids. Cells were collected and virus purified by lodixanol gradient ultracentrifugation.

The following timeline was used for infection of the mouse hearts. Mice were genotyped at 10 days postnatally. They were then subjected to 1 IP injection of Tmx (40mg/kg of mouse weight) at 14 days postnatally, followed by a concentration of 5×10^10 vg/g AAV9-DN-Sun1 or AAV9-GFP virus injected into the thoracic cavity at 15 days postnatally. Adult mice (3-5 months old) were injected IP with a single dose of Tmx (40mg/kg of mouse weight), followed by injection of AAV at a concentration of 5×10^10 vg/g AAV9-DN-Sun1 or AAV9-GFP virus into the thoracic cavity. Young and adult mice were anesthetised with a gaseous mixture of 1.5% Isoflurane (BioMac) and 1.5L O₂ before virus injections.

### 1.7 Plasmid construction and generation of Cas9 mRNA and sgRNAs

pX330 was obtained from Addgene (#42230, Cambridge, MA, USA). The 20nt Sun1 and Syne1 single guide RNA (sgRNA) sequences were designed with the help of CRISPR Design Tool (crispr.genome-engineering.org). A region of the gene of interest was submitted to the tool to identify suitable target sites. Since off-target mutations are possible in CRISPR/Cas9-mediated targeted mutagenesis in the mouse, the CRISPR Design Tool is able to experimentally assess off-target genomic modifications for each gRNA target sites and provide computationally predicted off-target sites for each intended target, ranking the target sequence according to quantitative specificity analysis on the effects of base-pairing mismatch identity, position and distribution. Complimentary oligonucleotides containing the gRNA target sequences were annealed and cloned into the Bbsl site of pX330. Guide RNA sequences were as follows:

| | |
|---|---|
| 5'-GCACAATAGCCTCGGATGTCG-3' for Sun1ΔSUN, | (SEQ ID NO:33) |
| 5'-CCGTTGGTATATCTGAGCAT-3' for Syne1-stop, | (SEQ ID NO:34) |
| 5'-GGTTATGGCCGATAGGTGCAT-3' for Tyrosinase4a | (SEQ ID NO:35) |

These plasmids (pSun1ΔSUN, pSyne1-stop and pTyrosinase4a) were then sequenced to verify correct insertion of the target sequences. For in vitro transcription, PCR was performed to generate the appropriate transcription templates using a common reverse primer (AAAAGCACCGACTCGGTGCC-3'; SEQ ID NO:36) and gRNA-specific forward primers that encoded the T7 promoter sequence as follows:

| | |
|---|---|
| Sun1ΔSUN: 5'-TTAATACGACTCACTATAGCACAATAGCCTCGGATGTCG-3' | (SEQ ID NO:37) |
| Syne1-stop: 5'-TTAATACGACTCACTATAGCCGTTGGTATATCTGAGCAT-3' | (SEQ ID NO:38) |
| Tyrosinase4a: 5'-TTAATACGACTCACTATAGGTTATGGCCGATAGGTGCAT-3' | (SEQ ID NO:39) |

The gRNA PCR products were then subjected to agarose gel electrophoresis (1.5% agarose) to confirm successful PCR, gel purified and used as templates for in vitro transcription using the MEGAshortscript T7 kit (Life Technologies). The gRNAs were purified using MEGAclear kit (Life Technologies) and eluted in RNase-free water. A sample of purified gRNAs were then subjected to agarose gel electrophoresis for quality checks before injecting into zygotes.

### 1.8 Generation of mutant mice using CRISPR/Cas9

3 to 4 weeks old C57BL/6N females were superovulated with Pregnant Mare Serum gonadotropin (Calbiochem, 36722, 5IU/ml). 48 hours later, the females were injected with human chorionic gonadotropin (Sigma, CG10, 5IU/ml) and were mated with C57BL6 males. The following day, fertilized 0.5dpc embryos were collected from the oviducts. Cas9 mRNA (Sigma, CAS9MRNA, 100ng/ul), Tyrosinase4a gRNA (50ng/ul) and gene-specific gRNA (50 ng/ul) were co-injected into the cytoplasm of the embryos in M2 medium (EmbryoMax^{®} Sigma) using a microinjection system (Nikon). Syne1-stop sgRNA were used to derive Syne1 C'T mutant mice and Sun1ΔSUN sgRNA were used to derive Sun1ΔSUN mutant mice. The injected zygotes were cultured in KSOM with amino acids (EmbryoMax^{®} Sigma) in an incubator maintained at 37°C with 5% CO₂ and 5% O₂ for 2 hours before implanting into 0.5dpc pseudopregnant C3H-ICR females.

### 1.9 DNA extraction for genotyping of CRISPR/Cas9 mice

Mouse tails were clipped and each placed in a 1.5 ml Eppendorf tube. 80 µl of lysis buffer (25 mM NaOH, 0.2 mM EDTA, pH 12) was dispensed into the tube and heated at 95°C for 60 minutes. After heating, the buffer was neutralized with an equal volume of 40 mM Tris-HCl, pH 5. For certain applications, DNA was extracted and purified from mouse tails using DNeasy Blood and Tissue Kit (QIAGEN).

### 1.10 Genotyping of CRISPR/Cas9 mice

CRISPR modified mutant mice were genotyped by PCR followed by gel electrophoresis using a high resolution agarose (2% MetaPhor agarose, Lonza).

Primers for Syne1CT'Δ8 mice were:

| | |
|---|---|
| Forward: 5'-TGCTCCTGCTGCTGCTTATT-3' | (SEQ ID NO:40) |
| Reverse: 5'- ACATGGTGGAGCATTTGTCTCC -3' | (SEQ ID NO:41) |

Primers for Sun1 CRISPR mice were:

| | |
|---|---|
| Forward: 5'-TGACCTTGAGCTGAAACTGC-3' | (SEQ ID NO:42) |
| Reverse: 5'-TCAGAACACTGGCACACACA-3' | (SEQ ID NO:43) |

Lmna mutant mice were genotyped as described in Example 1. To determine sequence of CRISPR-induced mutations, PCR products from mouse tail DNA were subjected to TOPO cloning (Zero BluntTM TOPOTM PCR Cloning Kit, 450245, Thermo Fisher Scientific). Plasmid DNA from at least 10 bacterial colonies were isolated using a mini-prep kit (QIAGEN, QIAprepSpin, Miniprep Kit) and sent for Sanger sequencing.

### 1.11 Derivation of myoblasts, fibroblasts and cell culture for CRISPR/Cas9 study

To isolate myoblasts, limbs were obtained from euthanized mice and muscles were dissected from bone. Tissue digestion was performed by incubating the muscle tissues in enzyme solution consisting of equal volumes of dispase II (Roche, cat. 04942078001) at a concentration of 2.4 U/ml and 1% collagenase II (GIBCO^{®} Invitrogen, cat 17101-015) in a 37°C water bath for 30 minutes, with occasional mixing at 10 minutes interval. After 30 minutes, enzyme solution was neutralized in D10 media (Dulbeco's Modified Eagle Medium (DMEM) with 10% fetal bovine serum). Mixture is then filtered through 70 µm sterile filter (BD FalconTM, cat 352350) and 40 µm sterile filter (BD FalconTM, cat 352340). The suspension was then centrifuged, supernatant removed and subsequently resuspended in F10 media (GIBCO^{®} Invitrogen, cat. 11550043) supplemented with 10 µg/ml bFGF (GIBCO^{®}, cat PHG0264) and plated in 100 mm plates. Mouse adult fibroblasts were allowed to settle for 2 to 3 hours before collecting the supernatant (with floating myoblasts) and replated into 60 mm plates coated with 0.15% Gelatin (Sigma, cat G1393). D10 media was added to the 100 mm plates with MAFs. To terminally differentiate myoblasts to myotubes, the media was changed to DMEM supplemented with 2% horse serum (Thermo Fisher Scientific GIBCO^{®}, cat 16050122).

### 1.12 Immunoblotting for CRISPR/Cas9 study

Whole cell lysates were generated using the Lysis-M kit solution (cOmplete; Roche). Cells were washed in ice-cold PBS and lysed with Roche Lysis M buffer, and centrifuged at 14,000g for 10 minutes to remove cell debris. To extract protein from tissue sample, small slices of tissue were rapidly placed into Lysing Matrix D tubes (MP Biomedicals), and snap frozen in liquid nitrogen. After snap freezing, the tubes were either stored at -80°C or used directly for protein analysis. Protein extraction buffer (50 mM Tris (pH7.4), 500 mM NaCl, 0.4% SDS, 5 mM EDTA (pH7.4), 1x Protease inhibitor (cOmpleteTM EDTA-free Protease Inhibitor cocktail, Cat no. 04693159001, Roche), 2% Triton, 1 mM Dithiothreitol, in distilled water) was added to tissues, which were then homogenized using the FastPrepTM-24 Instrument (MP Biomedicals). Samples were then centrifuged at 14,000g for 10 minutes to remove cell debris. Protein concentration was quantified using bicinchoninic acid (BCA) protein kit (Bio-Rad) before loading protein samples onto a polyacrylamide gel to ensure equal amounts were being analyzed. All protein samples were resolved by SDS-PAGE gel analysis and transferred onto polyvinylidene fluoride (PVDF) membrane (Millipore) by wet transfer for 48 hours at 20V at 4°C. Membranes were blocked in TBS containing 0.1% Tween 20 (TBST) supplemented 5% milk powder (Anlene) for 1 hour at room temperature. Western Blot analysis was performed using primary antibodies diluted in 5% milk powder (diluted in TBST). Membranes were incubated for 2 hours at room temperature or overnight at 4°C. For secondary antibodies, horseradish-peroxidase (HRP) (Invitrogen) conjugated antibodies were used for chemiluminescent imaging. The membranes were incubated for 1 hour at room temperature with the secondary antibodies. For immunoblots visualized by chemiluminescence, membranes were incubated in ECL substrate (Pierce) for 1 minute before being exposed to a chemiluminescence sensitive film (Thermo Scientific) and subsequently processed.

### 1.13 Immunofluorescence for CRISPR/Cas9 study

Cells were grown in 8-well slides (lbidi) and fixed in ice-cold methanol for 15 minutes at -20°C. They were then rinsed in PBS twice and permeabilized and blocked with 0.1% Triton X, 3% BSA in PBS for 15 minutes at room temperature. The fixed and permeabilized cells were then rinsed in PBS three times. Samples were then incubated with primary antibodies (Table 2) for 2 hours at room temperature or overnight at 4°C. Samples were then washed with PBS three times and subsequently incubated with secondary antibodies (Life Technologies) and DAPI (Life Technologies) for 1 hour at room temperature. After three washes in PBS, cells were mounted in Anti-fade (1% DABCO, 90% Glycerol, 10% PBS) and inspected using a Zeiss 510 Meta Confocal microscope or Axiovert 200 inverted epifluorescence microscope (Zeiss). Images were recorded and analysed using Zeiss ZEN, Metamorph or Image J (NIH) software.

**Table 2: Antibodies used for immunofluorescence study.**

| **Antibody** | **Type and Source** | **Concentration** |
|---|---|---|
| Akap450, HPA-026109 | Polyclonal, Sigma | 1:500 |
| MF20 | Monoclonal, DSHB | 1:25 |
| Nesp1 (MANNES1A) | Monoclonal, Glenn Morris | 1:1000 (Western) 1:50 (IF) |
| Nesp1-C'T | Monoclonal, Brian Burke | Undiluted supernatant |
| LaminA, ab8984 | Monoclonal, Abeam | 1:200 |
| LaminA, SSD | Monoclonal, Brian Burke | 1:200 |
| Pcm-1, HPA-023374 | Polyclonal, Sigma | 1:100 |
| Pent, ab4448 | Polyclonal, Abcam | 1:100 |
| Sun1-9F10 | Monoclonal, Brian Burke | 1:200 |
| Sun2-3.1E | Monoclonal, Brian Burke | 1:500 |
| Nesprin-2 | Polyclonal, MyBiosource.com | 1:500 |

### 1.14 Mouse genetics

Lmna mice and tamoxifen injection were described in Example 1. To obtain Lmna^{Δ/Δ}:Syne1^{C'TΔ8/C'TΔ8} and Lmna^{Flx/Flxmcm}:Syne1^{C'TΔ8/C'TΔ8} double mutant mice, Lmna^{Δ/+} or Lmna^{Flx/Flxmcm} mice were intercrossed with Syne1^{C'TΔ8/C'TΔ8} mice. In the Syne2 mouse model, a IRES-β-gal neomycin selectable cassette (PgkNeo) flanked by IoxP sites was inserted into the Syne2 gene, resulting in deletion of part of exon 102 and all of exons 103-104. The neomycin cassette was subsequently removed by crossing with Cre recombinase mice. Syne1^{C'TΔ8/+} or Syne1^{C'TΔ8/C'TΔ8} mice were crossed with Syne2^{+/-} or Syne2^{-/-} mice to obtain mice with mutant Syne1 and Syne2 alleles, which were intercrossed to obtain double mutant mice. Kaplan-Meier method was used to draw the survival curves.

### 1.15 Human guide RNA sequences

Potential guide RNA sequences to disrupt human SYNE1 KASH domain or SUN1 SUN domain were determined using CRISPR tool in Benchling software (Benchling Inc. USA) and are shown in Table 3.

**Table 3: Potential guide RNA sequences to target final exons in human SYNE1 (Nesprin-1) or SUN1 genes**

| **Gene Name** | **ENSEMBL gene ID** | **Chromosome** | **Position** | **Strand** | **Sequence** | **PAM** | **CRISPR enzyme** | **SEQ ID NO:** |
|---|---|---|---|---|---|---|---|---|
| SYNE1 | ENSG00000131018 | 6 | 515330 | - | TCGTGTATCTGAGCATGGGG | TGGAAT | saCas9 | 44 |
| SYNE1 | ENSG00000131018 | 6 | 515335 | - | GCCATTCGTGTATCTGAGCA | TGGGGT | saCas9 | 45 |
| SYNE1 | ENSG00000131018 | 6 | 515340 | + | TCCACCCCATGCTCAGATAC | ACGAAT | saCas9 | 46 |
| SYNE1 | ENSG00000131018 | 6 | 515320 | - | GAGCATGGGGTGGAATGACC | GGG | spCas9 | 47 |
| SYNE1 | ENSG00000131018 | 6 | 515321 | - | TGAGCATGGGGTGGAATGAC | CGG | spCas9 | 48 |
| SYNE1 | ENSG00000131018 | 6 | 515330 | - | TCGTGTATCTGAGCATGGGG | TGG | spCas9 | 49 |
| SYNE1 | ENSG00000131018 | 6 | 515333 | - | CATTCGTGTATCTGAGCATG | GGG | spCas9 | 50 |
| SYNE1 | ENSG00000131018 | 6 | 515334 | - | CCATTCGTGTATCTGAGCAT | GGG | spCas9 | 51 |
| SYNE1 | ENSG00000131018 | 6 | 515335 | - | GCCATTCGTGTATCTGAGCA | TGG | spCas9 | 52 |
| SYNE1 | ENSG00000131018 | 6 | 515345 | + | CCCATGCTCAGATACACGAA | TGG | spCas9 | 53 |
| SYNE1 | ENSG00000131018 | 6 | 515333 | + | CCCGGTCATTCCACCCCATG | TTTG | Cpf1 | 54 |
| SUN1 | ENSG00000164828 | 7 | 873276 | + | TTTTTCTAACTGGGGCCATC | CTGAGT | saCas9 | 55 |
| SUN1 | ENSG00000164828 | 7 | 873285 | - | CCGATACAGACAGGTATACT | CAGGAT | saCas9 | 56 |
| SUN1 | ENSG00000164828 | 7 | 873266 | + | AACTTCGGATTTTTTCTAAC | TGG | spCas9 | 57 |
| SUN1 | ENSG00000164828 | 7 | 873267 | + | ACTTCGGATTTTTTCTAACT | GGG | spCas9 | 58 |
| SUN1 | ENSG00000164828 | 7 | 873268 | + | CTTCGGATTTTTTCTAACTG | GGG | spCas9 | 59 |
| SUN1 | ENSG00000164828 | 7 | 873280 | - | ACAGACAGGTATACTCAGGA | TGG | spCas9 | 60 |
| SUN1 | ENSG00000164828 | 7 | 873296 | + | CTGAGTATACCTGTCTGTAT | CGG | spCas9 | 61 |
| SUN1 | ENSG00000164828 | 7 | 873281 | + | TTCTAACTGGGGCCATCCTG | TTTT | Cpf1 | 62 |
| SUN1 | ENSG00000164828 | 7 | 873282 | + | TCTAACTGGGGCCATCCTGA | TTTT | Cpf1 | 63 |
| SUN1 | ENSG00000164828 | 7 | 873283 | + | CTAACTGGGGCCATCCTGAG | TTTT | Cpf1 | 64 |
| SUN1 | ENSG00000164828 | 7 | 873284 | + | TAACTGGGGCCATCCTGAGT | TTTC | Cpf1 | 65 |

| | | | | | | | | |
|---|---|---|---|---|---|---|---|---|
| PAM, protospacer adjacent motif; saCas9, *Staphylococcus aureus* Cas9; spCas9,_*Streptococcus pyogenes* Cas9; Cpf1, CRISPR from *Prevotella* and *Francisella* 1. | | | | | | | | |

### 1.16 Statistical analysis

All statistical analysis was performed using Graphpad Prism software.

### Example 2: Cardiomyocyte specific loss of Lmna results in the rapid onset of heart failure

To further define the interaction between *Sun1* and *Lmna* in postnatal pathology in mice, we specifically ablated the *Lmna* gene in different tissues by using a conditional *Lmna*^{*Flx*/*Flx*} line of mice (Figure 33), that when recombined by Cre activation, results in the complete loss of LaminA/C protein [A. S. Wang, et al., Differentiation; research in biological diversity, (2015); I. Solovei et al., Cell 152: 584-598 (2013)]. When *Lmna*^{*Flx*/*Flx*} was constitutively deleted in all tissues by crossing the *Lmna*^{*Flx*/*Flx*} mice with *Zp3-Cre* mice [W. N. de Vries et al., Genesis 26: 110-112 (2000)], the mean postnatal lifespan was 17.5 days (Figure 26A). When the same deletion was induced in the absence of *Sun1,* the *Lmna*^{*Δ*/}*^{Δ}:_{Sun1}*^{*-*/*-*} mice lived to a mean of 32.5 days, almost a doubling in longevity (Fig 26A). Performing the same *Lmna* deletion on a *Sun2* null background did not extend the longevity of *Lmna*^{*Δ*/*Δ*} mice, revealing the longevity extension is specific to the loss of *Sun1* (Fig 36). Since the A-type lamins are widely expressed in almost all adult tissues, we then determined to what extent, *Lmna* deletion, specifically in cardiomyocytes, contributes to the early postnatal death of *Lmna*^{*Δ*/*Δ*} mice. Furthermore, the inventors wished to ascertain whether loss of *Sun1* would increase longevity in these mice harbouring *Lmna* deficient cardiomyocytes. We first crossed the *Lmna*^{*Flx*/*Flx*} with a constitutive myh6 *Cre* [R. Agah et al., J Clin Invest 100: 169-179 (1997)], in which Cre expression, though constitutive, is restricted to cardiomyocytes but commences during embryogenesis. These mice survived slightly longer than the *Lmna*^{*Δ*/*Δ*} to an average of 26.5 days postnatally (Figure 26C). When the same cardiomyocyte specific deletion was performed on a *Sun1*^{*-*/*-*} background, this resulted in a significant increase in longevity to at least 6 months and beyond after birth (Figure 26C). To further define the loss of *Lmna* and its effect in postnatal/adult cardiomyocytes we derived mice homozygous for the *Lmna*^{*Flx*/*Flx*} allele carrying the inducible cardiomyocyte specific Cre Tg(Myh6-cre/Esr1), (here abbreviated to *mcm*) in which Cre is induced by a single injection of tamoxifen (Tmx) [D. S. Sohal et al., Circ Res 89: 20-25 (2001)]. From this cross, the average lifespan of 3-5 month old *Lmna*^{*Flx*/*Flx:mcm*} mice, following Cre induction was 27 days (Figure 27A). Controls were unaffected by Tmx injection. PCR and immunofluorescence analysis confirmed the *Lmna* deletion was specific to the *Lmna*^{*Flx*/*Flx:mcm*} cardiomyocytes, with no detectable recombination occurring in the brain, diaphragm, lung, liver and skeletal muscle, or in wild-type control animals (Figure 27B). By 21 days post injection, *Lmna*^{*Flx*/*Flx:mcm*} mice showed laboured breathing, a dishevelled, ungroomed appearance, increased lethargy and kyphosis (Figure 27C). Immunofluorescence analysis of isolated cardiomyocytes (CM) and sections of *Lmna*^{*Flx*/*Flx:mcm*} hearts showed reduced levels of LaminA protein and cardiomyocyte nuclei without any LaminA expression (Figure 27D). LaminA protein levels were decreased 3.5 fold in *Lmna*^{*Flx*/*Flx:mcm*} hearts after Cre induction compared to uninduced *Lmna*^{*Flx*/*Flx:mcm*}and *Lmna*^{+/+/*mcm*} hearts (Figure 27E). By sampling *Lmna*^{*Flx*/*Flx:mcm*} mice at specific time points after Tmx injection, it was estimated that it takes 7-14 days after Cre induction for LMNA protein levels to fall by 50% (data not shown), a rate consistent with a study using siRNA *LMNA* knockdown in human fibroblasts by 1.3-fold after 48 hrs and a further 4-fold reduction after 10.5 days [A. Buchwalter and M. W. Hetzer, Nature communications 8: 328 (2017); T. Sieprath et al., Nucleus 6: 236-246 (2015)]. Echocardiograms (ECGs) performed at 21 days after *Cre* induction revealed poor cardiac contractility in *Lmna*^{*Flx*/*Flx:mcm*} mice compared to *Lmna*^{*Flx*/*Flx:mcm*} controls (Figure 28A). There was a significant reduction in the Ejection Fraction (EF%) and Fractional shortening (FS%) (P<0.0001) (Figure 28B). The left systolic and diastolic ventricular internal diameters (LVID) were enlarged, compared to *Lmna*^{*Flx*/*Flx:mcm*}controls (Figure 28B). Significantly fewer viable (brick-like) cardiomyocytes were isolated from *Lmna*^{*Flx*/*Flx:mcm*}+Tmx hearts compared to *Lmna*^{*Flx*/*Flx:mcm*} controls (Figure 28C). Visual analysis revealed the isolated cardiomyocytes from *Lmna*^{*Flx*/*Flx:mcm*} +Tmx hearts contained large intracellular vacuoles (Figure 28C). Histological analysis of *Lmna*^{*Flx*/*Flx:mcm*} +Tmx hearts, revealed infiltration of nucleated cells and increased intercellular spaces between cardiomyocytes compared to *Lmna*^{*Flx*/*Flx:mcm*}control hearts (Figure 28D). The left ventricular lumen in *Lmna*^{*Flx*/*Flx:mcm*}+Tmx hearts was significantly enlarged, together with significantly increased levels (P=0.0098) of fibrosis were noted in *Lmna*^{*Flx*/*Flx:mcm*}+Tmx hearts compared to in *Lmna*^{*Flx*/*Flx:mcm*} controls (Figure 28D). Increased numbers of apoptotic cells were also identified in *Lmna*^{*Flx*/*Flx:mcm*} +Tmx hearts compared to control hearts (Figure 28D). However there was no evidence of extensive DNA damage detectable in the cardiomyocytes, as assessed by Rad51 , MRE11 , H2AX phosphor-Ser and 53BP1 immunostaining (Data not shown).

### Example 3: Deletion of Sun1 ameliorates cardiac pathology induced by Lmna loss

Mice with *Lmna* mutations show a significant increase in longevity and health in the absence of Sun1 [C. Y. Chen et al., Cell 149: 565-577 (2012)]. As described, induced deletion of *Lmna* in cardiomyocytes (*Lmna*^{*Flx*/*Flx:mcm*} + Tmx) results in death within 1 month post Cre induction (Figure 26C). Strikingly, when the same deletion was induced on a *Sun1* null background the mice survived for more than 1 year after *Cre* induction (Figure 26C). Hearts from *Lmna*^{*Flx*/}*^{Flx:mcm}Sun1*^{*-*/*-*}+Tmx mice, 3 weeks after induction, were compared to those from *Lmna^{FlxlFlx:mcm}Sun1*^{+/+}+Tmx to determine the extent to which SUN1 loss ameliorated the pathological changes induced by *Lmna* loss in cardiomyocytes. Immunofluorescent imaging for Lamin A/C identified many elongated and distorted nuclei. In some of these, residual Lamin A/C was displaced to one pole of the nucleus (Figure 29A panel 1 and insert) in the *Lmna*^{*Flx*/}*^{Flx:mcm}Sun1*^{+/+}+Tmx hearts. In contrast in the *Lmna^{FlxlFlx:mcm}Sun1*^{-/-} +Tmx hearts, while there were many elongated nuclei, these showed few if any distortions, even when there was no Lamin A/C staining (Figure 29A panel 3 yellow arrow heads). Western analysis of whole hearts revealed a significant reduction in Lamin A/C in the *Lmna^{FlxlFlx:mcm}Sun1*^{*-*/*-*} +Tmx hearts (P=0.0359) lysates compared to *Lmna*^{*Flx*/}*^{Flx:mcm}Sun1*^{+/+} controls (Figure 29A lower panels). The *Lmna*^{*Flx*/}*^{Flx:mcm}Sun1*^{+/+}+Tmx cardiomyocyte nuclei exhibited increased longitudinal length, together with a segmented appearance, with the segments connected by narrow bridges (Figure 29A and C, panel 1 arrows). However, in the absence of *Sun1, Lmna*^{*Flx*/}*^{Flxmcm}Sun1*^{*-*/*-*} cardiomyocyte nuclei exhibited no abnormalities or segmentation (Figure 29C panels 3 and 4). In total, 70% of cardiomyocytes in *Lmna^{FlxlFlx:mcm}Sun1*^{+/+} mice had ruptured or misshapen nuclei compared to fewerthan 1% of the cardiomyocytes from the *Lmna*^{*Flx*/}*^{Flx:mcm}Sun1*^{-/-} (Figure 29C panel 5). Clear enlargement of the left ventricle (LV) was evident in the *Lmna*^{*Flx*/}*^{Flx:mcm}Sun1*^{+/+} mice but not in the LVs of the *Lmna^{FlxlFlx:mcm}Sun1*^{-/-} +Tmx hearts (Figure 29B, panels 1 and 2). The *Lmna*^{*Flx*/}*^{Flx:mcm} Sun1*^{+/+} hearts exhibited significantly increased levels of fibrosis (P<0.0001) compared to controls, whereas there was no significant fibrosis in the *Lmna*^{*Flx*/}*^{Flx:mcm}Sun1*^{-/-} hearts (Figure 29B, panels 3-5).

As a model for ventricular muscle mechanics we measured the active force in cardiac papillary muscle. The active force was significantly reduced by 66% in *Lmna*^{*Flx*/}*^{Flx:mcm}:Sun1*^{+/+}+Tmx papillary muscle (P=0.0028) compared to *Lmna*^{*Flx*/}*^{Flx:mcm} Sun1*^{+/+}+CTL. In the absence of SUN1, *Lmna*^{*Flx*/}*^{Flx:mcm}Sun1*^{-/-}+Tmx cardiac papillary active force was maintained at levels not significantly different from those of controls (Figure 29B panel 6).

Echocardiograms performed before and after Cre induction revealed progressive worsening of cardiac contractility in the *Lmna*^{*Flx*/}*^{Flx:mcm} Sun1*^{+/+} +Tmx mice compared to *Lmna*^{*Flx*/}*^{Fl:mcm}Sun1*^{-/-}+Tmx mice (Figure 29D). Loss of SUN1 preserved both EF, FS and Global Longitudinal Strain (GLS) (GLS is a separate parameter used to assess myocardial contractility, and is a better predictor of heart failure) in *Lmna*^{*Flx*/}*^{Flx:mcm}Sun1*^{-/-} + Tmx mice compared to *Lmna*^{*Flx*/}*^{Flx:mcm} Sun1*^{+/+} +Tmx mice.

PCR analysis of the aged *Lmna*^{*Flx*/}*^{Flx:mcm}Sun1*^{-/-}+Tmx hearts 12-14 months after Tmx injection confirmed the sustained deletion of *Lmna* gene (Figure 37C), while protein quantification revealed a significant reduction of LMNA levels in *Lmna*^{*Flx*/}*^{Flx:mcm} Sun1*^{-/-}+ Tmx hearts 12-14 months after Tmx (Figure 37D). Histological analysis of the 12-14 month *Lmna*^{*Flx*/}*^{Flx:mcm} Sun1*^{-/-}+ Tmx hearts revealed no significant increase in fibrosis compared to controls (Figures 37A and B). However, echocardiograms on these aged mice showed reduced EF and FS in both *Lmna*^{*Flx*/}*^{Flx:mcm} Sun1*^{+/+} + CTL and *Lmna*^{*Flx*/}*^{Flxmcm}Sun1*^{-/-} + Tmx mice (Figure 37E), although the average lifespan of *Lmna*^{*Flx*/*Flx*} mice is 13-14 months (Figure 26C and so the reduced contractile function may have been due to ageing. Together these findings demonstrate that loss of *Lmna,* in adult (2-3 month old) cardiomyocytes is sufficient to result in cardiac failure within 3-4 weeks after Cre activation, but the pathology is strikingly reduced by deleting *Sun1,* with this reduction being sustained for a year.

### Example 4: Loss of SUN1 extends longevity of Lmna missense mutants

As most cases of *LMNA* induced DCM result from missense mutations, we determined what effect loss of SUN1 had on the longevity and cardiac function of a previously described *Lmna* mutant mouse line carrying the *N195K* missense mutation that dies from DCM [L. C. Mounkes, et al., Hum Mol Genet 14: 2167-2180 (2005)], with this mutation having been identified in 2 unrelated patients diagnosed with AD-EDMD [D. Fatkin et al., N Engl J Med 341: 1715-1724 (1999); J. P. van Tintelen et al., Am Heart J 154: 1130-1139 (2007)]. Here too, we found that the absence of SUN1 significantly extended the lifespan of this mutant mouse line with improved cardiac function (Figure 26D). We extended these findings by deriving mice heterozygous for the *N195K* mutation, with the *WT-Lmna* allele being floxed i.e. *Lmna*^{*N195K*/}*^{Flx}xSun1*^{+/+}*.* Inducing the Tmx inducible cardiomyocyte Cre allele in these mice (*Lmna*^{*N195K*/*Flx:mcm*} + Tmx) resulted in the deletion of the WT floxed *Lmna* allele making the cardiomyocytes hemizygous for the *Lmna*^{*N195K*/*-*} mutation. These mice had a mean lifespan of less than 50 days, a longevity half that of the original *Lmna*^{*N195K*/*N195K*} homozygotes (Figure 30A). When the *Lmna* ^{*N195K*/*Flx:mcm*} +Tmx mutation was induced on a *Sun1* null background longevity was significantly extended from <50days to >200days (Figure 30A), revealing that loss of *Sun1* is also effective at preventing DCM caused by *Lmna* missense mutations specifically in cardiomyocytes.

Echocardiograms performed before and after Cre induction revealed progressive worsening of cardiac contractility in the *Lmna*^{*N195K*/}*^{Flx:mcm} Sun1*^{+/+} mice compared to *Lmna*^{*N195K*/}*^{Flx:mcm} Sun1*^{*-*/*-*} mice (Figure 30B). Loss of SUN1 preserved both EF, FS and Global Longitudinal Strain (GLS) in *Lmna*^{*N195K*/}*^{-:mcm}Sun1*^{*-*/*-*} mice compared to *Lmna*^{*N195K*/}*^{-:mcm} Sun1*^{+/+} mice (Figure 30B).

### Example 5: AAV9 mediated transduction and expression of a DNSun1 prolongs the lifespan of the Lmna^{Flx/Flx:mcm}+Tmx mice.

The above results demonstrated that genetically ablating SUN1's functions or reducing SUN1 levels could be of therapeutic value in treating DCM. We then tested whether this was due to the complete ablation of SUN1's functions to overcome its toxic over-abundance versus leaving its levels untouched and specifically disrupting its LINC complex-associated role in tethering KASH-domain proteins in the ONM, thereby tethering the nucleus to components of the cytoskeleton. To distinguish between these 2 possibilities, Adenovirus Associated Virus (AAV) was utilized to transduce and express, specifically in cardiomyocytes, a dominant negative SUN1 minigene whose protein product would compete with both SUN1- and SUN2-KASH binding in the cardiomyocyte perinuclear space [M. Crisp et al., J Cell Biol 172: 41-53 (2006)]. A schematic representation of an AAV viral capsid and its genomic payload is shown in Figures 48A and 48B (derived from Figures 1B and 1C of Lipinski et al., Prog Retin Eye Res. (2013) 32:22-47).

A region corresponding to the entire lumenal domain of the *Sun1* gene was tagged at its N terminus with an HA (HA-Sun1L) epitope. To localize the resulting protein product to the endoplasmic reticulum (ER) and perinuclear space (between the INM and ONM - PNS), the signal sequence and signal peptidase cleavage site of human serum albumin was fused to the N terminus of HA-Sun1L to yield SS-HA-Sun1L. To prevent the miniprotein's secretion, a KDEL tetrapeptide was linked to the C- terminus of SS-HA-Sun1L, forming SS-HA-Sun1L-KDEL (Figure 34). The signal sequence would ensure the HA-Sun1KDEL accumulates intracellularly within the contiguous peripheral ER and PNS lumen. The cDNA sequence encoding the minigene was fused to the chicken cardiotroponin promoter (cTnT) to ensure the minigene is only transcribed in cardiomyocytes [K. M. Prasad, et al., Gene Ther 18: 43-52 (2011)]. A diagram of how SS-HA-Sun1L (DN-Sun1) displaces the KASH domain proteins from the LINC complex in the PNS to the ER is presented in Figure 15 (third panel) and Figure 31B.

To verify that the DN-Sun1 functioned in cardiomyocytes (CM) we initially transduced human CMs derived from iPS stem cells using the AAV-DJ system [D. Grimm, et al., J Virol. 82(12):5887-911 (2008)] that provides for a higher infectivity rate in cultured cells than the AAV9 serotype used to transduce the DN-Sun1, under transcriptional control of the cTnT promoter, in the mouse hearts. The DN-Sun1 was effective at displacing Nesprin-1 from the nuclear envelopes in the CMs that were expressing the DN-Sun1 as shown in Figure 31D. Cells expressing high levels and low levels of DN-Sun1 are indicated by grey and white arrowheads respectively. High levels of DN-Sun1 expression resulted in the displacement of Nesprin-1 from the nuclear envelope. This confirmed that the DN-Sun1 was effective at disrupting the LINC complex in CMs.

We used AAV (serotype 9) to transduce and express the DN-Sun1 minigene in the hearts of postnatal mice by intrathoracic injection. The procedure is summarized in Figure 31A and all mice were sacrificed at 100 days after Tmx injection for analysis. Detection by PCR of the *Lmna* deletion in the hearts confirmed Cre induction by Tmx injection (Figure 31C). To determine the localization and expression levels of the DN-Sun1 minigene, total protein was extracted from half the heart. Western analysis revealed robust expression of both AAV9-DNSun1 and AAV9-GFP control protein (Dose injected: 5×10^10vg/g of mouse) 99 days after AAV injection (Figure 31C) with the expression levels of both proteins being dependent on the dose of viral particles injected (Figure 38). The expression of either AAV9-DNSun1 or AAV9-GFP proteins did not affect LMNA protein levels (Figure 39A).

Immunofluorescence analysis revealed that a larger percentage of cardiomyocytes were expressing GFP with 5x10^10vg/g of AAV9-GFP compared to the levels resulting from a 10-fold lower dose of viral particles (5×10^9 AAV9-GFP) (Fig. 39B and Fig. 39C).

The *Lmna*^{*Flx*/*Flx:mcm*} +Tmx mice, injected with AAV9-GFP control, lived an average of 34.5 days after Tmx, whereas *Lmna*^{*Flx*/*Flx:mcm*}+Tmx mice injected with AA9-DNSun1 (5×10^10 vg/g of mouse) lived significantly longer with the majority surviving at least 100 days after Tmx, before their termination for analysis (P=0.0002) (Figure 20 shows early time period results with male and female mice; Figure 31E shows results at 100 days with separate graphs for male and female mice and mice with different virus injection titre removed). Echo analysis confirmed *Lmna*^{*Flx*/*Flx:mcm*}+Tmx+AAV9-DNSun1 hearts were functioning better than *Lmna*^{*Flx*/*Flx:mcm*} +Tmx +AAV9-GFP hearts at 35 days post Tmx (Figure 31G). Although the *Lmna*^{*Flx*/*Flx:mcm*} +Tmx+AAV9-DNSun1 mice were alive at 100 days after induction both EF% and FS% were significantly lower compared to control *Lmna*^{*Flx*/*FlxWT*}+Tmx mice (Figure 31G). At 35 days post Tmx, increased fibrosis was detected in both the *Lmna*^{*Flx*/*Flx:mcm*} +Tmx+AAV9-DNSun1 and *Lmna*^{*Flxx*/*Flxxmcm*}+Tmx+AAV9-GFP hearts (Figure 31F), although fibrosis in the *Lmna*^{*Flx*/*Flx:mcm*} +Tmx+AAV9-DNSun1 hearts was significantly lower than in *Lmna*^{*Flxx*/*Flxxmcm*}+Tmx+AAV9-GFP hearts (Figure 31F lower panels).

### Example 6: Disruption of the LINC complex in mice using CRISPR/Cas9

Mice harboring a variety of Lmna mutations, both global and cardiac-specific, show a significant increase in longevity and health in the absence of Sun1 [(Chen et al., Cell 149: 565-577 (2012) and Examples 2-4]. Prior to the findings described in Examples 2-5, the mechanism of this rescue was unclear, but was speculated to be due to the toxic effects of excess Sun1 in Lmna mutants [Chen et al., Cell 149: 565-577 (2012)]. AAV-mediated expression of a dominant negative LINC-complex-disrupting transgene ameliorates the pathology associated with *Lmna* mutation [Example 5]. The findings in Examples 2-5 are consistent with the idea that LINC complex function, rather than excess Sun1, is the molecular driver of Lmna pathology. This was surprising, as genetic disruption of the LINC complex via loss of Sun1 and Sun2 [K. Lei et al., Proc Natl Acad Sci USA 106: 10207-10212 (2009)], or cardiac-specific disruption of Nesprin-1 and Nesprin-2 [Banerjee et al., PLOS Genet 10(2): e1004114 (2014)], in mice, resulted in various pathologies.

To develop alternative means of disrupting the LINC complex *in vivo,* the possibility of using CRISPR/Cas9 genome editing to disable the SUN and KASH domains of the proteins constituting the LINC complex was examined. As both the SUN domain and the KASH domain are located at the C-termini of their respective proteins, we hypothesized that a CRISPR guide RNA targeted to the 3' end of the genes encoding SUN or KASH domain proteins would result in a premature stop codon following CRISPR-induced non-homologous end joining. This would result in a truncated protein with its C-terminal SUN or KASH domain mutated. The truncated protein would be expressed and membrane-localized, but unable to interact with its cognate LINC complex partners. In Example 2, we found that loss of Sun2 did not ameliorate Lmna-associated pathologies. Thus we chose to target the Sun1 SUN domain using CRISPR as Sun1 appears to be the dominant SUN domain protein mediating *Lmna* pathology. Of the KASH domain proteins, only Nesprin-1, Nesprin-2 and Nesprin-3 are broadly expressed [H. F. Horn, Current topics in developmental biology 109: 287-321 (2014)). Nesprin-1 and Nesprin-2 are close paralogues that are functionally redundant. They interact with the actin and microtubule cytoskeleton, whereas Nesprin-3 appears to interact specifically with intermediate filaments [Kim et al., Biol. Chem. 396: 295-310 (2015)]. As we already had Nesprin-2 and Nesprin-3 mutant mouse strains derived by conventional gene targeting available in the laboratory, we chose to target the KASH domain of Nesprin-1 using CRISPR to test the possibility of using CRISPR/Cas9 *in vivo* for treatment of laminopathies. The Sun1 gene and the Syne1 gene encoding Nesprin-1 protein were directly targeted *in vivo* by microinjecting C57/BI6 mouse zygotes with Cas9 mRNA and gRNA targeting the SUN1 (5'-GCACAATAGCCTCGGATGTCG-3'; SEQ ID NO:66) or KASH1 (5'-CCGTTGGTATATCTGAGCAT-3' SEQ ID NO:67) domains, followed by implantation into surrogate mothers. Note the SUN1 gRNA targeted Sun1 upstream of the SUN domain so as to ablate the SUN domain. A gRNA (5'-GGTTATGGCCGATAGGTGCAT-3'; SEQ ID NO:68) targeting the tyrosinase gene was co-injected - progeny that had undergone CRISPR genome editing would have white or mosaic coat color resulting from tyrosinase disruption. These pups were genotyped to confirm successful gene disruption and used as founder animals to establish Sun1 or Nesprin-1 mutant colonies.

### 6.1 Characterization of mutant mice

Following Sanger sequencing of founder animals and F1 progeny, we focused on characterizing Sun1 mutant alleles (Figure 40A) with a 7 bp deletion (Sun1_del7, or Sun1Δ7; SEQ ID NO:71) and 4 bp insertion (Sun1_plus4; SEQ ID NO:70), and a Syne1 (Nesprin-1) mutant allele (Figure 41A) with a 8 bp deletion (Syne1_CTdel8, or Syne1 C'TΔ8; SEQ ID NO:76). The Sun1 mutant alleles were predicted to produce mRNA with premature stop codons resulting in a truncated Sun1 protein lacking a SUN domain (Figure 40B). Tail tip fibroblasts were isolated from Sun1 homozygous mutant animals.

Immunofluorescence staining revealed loss of Sun1 protein (Figure 40C), suggesting that the indels generated by CRISPR caused nonsense-mediated decay of Sun1 mRNA. It is unclear whether the site of mutation, being outside the SUN domain rather than inside the SUN domain, had an effect on the expression of the mutated gene. As we were unable to obtain Sun1 mutant alleles that produced Sun1 protein lacking the SUN domain, instead obtaining essentially Sun1 null animals, we did not further characterize these mutant lines.

The Syne1 C'TΔ8 allele is predicted to produce a protein where the final 11 amino acids in the wildtype sequence (SEQ ID NO:77) are mutated and are followed by an additional 50 amino acids encoded by an alternate reading frame (Figure 41B; SEQ ID NO:78). Immunoblotting performed on Syne1WT and Syne1C'TΔ8 heart and muscle tissues revealed a ~120kDa band in WT corresponding to the striated muscle-enriched Nesprin-1α isoform of the Syne1 gene (Figure 41C, D). In the C'TΔ8 heart and muscle tissues, the presumptive Nesprin-1α polypeptide appeared to be less abundant and of lower electrophoretic mobility than in the wildtype (Figure 41C, D). This is consistent with the 8bp deletion in the Syne1C'TΔ8 allele introducing a novel stop codon downstream, resulting in a protein of higher molecular weight. In addition, a ~1 MDa band likely corresponding to Nesprin-1Giant was observed in heart tissue from both Syne1WT and Syne1C'TΔ8 mice.

Immunofluorescence analysis of mouse adult fibroblasts (MAFs) derived from 12 week old mice revealed that Nesprin-1 was mis-localized from the nuclear envelope to the cytoplasm in the Syne1C'TΔ8 MAFs (Figure 42A). Similarly, in myotubes, Nesp-1 redistributes to the cytoplasm in the Syne1C'TΔ8 myotubes as compared to Syne1WT (Figure 42B). Other LINC complex and NE proteins such as SUN1, SUN2, Emerin and LaminA remained localized to the NE (Figure 43A-C). Consistent with previous reports [Gimpel et al., Curr. Biol. 27: 2999-3009.e9. (2017)], disruption of Nesprin-1 in myotubes led to mislocalization of centrosomal proteins PCM1, Pent and Akap450 from the myotube nuclear envelope (Figure 44A-C). Mislocalization of Nesprin-1 from the nuclear envelope is consistent with disruption of the Nesprin-1 KASH domain, preventing Nesprin-1C'TΔ8 mutant protein from interacting with the SUN domains of Sun1 and Sun2, which would normally restrict Nesprin-1 to the nuclear envelope. As the transmembrane region is not disrupted, it is likely that Nesprin-1 is mislocalized to the endoplasmic reticulum (ER) in the C'TΔ8 mutant, as the ER and the perinuclear space form a contiguous membrane system.

Similar to one previously reported Nesprin-1 mouse model [Zhang et al., Development 134(5): 901-8 (2007)], and in contrast to two other models [Puckelwartz et al., Hum Mol Genet 18: 607-620 (2009); Zhang et al., Hum Mol Genet 19: 329-341 (2010)], the disrupted KASH domain of Nesprin-1 results in no overt phenotypic differences between the Syne1 wildtype (WT) and Syne1C'TΔ8 mutant (Figure 45A-B). Both male and female homozygous mutants were fertile with no significant differences in body weight between the Syne1WT and Syne1C'TΔ8 mice (Figure 45C). Syne1C'TΔ8 mice also did not exhibit any growth retardation or obvious muscle dystrophy, nor did they display any difficulty in movement or grooming, which can be indications of muscle deterioration.

In order to probe the role of other KASH domain proteins in *Lmna* pathology, mice mutant for Syne2, encoding Nesprin-2, were generated by conventional gene targeting (Fig. 46A). To characterize the mutation, immunofluorescence microscopy of tail tip fibroblasts was carried out. Syne2^{-/-} homozygous mutant fibroblasts expressed little to no Nesprin-2 (Fig. 46B). Consistent with previous findings [Zhang et al., Development 134(5): 901-8 (2007)], while Syne2^{-/-} mice were overtly normal, with no growth retardation or infertility, Nesprin-1/2 double mutant mice (Syne1^{C'TΔ8/C'TΔ8}:Syne2^{-/-}) were perinatal lethals (Fig. 46C).

### 6.2 Disruption of Nesprin-1 KASH domain ameliorates Lmna pathologies

Even though Nesprin-1 was still expressed, Nesprin-1-containing LINC complexes would not be formed in Syne1^{C'TΔ8/C'TΔ8} cells and animals. Since AAV-mediated disruption of the LINC complex using dominant negative Sun1 *in vivo* rescues *Lmna* pathologies (Example 5), we reasoned that the "KASH-less" Nesprin-1 mutant allele we generated might also rescue *Lmna* pathology. To test this hypothesis, mice heterozygous for a Lmna null (Lmna^{Δ/Δ}) allele (Example 1) were intercrossed with Syne1C'TΔ8 mice to obtain Lmna^{Δ/Δ}:Syne1^{C'TΔ8/C'TΔ8}double mutant mice. While Lmna^{Δ/Δ} mice lived for 15-17 days, Lmna^{Δ/Δ}:Syne1^{C'TΔ8/C'TΔ8} double mutant mice lived for up to 42 days (Figure 24). Lmna null mice heterozygous for the Syne1^{C'TΔ8} allele did not experience any lifespan extension. Lmna^{Δ/Δ} mice on a Syne2^{-/-} homozygous mutant background also did not experience lifespan extension (Figure 47), indicating that Lmna pathology is mediated primarily by Nesprin-1/Sun1 LINC complexes.

To examine the effect of the Syne1^{C'TΔ8/C'TΔ8} allele in mice with cardiac-specific loss of Lmna, mice homozygous for a conditional *Lmna*^{*Flx*/*Flx*} allele carrying the inducible cardiomyocyte specific Cre Tg(Myh6-cre/Esr1) (here abbreviated to mcm), in which Cre is induced by a single injection of tamoxifen (Tmx), were used as described in Examples 1-2. Cardiac-specific deletion of Lmna results in death within a month, but mice with the same deletion induced on a homozygous Syne1^{C'TΔ8/C'TΔ8} background lived for at least 120 days after Tmx induction (Figure 25; no change from day 80-120).

### Example 7: Method for screening small molecules that block SUN-KASH interactions

Crystallographic studies of human SUN2 reveal that the SUN domain is assembled as a clover-like trimeric structure [Sosa et al., Cell 149(5): 1035-47 (2012)]. Trimerization is mediated by a triple-helical coiled-coil, with an estimated length of 40-45nm. This is sufficient to bridge the perinuclear space (PNS), allowing SUN and KASH domains to directly interact [Sosa et al., Cell 149(5): 1035-47 (2012)]. The KASH binding site is formed primarily within a groove formed at the interface between adjacent SUN domains (Figure 5B; Figure 21 left panel). This groove accommodates part of the KASH domain, about 18 residues, in an extended conformation. However, it is the C-terminal tetrapeptide of the KASH domain, featuring three proline residues followed by a terminal aliphatic reside, Leu or Thr (for Nesp1 and Nesp2 respectively), that is crucial for the SUN-KASH interaction (Figure 21 right panel, adapted from Figure 1 of Sosa et al, Cell 149(5): 1035-47 (2012). The significance of this tetrapeptide is that it is situated in a well-defined pocket formed within a single SUN monomer. Modification of this peptide in any way, including the addition of a single residue (an Ala) at the C-terminus, completely eliminates the SUN-KASH association over the entire SUN-KASH contact region (Sosa et al., Cell 149(5): 1035-47 (2012) and Figure 22 left panel). The conclusion is that while stable binding of the KASH domain requires 18-20 residues, it is the C-terminal tetrapeptide that actually initiates binding. Thus, blocking the tetrapeptide binding-pocket within the SUN monomer will abolish SUN-KASH association. We have described in this disclosure an AAV-based gene therapy strategy to break endogenous SUN-KASH interactions as a treatment for laminopathies, including dilated cardiomyopathy. Alternatively, a small molecule that blocks the SUN-KASH interaction at the SUN binding pocket would disrupt LINC complexes and similarly treat laminopathies. A variety of standard methods exist to screen for small molecule drugs *in vitro.*

An *in vitro* screen can be set up employing recombinant SUN and KASH domains or KASH peptide, for which methods of production have been previously published [Sosa et al., Cell 149(5): 1035-47 (2012)]. One such screen involves an assay technique analogous to an enzyme-linked immunosorbent assay (Figure 22 right panel, similar to Lepourcelet et al., Cancer Cell. 5(1): 91-102 (2004)). Recombinant SUN domain is immobilized on a solid surface, typically in 96-well plates, and then complexed with recombinant KASH domain linked to an enzyme that can generate a colorimetric or chemiluminescent readout. One method for enabling this linkage is to synthesize a biotinylated KASH peptide, which can then be linked with commercially available streptavidin-horseradish peroxidase (HRP) conjugate. Candidate compounds are obtained from appropriate suppliers and screened for their ability to inhibit KASH-SUN associations *in vitro.* Compounds that fail to inhibit the SUN-KASH interaction will result in a well in the plate where the recombinant SUN binds to the enzyme-linked KASH domain. Following wash steps and incubation with colorimetric or chemiluminescent HRP substrates, the presence of the SUN-KASH interaction is detected in standard plate readers. If the compound can inhibit SUN-KASH interaction then, following the wash step, the KASH domain is removed and there would be reduced or no enzymatic reaction in the well.

Alternatively, fluorescence anisotropy or polarization can be used to screen for small molecule inhibitors of SUN-KASH interactions *in vitro* [Lea, W.A., and Simeonov, A.. Expert Opin Drug Discov 6: 17-32 (2011)]. This assay also employs recombinant SUN and KASH domains. The KASH domain is fluorescently labeled; for example a chemically synthesized KASH peptide could be readily functionalized with a fluorescein moiety. Fluorescence anisotropy of the interacting KASH domain interacting with SUN domain can be measured using standard equipment such as a plate reader. A small molecule inhibitor that disrupts the SUN-KASH interaction can be readily detected as the fluorescence anisotropy of the fluorescent KASH will change if it is not bound to SUN.

As is typical in drug screening campaigns, the compounds which successfully pass the *in vitro* primary screen will then be subjected to cell-based secondary screens (Figure 23). In this case, immunofluorescence microscopy will be employed to identify those compounds that can dissociate LlNC complexes. This is manifest as dispersal of the KASH component to the peripheral endoplasmic reticulum while the cognate SUN protein is retained in the inner nuclear membrane. This microscopy-based assay can be performed first on HeLa cells. Active compounds are then evaluated on cultured cells from disease-relevant tissue, such as cardiac cells. An additional secondary screen may include the ability of the identified compound to rescue proliferation defects in Lmna knockout cells. Following hit-to-lead optimization of the identified compound using standard methods, the compound can be tested in mouse models of laminopathies such as those described herein for *Lmna* dilated cardiomyopathy. Efficacy of the leads can be evaluated using lifespan of the mutant mice and echocardiograms, as described herein, to assess heart function.

### Example 8: Discussion

DCM caused by *LMNA* is regarded as being aggressive, and often leads to premature death or cardiac transplantation [M. Pasotti et al., J Am Coll Cardiol 52: 1250-1260 (2008); M. R. Taylor et al., J Am Coll Cardiol 41: 771-780 (2003)]. By 60 years, 55% of *LMNA* mutation carriers die of cardiovascular failure or receive a heart transplant, compared with 11% of patients with idiopathic cardiomyopathy. Attempts to ameliorate DCM by fitting a pacemaker have been at best of transient benefit. Consequently it is necessary develop new therapeutic avenues to treat DCM caused by *LMNA* mutations.

The majority of *LMNA* mutations causing DCM are dominant negative missense. Treatment by conventional gene therapy to repair each mutation would be daunting and removal of the mutated allele, leaving the patient hemizygous for the remaining normal WT allele may also result in heart failure [G. Bonne et al., Nature genetics 21: 285-288 (1999)]. Various other routes downstream of the Lamin gene have been explored for potential therapeutic intervention, and have included mTOR inhibition with rapamycin/rapalogues [J. C. Choi et al., Science translational medicine 4: 144ra102 (2012); F. J. Ramos et al., Science translational medicine 4: 144ra103 (2012)] and inhibition of the MEK1 /2 kinase pathway [ W. Wu, et al., Circulation 123: 53-61 (2011*)*]*.* Both avenues, resulted in improved ventricular function and increased longevity (10-40%) but the extent and long-term efficacy was significantly less than that we observed with the loss of *Sun1.*

The molecular mechanisms underlying the varied phenotypes of the laminopathies are still not well understood, though two alternative hypotheses have been proposed to explain the tissue-specific pathologies. The first "gene regulation hypothesis" proposes that LMNA mutations/loss disrupt the equilibrium of various molecular pathways due to the mutations altering interactions with NE proteins and chromatin, which in turn alter gene expression. Evidence in support of this hypothesis comes from studies reporting changes in signalling pathways including the AKT-MTOR pathway [J. C. Choi et al., Science translational medicine 4: 144ra102 (2012)], WNT/β-catenin pathway [L. Hernandez et al., Dev Cell 19: 413-425 (2010); C. Le Dour et al., Hum Mol Genet 26: 333-343 (2017)], TGF-β/Smad [J. H. Van Berlo et al., Hum Mol Genet 14: 2839-2849 (2005); T. V. Cohen et al., Hum Mol Genet 22: 2852-2869 (2013)], MAP Kinase pathway [A. Brull, et al., Front Physiol 9: 1533 (2018)] and the ERK1/2 - CTGF/CCN2 pathway [M. Chatzifrangkeskou et al., Hum Mol Genet 25: 2220-2233 (2016)]. While these changes have been documented, none has clearly established whether these changes are not a secondary compensatory effect of a diseased tissue. Sun1 also fits into this rubric of disrupted expression levels as Sun1 protein, but not mRNA, is upregulated in laminopathies, leading to the proposal that laminopathy phenotypes are caused by toxicity from excess Sun1 [C. Y. Chen et al., Cell 149: 565-577 (2012)].

The second hypothesis suggested Lmna loss or mutation leads to increased nuclear fragility. As a result mechanical stress and tension forces transmitted via the LINC complex from the cytoplasm to the NE causes damage to the NE [J. Lammerding et al., J Clin Invest 113: 370-378 (2004)]. This hypothesis is similar to that proposed for Duchenne muscular dystrophy (DMD), where loss of dystrophin increases the fragility of the muscle cell membrane and when tension-stress forces are applied during muscle contraction this results muscle cell rupture and death [D. J. Blake, et al., Physiol Rev 82: 291-329 (2002)]. *Lmna* mutant fibroblasts show nuclear deformation, defective mechanotransduction, and reduced viability when subjected to mechanical strain, together with increased nuclear rupture at low and moderate pressures when compared to NMD [Popp, M.W., and Maquat, L.E. Cell 165: 1319-1322 (2016)]. In contracting mouse cardiomyocytes, mechanical stress and tension forces caused by 500-600 contractions per minute are transmitted to the NE via the LINC complex, resulting in nuclear distortion, damage and eventual death/loss as described in Figures 28 and 29. Presumably, such forces would cause significant damage to the fragile NE of *Lmna* null cardiomyocytes, resulting in CM death. If the tension-stress hypothesis is damaging to the NE, then unlinking the LINC complex, by disrupting SUN1, would reduce the tension-stress on the CM nuclei, and prevent CM cell death in the mutant CMs (Figure 32A-C). One caveat here is that complete disruption of the LINC complex, as would be the case following overexpression of DN-Sun1, could potentially be deleterious rather than therapeutic. At the cellular level, multiple mechanical phenomena including intracellular force transmission, cell polarization and migration, were impacted following LINC complex disruption by dominant negative SUN and KASH constructs [Lombardi et al., J Biol Chem 286(30):26743-53 (2011)]. In animal models, Sun1/Sun2 [Lei etal., Proc Natl Acad Sci 106(25):10207-12 (2009)] and Nesprin-1/Nesprin-2 [Zhang et al., Development 134(5):901-8 (2007)] double mutant mice experience perinatal lethality and cardiac-specific disruption of the KASH domains of Nesprin-1 and Nesprin-2 using an embryonic cardiac Cre driver (Nkx2.5-Cre) results in early onset cardiomyopathy [Banerjee et al., PLOS Genet 10(2):e1004114 (2014)].

We attempted to distinguish the tension-stress hypothesis from the expression level hypothesis in cardiomyocytes, using a DN-Sun1 construct to compete with endogenous Sun1 and Sun2 proteins for KASH-domain-binding and so unlink the LINC complex without directly altering Sun1 levels (Figures 32D & 34). The AAV9 vector, which has a high affinity for CM, was used to deliver DN-Sun1 under the cTnT promoter to CMs [C. Zincarelli, et al., Mol Ther 16: 1073-1080 (2008)]. Our results showed the successful delivery of GFP to cardiomyocytes (Figure 31C), and robust expression of both the control GFP and DN-Sun1 proteins (Figure 31C) with the latter resulting in the dispersal of the KASH domain proteins from the cardiomyocyte nuclei (Figure 31D). Surprisingly, not only did AAV-DN-Sun1 ameliorate the pathology in mice with depleted cardiac Lmna levels, it also had no discernible effect on the cardiac health of wildtype mice, which would be expected to also experience complete LINC complex disruption in their hearts (Figures 31E & G). This suggests that an intact LINO complex may be required in embryonic development, but not postnatally.

In addition, using CRISPR/Cas9 in mice, we generated a Syne1 mutant allele (C'TΔ8) that gave rise to a truncated Nesprin-1 protein with a disrupted, non-functional, KASH domain. Mice lacking Lmna globally or in the heart have a shortened lifespan, but the presence of a homozygous Synel^{C'TΔ8/C'TΔ8} mutation resulted in significant lifespan extension. Loss of Sun1 or AAV-mediated disruption of the LINC complex by dominant negative transgenes *in vivo* resulted in similar rescue of *Lmna* pathology (Example 2-5), while Sun2 and Nesprin-2 mutations did not. Taken together, these data suggest that LINC complexes comprised of Sun1 and Nesprin-1 drive the pathology in *Lmna* mutant cells and animals.

There have been a number of reports on the use of AAV to deliver CRISPR/Cas components *in vivo* for treating diseases. Our results predict that AAV-mediated CRISPR/Cas, such as CRISPR/Cas9, delivery to target the Nesprin-1 KASH domain in disease-affected tissue can be used to treat laminopathies, including dilated cardiomyopathy. For instance, cardiotropic AAVs (e.g. AAV9) can be used to deliver transgene cassette(s) containing a cardiac-specific promoter (e.g. cTnT) driving Cas endonuclease enzyme expression and an appropriate promoter (e.g. U6) driving gRNA expression to treat *LMNA* DCM. Since the packaging capacity of AAV is limited to 4.7 kb, a smaller Cas9 derived from *Staphylococcus aureus* (saCas9) rather than the larger, more commonly used, *Streptococcus pyogenes* Cas9 may be preferred. Alternatively, other CRISPR enzymes such as Cpf1, which is small enough for AAV packaging and has a more commonly found protospacer adjacent motif (PAM) than saCas9, could be used [Zetsche, B., et al., Cpf1 Is a Single RNA-Guided Endonuclease of a Class 2 CRISPR-Cas System. Cell 163, 759-771 (2015)].

Guide RNAs would target the 3' region of the Nesprin-1 gene encoding the KASH domain (Table 3). While we have targeted the region adjacent to the stop codon, in principle any gene region encoding the KASH domain could be targeted as indels generated by CRISPR would likely result in frameshift mutations that disable the KASH domain. However, as the final 4 amino acids in the KASH domain are known to be absolutely required for SUN domain interaction and hence LINC complex formation [Sosa et al., Cell 149(5):1035-47 (2012)], it is prudent to select gRNA in the vicinity of the stop codon as even indels that do not result in a frameshift could still mutate the relevant KASH amino acids required for the SUN-KASH interaction. Furthermore, it should be noted that because the Syne1 gene encoding Nesprin-1 is very large and has multiple splice isoforms and alternative start sites, guide RNAs targeted outside the KASH domain, while giving rise to some mutant Nesprin-1 isoforms, may not perturb expression of other isoforms of Nesprin-1 protein, including KASH-containing isoforms. This would result in formation of functional or partially functional Nesprin-1/Sun1 LINC complexes that would still be able to drive pathology in Lmna mutants.

We did not further investigate the Sun1 mutant mice generated in this study as instead of mice with Sun1 lacking the SUN domain, we essentially obtained Sun1 null mice, which have already been well characterized. We suspect that inducing CRISPR mutation in Sun1 resulted in nonsense-mediated decay (NMD) of Sun1 transcript. Occurrence of a premature termination codon (PTC) 50-55 nucleotides upstream of a exon-exon junction is a trigger for NMD [Popp, M.W., and Maquat, L.E. Cell 165: 1319-1322 (2016)]. Also, PTCs occurring in the middle of a transcript are more likely to result in NMD [Eberle et al., PLOS Biology 6: e92 (2008); Reber et al., MBoC 29: 75-83 (2018)]. In the Sun1_plus4 mutant, the PTC is more than 55 nucleotides upstream of the exon-exon junction and is likely to trigger NMD. For the Sun1Δ7 mutant, the PTC is less than 50 nucleotides from the exon-exon junction. However for both mutants, since we targeted upstream of the sizeable SUN domain, the PTCs are roughly 2/3 of the way along the length of the transcript, and hence also likely to trigger NMD. In order to specifically disrupt the SUN domain in Sun1 without inducing a null mutation, we can adopt a similar strategy as for Nesprin-1 - directing the guide RNA at the very 3' end of the coding region of the transcript (Table 3). Earlier work demonstrated that mutation of a tyrosine residue to phenylalanine at the C-terminus of SUN2 (Y707F) abolished KASH binding [Sosa et al., Cell 149(5):1035-47 (2012)]. This critical tyrosine residue is conserved in SUN1 (Y812 in Uniprot E9PHI4) and present in the final coding exon of the SUN1 transcript. Selection of a gRNA 5' proximal to the codon for Y812 would produce indel mutations that cause a frameshift mutation that would mutate Y812 and disrupt KASH binding. As the gRNA would be in the final coding exon, the likelihood of triggering NMD would be low. One can thus envision a CRISPR/Cas9-based strategy to treat laminopathies by targeting a critical residue required for KASH-binding in the SUN1 SUN domain. AAVs could be used to deliver CRISPR enzyme and gRNA targeting SUN1 in appropriate disease tissue, such as the heart. Incapacitation of SUN1 KASH binding would then ameliorate the deleterious effects of *Lmna* mutations.

From these results we propose that the loss of or mutations within *Lmna* causes instability in the CM nuclei due to loss or incorrect assembly of the nuclear lamina. This makes the nuclei susceptible to the tension/stress forces exerted via the LINC complex from the contractile sarcomeres of the CMs. In the absence of SUN1, or following mutation of Nesprin-1 KASH domain, the untethered LINC complexes exert less tensional force on the CM nuclei, enabling survival of the lamin deficient cardiomyocyte.

These results provide an opportunity to use the AAV-mediated delivery of DN-Sun, DN-KASH, or direct mutation of endogenous SUN or KASH proteins as potential therapeutics for laminopathy-related DCM in patients. The AAV system, as a therapeutic delivery route in patients is established and has been approved by the FDA for treating some diseases. It is becoming more widely used with multiple on-going clinical trials, including the introduction into patients with heart disease. Our results show that disrupting the LINC complex in CMs could be effective at preventing heart failure for an extended period.

### Example 9: References to Examples 1 to 8

1. M. Ackers-Johnson et al., A Simplified, Langendorff-Free Method for Concomitant Isolation of Viable Cardiac Myocytes and Nonmyocytes From the Adult Mouse Heart. Circulation Research 119: 909 (2016).
2. R. Agah et al., Gene recombination in postmitotic cells. Targeted expression of Cre recombinase provokes cardiac-restricted, site-specific rearrangement in adult ventricular muscle in vivo. The Journal of clinical investigation 100: 169-179 (1997).
3. S. G. Alam et al., The mammalian LINC complex regulates genome transcriptional responses to substrate rigidity. Scientific reports 6: 38063 (2016).
4. A. T. Bertrand et al., DelK32-lamin A/C has abnormal location and induces incomplete tissue maturation and severe metabolic defects leading to premature death. Hum Mol Genet 21: 1037-1048 (2012).
5. D. J. Blake, A. Weir, S. E. Newey, K. E. Davies, Function and genetics of dystrophin and dystrophin-related proteins in muscle. Physiol Rev 82: 291-329 (2002).
6. G. Bonne et al., Mutations in the gene encoding lamin A/C cause autosomal dominant Emery-Dreifuss muscular dystrophy. Nature genetics 21: 285-288 (1999).
7. A. Brull, B. Morales Rodriguez, G. Bonne, A. Muchir, A. T. Bertrand, The Pathogenesis and Therapies of Striated Muscle Laminopathies. Front Physiol 9: 1533 (2018).
8. A. Buchwalter, M. W. Hetzer, Nucleolar expansion and elevated protein translation in premature aging. Nature communications 8: 328 (2017).
9. B. Burke, C. L. Stewart, The nuclear lamins: flexibility in function. Nat Rev Mol Cell Biol 14: 13-24 (2013).
10. G. Captur et al., Lamin and the heart. Heart 104: 468-479 (2018).
11. M. Chatzifrangkeskou et al., ERK1/2 directly acts on CTGF/CCN2 expression to mediate myocardial fibrosis in cardiomyopathy caused by mutations in the lamin A/C gene. Hum Mol Genet 25: 2220-2233 (2016).
12. C. Y. Chen et al., Accumulation of the inner nuclear envelope protein Sun1 is pathogenic in progeric and dystrophic laminopathies. Cell 149: 565-577 (2012).
13. Y. H. Chi et al., Requirement for Sun1 in the expression of meiotic reproductive genes and piRNA. Development 136: 965-973 (2009).
14. J. C. Choi et al., Temsirolimus activates autophagy and ameliorates cardiomyopathy caused by lamin A/C gene mutation. Science translational medicine 4: 144ra102 (2012).
15. M. Crisp et al., Coupling of the nucleus and cytoplasm: role of the LINC complex. J Cell Biol 172: 41-53 (2006).
16. T. V. Cohen et al., Defective skeletal muscle growth in lamin A/C-deficient mice is rescued by loss of Lap2alpha. Hum Mol Genet 22: 2852-2869 (2013).
17. W. N. de Vries et al., Expression of Cre recombinase in mouse oocytes: a means to study maternal effect genes. Genesis 26: 110-112 (2000).
18. Eberle, A.B., Stalder, L., Mathys, H., Orozco, R.Z., and Mühlemann, O. Posttranscriptional Gene Regulation by Spatial Rearrangement of the 3' Untranslated Region. PLOS Biology 6: e92 (2008).
19. D. Fatkin et al., Missense mutations in the rod domain of the lamin A/C gene as causes of dilated cardiomyopathy and conduction-system disease. N Engl J Med 341: 1715-1724 (1999).
20. Gimpel, P., et al., Nesprin-1α-Dependent Microtubule Nucleation from the Nuclear Envelope via Akap450 Is Necessary for Nuclear Positioning in Muscle Cells. Curr. Biol. 27: 2999-3009.e9. (2017).
21. F. Haque et al., Mammalian SUN protein interaction networks at the inner nuclear membrane and their role in laminopathy disease processes. J Biol Chem 285: 3487-3498 (2010).
22. L. Hernandez et al., Functional coupling between the extracellular matrix and nuclear lamina by Wnt signaling in progeria. Dev Cell 19: 413-425 (2010).
23. R. E. Hershberger, A. Morales, in GeneReviews((R)), M. P. Adam et al., Eds. (Seattle (WA), 1993).
24. R. E. Hershberger, D. J. Hedges, A. Morales, Dilated cardiomyopathy: the complexity of a diverse genetic architecture. Nat Rev Cardiol 10: 531-547 (2013).
25. D. S. Herman et al., Truncations of titin causing dilated cardiomyopathy. N Engl J Med 366: 619-628 (2012).
26. H. F. Horn, LINC complex proteins in development and disease. Current topics in developmental biology 109: 287-321 (2014).
27. J. L. Jefferies, J. A. Towbin, Dilated cardiomyopathy. Lancet 375: 752-762 (2010).
28. Kim, D.I., Birendra, K.C., and Roux, K.J. (2015). Making the LINC: SUN and KASH protein interactions. Biol. Chem. 396: 295-310.
29. T. J. Kirby, J. Lammerding, Emerging views of the nucleus as a cellular mechanosensor. Nature cell biology 20: 373-381 (2018).
30. J. Lammerding et al., Lamin A/C deficiency causes defective nuclear mechanics and mechanotransduction. The Journal of clinical investigation 113: 370-378 (2004).
31. J. Lammerding et al., Abnormal nuclear shape and impaired mechanotransduction in emerin-deficient cells. J Cell Biol 170: 781-791 (2005).
32. J. Lammerding et al., Lamins A and C but not lamin B1 regulate nuclear mechanics. J Biol Chem 281: 25768-25780 (2006).
33. Lea, W.A., and Simeonov, A. Fluorescence Polarization Assays in Small Molecule Screening. Expert Opin Drug Discov6: 17-32 (2011).
34. C. Le Dour et al., Decreased WNT/beta-catenin signalling contributes to the pathogenesis of dilated cardiomyopathy caused by mutations in the lamin a/C gene. Hum Mol Genet 26: 333-343 (2017).
35. K. Lei et al., SUN1 and SUN2 play critical but partially redundant roles in anchoring nuclei in skeletal muscle cells in mice. Proc Natl Acad Sci U S A 106: 10207-10212 (2009).
36. Lepourcelet et al., Small-molecule antagonists of the oncogenic Tcf/beta-catenin protein complex. Cancer Cell. 5(1): 91-102 (2004).
37. C. J. Malone, W. D. Fixsen, H. R. Horvitz, M. Han, UNC-84 localizes to the nuclear envelope and is required for nuclear migration and anchoring during C. elegans development. Development 126: 3171-3181 (1999).
38. L. C. Mounkes, S. V. Kozlov, J. N. Rottman, C. L. Stewart, Expression of an LMNA-N195K variant of A-type lamins results in cardiac conduction defects and death in mice. Hum Mol Genet 14: 2167-2180 (2005).
39. M. D. Muzumdar, B. Tasic, K. Miyamichi, L. Li, L. Luo, A global double-fluorescent Cre reporter mouse. Genesis 45: 593-605 (2007).
40. V. Nikolova et al., Defects in nuclear structure and function promote dilated cardiomyopathy in lamin A/C-deficient mice. The Journal of clinical investigation 113: 357-369 (2004).
41. M. Pasotti et al., Long-term outcome and risk stratification in dilated cardiolaminopathies. J Am Coll Cardiol 52: 1250-1260 (2008).
42. Popp, M.W., and Maquat, L.E. Leveraging Rules of Nonsense-Mediated mRNA Decay for Genome Engineering and Personalized Medicine. Cell 165: 1319-1322 (2016).
43. K. M. Prasad, Y. Xu, Z. Yang, S. T. Acton, B. A. French, Robust cardiomyocyte-specific gene expression following systemic injection of AAV: in vivo gene delivery follows a Poisson distribution. Gene Ther 18: 43-52 (2011).
44. Puckelwartz, M.J., et al., Disruption of nesprin-1 produces an Emery Dreifuss muscular dystrophy-like phenotype in mice. Hum Mol Genet 18: 607-620 (2009).
45. Ran FA, et al., Genome engineering using the CRISPR-Cas9 system. Nat. Protoc 8: 2281-2308 (2013);
46. Ran FA, et al., Double nicking by RNA-guided CRISPR Cas9 for enhanced genome editing specificity. Cell 154: 1380-1389 (2013)
47. F. J. Ramos et al., Rapamycin reverses elevated mTORC1 signaling in lamin A/C-deficient mice, rescues cardiac and skeletal muscle function, and extends survival. Science translational medicine 4: 144ra103 (2012).
48. Reber, S., et al., CRISPR-Trap: a clean approach for the generation of gene knockouts and gene replacements in human cells. MBoC 29: 75-83 (2018).
49. T. Sieprath et al., Sustained accumulation of prelamin A and depletion of lamin A/C both cause oxidative stress and mitochondrial dysfunction but induce different cell fates. Nucleus 6: 236-246 (2015).
50. D. S. Sohal et al., Temporally regulated and tissue-specific gene manipulations in the adult and embryonic heart using a tamoxifen-inducible Cre protein. Circ Res 89: 20-25 (2001).
51. I. Solovei et al., LBR and lamin A/C sequentially tether peripheral heterochromatin and inversely regulate differentiation. Cell 152: 584-598 (2013).
52. Sosa BA, Rothballer A, Kutay U, Schwartz TU, LINC complexes form by binding of three KASH peptides to domain interfaces of trimeric SUN proteins. Cell 149(5): 1035-47 (2012).
53. C. L. Stewart, S. Kozlov, L. G. Fong, S. G. Young, Mouse models of the laminopathies. Exp Cell Res 313: 2144-2156 (2007).
54. C. Stewart and B. Burke, RNAi-based therapies for cardiomyopathies, muscular dystrophies and laminopathies. WO2013/158046.
55. T. Sullivan et al., Loss of A-type lamin expression compromises nuclear envelope integrity leading to muscular dystrophy. J Cell Biol 147: 913-920 (1999).
56. E. C. Tapley, D. A. Starr, Connecting the nucleus to the cytoskeleton by SUN-KASH bridges across the nuclear envelope. Curr Opin Cell Biol 25, 57-62 (2013).
57. U. Tayal, S. Prasad, S. A. Cook, Genetics and genomics of dilated cardiomyopathy and systolic heart failure. Genome Med 9: 20 (2017).
58. M. R. Taylor et al., Natural history of dilated cardiomyopathy due to lamin A/C gene mutations. J Am Coll Cardiol 41: 771-780 (2003).
59. C. Toepfer et al., Myosin regulatory light chain (RLC) phosphorylation change as a modulator of cardiac muscle contraction in disease. J Biol Chem 288: 13446-13454 (2013).
60. C. N. Toepfer, T. G. West, M. A. Ferenczi, Revisiting Frank-Starling: regulatory light chain phosphorylation alters the rate of force redevelopment (ktr) in a length-dependent fashion. J Physiol 594: 5237-5254 (2016).
61. J. H. Van Berlo et al., A-type lamins are essential for TGF-beta1 induced PP2A to dephosphorylate transcription factors. Hum Mol Genet 14: 2839-2849 (2005).
62. B. van Steensel, A. S. Belmont, Lamina-Associated Domains: Links with Chromosome Architecture, Heterochromatin, and Gene Repression. Cell 169: 780-791 (2017).
63. J. P. van Tintelen et al., High yield of LMNA mutations in patients with dilated cardiomyopathy and/or conduction disease referred to cardiogenetics outpatient clinics. Am Heart J 154: 1130-1139 (2007).
64. H. Wakimoto, J. G. Seidman, R. S. Y. Foo, J. Jiang, in Current Protocols in Molecular Biology. (John Wiley & Sons, Inc., 2001).
65. A. S. Wang, S. V. Kozlov, C. L. Stewart, H. F. Horn, Tissue specific loss of A-type lamins in the gastrointestinal epithelium can enhance polyp size. Differentiation 89: 11-21 (2015).
66. H. J. Worman, C. Ostlund, Y. Wang, Diseases of the nuclear envelope. Cold Spring Harbor perspectives in biology 2: a000760 (2010).
67. W. Wu, A. Muchir, J. Shan, G. Bonne, H. J. Worman, Mitogen-activated protein kinase inhibitors improve heart function and prevent fibrosis in cardiomyopathy caused by mutation in lamin A/C gene. Circulation 123: 53-61 (2011).
68. Zetsche, B., et al., Cpf1 Is a Single RNA-Guided Endonuclease of a Class 2 CRISPR-Cas System. Cell 163, 759-771 (2015).
69. Zhang, J., Felder, A., Liu, Y., Guo, L.T., Lange, S., Dalton, N.D., Gu, Y., Peterson, K.L., Mizisin, A.P., Shelton, G.D., et al. (2010). Nesprin 1 is critical for nuclear positioning and anchorage. Hum Mol Genet 19: 329-341.
70. Zhang, X., Xu, R., Zhu, B., Yang, X., Ding, X., Duan, S., Xu, T., Zhuang, Y., and Han, M. (2007). Syne-1 and Syne-2 play crucial roles in myonuclear anchorage and motor neuron innervation. Development 134: 901-908.
71. C. Zincarelli, S. Soltys, G. Rengo, J. E. Rabinowitz, Analysis of AAV serotypes 1-9 mediated gene expression and tropism in mice after systemic injection. Mol Ther 16: 1073-1080 (2008).

### Example 10: LlNC complex disruption via loss of Sun1 extends longevity in a model of Lmna mutation-associated muscular dystrophy

All experiments were approved by the Biomedical Sciences Institute (BMSI) Singapore Institutional Animal Care Committee. The generation of the *Lmna*^{*Flx*/*Flx*} mice and MLC-Cre mice is described in (Wang et al., Differentiation. (2015) 89(1-2):11-21 and Mourkioti et al., Genesis. (2008) 46(8):424-30, respectively. *Lmna*^{*Flx*/*Flx*} mice were crossed to MLC-Cre mice to obtain *Lmna*^{*Flx*/+}*;MLC-Cre* mice, which were then crossed with *Lmna*^{*Flx*/+} mice to generate experimental cohorts of *Lmna*^{*Flx*/}*^{Flx};MLC-Cre, Lmna*^{*Flx*/*Flx*} and *Lmna*^{+/+}*;MLC-Cre* mice. *Lmna*^{*Flx*/+}*;MLC-Cre* mice were crossed with *Sun1*^{+/-} mice to obtain *Lmna*^{*Flx*/+}*;MLC-Cre;Sun1*^{+/-} mice, which were intercrossed to obtain *Lmna*^{*Flx*/}*^{Flx};MLC-Cre;Sun1*^{+/-} mice. *Lmna*^{*Flx*/}*^{Flx};MLC-Cre;Sun1*^{+/-} mice were intercrossed to generate experimental cohorts of *Lmna*^{*Flx*/}*^{Flx};MLC-Cre;Sun1*^{+/+}, *Lmna*^{*Flx*/}*^{Flx};MLC-Cre;Sun1*^{+/-} and *Lmna^{FlxlFlx};MLC-Cre;Sun1*^{-/-} mice. The survival rate of the various genotypes was analyzed by the Kaplan-Meier method, and statistical comparison was performed by log-rank test.

Example 2 and Figures 26C and 27A show that cardiac-specific constitutive (*Lmna*^{*Flx*/}*^{Flx};αMyHC-Cre, abbreviated Lmna*^{*Flx*/*Flx:αMyHC*}) and inducible (*Lmna*^{*Flx*/}*^{Flx};*-*Myh6-cre*/*Esr1*, abbreviated *Lmna*^{*Flx*/*Flx:mcm*}) knockout of *Lmna* in mice resulted in mortality - 26.5 days postnatally and ~27 days post tamoxifen induction respectively. LINC complex inhibition by genetic disruption of Sun1 or Nesprin-1 in cardiac cells, or expression of a dominant-negative SUN protein, is demonstrated in the present examples to ameliorate cardiac dysfunction in tamoxifen-treated *Lmna*^{*Flx*/*Flx:mcm*} mice.

In order to establish a mouse model for *Lmna* muscular dystrophy, *Lmna*^{*Flx*/*Flx*} mice were crossed with mice harbouring a Cre transgene under the control of the myosin light chain promoter (MLC-Cre), generating mice with constitutive deletion of *Lmna* in skeletal muscle. These mice survived for an average of 18 days after birth (Figure 49A).

To determine if LINC complex disruption suppresses lethality in this mouse model of *Lmna* muscular dystrophy, as it does for *Lmna* dilated cardiomyopathy, *Lmna*^{*Flx*/}*^{Flx};MLC-Cre* mice were derived on a *Sun1*^{+/-} heterozygous or *Sun1*^{-/-} homozygous background. LINC complex disruption mediated by loss of one copy of Sun1 extended the lifespan of the mice from 18 to 24 days, while more severe disruption via loss of both copies of Sun1 extended lifespan of the mice from 18 to 35 days (Figure 49B).

### Example 11: LINC complex disruption via loss of Sun1 extends longevity in a model of Hutchinson-Gilford Progeria

In order to generate a knock-in mouse model of Hutchinson-Gilford Progeria syndrome without extraneous sequence, CRISPR-Cas9-mediated homology-directed repair of the *Lmna* gene locus was carried out using plasmid-encoded Cas9 enzyme and guide RNA, and an oligo template for homologous recombination.

To generate *Lmna^{G609G-CR}* mice, C57BL/6 female mice were used as embryo donors and foster mothers. 3-8 week old C57BL/6 female mice superovulated by injecting pregnant mare serum gonadotrophin followed 48 h later by human chorionic gonadotropin and mated to C57BL/6 stud males. Fertilized embryos were collected from oviducts and the pronuclei injected with the pX330 plasmid encoding Cas9 and a guide RNA (gRNA) targeting the *Lmna* gene (5 ng/µl), and an oligo for homology-directed repair of the *Lmna* gene (5 ng/µl). The gRNA sequence is: AGGAGATGGATCCGCCCACC (SEQ ID NO:109) and the repair oligo sequence for inducing *Lmna-G609G* progeria mutation is shown in SEQ ID NO:110. Injected embryos were transferred on the same day to recipient dames (on average 15 zygotes / female).

Founder animals were identified by genotyping PCR followed by Sanger sequencing. Genotyping primers were as follows:

| | | |
|---|---|---|
| Lmna GT up: | 5'- CCACAGGTCTCCCAAGTCCCCATC-3' | (SEQ ID NO:111) |
| Lmna GT down: | 5'- TCCTCTCCCTCCCTGACCCCAAA-3' | (SEQ ID NO:112) |

*Lmna*^{*G609G-CR*/+}*;Sun1*^{+/-} mice were intercrossed to generate experimental cohorts of *Lmna*^{*G069G-CR*/}*^{G069G-CR};Sun1*^{+/+}*, Lmna*^{*G069G-CR*/}*^{G069G-CR};Sun1*^{+/-}*, Lmna*^{*G069G-CR*/}*^{G069G-CR};Sun1*^{-/-}*, Lmna*^{*G069G-CR*/+}*;Sun1*^{-/-}*, Lmna*^{*G069G-CR*/+}*;Sun1*^{+/+}, *Lmna*^{+/+}*;Sun1*^{+/+} and *Lmna*^{+/+}*;Sun1*^{-/-} mice. The survival rate of the various genotypes was analyzed by the Kaplan-Meier method, and statistical comparison was performed by log-rank test.

Similar to previously established Lmna-G609G mice, mice heterozygous for the *Lmna^{G609G-CR}* allele survived for 290 days, while mice homozygous for the mutant allele survived for 116 days (Figure 50).

To determine if LINC complex disruption suppresses lethality in this novel knock-in model of HGPS, as it does for *Lmna* dilated cardiomyopathy, we derived Lmna^{G609G-CR} mice in a Sun1 mutant background. *Lmna*^{*G609G-CR*/*G609G-CR*} mice on a *Sun1*^{+/-} heterozygous mutant background survived for an average of 152 days, while *Lmna*^{*G609G-CR*/*G609G-CR*} mice on a *Sun1*^{-/-} heterozygous mutant background survived for 174 days (Figure 50). Thus loss of the LINC complex component Sun1 suppresses the deleterious effect of the progeria mutation in a dose-dependent manner. Similarly, mice heterozygous for the progeria mutation on a Sun1 null background survived for more than a year, unlike the progeria mutant mice with wildtype Sun1, which died in just under a year (Figure 50).

### Example 12: LINC complex disruption by expression of dominant-negative LINC complex proteins alleviates pathophysiology in Lmna mutation-associated muscular dystrophy and Lmna mutation-associated progeria

Mice having mouse models of *Lmna* mutation-associated muscular dystrophy and *Lmna* mutation-associated progeria are administered with nucleic acid encoding dominant-negative Sun1, Sun2, or KASH1-5.

The mouse models of *Lmna* mutation-associated muscular dystrophy include the *Lmna* knockout model described in Example 10, and *Lmna* point mutant models such as Lmna-H222P and Lmna-N195K.

The mouse models of *Lmna* mutation-associated progeria include the Lmna-G609G model described in Example 11.

The nucleic acids are delivered using adeno-associated virus, adenovirus or lentivirus vectors, or nanoparticles.

Mice having *Lmna* mutation-associated muscular dystrophy treated with a LINC complex inhibitor have an extended lifespan as compared to untreated mice, or mice treated with vehicle only.

Mice having *Lmna* mutation-associated progeria treated with a LINC complex inhibitor have an extended lifespan as compared to untreated mice, or mice treated with vehicle only.

### Example 13: Gene editing of LINC complex components alleviates pathophysiology in Lmna mutation-associated muscular dystrophy and Lmna mutation-associated progeria

Mice having mouse models *of Lmna* mutation-associated muscular dystrophy and *Lmna* mutation-associated progeria are administered with nucleic acid encoding gene editing systems (including CRISPR/Cas9 systems) for disrupting LINC complex components.

The gene editing systems target an exon of a SUN domain-containing protein encoding a SUN domain, or target an exon of a KASH domain-containing protein encoding a KASH domain.

The mouse models of *Lmna* mutation-associated muscular dystrophy include the *Lmna* knockout model described in Example 10, and *Lmna* point mutant models such as *Lmna*-H222P and *Lmna*-N195K.

The mouse models of *Lmna* mutation-associated progeria include the *Lmna*-G609G model described in Example 11.

The nucleic acids are delivered using adeno-associated virus, adenovirus or lentivirus vectors, or nanoparticles.

Mice having *Lmna* mutation-associated muscular dystrophy treated with a LINC complex inhibitor have an extended lifespan as compared to untreated mice, or mice treated with vehicle only.

Mice having *Lmna* mutation-associated progeria treated with a LINC complex inhibitor have an extended lifespan as compared to untreated mice, or mice treated with vehicle only.

### Example 14: RNAi-mediated knockdown of LINC complex component expression alleviates pathophysiology in Lmna mutation-associated muscular dystrophy and Lmna mutation-associated progeria

Mice having mouse models of *Lmna* mutation-associated muscular dystrophy and *Lmna* mutation-associated progeria are administered with nucleic acid encoding shRNA or siRNA targeting LINC complex components.

The shRNA/siRNA target a SUN domain-containing protein or a KASH domain-containing protein.

The mouse models of *Lmna* mutation-associated muscular dystrophy include the *Lmna* knockout model described in Example 10, and *Lmna* point mutant models such as *Lmna*-H222P and *Lmna*-N195K.

The mouse models of *Lmna* mutation-associated progeria include the *Lmna*-G609G model described in Example 11.

The nucleic acids are delivered using adeno-associated virus, adenovirus or lentivirus vectors, or nanoparticles.

Mice having *Lmna* mutation-associated muscular dystrophy treated with a LINC complex inhibitor have an extended lifespan as compared to untreated mice, or mice treated with vehicle only.

Mice having *Lmna* mutation-associated progeria treated with a LINC complex inhibitor have an extended lifespan as compared to untreated mice, or mice treated with vehicle only.

### Example 15: LINC Complex disruption using small molecule drugs alleviates pathophysiology in Lmna mutation-associated muscular dystrophy and Lmna mutation-associated progeria

Mice having mouse models of *Lmna* mutation-associated muscular dystrophy and *Lmna* mutation-associated progeria are administered with small molecule inhibitors of interaction between SUN and KASH domains.

The mouse models of *Lmna* mutation-associated muscular dystrophy include the *Lmna* knockout model described in Example 10, and *Lmna* point mutant models such as *Lmna-H222P* and *Lmna*-N195K.

The mouse models of *Lmna* mutation-associated progeria include the *Lmna*-G609G model described in Example 11.

The small molecule inhibitors inhibit association between a KASH domain and a SUN domain, and are identified e.g. as described in Example 7.

Mice having *Lmna* mutation-associated muscular dystrophy treated with a LINC complex inhibitor have an extended lifespan as compared to untreated mice, or mice treated with vehicle only.

Mice having *Lmna* mutation-associated progeria treated with a LINC complex inhibitor have an extended lifespan as compared to untreated mice, or mice treated with vehicle only.

### Example 16: LINC Complex disruption using small molecule drugs alleviates pathophysiology in Lmna mutation-associated dilated cardiomyopathy

Mice having mouse models of *Lmna* mutation-associated dilated cardiomyopathy are administered with small molecule inhibitors of interaction between SUN and KASH domains.

The mouse models of *Lmna* mutation-associated dilated cardiomyopathy include the *Lmna* knockout model described in Example 10, and *Lmna* point mutant models such as *Lmna-H222P* and *Lmna*-N195K.

The small molecule inhibitors inhibit association between a KASH domain and a SUN domain, and are identified e.g. as described in Example 7.

Mice having *Lmna* mutation-associated dilated cardiomyopathy treated with a LINC complex inhibitor have an extended lifespan and/or improved cardiac function (e.g. increased myocardial contractility, increased ejection fraction and/or increased fractional shortening) as compared to untreated mice, or mice treated with vehicle only.

### Example 17: RNAi-mediated knockdown of LINC complex component expression alleviates pathophysiology in Lmna mutation-associated dilated cardiomyopathy

Mice having mouse models of *Lmna* mutation-associated dilated cardiomyopathy are administered with small molecule inhibitors of interaction between SUN and KASH domains.

The shRNA/siRNA target a SUN domain-containing protein or a KASH domain-containing protein.

The mouse models of *Lmna* mutation-associated dilated cardiomyopathy include the *Lmna* knockout model described in Example 10, and *Lmna* point mutant models such as *Lmna*-H222P and *Lmna*-N195K.

The nucleic acids are delivered using adeno-associated virus, adenovirus or lentivirus vectors, or nanoparticles.

Mice having *Lmna* mutation-associated dilated cardiomyopathy treated with a LINC complex inhibitor have an extended lifespan and/or improved cardiac function (e.g. increased myocardial contractility, increased ejection fraction and/or increased fractional shortening) as compared to untreated mice, or mice treated with vehicle only.

### Example 18: LINC complex disruption via loss of Sun1 alleviates atherosclerosis In Ldlr^{-/-} mutant mice

### 18.1 Materials and methods

### Sun1^{-/}⁻;Ldlr^{-/-} mice and Western-type diet model of atherosclerosis

All experiments were approved by the Biomedical Sciences Institute (BMSI) Singapore Institutional Animal Care Committee. *Sun1*^{*-*/*-*} mice and their generation is described in Chi et al., Development (2009) 136(6):965-73. *Ldlr*^{*-*/*-*} mice (*C57BL*/*6Jlnv,* Jackson Laboratory) were crossed to *Sun1*^{+/-} mice to generate *Sun1*^{+/-}*;Ldlr*^{+/-} mice, which were then intercrossed to generate *Sun1*^{+/-}*,Ldlr*^{-/-} mice. These *Sun1*^{+/-}*,Ldlr*^{-/-}mice were then intercrossed to generate the experimental cohorts of *Sun1*^{*-*/}*⁻;Ldlr*^{*-*/*-*} and *Sun1*^{+/+};*Ldlr*^{*-*/*-*} mice.

Male experimental mice on a 12h light-dark cycle were maintained on chow diet (1324_modified, Altromin GmbH & Co.) until 12 weeks of age, followed by a western type diet (WTD; D12079B, Research Diets, NJ) for 15 weeks. The mice had free access to food and water except during a 4-5 h fasting period prior to blood sample collection. Mice were anesthetized at 27 weeks (100mg/kg ketamine hydrochloride/10mg/kg xylazine i.p.), bled retro-orbitally, perfused transcardially with 1xPBS, and hearts fixed in 4% paraformaldehyde (Sigma) and embedded in paraffin. The aortic arch and thoracic aorta were also dissected and fixed in 4% paraformaldehyde.

### Plasma lipid quantification

Plasma HDL cholesterol (HDLc) and total cholesterol were measured using the Infinity cholesterol kit (401-25P, Sigma), following manufacturer's instructions. For HDLc quantification, 20µl of plasma was mixed with an equal volume of 20% polyethylene glycol made in 0.2M glycine. The mix was vortexed immediately, incubated at room temperature for 5 minutes, centrifuged at 14,000rpm for 6 minutes, and 10µl of the supernatant quantified by Infinity cholesterol kit. Non-HDLc was quantified by subtracting HDLc from the total cholesterol values for each mouse.

### Aortic sinus atherosclerotic lesion analyses

Paraformaldehyde fixed hearts were dehydrated and embedded in paraffin following standard protocols. Serial cross sections (5µm-thick) were obtained throughout the entire aortic root for histological analyses as described^{26,27}. Briefly, aortic cross-sections were stained with hematoxylin-phloxine-saffron and atherosclerotic lesion area was analyzed in 4 cross-sections/mouse. Aperio Imagescope (Leica Biosystems, USA) and imaged were used for morphometric quantification of lesion number, area and severity according to the American Heart Association (Stary et al., Arterioscler Thromb Vasc Biol. 1994;14:840-856; Stary et al., Arterioscler Thromb Vasc Biol. 1995;15:1512-1531) classification. MAC-3 (550292, BD-Pharmigen, USA) antibody was used to determine lesional macrophage content.

### Aortic arch en face lesion analyses

Paraformaldehyde fixed aortas were cleaned by removing adventitial tissues using saline to keep the tissue moist during the cleaning. The aortas were opened longitudinally to the iliac bifurcation and pinned out flat on the surface of black wax with 0.2-mm steel pins (Fine Science Tools #26002-20). Aortas were then stained en face with oil red O solution (Sigma Aldrich #1516) for 30 min at room temperature and washed four times with PBS. imaged was used for the quantification of the lesion area.

### Statistical analyses

Data were analyzed using Student's T-tests. Values of p<0.05 were considered to represent significant differences between groups. Results are shown as mean ± SEM.

### 18.2 Results

### Lower atherosclerotic lesion area in the aortic arches of Sun1^{-/}⁻;Ldlr^{-l-} mice

To determine the impact of Sun1 deletion on atherosclerotic lesion development, *Sun1*^{*-*/*-*} mice generated on the WTD fed *Ldlr*^{*-*/*-*} mouse background were utilized (Breslow et al., Science 1996;272:685-688). Atherosclerotic lesions were analyzed after 15 weeks of WTD administration, in en face sections of mice aortas stained with Oil Red O, which stains neutral lipids (Figure 51A).

A significant decrease in percentage lesion area was observed in the *Sun1*^{*-*/}*⁻;Ldlr^{-l-}* mice (*Sun1*^{*-*/}*⁻;Ldlr*^{*-*/*-*}: 16.33±2.01, *Sun*^{+/+}*;Ldlr*^{*-*/}*⁻.* 23.88±1.60 % lesion area, p=0.013) (Figure 51B). Percentage lesion area was quantified as the lesion area (Oil Red O positive area) as a fraction of the total area of the aortic arch. A significant decrease in absolute lesion area was also observed in the *Sun1*^{*-*/}*⁻;Ldlr^{-l-}* mice (*Sun1*^{*-*/}*⁻;Ldlr^{-l-}*: 60393±8343, *Sun*^{+/+}*;Ldlr*^{*-*/*-*}: 100702±14637, µm², p=0.029) (Figure 51C).

These data show that inhibition of Sun1 significantly reduces atherosclerotic lesion size in mice.

### Lower atherosclerotic lesion area in the aortic sinus of Sun1^{-/-};Ldlr^{-/-} mice

Since atherosclerotic lesion area in the aortic arches was significantly decreased, the inventors next assessed lesion area in the aortic sinus of the mice. Previous studies characterizing the sites of atherosclerotic lesions in the *Ldlr*^{*-*/*-*} mice have shown unequivocally that the earliest lesions form at the aortic sinus, and lesions are seen as early as after 4 weeks of high fat diet feeding (Ma et al., PLoS ONE. 2012:7(4):e35835; Paigen et al., Atherosclerosis. 1987:68:231-240). Indeed, initiation timing of lesions at different locations in the *Ldlr*^{*-*/*-*} mice were rank-ordered as aortic sinus>innominate artery>thoracic aorta>abdominal aorta (Ma et al., PLoS ONE. 2012:7(4):e35835).

Hematoxylin phloxine saffron (HPS) stained sections (Figure 52A) of the aortic sinus were next quantified for atherosclerotic lesions. A significant decrease in % lesion area was observed in the *Sun1*^{*-*/}*⁻;Ldlr*^{-/-} mice (*Sun1*^{*-*/}*⁻;Ldlr*^{*-*/*-*}: 17.57 ±1.27, *Sun*^{+/+}*;Ldlr*^{*-*/*-*}: 22.92±0.94, % lesion area, p=0.0045) (Figure 52B). No changes were found in the total number of atherosclerotic lesions in the *Sun1*^{*-*/}*⁻;Ldlr*^{*-*/*-*} mice (*Sun1*^{-/-}*;Ldlr*^{*-*/}*⁻.* 12.63±1.54, *Sun*^{+/+}*;Ldlr*^{-/-}*.* 12.78±0.85, # of lesions, p=0.93) (Figure 52C).

The inventors next classified each individual lesion as mild to severe using the American Heart Association criteria for lesion severity (Stary et al., Arterioscler Thromb Vasc Biol. 1994;14:840-856; Stary et al., Arterioscler Thromb Vasc Biol. 1995;15:1512-1531). A significant 26% decrease in the number of severe lesions (class IV and V) was observed in the *Sun1*^{*-*/}*⁻;Ldlr*^{*-*/*-*} mice (*Sun*^{*-*/}*⁻;Ldlr*^{*-*/*-*}: 7.75±1.01, *Sun*^{+/+}*;Ldlr*^{-/-}*.* 10.56±0.73, lesion #, p=0.037) (Figure 52D). A significant increase in the number of mild lesions (class I to III) were observed in the *Sun1*^{*-*/}*⁻;Ldlr*^{*-*/*-*} mice (*Sun1*^{*-*/}*⁻;Ldlr^{-l-}*: 4.87±0.95, *Sun*^{+/+}*;Ldlr*^{*-*/*-*}: 2.22±0.57, # of lesions, p=0.027) (Figure 52E).

Together, these data show that inhibition of Sun1 significantly decreases atherosclerotic lesion size, and prevents the progression of lesions from mild to severe.

### Lower atherosclerotic lesion complexity in Sun1^{-/}⁻;Ldlr^{-/-} mice

Since a significantly lower lesion area, and a significant decrease in advanced lesions were observed in the *Sun1*^{*-*/}*⁻;Ldlr*^{*-*/*-*} mice, the inventors next assessed lesion complexity by assessing macrophage infiltration (Figure 53A). Significantly lower (by 45%) macrophage (MAC3) positive lesion area was observed in the *Sun1*^{*-*/}*⁻;Ldlr*^{*-*/*-*} mice (*Sun1*^{*-*/}*⁻;Ldlr*^{*-*/*-*}: 27796±1868, *Sun*^{+/+}*;Ldlr*^{*-*/*-*}: 50418±3887, µm², p=0.0001) (Figure 53B), suggesting that in the absence of Sun1, less complex lesions were formed in the mice.

### Plasma lipid levels were unchanged in the Sun1^{-/}⁻;Ldlr^{-/-} mice

The results show that mice with Sun1 inhibition are protected against atherosclerotic lesion progression, displaying smaller lesion area, and less advanced and complex lesions.

To exclude that the smaller lesions in the *Sun1*^{*-*/}*⁻;Ldlr*^{*-*/*-*} mice resulted from the *Sun1*^{*-*/}*⁻;Ldlr*^{*-*/*-*} mice being smaller compared to their wildtype littermates, body weights of the mice were assessed. No changes were observed in body weights in the *Sun1*^{*-*/}*⁻;Ldlr*^{*-*/*-*} mice (*Sun1*^{*-*/}*⁻;Ldlr*^{*-*/*-*}: 39.54±2.01, *Sun*^{+/+}*;Ldlr*^{-/-}*.* 41.98±2.31, g, p=0.45) (Figure 54A), suggesting that the reduction in aortic lesion size did not result from lower body weights in the *Sun1*^{*-*/}*⁻;Ldlr*^{*-*/*-*} mice.

The inventors next analysed the plasma lipid composition of the mice, in order to determine if an alteration in plasma lipid levels contributed to the protection against atherosclerosis in these mice. No changes in total cholesterol were observed in *Sun1*^{-/-}*;Ldlr*^{-/-} mice (*Sun1*^{*-*/}*⁻;Ldlr^{-l-}*: 1075±69, *Sun*^{+/+}*;Ldlr*^{-/-}: 1182±59, mg/dL, p=0.25) (Figure 54B). As well, no differences in plasma HDLc levels (*Sun1*^{*-*/}*⁻;Ldlr*^{*-*/*-*}: 109.3±6.5, *Sun*^{+/+}*;Ldlr*^{-/-}*.* 108.9±6.2, mg/dL, p=0.93) (Figure 54C) or in ApoB-containing non-HDLc levels were observed in the *Sun1*^{-/-}*;Ldlr*^{-/-} mice (*Sun1*^{*-*/}*⁻;Ldlr^{-l-}*: 965.9±65.6, *Sun*^{+/+}*;Ldlr*^{*-*/*-*}: 1073.0±54.6, mg/dL, p=0.23) (Figure 54D).

These data suggest that protection against atherosclerosis observed in the *Sun1*^{-/-}*;Ldlr*^{-/-} mice was not a consequence of alterations in plasma lipid levels.

### Example 19: LINC complex disruption by expression of dominant-negative LINC complex proteins alleviates pathophysiology in mouse models of atherosclerosis and familial hypercholesterolemia

Mice having mouse models of atherosclerosis and familial hypercholesterolemia are administered with nucleic acid encoding dominant-negative Sun1, Sun2, or KASH1-5.

A mouse model of atherosclerosis and familial hypercholesterolemia is establish by feeding *Ldlr*^{*-*/*-*} mice a Western-type diet as described in Example 18.

The nucleic acids are delivered using adeno-associated virus, adenovirus or lentivirus vectors, or nanoparticles.

Mice treated with a LINC complex inhibitor have smaller, less advanced and less complex atherosclerotic lesions as compared to untreated mice, or mice treated with vehicle only.

### Example 20: Gene editing of LINC complex components alleviates pathophysiology in mouse models of atherosclerosis and familial hypercholesterolemia

Mice having mouse models of atherosclerosis and familial hypercholesterolemia are administered with nucleic acid encoding gene editing systems (including CRISPR/Cas9 systems) for disrupting LINC complex components.

The gene editing systems target an exon of a SUN domain-containing protein encoding a SUN domain, or target an exon of a KASH domain-containing protein encoding a KASH domain.

A mouse model of atherosclerosis and familial hypercholesterolemia is establish by feeding *Ldlr*^{*-*/*-*} mice a Western-type diet as described in Example 18.

The nucleic acids are delivered using adeno-associated virus, adenovirus or lentivirus vectors, or nanoparticles.

Mice treated with a LINC complex inhibitor have smaller, less advanced and less complex atherosclerotic lesions as compared to untreated mice, or mice treated with vehicle only.

### Example 21: LINC Complex disruption using small molecule drugs alleviates pathophysiology in mouse models of atherosclerosis and familial hypercholesterolemia

Mice having mouse models of atherosclerosis and familial hypercholesterolemia are administered with small molecule inhibitors of interaction between SUN and KASH domains.

A mouse model of atherosclerosis and familial hypercholesterolemia is establish by feeding *Ldlr*^{*-*/*-*} mice a Western-type diet as described in Example 18.

The small molecule inhibitors inhibit association between a KASH domain and a SUN domain, and are identified e.g. as described in Example 7.

Mice treated with a LINC complex inhibitor have smaller, less advanced and less complex atherosclerotic lesions as compared to untreated mice, or mice treated with vehicle only.

### Example 22: RNAi-mediated knockdown of LINC complex component expression alleviates pathophysiology in mouse models of atherosclerosis and familial hypercholesterolemia

Mice having mouse models of atherosclerosis and familial hypercholesterolemia are administered with nucleic acid encoding shRNA or siRNA targeting LINC complex components.

A mouse model of atherosclerosis and familial hypercholesterolemia is establish by feeding *Ldlr*^{-/-} mice a Western-type diet as described in Example 18.

The shRNA/siRNA target a SUN domain-containing protein or a KASH domain-containing protein.

The nucleic acids are delivered using adeno-associated virus, adenovirus or lentivirus vectors, or nanoparticles.

Mice treated with a LINC complex inhibitor have smaller, less advanced and less complex atherosclerotic lesions as compared to untreated mice, or mice treated with vehicle only.

### Example 23: AAV9 mediated transduction and expression of a DNSun1 prolongs the lifespan and improves cardiac function of LmnaFlx/Flx:mcm+Tmx mice

In further experiments, the inventors investaged the effects of treatment of mice having cardiomyocyte-specific deletion of *Lmna* with AAV9-vectored dominant-negative human SUN1 protein expressed under the control of a cardiomyocyte-specific promoter.

### 23.1 Materials and methods

### Molecular cloning of humanized SUN1 dominant negative transgene

The following sequence was obtained as a gBlock from IDT:

5'-tat agg cta gcG GCA CAA TGA AGT GGG TAA CCT TTA TTT CCC TTC TTT TTC TCT TTA GCT
CGG CCT ATT CCA GGG GTG TGT TTC GTC GAG ATG GGC CCt ccg gag gtG AAC AAA AAC TCA
TCT CAG AAG AGG ATC Tca ccg gtg gaG ATG AGG GCT GGG AAG CCA GAG-3' (SEQ ID NO:117)

The gBlock contains the following sequence features: Nhel restriction enzyme (RE) site, human serum albumin signal sequence, Apal and BspEI RE sites, Myc epitope tag, Agel RE site, and sequence complementary to the start of the lumenal domain of human SUN2. Primers of sequence 5'-TAT AGG CTA GCG GCA CAA TG-3' (SEQ ID NO:118) and 5'-CTC TGG CTT CCC AGC CCT CA-3' (SEQ ID NO:119) were used to PCR amplify the gBlock to form a megaprimer. The megaprimer and a reverse primer encoding the KDEL sequence and the C-terminus of SUN2 (5'-CCG GGT CGA CCT ACA ACT CAT CTT TGT GGG CGG GCT CCC CAT GCA C-3' (SEQ ID NO:120)) were used to amplify SUN2 lumenal domain from a SUN2 cDNA clone (AM392760). The PCR product and pENN-AAV-cTnT-EGFP-RBG (UPEnn Vector Core, Philadelphia, PA) were digested with Nhel and Sall and ligated to obtain pAAV-cTnT-Myc-SUN2DN.

To obtain SUN1 dominant negative construct, 5'-TCT CAC CGG TGG AGA TGA CCC CCA GGA CGT GTT TAA AC-3' (SEQ ID NO:121) and 5'-CCG GGT CGA CCT ACA ACT CAT CTT TCT TGA CAG GTT CGC CAT GAA CTC-3' (SEQ ID NO:122) were used to PCR amplify human SUN1 lumenal domain from a cDNA clone (Loo et al., ELife (2019) 8; 1-25). The SUN1 PCR product and pAAV-cTnT-Myc-huSUN2DN were digested with Agel and Sall, and ligated to obtain pAAV-cTnT-Myc-huSUN1DN, the nucleotide sequence of which is shown in SEQ ID NO:125.

### Production of AAV for humanized SUN1 dominant negative experiments

293T cells were cultured in 15 cm dishes or T-flasks before being seeded into a 10-chamber CellSTACK (Corning Inc., Corning, NY, USA). pAAV2/9 (gift of James M. Wilson, UPEnn Vector Core, Philadelphia, PA), pHelper (Part No. 340202, Cellbiolabs, Inc., San Diego, CA, USA) and plasmids containing the transgenes (pENN-AAV-cTnT-EGFP-RBG and pAAV-cTnT-Myc-SUN1DN) were co-transfected into the cells using PEI Max (Polysciences, Warrington, PA, USA). 4 days after transfection, cell pellets and cell culture supernatant were harvested. Supernatant was clarified by filtration and applied by gravity flow to ~1 ml POROS^{™} CaptureSelect^{™} AAV9 Affinity Resin (Thermo Fisher Scientific, Waltham, MA, USA). Cells were resuspended in lysis buffer (phosphate buffered saline, 200 mM NaCl, 0.001% Pluronic F-68), lysed by 4-5 rounds of freeze/thaw, sonicated, and treated with benzonase to shear and digest DNA. Cell debris was pelleted by centrifugation, and cell lysate collected and filtered through 0.45 µm syringe filters. Filtered lysates were then applied to AAV9 affinity resin by gravity flow. Following washing in wash buffer (phosphate buffered saline, 500 mM NaCl), AAV9 virions were eluted using 100 mM glycine, pH 2.5, and collected into microfuge tubes containing 1/10^{th} volume of 1M Tris, pH 8. Following 2 rounds of buffer exchange into PBS containing 0.01% Pluronic F-68 and concentration via Amicon^{®} Ultra 100 kDA concentrators (Merck KGaA, Darmstadt, Germany), ~ 1 ml solution containing AAV virions was filtered through 0.22 µm 4 mm Millex syringe filter units (Merck KGaA, Darmstadt, Germany) and stored in 4 °C or -80 °C. For the quantification of viral genomes, dye-based quantitative real-time PCR was performed using primers 5'-acagtctcgaacttaagctgca-3' (SEQ ID NO:123) and 5'-gtctcgacaagcccagtttcta-3' (SEQ ID NO:124), Pacl-digested and PCR-purified pENN-AAV-cTnT-EGFP-RBG (for the generation of a standard curve), and PerfeCTa SYBR Green FastMix Low ROX qPCR mastermix (Quanta BioSciences, Beverly, MA, USA).

### In vivo gene delivery

AAV transduction was performed by retro-orbital injection. For evaluation of the cardiac function only male animals were used. The experimental design was as follows: On postnatal day 10 mice were genotyped. To induce deletion of the *Lmna* gene in cardiomyocytes tamoxifen was administered by intraperitoneal injection on postnatal day 14 to all animals (40 mg/kg). On postnatal day 15, animals were injected intravenously with AAV9-huSUN1DN or AAV9-GFP (2x 10^10 vg/g, 5 µl/g) into the retro-orbital sinus using an insulin syringe. For the dose-response experiment, mice were administered with 2 × 10^10 viral genome/g bodyweight ('standard dose'), or 5 × 10^10 viral genome/g bodyweight (`high dose'). Animals were selected randomly. TMX and AAV9 injections were performed under anaesthesia using 1.5 % Isoflurane mixed with oxygen. The AAV9 working solution was prepared freshly prior to administration. Depending on the concentration of viral genomes the respective AAV9 stock solutions were diluted with PBS containing 0.001 % Pluronic F-68.

### 23.2 Results

AAV9 was used to introduce and express a dominant negative human SUN1 minigene expressed specifically in cardiomyocytes under the control of the chicken cardiac troponin promotor (Figure 55A).

*Lmna*^{*flx*/}*^{flx} Mcm* mice were administered with a single IP injection of tamoxifen (40 mg/kg) at postnatal day 14 to induce *Lmna* deletion in cardiomyocytes (Figure 55B). At postnatal day 15, AAV9-huSUN1DN or a control AAV9 virus encoding GFP ((5×10^10 viral genome/g bodyweight) were administered by retro-orbital injection. *Lmna*^{*flx*/}*^{flx} WT* control groups received the same treatments (i.e. AAV9-huSUN1DN or AAV9-GFP).

Administration of the AAV9-vectored huSUN1DN minigene to the tamoxifen-treated *Lmna*^{*flx*/}*^{flx} Mcm* mice (therefore having cardiomyocyte-specific deletion of *Lmna*) mice approximately doubled their lifespan (66 days) as compared to their AAV9-GFP-treated counterparts (36 days) - see Figure 55C.

Echocardiogram analysis revealed that treatment of AAV9-huSUN1DN was also associated with improved cardiac function (FS, GLS, EF) at day 28 after tamoxifen treatment of the *Lmna*^{*flx*/}*^{flx} Mcm* mice, as compared to treatment with AAV9-GFP (Figures 55D, 55E and 55F). However, in comparison to the *Lmna*^{*flx*/}*^{flx} WT* controls the AAV9-huSUN1DN-treated mice having cardiomyocyte-specific deletion of *Lmna* displayed weaker heart function, which might explain their shorter lifespan.

No differences in lifespan or cardiac function were observed between *Lmna*^{*flx*/}*^{flx} WT* mice treated with AAV9-huSUN1DN or AAV9-GFP (Figures 55C, 55D, 55D, 55E and 55F), indicating no adverse effects associated with administration of AAV9-huSUN1DN.

In further experiments, the inventors investigated whether the improvement in survival and cardiac function observed in AAV9-huSUN1DN-treated mice mice having cardiomyocyte-specific deletion of *Lmna* could be increased further by administration of a larger dose of AAV9-huSUN1DN.

Briefly, *Lmna*^{*flx*/}*^{flx} Mcm* mice were administered with a single IP injection of tamoxifen (40 mg/kg) at postnatal day 14 to induce *Lmna* deletion in cardiomyocytes, and at postnatal day 15, AAV9-huSUN1DN was administered at (i) 2 × 10^10 viral genome/g bodyweight, or (ii) 5 × 10^10 viral genome/g bodyweight, by retro-orbital injection.

Mice having cardiomyocyte-specific deletion of *Lmna* and administered with 5 × 10^10 viral genome/g bodyweight of AAV9-huSUN1DN had improved survival (lifespan ~200 days) as compared to their counterparts treated with AAV9-huSUN1DN at a dose of 2 × 10^10 viral genome/g bodyweight (Figure 56A).

Echocardiogram analysis revealed that mice having cardiomyocyte-specific deletion of *Lmna* and administered with 5 × 10^10 viral genome/g bodyweight of AAV9-huSUN1DN also had improved cardiac function as compared to their counterparts treated with AAV9-huSUN1DN at a dose of 2 × 10^10 viral genome/g bodyweight, with a delay to the decline in cardiac function (Figures 56B, 55C and 56D).

The results indicate dose-depenent effects for AAV9-huSUN1 DN treatment on improvement of lifespan and the cardiac function in mice having cardiomyocyte-specific deletion of *Lmna.*

Overall, the results of this Example establish AA9-vectored huSUN1 DN as a promising therapy for the treatment of *LMNA* mutation-associated dilated cardiomyopathy.

The following numbered clauses, describing aspects of our proposals, are part of the description:
1. A LINC complex inhibitor for use in a method of treating or preventing a laminopathy.
2. Use of a LINC complex inhibitor in the manufacture of a medicament for use in a method of treating or preventing a laminopathy.
3. A method of treating or preventing a laminopathy, comprising administering a therapeutically or prophylactically effective amount of a LINC complex inhibitor to a subject.
4. The LINC complex inhibitor for use according to clause1, the use according to clause2, or the method according to clauses, wherein the laminopathy is characterised by one or more of myopathy, cardiomyopathy, dilated cardiomyopathy, muscular dystrophy, cardiac muscular dystrophy, skeletal muscular dystrophy, progeria, neuropathy, lipoatrophy, skeletal dysplasia, lipodystrophy, leukodystrophy or dermopathy.
5. The LINC complex inhibitor for use, the use or the method according to any one of clauses 1 to 4, wherein the laminopathy is associated with mutation to *LMNA.*
6. The LINC complex inhibitor for use, the use or the method according to any one of clauses 1 to 5, wherein the laminopathy is selected from: Hutchinson-Gilford Progeria Syndrome; Dilated Cardiomyopathy; Muscular Dystrophy, Congenital, Lmna-Related; Emery-Dreifuss Muscular Dystrophy 2, Autosomal Dominant; Muscular Dystrophy; Mandibuloacral Dysplasia with Type a Lipodystrophy; Cardiomyopathy, Dilated, 1a; Charcot-Marie-Tooth Disease; Limb-Girdle Muscular Dystrophy; Cardiomyopathy, Dilated, with Hypergonadotropic Hypogonadism; Emery-Dreifuss Muscular Dystrophy 3, Autosomal Recessive; Lipodystrophy, Familial Partial, Type 2; Emery-Dreifuss Muscular Dystrophy; Charcot-Marie-Tooth Disease, Axonal, Type 2b1; Heart-Hand Syndrome, Slovenian Type; Aging; Familial Partial Lipodystrophy; Restrictive Dermopathy, Lethal; Arrhythmogenic Right Ventricular Cardiomyopathy; Tooth Disease; Heart Disease; Werner Syndrome; Hypertrophic Cardiomyopathy; Left Ventricular Noncompaction; Atrioventricular Block; Calcinosis; Acroosteolysis; Autosomal Dominant Limb-Girdle Muscular Dystrophy; Diabetes Mellitus, Noninsulin-Dependent; Osteoporosis; Atrial Fibrillation; Atrial Standstill 1; Acanthosis Nigricans; Cardiac Conduction Defect; Catecholaminergic Polymorphic Ventricular Tachycardia; Mandibular Hypoplasia, Deafness, Progeroid Features, and Lipodystrophy Syndrome; Sick Sinus Syndrome; Pelger-Huet Anomaly; Charcot-Marie-Tooth Disease, Axonal, Type 2e; Congenital Generalized Lipodystrophy; Restrictive Cardiomyopathy; Congenital Fiber-Type Disproportion; Lipodystrophy, Congenital Generalized, Type 1; Myofibrillar Myopathy; Lipodystrophy, Familial Partial, Type 1; Axonal Neuropathy; Atypical Werner Syndrome; Ovarian Cystadenoma; Fanconi Anemia, Complementation Group a; Body Mass Index Quantitative Trait Locus 11; Skin Disease; Rigid Spine Muscular Dystrophy 1; Neuromuscular Disease; Hallermann-Streiff Syndrome; Bethlem Myopathy 1; Acquired Generalized Lipodystrophy; Cardiomyopathy, Dilated, 1e; Lipodystrophy, Congenital Generalized, Type 4; Undifferentiated Pleomorphic Sarcoma; Lipodystrophy, Familial Partial, Type 3; Muscular Dystrophy, Congenital Merosin-Deficient, 1a; Proximal Spinal Muscular Atrophy; Muscular Dystrophy-Dystroglycanopathy , Type B, 5; Muscular Dystrophy, Congenital, 1b; Reynolds Syndrome; Wiedemann-Rautenstrauch Syndrome; Emery-Dreifuss Muscular Dystrophy 1, X-Linked; Lipodystrophy, Congenital Generalized, Type 2; Monogenic Diabetes; Cardiomyopathy, Dilated, 1d; Myopathy, Proximal, and Ophthalmoplegia; Muscle Tissue Disease; Lipodystrophy, Familial Partial, Type 4; Cardiomyopathy, Dilated, 1h; Second-Degree Atrioventricular Block; Median Neuropathy; Intrinsic Cardiomyopathy; Prolapse of Female Genital Organ; Complete Generalized Lipodystrophy; Rigid Spine Muscular Dystrophy; Emerinopathy; Ulnar Nerve Lesion; Limb-Girdle Muscular Dystrophy Type 1b; Lmna-Related Dilated Cardiomyopathy; Pelvic Muscle Wasting; Generalized Lipodystrophy-Associated Progeroid Syndrome; Muscular Disease; Cardiomyopathy, Dilated, 1b; Autosomal Genetic Disease; Familial Isolated Arrhythmogenic Ventricular Dysplasia, Right Dominant Form; Familial Isolated Arrhythmogenic Ventricular Dysplasia, Biventricular Form; Familial Isolated Arrhythmogenic Ventricular Dysplasia, Left Dominant Form; Lmna-Related Cardiocutaneous Progeria Syndrome; and Autosomal Semi-Dominant Severe Lipodystrophic Laminopathy.
7. A LINC complex inhibitor for use in a method of treating or preventing a disease characterised by hyperlipidemia.
8. Use of a LINC complex inhibitor in the manufacture of a medicament for use in a method of treating or preventing a disease characterised by hyperlipidemia.
9. A method of treating or preventing a disease characterised by hyperlipidemia, comprising administering a therapeutically or prophylactically effective amount of a LINC complex inhibitor to a subject.
10. The LINC complex inhibitor for use according to clause 7, the use according to clause 8, or the method according to clause 9, wherein the disease characterised by hyperlipidemia is selected from atherosclerosis, cardiovascular disease, stroke and a familial hyperlipidemia.
11. The LINC complex inhibitor for use, the use or the method according to any one of clauses 1 to 10, wherein the LINC complex inhibitor is capable of binding to a LINC complex, a LINC complex protein or an interaction partner for a LINC complex protein, or wherein the LINC complex inhibitor is capable of reducing expression of a LINC complex protein.
12. The LINC complex inhibitor for use, the use or the method according to clause 11, wherein the LINC complex inhibitor is capable of inhibiting interaction between a LINC complex protein and an interaction partner for a LINC complex protein.
13. The LINC complex inhibitor for use, the use or the method according to any one of clause 11 or clause 12, wherein the LINC complex inhibitor is a peptide/polypeptide, nucleic acid or small molecule.
14. The LINC complex inhibitor for use, the use or the method according to clause 11, wherein the LINC complex inhibitor is capable of modifying a gene encoding a LINC complex protein to reduce its expression.
15. The LINC complex inhibitor for use, the use or the method according to clause 14, wherein the LINC complex inhibitor comprises a site-specific nuclease (SSN) targeting a gene encoding a LINC complex protein.
16. The LlNC complex inhibitor for use, the use or the method according to clause 11, wherein the LINC complex inhibitor is an inhibitory nucleic acid capable of reducing expression of a LINC complex protein by RNA interference (RNAi).
17. The LINC complex inhibitor for use, the use or the method according to any one of clauses 11 to 16, wherein the method comprises administering nucleic acid encoding the LINC complex inhibitor, or nucleic acid encoding factors required for production of the LINC complex inhibitor, to the subject.

## Claims

1. A LINC complex inhibitor for use in a method of treating or preventing a laminopathy.

2. The LINC complex inhibitor for use according to claim 1, wherein the laminopathy is **characterised by** one or more of myopathy, cardiomyopathy, dilated cardiomyopathy, muscular dystrophy, cardiac muscular dystrophy or skeletal muscular dystrophy.

3. The LINC complex inhibitor for use according to claim 1 or claim 2, wherein the laminopathy is associated with mutation to *LMNA.*

4. The LINC complex inhibitor for use according to any one of claims 1 to 3, wherein the laminopathy is **characterised by** one or more of progeria, neuropathy, lipoatrophy, skeletal dysplasia, lipodystrophy, leukodystrophy or dermopathy, or is selected from: Hutchinson-Gilford Progeria Syndrome; Dilated Cardiomyopathy; Muscular Dystrophy, Congenital, Lmna-Related; Emery-Dreifuss Muscular Dystrophy 2, Autosomal Dominant; Muscular Dystrophy; Mandibuloacral Dysplasia with Type a Lipodystrophy; Cardiomyopathy, Dilated, 1a; Charcot-Marie-Tooth Disease; Limb-Girdle Muscular Dystrophy; Cardiomyopathy, Dilated, with Hypergonadotropic Hypogonadism; Emery-Dreifuss Muscular Dystrophy 3, Autosomal Recessive; Lipodystrophy, Familial Partial, Type 2; Emery-Dreifuss Muscular Dystrophy; Charcot-Marie-Tooth Disease, Axonal, Type 2b1; Heart-Hand Syndrome, Slovenian Type; Aging; Familial Partial Lipodystrophy; Restrictive Dermopathy, Lethal; Arrhythmogenic Right Ventricular Cardiomyopathy; Tooth Disease; Heart Disease; Werner Syndrome; Hypertrophic Cardiomyopathy; Left Ventricular Noncompaction; Atrioventricular Block; Calcinosis; Acroosteolysis; Autosomal Dominant Limb-Girdle Muscular Dystrophy; Diabetes Mellitus, Noninsulin-Dependent; Osteoporosis; Atrial Fibrillation; Atrial Standstill 1; Acanthosis Nigricans; Cardiac Conduction Defect; Catecholaminergic Polymorphic Ventricular Tachycardia; Mandibular Hypoplasia, Deafness, Progeroid Features, and Lipodystrophy Syndrome; Sick Sinus Syndrome; Pelger-Huet Anomaly; Charcot-Marie-Tooth Disease, Axonal, Type 2e; Congenital Generalized Lipodystrophy; Restrictive Cardiomyopathy; Congenital Fiber-Type Disproportion; Lipodystrophy, Congenital Generalized, Type 1; Myofibrillar Myopathy; Lipodystrophy, Familial Partial, Type 1; Axonal Neuropathy; Atypical Werner Syndrome; Ovarian Cystadenoma; Fanconi Anemia, Complementation Group a; Body Mass Index Quantitative Trait Locus 11; Skin Disease; Rigid Spine Muscular Dystrophy 1; Neuromuscular Disease; Hallermann-Streiff Syndrome; Bethlem Myopathy 1; Acquired Generalized Lipodystrophy; Cardiomyopathy, Dilated, 1e; Lipodystrophy, Congenital Generalized, Type 4; Undifferentiated Pleomorphic Sarcoma; Lipodystrophy, Familial Partial, Type 3; Muscular Dystrophy, Congenital Merosin-Deficient, 1a; Proximal Spinal Muscular Atrophy; Muscular Dystrophy-Dystroglycanopathy , Type B, 5; Muscular Dystrophy, Congenital, 1b; Reynolds Syndrome; Wiedemann-Rautenstrauch Syndrome; Emery-Dreifuss Muscular Dystrophy 1, X-Linked; Lipodystrophy, Congenital Generalized, Type 2; Monogenic Diabetes; Cardiomyopathy, Dilated, 1d; Myopathy, Proximal, and Ophthalmoplegia; Muscle Tissue Disease; Lipodystrophy, Familial Partial, Type 4; Cardiomyopathy, Dilated, 1h; Second-Degree Atrioventricular Block; Median Neuropathy; Intrinsic Cardiomyopathy; Prolapse of Female Genital Organ; Complete Generalized Lipodystrophy; Rigid Spine Muscular Dystrophy; Emerinopathy; Ulnar Nerve Lesion; Limb-Girdle Muscular Dystrophy Type 1b; Lmna-Related Dilated Cardiomyopathy; Pelvic Muscle Wasting; Generalized Lipodystrophy-Associated Progeroid Syndrome; Muscular Disease; Cardiomyopathy, Dilated, 1b; Autosomal Genetic Disease; Familial Isolated Arrhythmogenic Ventricular Dysplasia, Right Dominant Form; Familial Isolated Arrhythmogenic Ventricular Dysplasia, Biventricular Form; Familial Isolated Arrhythmogenic Ventricular Dysplasia, Left Dominant Form; Lmna-Related Cardiocutaneous Progeria Syndrome; and Autosomal Semi-Dominant Severe Lipodystrophic Laminopathy.

5. A LINC complex inhibitor for use in a method of treating or preventing a disease **characterised by** hyperlipidemia.

6. The LINC complex inhibitor for use according to claim 5, wherein the disease **characterised by** hyperlipidemia is selected from atherosclerosis, cardiovascular disease, stroke and a familial hyperlipidemia.

7. The LINC complex inhibitor for use according to any one of claims 1 to 6, wherein the LINC complex inhibitor is capable of binding to a LINC complex, a LINC complex protein or an interaction partner for a LINC complex protein, or wherein the LINC complex inhibitor is capable of reducing expression of a LINC complex protein.

8. The LINC complex inhibitor for use according to claim 7, wherein the LINC complex inhibitor is capable of inhibiting interaction between a LINC complex protein and an interaction partner for a LINC complex protein.

9. The LINC complex inhibitor for use according to any one of claim 7 or claim 8, wherein the LINC complex inhibitor is a peptide/polypeptide, nucleic acid or small molecule.

10. The LINC complex inhibitor for use according to claim 9, wherein LINC complex inhibitor is an inhibitory nucleic acid capable of reducing expression of a LINC complex protein by RNA interference (RNAi), optionally wherein the inhibitory nucleic acid is a small interfering RNA (siRNA), a short hairpin RNA (shRNA), or a micro RNA (miRNA).

11. The LINC complex inhibitor for use according to any one of claims 1 to 10, wherein the LINC complex inhibitor is capable of reducing expression of a KASH domain-containing protein or a SUN-domain containing protein.

12. The LINC complex inhibitor for use according to any one of claims 1 to 9, wherein the LINC complex inhibitor is (i) a dominant-negative SUN domain-containing protein that inhibits interaction between a SUN domain-containing protein and a KASH domain-containing protein, or (ii) a dominant-negative KASH domain-containing protein that inhibits interaction between a SUN domain-containing protein and a KASH domain-containing protein.

13. The LINC complex inhibitor for use according to any of claims 1 to 12, wherein the treatment or prevention comprises administering nucleic acid encoding the LINC complex inhibitor, or nucleic acid encoding factors required for production of the LINC complex inhibitor, to a subject.

14. The LINC complex inhibitor for use according to claim 13, wherein the nucleic acid is comprised in a vector, optionally wherein the vector is an adeno-associated viral vector, optionally wherein the adeno-associated viral vector selected from one of the following serotypes: AAV9, AAV1, AAV6, AAV8, AAV2i8, AAV9.45, AAV10 and AAVrh.74.

15. The LINC complex inhibitor for use according to claim 14, wherein the vector comprises a promoter providing for expression of the nucleic acid in cardiac and/or skeletal muscle cells or tissue, optionally wherein the promoter is:
(i) a cardiac or cardiomyocte-specific promoter, optionally wherein the cardiac or cardiomyocte-specific promoter is selected from a cTNT, α-MHC or MLC2v promoter; or
(ii) a skeletal muscle or striated muscle cell-specific promoter, optionally wherein the skeletal muscle or striated muscle cell-specific promoter is selected from a MCK, MHCK7 or desmin promoter.
